# EUROPEAN PATENT SPECIFICATION

(11) **EP 2 634 264 B1**
(45) Date of publication and mention of the grant of the patent: **14.09.2016**
(21) Application number: 13154538.6
(22) Date of filing: 23.07.2007
(51) Int. Cl.: C12Q 1/68, G01N 33/574

(54) **Methods and nucleic acids related to the gene GLI3 for analyses of cellular proliferative disorders**
Mit dem Gen GLI3 assozierte Verfahren und Nukleinsäuren für Analysen von mit proliferativem Zellwuchs verbundenen Krankheiten
Procédés et acides nucléiques associes au gène GLI3 pour les analyses de troubles prolifératifs cellulaires

(30) Priority: 21.07.2006 US 832509 P; 20.10.2006 US 853097 P
(43) Date of publication of application: 04.09.2013
(62) Divisional of application: 07813223.0
(73) Proprietor: Epigenomics AG, 10829 Berlin (DE)
(72) Inventor: Lofton-Day, Catherine, Seattle, WA 98103 (US); Sledziewski, Andrew, Shoreline, WA 98177 (US); Model, Fabian, 12683 Berlin (DE); Cottrell, Susan, Seattle, WA 98115 (US); Distler, Jürgen, 12163 Berlin (DE); Tetzner, Reimo, 10439 Berlin (DE); Dietrich, Dimo, 10437 Berlin (DE)
(74) Representative: Zwicker, Jörk

(56) References cited:
- WO-A1-2007/073220
- WO-A2-2008/122447
- US-A1- 2006 024 692
- KAY L. POGUE-GEILE ET AL: "A New Microarray, Enriched in Pancreas and Pancreatic Cancer cDNAs to Identify Genes Relevant to Pancreatic Cancer", CANCER GENOMICS & PROTEOMICS, vol. 1, 1 January 2004 (2004-01-01), pages 371-386, XP055056279,
- DATABASE Geneseq [Online] 4 August 1995 (1995-08-04), "Reverse transcription primer used in cDNA analysis technique.", XP002703645, retrieved from EBI accession no. GSN:AAQ75611 Database accession no. AAQ75611
- DATABASE Geneseq [Online] 4 August 1995 (1995-08-04), "Reverse transcription primer used in cDNA analysis technique.", XP002703646, retrieved from EBI accession no. GSN:AAQ75735 Database accession no. AAQ75735
- DATABASE Geneseq [Online] 4 August 1995 (1995-08-04), "Reverse transcription primer used in cDNA analysis technique.", XP002703647, retrieved from EBI accession no. GSN:AAQ75608 Database accession no. AAQ75608
- DATABASE Geneseq [Online] 12 July 2002 (2002-07-12), "Oligonucleotide for detecting cytosine methylation SEQ ID NO 6941.", XP002704969, retrieved from EBI accession no. GSN:ABQ20350 Database accession no. ABQ20350
- DATABASE Geneseq [Online] 12 July 2002 (2002-07-12), "Oligonucleotide for detecting cytosine methylation SEQ ID NO 2679.", XP002704970, retrieved from EBI accession no. GSN:ABQ16088 Database accession no. ABQ16088
- TAKEICHI ET AL: "Cadherins in cancer: implications for invasion and metastasis", CURRENT OPINION IN CELL BIOLOGY, CURRENT SCIENCE, LONDON, GB, vol. 5, no. 5, 1 October 1993 (1993-10-01) , pages 806-811, XP023601983, ISSN: 0955-0674, DOI: 10.1016/0955-0674(93)90029-P [retrieved on 1993-10-01]
- A R DALLOSSO ET AL: "Long-range epigenetic silencing of chromosome 5q31 protocadherins is involved in early and late stages of colorectal tumorigenesis through modulation of oncogenic pathways", ONCOGENE, vol. 31, no. 40, 4 October 2012 (2012-10-04), pages 4409-4419, XP055071285, ISSN: 0950-9232, DOI: 10.1038/onc.2011.609

<110> Epigenomics AG
<120> METHODS AND NUCLEIC ACIDS FOR ANALYSES OF CELLULAR PROLIFERATIVE
   DISORDERS
<130> 536-115 EPT1
<150> US 60/832,509
   <151> 2006-07-21
<150> US 60/853,097
   <151> 2006-10-20
<160> 148
<210> 1
   <211> 2519
   <212> DNA
   <213> Homo Sapiens
<400> 1
   gatttatgag tgaatgacta aaagtgcagc tgagtcctgg cagagggcat ggggtcccac      60
   ccagagacag gcagagaaag ttgaagtccc aggattggag gccgttcttc ctcacctccc     120
   caccaggccc aggcagggct tgatctgaac ggaggcctgg gaacctgtgg ccagccttta     180
   cttgttggaa aagagcagtc cttaagctca attgctccag gttgatgctt ccctactttt     240
   ttttatttat ttatttttat tattattttt tttttattga gacggagtct tactctgttg     300
   ccaggttgga gtgcagtggc gcgatctcgg ctcactgcca cctccgcctg ctgagttcaa     360
   gcctcagcct cctgagtagc ctcctgggta gctgggacta caggcgtgcg ccaccacgcc     420
   aggctaattt tttgtatttt agtatagacg gagtttcacc actttggcca ggatggtctc     480
   gatctcctga cctcgtgata ctcccgcctc ggtatcccaa agtgccggga ttacaggcat     540
   gagccacagc gcccggcccc tagttctttt taaaaaacgc tagatccgtc cgctgcgctg     600
   agtggaggcg gggcaggcct ccgttctcca attggcctta tccaccgagc tcttcccttg     660
   tgccgggctc tgtgccaagc acatcacacg ctgtatcctg cggccaggtt gctgtggtcc     720
   agggtcgtac cctggtccaa ggtcgcaaac cgaggtggga ctccgatccg gcaaccacgc     780
   ccgtggcccg gaaacggcgt cccctgaggc ccaggagagg ccgggcggtg agcggctgtg     840
   gagccgagcg cgggcagtgc ggatgctgcc tatgggggag gcagccaagg acggagggcg     900
   agaggcggtt cttccaaggt caccctcttc cgggttgcaa gcaaaggtca ggggatcccg     960
   gaatggttag tgcaggagct tctctgtgcc ttccacgtcc tagatcctca gagcctcaga    1020
   aacggagatc atcgtcccca cccccatttt acagatgaag aaactgagcc gaggaaagga    1080
   agcgacttgg ccaaggtcgg agagctcatt ctttgcaggg cggggtttgg aacccggggt    1140
   ctggctctcg gcaacgcgcc ctcggcccgc agcctcctgc cccctgtgcc ccgcttcggc    1200
   ccccagcgca gctgcagagg ggcccccctc gacgcataca ctcaagagcc cgaccgcgcg    1260
   gctgaaatcg cggagctcgg agccgcggct ggctgagcga tcgcggttcc tgggctgcgt    1320
   gcgcgcccct tggagctgaa aggagcgcca ggatcggggg cgctgcaccg ggctgggccc    1380
   ctcaacgctc gcagaccggg ccgggctgca gctggagatg gcagcaatcc cgggaggtct    1440
   ccgggcctct tcagggtgcg tccaggaggc gggttccgtg cgacgcggtg cagcccaccc    1500
   ccccccccga gaccgcttaa cttcgcgggg gcagcctcgg gcgctcggag acgcggaggc    1560
   ccagactgca gcctccggat gctggaagcc cagactccct ggggtcaccg gctctcccgc    1620
   caccccagct gcagagagtc ccattgcttc accgtccgga gcttagtctc cttgttcctc    1680
   taccagtccc tccctccgca ggtctctggg gacttctgac cgcctgttct tactctcccc    1740
   ctgcccccat acttcccgcc cttgtctcag gaacggtgat acagtcaccg gattgctctc    1800
   catctcctgt tagtctacac tgcacacaac tcaataatcc gcgcccttcc atccgggtga    1860
   cagagacaca gataatctga gctagtggtg ctcaaagtac cggtcccaga acagcagcat    1920
   cagcatctct tgggaacttg ttaaaaatga gaatttgggc cgggcgcggt ggctcacgcc    1980
   tgtaatccca gcactttggg aggccgaggc gggcggatca cgaggtcagg agatcgagac    2040
   catcccggct aaaacggtga aaccccgtct ctactaaaaa tacaaaaaat tagccgggca    2100
   tagtggcggg cgcctgtagt cccagctact tgggaggctg aggcaggaga atggcgtgaa    2160
   cccgggaggc ggagcttgca gtgagccgag atcccgccac tgcactccag cctgggcgac    2220
   agagcgagac tccgtctcaa aaaaaaaaaa aatgcgaatt tgggggcccc accccagatc    2280
   tactgaacag aaactctgtg gagcccagca gatgattccc atgcacacta aagtttgcga    2340
   gccactgatc taaacattct ttcatccatt cattcttcac ctggcccacc cagcattgcc    2400
   agtgggagag acacccgcaa agcaccaggc tgtgagcccc accgccgtgc actctgagac    2460
   actgtccact agctttggga tggcaggcag aggtactcca gcttggtcta gtgcagacc     2519
<210> 2
   <211> 2283
   <212> DNA
   <213> Homo Sapiens
<400> 2
   aatgaagacg ctggagatcg ggcccctgcc cgtccccttt ctgcgccccg ggatgaggca      60
   gagactgaac agccggcgag caaatcaacg gcatccagaa agccatgtcg gactcggcgc     120
   ccagcgccca agcgctaacc cgctgaaagt ttctcagcga aatctcaggg acgatctgga     180
   ccccgctgag aggaactgct tttgagtgag atggtcccag aggcctggag gagcggactg     240
   gtaagcaccg ggagggtagt gggagttttg cttctgcttg gtgccttgaa caaggcttcc     300
   acggtcattc actatgagat cccggaggaa agagagaagg gtttcgctgt gggcaacgtg     360
   gtcgcgaacc ttggtttgga tctcggtagc ctctcagccc gcaggttccg ggtggtgtct     420
   ggagctagcc gaagattctt tgaggtgaac cgggagaccg gagagatgtt tgtgaacgac     480
   cgtctggatc gagaggagct gtgtgggaca ctgccctctt gcactgtaac tctggagttg     540
   gtagtggaga acccgctgga gctgttcagc gtggaagtgg tgatccagga catcaacgac     600
   aacaatcctg ctttccctac ccaggaaatg aaattggaga ttagcgaggc cgtggctccg     660
   gggacgcgct ttccgctcga gagcgcgcac gatcccgatg tgggaagcaa ctctttacaa     720
   acctatgagc tgagccgaaa tgaatacttt gcgcttcgcg tgcagacgcg ggaggacagc     780
   accaagtacg cggagctggt gttggagcgc gccctggacc gagaacggga gcctagtctc     840
   cagttagtgc tgacggcgtt ggacggaggg accccagctc tctccgccag cctgcctatt     900
   cacatcaagg tgctggacgc gaatgacaat gcgcctgtct tcaaccagtc cttgtaccgg     960
   gcgcgcgtcc tggaggatgc accctccggc acgcgcgtgg tacaagtcct tgcaacggat    1020
   ctggatgaag gccccaacgg tgaaattatt tactccttcg gcagccacaa ccgcgccggc    1080
   gtgcggcaac tattcgcctt agaccttgta accgggatgc tgacaatcaa gggtcggctg    1140
   gacttcgagg acaccaaact ccatgagatt tacatccagg ccaaagacaa gggcgccaat    1200
   cccgaaggag cacattgcaa agtgttggtg gaggttgtgg atgtgaatga caacgccccg    1260
   gagatcacag tcacctccgt gtacagccca gtacccgagg atgcccctct ggggactgtc    1320
   atcgctttgc tcagtgtgac tgacctggat gctggcgaga acgggctggt gacctgcgaa    1380
   gttccaccgg gtctcccttt cagccttact tcttccctca agaattactt cactttgaaa    1440
   accagtgcag acctggatcg ggagactgtg ccagaataca acctcagcat caccgcccga    1500
   gacgccggaa ccccttccct ctcagccctt acaatagtgc gtgttcaagt gtccgacatc    1560
   aatgacaacc ctccacaatc ttctcaatct tcctacgacg tttacattga agaaaacaac    1620
   ctccccgggg ctccaatact aaacctaagt gtctgggacc ccgacgcccc gcagaatgct    1680
   cggctttctt tctttctctt ggagcaagga gctgaaaccg ggctagtggg tcgctatttc    1740
   acaataaatc gtgacaatgg catagtgtca tccttagtgc ccctagacta tgaggatcgg    1800
   cgggaatttg aattaacagc tcatatcagc gatgggggca ccccggtcct agccaccaac    1860
   atcagcgtga acatatttgt cactgatcgc aatgacaatg ccccccaggt cctatatcct    1920
   cggccaggtg ggagctcggt ggagatgctg cctcgaggta cctcagctgg ccacctagtg    1980
   tcacgggtgg taggctggga cgcggatgca gggcacaatg cctggctctc ctacagtctc    2040
   ttgggatccc ctaaccagag cctttttgcc atagggctgc acactggtca aatcagtact    2100
   gcccgtccag tccaagacac agattcaccc aggcagactc tcacggtctt gatcaaagac    2160
   aatggggagc cttcgctctc caccactgct accctcactg tgtcagtaac cgaggactct    2220
   cctgaagccc gagccgagtt cccctctggc tctgcccccc gggagcagaa aaaaaatctc    2280
   acc                                                                  2283
<210> 3
   <211> 2236
   <212> DNA
   <213> Homo Sapiens
<400> 3
   caggtgccaa cgaccgcgcc agactggcgt tggaaccctt gggccataag gtcttagtgg      60
   gtggcgtcat tatctctgcg gtttgcaaaa gcggagtggc cctgtcaggc gggaagcctc     120
   tctcctctcc ccagatccgc gacaggccgc aggcaagaac cagcgcaacc agggcgcgtc     180
   cgcacagact tggaggcggc tgcatgctgc tacctgctcc agaaggcgtc cggtggccgc     240
   tccgcagttt cagagcctag agacaagcag aggaaggaag acaggacact tgggtcagct     300
   gcccgagcca agtcgctgca aacgctaata ccgcccgcag cctcttcgcc agtatccacg     360
   ctcaaaagta actcaagttt gcgcgccagt gggaaacttg gcgctcatga ctcgccagcg     420
   cgggtcgaaa ctccttcctg atgccctctc agctgttttt cttcccccgc gtggccagga     480
   ggggtaaata atatgcagtg gggcggggcg ttctggaagg ggtgtgccag ggagggaatg      540
   atgggggtcc caggcaaagc tccgaactct ttcacgtaac gggaggaata cagacacgtc     600
   ttagttaagc gtgcgtggga accgcggaat taaggcacct agaggccaag ggcttgtgtc     660
   aagctctgca ttttgccccg caggggaacc tctatacccc gcgattgaac tcgaaagact     720
   cctcccggcg gatgaaagcc gctactccct ggctggctga gctacagctc ccgcagcgcg     780
   cccaggagtg cgccggagat tcggaaaccc gcagagactt ctcaagtcag caggaacttg     840
   gaaaccgctg ttccctccaa acggcttcaa acactcgcgc tccttccttt acccaaccta     900
   attttaattt gccctctcta taggcttaat ttttattttt ttaaaatccc aaactagtaa     960
   gccaaaccga agccccagaa taaggttcaa tcaatgattt taattcaaca ccaagcccga    1020
   atgtttacct ctcggatctg acaaaccaga tgcagagcga cgaatggaga ccaccttccc    1080
   gggaggcgga acccagctgg gttccagcct gctctgggag aggagcacgc ctcccttgag    1140
   ctgcaggcgg gtccggctct gacgaaacgc tgcgagaatg ccagacagtg tagcctccac    1200
   cgtttgctcg gggtctctgc tgccatcaga caagtacttt tattcattca tcccttccca     1260
   tcaatcaccg ccagactcct cccgcgcctt ccgaccccgc tgcaaccttt ccccttgggc     1320
   gatgcctgga cagcatcagt agtagttgcc cgagggaggg gggcagtcct cgtccgggga    1380
   caggtcccaa aaggctgttt atttgttaga gttctacgct cttcaaatga acccaaatca    1440
   agggcaagtt atcacgcacc atgtgacacc cggcagctcc agactagaac aaggctctgc    1500
   actgaacgtg ataattgaac ttttaatgcc aagtgtcact tttctttcag gtaaatgttc    1560
   cgccgctgtg atatttaagg ttggtttgtg ggcgctgctc tttgctctgc ttcattttgc    1620
   cctgaagttt catttcaagc caacattaac cccaacacgg aacagctcga tcttttcttc    1680
   ccaggccctc tcactgcccc gagagaggat tacaagtact cctcattttc cccagtacag    1740
   ctcttctcct ctcttcctgc cacagtatcg gtgttaggca atctcaggca tttttatact    1800
   gtacctatga cagacttgat gcttatcgga gacatgaaaa taatcagaaa gctgccatgc    1860
   aaattcaaca tgatatttat ttaccaggta gagcaataca ataaggtctt tctatccatt    1920
   cttgagctcc atgtttaccg cagagaggaa aataacagcg actggtagtt ttgcccagag    1980
   attgtataaa agaggtcagg aagtcaggct tggagatgca ctggagagta ctgtgccgtc    2040
   ctccaatcag caaaatccct actggatctg tggaacttca aacccagaag cacccattcc    2100
   atgtagcagt ttcacccaga gacaggaggc tcgttttttg cttcatgtta gttaatggaa   2160
   tagatgggac aagaaccttg gatttttggc tcttaggcaa tgctttctgg gacagtggaa    2220
   gtagaaaatc aatttc                                                    2236
<210> 4
   <211> 2204
   <212> DNA
   <213> Homo Sapiens
<400> 4
   tccttctgca aatcggtcta agtttcggga ggtgggattg ggggaggagg gggttcaccc      60
   tggggccagg ccagggctgg gtggaggagg cggccttcga gagagtgagt cgggaaagaa     120
   ggggagtggg caaagggtga gatgtggggt cccggtcccc gcctcgctgg ctgccagttc     180
   cctctgcgtt tcctagtccg cgtgcgtgac acgagatagt gcagccctgg catggggctt     240
   ggcatctggc ggccgcgaga taaccagggc ccgtgcgatg ggaaacgtgc cgttggcagc     300
   ccgccacccc tccatcgtga cctcagtctg ccaaacaggg ttcgtgatgc tcagtttact     360
   agggaggagc cgcggctttg agatctcgcc cagcccgaag cagcggagac tggccaggag     420
   cccggctgtc agacctcgga gcgcacgctg cgggcggggg ctcggtccac gggggagggt     480
   caggggacgc gggactcacc cttgaaggag tcggggagcc gctgggcttc ggagtcccgt     540
   gtgtgccgct tctcggccat gctgcccgag acgcagtcgc actcccccgt cctcgttgcc     600
   aaacccggag ccgccgggaa tgccctgagg agccagaggc acaagaccca gattgccgcc     660
   ccgcccccaa tcgaggtccc gccctcgtga gagttggcca atcaacgccc aggatcgagg     720
   aggacccacc tcccaatccc cgccctcctg tccctccacg gcgcgactgc ttcgcagtca     780
   cttcccgcta gttctgtggg ctgaggggcg tggcctgagg ggcgtggcct gaggggcgag      840
   agcccccaag gctgtgggta tgaggccggg gccctgcacc ggaagggagg gcggaagcca     900
   cgggacgtgg agcagatggt gaggatagtg gggaggtagc agcagaggat gacccgaggc     960
   aaaagtgcaa gtgaagaaaa gtcgccttgc cgggcagagg tacgggacca aggcgcctcc    1020
   tctgggctga ggagttagag ggaagaaaat ggcgccctgt catgagggcg cgaggtccga    1080
   gttgagggga gaagactgag ggacacagaa gtggccgaag tgagggcgct gaggtcagcc    1140
   tgagctgaga gggccggcag atcacgccgc agccgaagtg aacgaactcg agtgaggtgt    1200
   gttaagaaaa tcaaaatgta taaatttgga aaaggagagg aggccgggcg cggtggctca    1260
   cgcctgtaat cccagcacgt tggaaggccg aggcgggtag atcatctgag gttagaagtt    1320
   cgagaccagc ttggccaaca tggtgaaacc ccgtctctgc taaaaataca aaaattagcc    1380
   gggcgtgtgg cgcgcgcctg taatcccagc tacttgggag gctgaggcag gagaatcgct    1440
   tgaacttggg aggcagaggt tgcagtgagc cgagatggcc tgggtgacag agcgagactc    1500
   cgtctcaaaa aaacaaaaca aaaaacaaaa aaacaggaga ggagacttta tttcttgtaa    1560
   ggggttacag cctgcaaggt ggccattccg caggctggag aatggccagg accagagaca    1620
   ggcatttcgt tggaggaggg gttggagtag cggctttatg ctgaacagct tgggtagaca    1680
   tgcatattca gcaggtgaca ggaggagcta tgaatattca tgagggtggt cctgacgcat    1740
   gcccattgag caaacatgca tgtaacatac aacccatgtt cattttggag tgaagagtta    1800
   ccaattaaat gttaaatgcc tggcctgcat ggtgacgtcc ccatctctac aaaaagtaaa    1860
   aaaaaaatag ccaggtgggg tggtgtgtgc ttgcggtccc aactagtcag gaggctgggg    1920
   caggaggatc acttgagccc aagagttgga ggctgcagtg atgtatgatc agatcactgc    1980
   actccagcct gatggactct aaaaaataat aacaatacat gtattacagt tagaccccat    2040
   gcgtcaaaag gtcttttcag gacaggaagg tgtgcaagta tgcagtctgt gtaaactagc    2100
   cagagccagt ccatggtcgg tagttttcct atgaggagaa agttactgaa atcagcctct    2160
   tgtccaatta aagctgtaat gatggctggt ggaatagggg ttcc                      2204
<210> 5
   <211> 2137
   <212> DNA
   <213> Homo Sapiens
<400> 5
   tctcattagt tagtacctaa ttatttaaaa gtgtggttta tgcttggaac tgggctataa     60
   agatggcaat gtgccaaatc agacatctgc ttcctgtaca ctcctataaa ttatatcagc    120
   ttattttaca actttgtccc tgtcagttat aaagaagaaa gtttaacatt ctagacgccc    180
   acacctcatc aaactgtggg tgaagagtca gtgagcggca aaaggtggcc gctttgagtt    240
   gtggtgatgg aattttcagg gaatagtcag gagcttggaa agtcactcct gagctggtcc    300
   attcaagcat tcgggataga agtggtcagt agaaattcct catggaatct caaaacacca    360
   ctaggacagt agctccacga cacttttaaa acctggagtt taaaaaataa tgaagtataa    420
   tttatttaga acttaagcat caagggaaaa cgttaagttc tgcttcactt caaagacttt    480
   tccctgagga aaacatgcca ctgtttttca gatgtgaatg aaatgatact ttttcccccg    540
   acatagtttc cttgcttacg actccacact gttctccttc aatccctttc accactgaaa    600
   cattacatgc aagatttgct ggcatgcccc ttgacttttc atgcttaagg ggaaaaatgt    660
   ttgtagctga tgtttgagtc ccaggagctc ggtgaagaaa aaaaaaaaaa gcaacgcaag    720
   gaaaattgct ctaatggtat atccttccta aagcttgaag tttcataggt aaaaggaggg    780
   ataaacattc cttcctgaaa gcaaacagtc aaggcttttt cccactcaac tatccctgaa    840
   ccttttgtaa tttccccgag gcctaaaccc agggcacaat taattccaga ccctatttta    900
   aatctaatcc gtctcctatt tgcaacctgc ctgcaggtgg gtcttttctt tctaaattat    960
   aaatctgaac ctgcgtcaac ctcttacaac acaaggcatt taaagcttcc tggagccact   1020
   gacctggtga cgctgtagtg cacgtctgca tttgaaagta agcaagagaa cagaaagcga   1080
   gcaagagaac cgcaaccttt accaccttgg cgaattcttg ccgggaccta catattaaac   1140
   agcacttgca cacagacgcc tcaagtcgtc ctctttagag tcgtcactta gacctgcaag   1200
   gggcggggga ccagtggaaa cccaaacaga aactttcacc attacaaagg cgagtcccca   1260
   ttgattatgg taattgtttt gctcaggttg tacgggtatg gccaattttg tttgaaagct   1320
   cgctctttct ctgcgctttc ctgtcgagtt cccttcaggt tctggtttcc tccgacggtg   1380
   gcaagggatg tttttcacgc ctttcaccac ttttgcccgc gcctgcgtcc cctgcccgag   1440
   gtcccaggcc gcggcggcgg cgcggtgggt tcattagcgg gcgcgccggg cccgcgcagc   1500
   gctgtctgca gccgcgtcgc cgcatccatc atcattacat cagcgcgggg aagtccacgc   1560
   cgagttctcc cggccccggc gcgccccagt cagcgcgcca gcaaacagaa catgctggat   1620
   tgctcagaga cggctctggg tgtctggggg cagaaggggt gctcccaggc ccggctttga   1680
   tccgcatttc tgaagctggc tttgggagac aatgcatctc cgccagccag gccgcgggaa   1740
   aagtgaaaaa agggctgggg gccatgtggg ggtggggagg tgagctccag cgccagcagc   1800
   gcctggcagt ctccctccag tgcagagaag cggtctgtat caaggcgtat cagagtggtc   1860
   accctgctcc tcggaatatt cacgggcatt tgcaaactct acagttggcc cagccaaatt   1920
   tccagcaaac actggcaagg gccggccctg gttgcgttca gagggctctt ttaactggct   1980
   gaattcaggg agctgcaacc ttccaggtac tttgcaatgc aaccaccctg agtgtcgcat   2040
   tataataaaa cattggggat cccttgaaaa gccttcacaa aaccaatagg tgtgtgcttc   2100
   gctcacaaaa cgtcgaattc accttctatg aacctgg                            2137
<210> 6
   <211> 2461
   <212> DNA
   <213> Homo Sapiens
<400> 6
   tttttttctt ttttcctttt tctctttctt ttcttttctt tctttctttc tttctttctt     60
   tctttctttc tttctttctt tttcgttttt ctctttcttt ctttttctgt ttcttatctc    120
   ctcagcttga aggttgcatc ctctttatct ccttaagtaa agctgggagt tctgtagtac    180
   tccaggagtg aggacaatta tgcttaagaa actttggttt cagtttagtc ccagaccaag    240
   aggaaaacat ttcaaaattt taaagtaaat cctaaaagtg taatttacac cagagcgatt    300
   gacctggcat ttatgtagac tgaagcttga gaagtaattc aagtggtttc cactcattcc    360
   cttggaagag tccttagaat gtacttccat tggtctgaaa aacgcctgaa aaagagtcct      420
   gatgctaaca gtaatggaaa taggagggct ggtggttcac gatcagccca gtggtatcct      480
   ctattctatt tgcatctagg taaagagact gtggcctgta tgtgatcaaa caatttgttg      540
   aagcgctttg ggcttagggc atttaatttc tgtgaagtag gaccatcagt tgtttgctct      600
   gtttttgtgt cacctctcag aatacattaa agtaggggca acccttgagc ccagacttct      660
   gccatgtgaa gaccctttga aaatcctgac aaacacaggt actgcgtaag tggtcagcta      720
   attaaagagg ggaggtggag ctgtcctttg tgtatccaat aagtacccat tatctcattt      780
   gagcatgaaa agaggccact gttattactt tcaagaagga aagtaagcag gatagctcat      840
   atttttagaa caattcctca ccaaatggaa taattccggt gaaaagtggg agtgaggaag      900
   aaagaaaaaa aaaacttcta atcataatgt ttgggaataa gaaaggaaga agaaactcac      960
   gtcaaagccg actttctcct gcagctgtaa aataaactct taagaccctt cctgctgaaa     1020
   ctctggagag gaaaactgga gtggcgggtg ggctttgcct gcagctcaac tctccctcgc     1080
   ggcgcgggcg cggctgggtt cagcacctcg gaaagcgccc ctcgcggcgc cccgggatta     1140
   cgcatgctcc ttggggcccg ccgccttggc cgtgcaagtg ccaccgtaac tggtgagagc     1200
   cgctggcaac ccacccggag ttgacaaccg cggagagacg cagacaccca ctgacctcca     1260
   ggaagctgag cgtggtggat ggaactctac gatctctttc tctccaagga cggaaacctc     1320
   atccaagcag tcccagagga aacggataaa ggtatttgaa agggagcgag cggccccaaa     1380
   tcgcacaatt gagcggctgg gggagttatg cgccagtgcc ccagtgaccg cgggacacgg     1440
   agaggggaag tctgcgttgt acataaggac ctagggactc cgagcttggc ctgagaaccc     1500
   ttggacgccg agtgcttgcc ttacgggctg cactcctcaa ctctgctcca aagcagccgc     1560
   tgagctcaac tcctgcgtcc agggcgttcg ctgcgcgcca ggacgcgctt agtacccagt     1620
   tcctgggctc tctcttcagt agctgctttg aaagctccca cgcacgtccc gcaggctagc     1680
   ctggcaacaa aactggggta aaccgtgtta tcttaggtct tgtcccccag aacatgacct     1740
   agaggtacct gcgcatgcag atggccgatg cagccacgat agccaccatg aataaggcag     1800
   caggcgggga caagctagca gaactcttca gtctggtccc ggaccttctg gaggcggcca     1860
   acacgagtgg taacgcgtcg ctgcagcttc cggacttgtg gtgggagctg gggctggagt     1920
   tgccggacgg cgcgccgcca ggacatcccc cgggcagcgg cggggcagag agcgcggaca     1980
   cagaggcccg ggtgcggatt ctcatcagcg tggtgtactg ggtggtgtgc gccctggggt     2040
   tggcgggcaa cctgctggtt ctctacctga tgaagagcat gcagggctgg cgcaagtcct     2100
   ctatcaacct cttcgtcacc aacctggcgc tgacggactt tcagtttgtg ctcaccctgc     2160
   ccttctgggc ggtggagaac gctcttgact tcaaatggcc cttcggcaag gccatgtgta     2220
   agatcgtgtc catggtgacg tccatgaaca tgtacgccag cgtgttcttc ctcactgcca     2280
   tgagtgtgac gcgctaccat tcggtggcct cggctctgaa gagccaccgg acccgaggac     2340
   acggccgggg cgactgctgc ggccggagcc tgggggacag ctgctgcttc tcggccaagg     2400
   cgctgtgtgt gtggatctgg gctttggccg cgctggcctc gctgcccagt gccattttct     2460
   c                                                                     2461
<210> 7
   <211> 1920
   <212> DNA
   <213> Homo Sapiens
<400> 7
   ccaggctgcc gtagacacag cctttgctct cccgaaaaac acgttctagg cgccgggatt      60
   ccagatacct gggaaataga gtgcacgcag ctgttgagag gcctcgcgct tggcttctcc     120
   tatcactgag gcgcagaggt gctgtggaca gcccagaccc acacggcgcc cgaggtgaaa     180
   cagaaccctc agtctcccta tgaggccact ggcactctcg gctgtcccca gagctctccg     240
   acttagagct gaatgcaaag taagcgctcg aaatgcagaa gtagccgggg ccgcccacgg     300
   cacctgcctc gctcggggcg agagaagacg ccaggctgag gtcccagcga cctcaggcac     360
   cagctccgaa ggagggcggg gagaccgcaa aggggaagtg cccggagggc caacggcccc     420
   cgcgcaccct gcgcccctct gaagcgcgcc gcctccccgc gccggggact gggacctgcc     480
   tctggggaat ccgcctagaa gacggcggcg gactggggtc gggcactctc cagggctgtc     540
   aggccctccc cagccctgca cctgccgcgc cgccccacct cgccaggaag tctcagagac     600
   cccggggatg gggtgggagc gccttcccat cgcgggctca aaaagaagga aggacgcccc     660
   caggggtcgt agaaggagga ctagctccaa gccacaactt tcttcggacc caaggcaggc     720
   cggctggggc tccgcgccta cacggcccct ggcgggggtc cgcgcgcccc gggagccccg     780
   cggctcgggg aggaaagagg agacaagaga caggcgagga ttacggggct gacccagccg     840
   gggtagggac catcgtggaa aaactttggc gaggtggggg gacgcggaaa gagagcggcc     900
   cgcgccctgc accttgcgcc gggcatcccg cgccagtgcc tcgctcccag tgccccgcgc     960
   cccgcgcccc gcgccttgcc ttcaccccgg gccagctgca tcgcgcccgc gccgcaggaa    1020
   ccgtggagtt ggaaagtggg ggcgccgcgg ctggggggct gcttcagctg cgcctcggcc    1080
   agcgatcggc gggccgggct caaatccagc caggctgggc aggcggtggc cgcgcgactg    1140
   gggaccgggc gccccgccct cctcgctccc ctcctccttc ctctccctcc ctccagcccc    1200
   ttggcctttt tcagccccta ccggatctgc tcgtccgctg tcctctcttt tctctcgctc    1260
   ttcatatcac tctccacccc ttcgccttgc cttcgccttt cttcctcccc ttgtctcctg    1320
   ccccctcctc ttctcccctc ccctctaggg gcggagcttc tcccctccct cccagacaat    1380
   gctgtggctg cgtccccttc cccgccagct cgtccaggct cccgccgcca gcgattcttc    1440
   cgggctgggg gtggggaggt ggggggggag tgcagggttg gggaggatga gctggctccc    1500
   ctcacctcct tgctgctgcc ctctccaaga gggatggaga cttggcccaa gctcctcggt    1560
   tcacccggag ctgtgacagc cactcccagg gaacagtcac gctgccctac caagcccacc    1620
   tccagcggcc tggattcccc aggcagaggt tgtgggattt tgttttttct aacatcccag    1680
   cttattccca aaagggtttg agccggacag gggctaaaca ggccccttcg acttggcggg    1740
   ccggccagac gtgacagcaa tgccaaggag gccaagtttc tttgtccatt tctcacctcc    1800
   cccttttcca tccctggacc tcctggcgcc cccagtacac agaggccctt gagcagcccg    1860
   gctgcaggtt ccctatctac tcagagttct ccccctcacg tgcctatccc caaccctgca    1920
<210> 8
   <211> 2225
   <212> DNA
   <213> Homo Sapiens
<400> 8
   atcttcctgt aaaccaaatt cctgctgccc agtccagtgc ctggtgcata gtgggggcta      60
   gacagacatc tgttgagtgc atgaccaggg cgctgtaggt ggaggaggtg gtggtggggt     120
   gggggagggg aattcccctg ctgctccccg ttctgtggga accagctcca gaaagaaatc     180
   ctctgccgcc cttcccaccc tactccaact ttggatctgc tctctgacct ccttcacttg     240
   gccctagttc tccacccaga gtcaaccctg cctgaaaggc accatgtcct gtcaccaagg     300
   tctcaaaata gggaaaatag tctctgaagc cctcagcccg caggagatag ggcaccccag     360
   ggtacaaggc ttcaccgagg ggctggaaaa agagcccaga tcccaacacc tatagaaagg     420
   ggccctggct gtccagctgt gagtggcctt cccctcgcct ctcagcacac ctggaaacga     480
   ctccacatgt gggcgccgcg ggatgctggg tagcaaggag atgacccagc tgaacaatgc     540
   atttggtcca ctcctgcgag gggaggagaa cgccgaggga caggaactgc gcgccagcaa     600
   aagacaccct aacctggggt gcgcccgcca gctgctttcc caaggtgaaa acctccaaga     660
   ggcgagatgc aaatcccagt ggcgtccaca cccggaggtg tcttctctcc aggtcgcccc     720
   tccgtctcat gcgacacccc aatccccgca gcctctgctc agcgtgggac caagaatggg     780
   gagtcctacc cctcctcggc tttccagccc cctaacccaa gacaagaggg cgcgcgcccg     840
   ggctgggaca ggtcccagcc gcattttcgt agattgcact ccccgcccgg gagcgacccg     900
   ttccagcagc ccccttcccg ccccatcaac gcggccgcac tgtcggcgtc tagaggcgac     960
   atcctggcaa agacaggaat caagtcagaa attcaggctc tagaagcaaa ggctgcggtg    1020
   cctcctggct ggaagaaaac ggcgggtggg tggaggatgc ggtgcgcggg aatttcccca    1080
   cggacgcttg ctgtttcctg ctcacctttc aggaaggtgc ccctctacct gcaccctccc    1140
   ctgcaatttg gagctcgtcc gcgagctcag ctacccgctg gctccctctg cccgcgcccg    1200
   cacaccacag actccgctta ccgtacccta cccggtccta gagggcccag aggggatctc    1260
   ctgaaaccaa ctcgcctgct tggagccagc cccgacgcat atctcgagag tggcgggagg    1320
   caaagcagcc ggaatgcccg agaaaaaagt gggcgtcaga ggcccttgtc ctagcgactc    1380
   ccccaccgca ccgactgagt cccgggactg cagggatcta agcccttacc tgtctcggcg    1440
   cctcctttct gtgcgctctg ctgctccgga agccggatcg agagtcacag tgtggttggg    1500
   cgcaggcggg ccgctcggtt ttcacctcga ggacccggac ttgcacttgc agagccgctg    1560
   cgtgggggat gtccctcttt tttcctcgcc ccggcgggct cctccggcgt gtcagggtct    1620
   ctgggttcca gctggagttg gcgctccggc cccacagctc tttccagcgt ctcggacctg    1680
   tgcgccccgc cccgcgctgg actccgcccc ggggtccccg cctgcctcct agtccaggcc    1740
   aagggtggat gcccagctcc cccaccccac cgcgtgctct cgggctggtg cgtccagggc    1800
   gcgggcaccg tagctccggc cgcgctgacg cgagtccccg gcagcggggg cagcctctcg    1860
   cacggatttc agcgccttcg catcccgtct gaatcggacc acacagtagc cgggtttcta    1920
   ccgtgcgctt gggaacagtg agcaccgccc tctgccgcca gtcgtggcac cgcacccggg    1980
   tcgcgcgcgc aggaggggag agtcctgtct tccggaggtg cccagtgcca gcgtccgcgc    2040
   ccactagaag gtgacccttt caaggaaagc ccggctccga cgagtgcggt gtgggccttt    2100
   ggagatggcg gtgaaggagg aggcccccag gaggaagctg gggatgggga cagtttgctc    2160
   tgctcaaagc ccctgaagtc ctcacccaaa ctttgcgatg accaccacta tcagattcca    2220
   tggcc                                                                2225
<210> 9
   <211> 2251
   <212> DNA
   <213> Homo Sapiens
<400> 9
   cttttcagcg tgactgaccg cgactcagag gagaatgggc aggtgcagtg cgagctactg      60
   ggagacgtgc ctttccgcct caagtcttcc tttaagaatt actacaccat cgttaccgaa     120
   gcccccctgg accgagaggc gggggactcc tacaccctga ctgtagtggc tcgggaccgg    180
   ggcgagcctg cgctctccac cagtaagtcg atccaggtac aagtgtcgga tgtgaacgac     240
   aacgcgccgc gtttcagcca gccggtctac gacgtgtatg tgactgaaaa caacgtgcct     300
   ggcgcctaca tctacgcggt gagcgccacc gaccgggatg agggcgccaa cgcccagctt     360
   gcctactcta tcctcgagtg ccagatccag ggcatgagcg tcttcaccta cgtttctatc     420
   aactctgaga acggctactt gtacgccctg cgctccttcg actatgagca gctgaaggac     480
   ttcagttttc aggtggaagc ccgggacgct ggcagccccc aggcgctggc tggtaacgcc     540
   actgtcaaca tcctcatagt ggatcaaaat gacaacgccc ctgccatcgt ggcgcctcta     600
   ccagggcgca acgggactcc agcgcgtgag gtgctgcccc gctcggcgga gccgggttac     660
   ctgctcaccc gcgtggccgc cgtggacgcg gacgacggcg agaacgcccg gctcacttac     720
   agcatcgtgc gtggcaacga aatgaacctc tttcgcatgg actggcgcac cggggagctg     780
   cgcacagcac gccgagtccc ggccaagcgc gacccccagc ggccttatga gctggtgatc     840
   gaggtgcgcg accatgggca gccgcccctt tcctccaccg ccaccctggt ggttcagctg     900
   gtggatggcg ccgtggagcc ccagggcggg ggcgggagcg gaggcggagg gtcaggagag     960
   caccagcgcc ccagtcgctc tggcggcggg gaaacctcgc tagacctcac cctcatcctc    1020
   atcatcgcgt tgggctcggt gtccttcatc ttcctgctgg ccatgatcgt gctggccgtg    1080
   cgttgccaaa aagagaagaa gctcaacatc tatacttgtc tggccagcga ttgctgcctc    1140
   tgctgctgct gctgcggtgg cggaggttcg acctgctgtg gccgccaagc ccgggcgcgc    1200
   aagaagaaac tcagcaagtc agacatcatg ctggtgcaga gctccaatgt acccagtaac    1260
   ccggcccagg tgccgataga ggagtccggg ggctttggct cccaccacca caaccagaat    1320
   tactgctatc aggtatgcct gacccctgag tccgccaaga ccgacctgat gtttcttaag    1380
   ccctgcagcc cttcgcggag tacggacact gagcacaacc cctgcggggc catcgtcacc    1440
   ggttacaccg accagcagcc tgatatcatc tccaacggaa gcattttgtc caacgaggta    1500
   aggctgaagc gaaaggacca ccatctctca tctcctccat cagaaagcct cctctagccc    1560
   ggcccttgta tctctggtgc actgtatcta tttttaggat attagcttat gtgtatcgtt    1620
   gtgggagcag agatgggcgg tcaccttctc ccactccttc gtgtgtaacc taactttcgc    1680
   gttgttccac cctttcacat ttattttcat tccgtcccct tggtactttg ccaccttgga    1740
   gctccctcct ttgctcttcc atcctgtcag tcctttccct tctcagtaac ctgggcatga    1800
   agggaaactg cgtgaaggga gagggaaatg tggaggaggg acttactttc tagcactggc    1860
   aaaggtcttt tttctttgcg tctgtcccag gcattaataa agttggctct attttgcttt    1920
   gtttaacgat gcttttagtc gcgtgtacaa gtaagctata gattgtttaa ctttacacag    1980
   ttgtctaacc tcgaattcat gttttagtga acaagttacc agatccttct cctctgaact    2040
   cgggttgcaa aaaaagcctt cagttcggct ctggacagca tttacagacg ctcttgaagc    2100
   cgagcgccca cacagtgtga atttgaatga agctgcgttg gcacaaaccc ctgttaagag    2160
   taaactaacg aaaggcttaa acaattgttg tcttaaatat atctttttag ctaataatca    2220
   ccttcttgcc actgttttgg ataaatcacg c                                   2251
<210> 10
   <211> 2553
   <212> DNA
   <213> Homo Sapiens
<400> 10
   ctggccaccc ctgccctccc ttagacctcc agagccccca gtgtagccac agaggatgct      60
   gttggcttca gccccaagaa gacgccgctt cctccagagg gctaagtaag tgggaatccc     120
   cctccctact tgtcctgggc tccaggcagg gcccctggtg taaggcctgg ggctggaagc     180
   cgacccacct aggtccaggc tctggggcag aactgaaact ccttggttac tgtcggctgc     240
   agcctgggag caggccactg ccaaagctgt gggtccttcc aggacagtct ccccatgagg     300
   ccggtcctcc acctgctgtt tcttcacacc tggtggccag ggatgtggcc ctgggtagaa     360
   cgatgattct ccactcctgt cattatggaa gccaccgctg tctcccagcc cagccagcca     420
   cctgggctgc agagcacccc tttcatgccc tccgggtgcc tcccccttct cctgccccag    480
   cctggctttg tcctaccctg ctctcaggga ggggtaccct ggagtggggc cagggcatgg     540
   ctctcccccg agggagttcc tctctggctg tccccagggc agctctgcac agcctcagta     600
   cctggcgcac ctcccttgac atccttctta gggacagtca ggcactctgt gtggggcact     660
   caagagagcc aggcccgtca gcctctagct cctgccagaa tgcaggcctg aggggtgagg     720
   ggcggggcag gggcagggac aggaactccg gcgtgctctc catccgcaaa ggttcactga     780
   ggccccgagc cccagccact gagccaccaa gtcagcctgg gccaggcctg ggtgccctgt     840
   ctgcaatgga ggcagagacg gggtctcggg gcagttctga ggatgctggg tgcacagcgg     900
   gggcctcgcc ggcaggaatc acttatgctc tctcctgggc caagctttgt ggatgcccag     960
   cctggggccg cggggagctg gcaggtcagt ggcagacact ggtgggcaga cctagtgtct    1020
   ggtagaacag gcatcaagga agtggtgacc ggagggaagc caagtgcact caaaccctcg    1080
   ggtgagtcat caccgccggg tctttcacag ctgctgaaag tgagcaacag tgatgaaggt    1140
   ttgtgagttt ctgcgtgagc gagtgaatgg accagtagca gtttccaggt tgtggaagag    1200
   cgttccctcc ccgggatggg gacacttggt tacagcaatt cctaatcccc cacccaccca    1260
   ccgcccactg cagaggtatg cgggggccct gcttcctgca ggcaggagtg aggggcactc    1320
   ctgtgatgtg gcacccctgt gaccgaggtc atgtgtgatc ggtgtaaggg caggaagcga   1380
   gtcattggtc tgcaccaggc gtgggggctt ctgcgagggc aggacccaaa gtcggcctgg   1440
   cctcccggct gcagcactcc tttccctttc gaattaggtt agagccctgg gacgggaggt    1500
   gccctgtaga ccacccccct caccaacttc cgtcctccgc cccacccccg cggtgatccg   1560
   gtgaactgcc ggccccctgc tgtgcaccga gtggggcagt gaccctgacg tggcgtctcc   1620
   tgccgcccct gccaccgcca ccacctccgg tggcccagcc tccgcattcc ccacccccat   1680
   ggaggaatgc accaggcctc ccttcctgga tgcacccctc acccacatgc ttccaaaccc   1740
   tggcattttc tgctccccct ttactcccac cccttcccct aggctcccag acaaagggga   1800
   agtggctgga tcctcttaaa gggacagtgt cccaccagct tactgctgaa ctcccctcct    1860
   caaccccagt tccctagtta cagttaatta gcattagcag acagcccatg agtgataccc    1920
   atgcaggccc caggctgtgg agagtttcct gggtaggaaa cagcccttaa ggtccctcat    1980
   ctcatccagg tcccagtctt tcctacctgc ctctctccta gattgtggcc ctttggagcc    2040
   tggttcttct gtccctgtgt gaccgacaca tagcacccaa acagtggcag agcgggacgg    2100
   accccctagc ctgttctctg tgtgggtctg taccctgacc cagacatgcc cccccacagc   2160
   aggacccagg ggggcacatg tgtgcctgcg ggttcactgg ggcacccgca tttggtttat    2220
   tttatttttt agagagaggg tcttgctgtg tcacccagct ggagtgcagt ggtgtaatca    2280
   tagcacactg cagccttcaa ctcctgggct caagcgatcc tccctcccca gcctccctag    2340
   tagctgggag tacaggaccc actgtatcct ggctaatttt ttaataattt tttaagagat    2400
   ggggtcttac tgtgttgccc aggctggcct caaacctctg gcctcaagtg atcctcccac    2460
   cttcgcctcc tgaagtgctg agattacagg catgagccac catgcccatc ccagactgac    2520
   atttctatat ttgttcatcc tggctgggca ggg                                 2553
<210> 11
   <211> 2001
   <212> DNA
   <213> Homo Sapiens
<400> 11
   ggcaaaagcc tgcctggact tcctggccac cagaaatatg agcatggtgg tggtccccag      60
   ttccctattc atgcttgggc tcaagagact gggagtctag gttcactgac tccctgagaa     120
   agactaagac cctgcatttt agaaagaggt ttggggatct ctgccctgcg caagggtaga     180
   aggatcagct gttcctctga gcaccttaac ccggaacccc ggtccgaagc cgagacagga     240
   gactggatgc gaggccctcc cagagctggt ttctctcaaa caacttccaa aactcctaga     300
   tcctaggggt acgccgaaat cccccaaagc agtccaaaga acacaacgag agtcctaaca     360
   tcccaggtgg cggcgcgctg gctccctgga gcggggcggg acgcggccgc gcggactcac     420
   gtgcacaacc gcgcgggacg gggccacgcg gactcacgtg cacaaccgcg ggaccccagc     480
   gccagcggga ccccagcgcc agcgggaccc cagcgccagc gggaccccag cgccagcggg     540
   accccagcgc cagcgggacc ccagcgccag cgggacccca gcgccagcgg gtctgtggcc     600
   cagtggagcg agtggagcgc tggcgacctg agcggagact gcgccctgga cgccccagcc     660
   tagacgtcaa gttacagccc gcgcagcagc agcaaagggg aaggggcagg agccgggcac     720
   agttggatcc ggaggtcgtg acccagggga aagcgtgggc ggtcgaccca gggcagctgc     780
   ggcggcgagg caggtgggct ccttgctccc tggagccgcc cctccccaca cctgccctcg     840
   gcgcccccag cagttttcac cttggccctc cgcggtcact gcgggattcg gcgttgccgc     900
   cagcccagtg gggagtgaat tagcgccctc cttcgtcctc ggcccttccg acggcacgag     960
   gaactcctgt cctgccccac agaccttcgg cctccgccga gtgcggtact ggagcctgcc    1020
   ccgccagggc cctggaatca gagaaagtcg ctctttggcc acctgaagcg tcggatccct    1080
   acagtgcctc ccagcctggg cgggagcggc ggctgcgtcg ctgaaggttg gggtccttgg    1140
   tgcgaaaggg aggcagctgc agcctcagcc ccaccccaga agcggccttc gcatcgctgc    1200
   ggtgggcgtt ctcgggcttc gacttcgcca gcgccgcggg gcagaggcac ctggagctcg    1260
   cagggcccag acctgggttg gaaaagcttc gctgactgca ggcaagcgtc cgggaggggc    1320
   ggccaggcga agccccggcg ctttaccaca cacttccggg tcccatgcca gttgcatccg    1380
   cggtattggg caggaaatgg cagggctgag gccgacccta ggagtataag ggagccctcc    1440
   atttcctgcc cacatttgtc acctccagtt ttgcaaccta tcccagacac acagaaagca    1500
   agcaggactg gtggggagac ggagcttaac aggaatattt tccagcagtg agcaggggct    1560
   gtatgggacg cgggaggagc tcagaggagg cgcggagagt gcccgaggtt gggtgagtgc    1620
   ctagagggga gatagttgaa ccgggttcaa gaggtgctta gtgggtgttt gttgaatgaa    1680
   tgagtgatgg gctttgaagt ctgagtgcat tgaaagaggg ggtgtgtaaa aagggctcct    1740
   ttcatcacac aggacacagc atatgcaaat cctctccctg tggaaaagcc agacaggtta    1800
   aaaaggttac aaacaaatta gccgggcatg gtggtgcgcg tctgtagtcc cagctactag    1860
   ggaggctgag ccaggggaat cgcttgaacc cgggaggcgg agattgcagt gagccaagat    1920
   cgcgccactg cactccagcc tggaaacaga gcgagactcc gtctcggaaa aaaaaaaaaa    1980
   aagttacaaa ccgtgtgtgg g                                              2001
<210> 12
   <211> 219909
   <212> DNA
   <213> Homo Sapiens
<400> 12
<210> 13
   <211> 1405
   <212> DNA
   <213> Homo Sapiens
<400> 13
   cgttacccga gttgtaaagg gcggctccct gtgtctgccc cgctgcaccg atacaccgag      60
   ctgcgcacgg tgcccagcgc agggagaaca aatgatcatc tgtccaacgc gcccatttac     120
   aggtgaggaa actaaggctc caactcaatc gacgcactct gcccttttga ttaccagaaa     180
   agtagcagga caggtgtcct gtcccgccct accccggccc actaagccgg caccccggct     240
   ccgacccccg gctgtgcccg gcgccgccgc ggtgcccggc gccgccgcct cgcccggcgg     300
   ggccgcccgg agcgcccgca cctccgcccg cttccacctg gccgggcccg ccccgcccgg     360
   actcgggact gggaagtgcg gcgactcccg gaaccagcca ttggcgccag cgcggggagc     420
   tgggggtgca gagctgcggg cgcggcgggc cacgcaggcg gcccccaccc ccggcctggc     480
   ctggtctggt ctggtctgcg ctgccgcgcg ggggcgcccc ctcccaggcc cggcgcccgc     540
   cagccccgct ccgccaggtg cagcgcagcg caggggtggg cgggggtggg gctcggcgcg     600
   cacgttcacg gggcggggag ggggcgggtc aggggcggga ccacagccgg ctgggccggg     660
   gttctatgcg catctccggg gaggggcggg gcgggggcgg ggccggggcg gggcccggtc     720
   ggtgcactcc agacggcggg ccgccccctc ttcccgcctt cctactaccg gcccaggatt     780
   agcgccctgg gagcgcgcgc cccgctgcct cgccgccaca ctttcctggg agcggcggcc     840
   acggaggcac catgaagaag tcttactcag gtgggcttcg cgcccggggt ggggaggggt     900
   cggtgtcccg ggaccagcgc tgctcacctg agtgcctgcg gccgggagtg gcgaggcgcc     960
   cccggagctg agcgagtccc cgcggcgggc acactgcagg tcgagttcct cccaggacag    1020
   ggccgctgtc gggccgcttt cgacctgagc cgaccgtccc ctgcgctgtc tccagccctt    1080
   gctcgagtgt cggaggggct gccctggggg acgctccctc ttcctcgccc cttgcaccct    1140
   cgcaggaatc gctgactttc caggtcggcc gggtgctttg ggtccctgtg cgtctgtgtg    1200
   ggtgaatggg gtcggggcta ggtggagggg tgtccttggg ttcagcctct agggctggtg    1260
   gtccaggccg cagcatcctt tcttcggatt ctcttcggtt tctcctctac ttagtggggc    1320
   acgggacggc ctccagatgg gaccgtccag cagcgcccaa acttggcgac tcgggttcac    1380
   gttttgcgct caggacgccg cccgc                                          1405
<210> 14
   <211> 1818
   <212> DNA
   <213> Homo Sapiens
<400> 14
   cgtcgttcgt ttttggtttt tttgatagtc gtgttttatt ttcgttttgt gttttttttt      60
   tcggatagtg ttttttttag ggtttattta ggagggtgta gcggtggttt tcggggcggt     120
   ggtcgtggtg ggggtgttag ttgtaggggt gttttcggtg ggtgggagtt ggtggttttt     180
   cgttggtgtt atgggattcg tatgttcgtt ttgcgttttt cggtttttga gtttataggt     240
   cgggattttg tttgttagtc gcgtgcgttg tcgtttaatt tttgtaggcg tagagcgcgc     300
   ggcggcggtg atagagaatt ttgtttggtt gtttaaatat agttttttgt agaaggattt     360
   tgcgttcggg gaaggggagg aatttttttt tttttgggcg ttcgtttttt tcgttatggt     420
   tcggttttta tattcgttta tatttggtcg tagcggggcg ttcgggggga ggggttgagg     480
   tcgcgttttt cgtcgttttt tgggcgcggg ttaggcgggg aggagggggg cgtttcggtc     540
   gtgtgtttag gattgttttt tagcggttat tcgggtttta gttttttagg tttggttttg     600
   ataggcgggc ggagtagtta gtgcgagata gggaggtcgg tgcgggtgcg ggaatttgat     660
   tcgttcggga ggcgggggcg gggcgggggc gtagcgcgcg gggaggggtc ggcgttcgtt     720
   tttttttttt atttatttag ttgagttagg gggtttaggg gttttttcgg cggttagttt     780
   tgtattgtag gagcgcgggc gcggcgtttt agttagcgcg tagggttcgg gtttcgtcgg     840
   gggcgttttt tcgtcgttgt ttttcgcgcg attcgttgtt tattagttat tatgtcggat     900
   ttcgcggtta acgcgtagtt ggatgggatt atttcggatt tcgaaggtgg gtgttgggtt     960
   ggttgttgcg gtcgcggacg tgttggagag gattttgcgg gtgggtttgg cgcgggacgg    1020
   gggtgcgttg aggggagacg ggagtgcgtt gaggggagac gggattttta atttaggcgt    1080
   tttttcgttg agagcgtcgc gcgttttcgg tttcgtgttc gcgtcgttta cgtgggggat    1140
   tttgttaggg gtattcgcgt agattttgcg cgtttttata ggattttgtg ttcgttttgc    1200
   gtattgtcgt ttgggttttt ttttttttat tgttgtttgt gtttgttaag cgatagcgat    1260
   tttttcgagg gttcgcgagg ttgtttcgga attttttagg acgtatagtt ttattttggg    1320
   aaatttatcg gttttttttt tttggttttt ttcggcggtt ttcgggtttc gtttggattc    1380
   ggtaacggga tagggaggtc gttttttatt ttcgattgag tggatagtcg cgttttgttc    1440
   gggtggatag tttttttttt ttttacgtta gtttcggggt cgttaagttg tgtagttcgt    1500
   gggtcgggag tatcgaacgg atatagttta ggtcgtggta gggtttagag tgggatgttt    1560
   tatggttttt atttaggttt ggggatattt ttattcgttt tttagaatcg ggtcgtgggg    1620
   gatagaaggg gtttgcgtgc gggtagggag agtattttgg tttttttttg ttttcggggt    1680
   ttataaagtg tgttgggatt tgcggggttg ttttgtttaa gtttgggttt ggcgttcgcg    1740
   tttttgagtt tgtgagtgcg tgcgtttttt tgcgtttttt tgattgtcgg tgttggggtt    1800
   ttgcgttttg cgttcgcg                                                  1818
<210> 15
   <211> 2519
   <212> DNA
   <213> Artificial Sequence
<220>
   <223> chemically treated genomic DNA (Homo sapiens)
<400> 15
   gatttatgag tgaatgatta aaagtgtagt tgagttttgg tagagggtat ggggttttat      60
   ttagagatag gtagagaaag ttgaagtttt aggattggag gtcgtttttt tttatttttt     120
   tattaggttt aggtagggtt tgatttgaac ggaggtttgg gaatttgtgg ttagttttta     180
   tttgttggaa aagagtagtt tttaagttta attgttttag gttgatgttt ttttattttt     240
   ttttatttat ttatttttat tattattttt tttttattga gacggagttt tattttgttg     300
   ttaggttgga gtgtagtggc gcgatttcgg tttattgtta ttttcgtttg ttgagtttaa     360
   gttttagttt tttgagtagt tttttgggta gttgggatta taggcgtgcg ttattacgtt     420
   aggttaattt tttgtatttt agtatagacg gagttttatt attttggtta ggatggtttc     480
   gattttttga tttcgtgata ttttcgtttc ggtattttaa agtgtcggga ttataggtat     540
   gagttatagc gttcggtttt tagttttttt taaaaaacgt tagattcgtt cgttgcgttg     600
   agtggaggcg gggtaggttt tcgtttttta attggtttta tttatcgagt tttttttttg     660
   tgtcgggttt tgtgttaagt atattatacg ttgtattttg cggttaggtt gttgtggttt     720
   agggtcgtat tttggtttaa ggtcgtaaat cgaggtggga tttcgattcg gtaattacgt     780
   tcgtggttcg gaaacggcgt tttttgaggt ttaggagagg tcgggcggtg agcggttgtg      840
   gagtcgagcg cgggtagtgc ggatgttgtt tatgggggag gtagttaagg acggagggcg      900
   agaggcggtt tttttaaggt tatttttttt cgggttgtaa gtaaaggtta ggggatttcg      960
   gaatggttag tgtaggagtt tttttgtgtt ttttacgttt tagattttta gagttttaga     1020
   aacggagatt atcgttttta tttttatttt atagatgaag aaattgagtc gaggaaagga     1080
   agcgatttgg ttaaggtcgg agagtttatt ttttgtaggg cggggtttgg aattcggggt     1140
   ttggttttcg gtaacgcgtt ttcggttcgt agttttttgt tttttgtgtt tcgtttcggt     1200
   ttttagcgta gttgtagagg ggtttttttc gacgtatata tttaagagtt cgatcgcgcg     1260
   gttgaaatcg cggagttcgg agtcgcggtt ggttgagcga tcgcggtttt tgggttgcgt     1320
   gcgcgttttt tggagttgaa aggagcgtta ggatcggggg cgttgtatcg ggttgggttt     1380
   tttaacgttc gtagatcggg tcgggttgta gttggagatg gtagtaattt cgggaggttt     1440
   tcgggttttt ttagggtgcg tttaggaggc gggtttcgtg cgacgcggtg tagtttattt     1500
   tttttttcga gatcgtttaa tttcgcgggg gtagtttcgg gcgttcggag acgcggaggt     1560
   ttagattgta gttttcggat gttggaagtt tagatttttt ggggttatcg gttttttcgt     1620
   tattttagtt gtagagagtt ttattgtttt atcgttcgga gtttagtttt tttgtttttt     1680
   tattagtttt ttttttcgta ggtttttggg gatttttgat cgtttgtttt tatttttttt     1740
   ttgtttttat atttttcgtt tttgttttag gaacggtgat atagttatcg gattgttttt     1800
   tattttttgt tagtttatat tgtatataat ttaataattc gcgttttttt attcgggtga     1860
   tagagatata gataatttga gttagtggtg tttaaagtat cggttttaga atagtagtat     1920
   tagtattttt tgggaatttg ttaaaaatga gaatttgggt cgggcgcggt ggtttacgtt     1980
   tgtaatttta gtattttggg aggtcgaggc gggcggatta cgaggttagg agatcgagat     2040
   tatttcggtt aaaacggtga aatttcgttt ttattaaaaa tataaaaaat tagtcgggta     2100
   tagtggcggg cgtttgtagt tttagttatt tgggaggttg aggtaggaga atggcgtgaa     2160
   ttcgggaggc ggagtttgta gtgagtcgag atttcgttat tgtattttag tttgggcgat     2220
   agagcgagat ttcgttttaa aaaaaaaaaa aatgcgaatt tgggggtttt attttagatt     2280
   tattgaatag aaattttgtg gagtttagta gatgattttt atgtatatta aagtttgcga     2340
   gttattgatt taaatatttt tttatttatt tattttttat ttggtttatt tagtattgtt     2400
   agtgggagag atattcgtaa agtattaggt tgtgagtttt atcgtcgtgt attttgagat     2460
   attgtttatt agttttggga tggtaggtag aggtatttta gtttggttta gtgtagatt      2519
<210> 16
   <211> 2519
   <212> DNA
   <213> Artificial Sequence
<220>
   <223> chemically treated genomic DNA (Homo sapiens)
<400> 16
   ggtttgtatt agattaagtt ggagtatttt tgtttgttat tttaaagtta gtggatagtg      60
   ttttagagtg tacggcggtg gggtttatag tttggtgttt tgcgggtgtt ttttttattg      120
   gtaatgttgg gtgggttagg tgaagaatga atggatgaaa gaatgtttag attagtggtt     180
   cgtaaatttt agtgtgtatg ggaattattt gttgggtttt atagagtttt tgtttagtag     240
   atttggggtg gggtttttaa attcgtattt tttttttttt tgagacggag tttcgttttg     300
   tcgtttaggt tggagtgtag tggcgggatt tcggtttatt gtaagtttcg tttttcgggt     360
   ttacgttatt tttttgtttt agttttttaa gtagttggga ttataggcgt tcgttattat    420
   gttcggttaa ttttttgtat ttttagtaga gacggggttt tatcgtttta gtcgggatgg     480
   tttcgatttt ttgatttcgt gattcgttcg tttcggtttt ttaaagtgtt gggattatag     540
   gcgtgagtta tcgcgttcgg tttaaatttt tatttttaat aagtttttaa gagatgttga     600
   tgttgttgtt ttgggatcgg tattttgagt attattagtt tagattattt gtgtttttgt     660
   tattcggatg gaagggcgcg gattattgag ttgtgtgtag tgtagattaa taggagatgg     720
   agagtaattc ggtgattgta ttatcgtttt tgagataagg gcgggaagta tgggggtagg     780
   gggagagtaa gaataggcgg ttagaagttt ttagagattt gcggagggag ggattggtag     840
   aggaataagg agattaagtt tcggacggtg aagtaatggg attttttgta gttggggtgg    900
   cgggagagtc ggtgatttta gggagtttgg gtttttagta ttcggaggtt gtagtttggg     960
   ttttcgcgtt ttcgagcgtt cgaggttgtt ttcgcgaagt taagcggttt cggggggggg    1020
   ggtgggttgt atcgcgtcgt acggaattcg ttttttggac gtattttgaa gaggttcgga    1080
   gatttttcgg gattgttgtt atttttagtt gtagttcggt tcggtttgcg agcgttgagg    1140
   ggtttagttc ggtgtagcgt tttcgatttt ggcgtttttt ttagttttaa ggggcgcgta    1200
   cgtagtttag gaatcgcgat cgtttagtta gtcgcggttt cgagtttcgc gattttagtc    1260
   gcgcggtcgg gtttttgagt gtatgcgtcg aggggggttt ttttgtagtt gcgttggggg    1320
   tcgaagcggg gtataggggg taggaggttg cgggtcgagg gcgcgttgtc gagagttaga    1380
   tttcgggttt taaatttcgt tttgtaaaga atgagttttt cgattttggt taagtcgttt     1440
   ttttttttcg gtttagtttt tttatttgta aaatgggggt ggggacgatg attttcgttt    1500
   ttgaggtttt gaggatttag gacgtggaag gtatagagaa gtttttgtat taattatttc    1560
   gggatttttt gatttttgtt tgtaattcgg aagagggtga ttttggaaga atcgtttttc    1620
   gtttttcgtt tttggttgtt ttttttatag gtagtattcg tattgttcgc gttcggtttt    1680
   atagtcgttt atcgttcggt tttttttggg ttttagggga cgtcgttttc gggttacggg   1740
   cgtggttgtc ggatcggagt tttatttcgg tttgcgattt tggattaggg tacgattttg    1800
   gattatagta atttggtcgt aggatatagc gtgtgatgtg tttggtatag agttcggtat    1860
   aagggaagag ttcggtggat aaggttaatt ggagaacgga ggtttgtttc gtttttattt    1920
   agcgtagcgg acggatttag cgttttttaa aaagaattag gggtcgggcg ttgtggttta     1980
   tgtttgtaat ttcggtattt tgggatatcg aggcgggagt attacgaggt taggagatcg    2040
   agattatttt ggttaaagtg gtgaaatttc gtttatatta aaatataaaa aattagtttg    2100
   gcgtggtggc gtacgtttgt agttttagtt atttaggagg ttatttagga ggttgaggtt    2160
   tgaatttagt aggcggaggt ggtagtgagt cgagatcgcg ttattgtatt ttaatttggt    2220
   aatagagtaa gatttcgttt taataaaaaa aaaataataa taaaaataaa taaataaaaa    2280
   aaagtaggga agtattaatt tggagtaatt gagtttaagg attgtttttt tttaataagt    2340
   aaaggttggt tataggtttt taggttttcg tttagattaa gttttgtttg ggtttggtgg    2400
   ggaggtgagg aagaacggtt tttaattttg ggattttaat tttttttgtt tgtttttggg    2460
   tgggatttta tgttttttgt taggatttag ttgtattttt agttatttat ttataaatt     2519
<210> 17
   <211> 2283
   <212> DNA
   <213> Artificial Sequence
<220>
   <223> chemically treated genomic DNA (Homo sapiens)
<400> 17
   aatgaagacg ttggagatcg ggtttttgtt cgtttttttt ttgcgtttcg ggatgaggta      60
   gagattgaat agtcggcgag taaattaacg gtatttagaa agttatgtcg gattcggcgt     120
   ttagcgttta agcgttaatt cgttgaaagt tttttagcga aattttaggg acgatttgga     180
   tttcgttgag aggaattgtt tttgagtgag atggttttag aggtttggag gagcggattg     240
   gtaagtatcg ggagggtagt gggagttttg tttttgtttg gtgttttgaa taaggttttt     300
   acggttattt attatgagat ttcggaggaa agagagaagg gtttcgttgt gggtaacgtg     360
   gtcgcgaatt ttggtttgga tttcggtagt tttttagttc gtaggtttcg ggtggtgttt     420
   ggagttagtc gaagattttt tgaggtgaat cgggagatcg gagagatgtt tgtgaacgat     480
   cgtttggatc gagaggagtt gtgtgggata ttgttttttt gtattgtaat tttggagttg     540
   gtagtggaga attcgttgga gttgtttagc gtggaagtgg tgatttagga tattaacgat     600
   aataattttg ttttttttat ttaggaaatg aaattggaga ttagcgaggt cgtggtttcg     660
   gggacgcgtt tttcgttcga gagcgcgtac gatttcgatg tgggaagtaa ttttttataa     720
   atttatgagt tgagtcgaaa tgaatatttt gcgtttcgcg tgtagacgcg ggaggatagt     780
   attaagtacg cggagttggt gttggagcgc gttttggatc gagaacggga gtttagtttt     840
   tagttagtgt tgacggcgtt ggacggaggg attttagttt ttttcgttag tttgtttatt     900
   tatattaagg tgttggacgc gaatgataat gcgtttgttt ttaattagtt tttgtatcgg     960
   gcgcgcgttt tggaggatgt atttttcggt acgcgcgtgg tataagtttt tgtaacggat    1020
   ttggatgaag gttttaacgg tgaaattatt tattttttcg gtagttataa tcgcgtcggc    1080
   gtgcggtaat tattcgtttt agattttgta atcgggatgt tgataattaa gggtcggttg     1140
   gatttcgagg atattaaatt ttatgagatt tatatttagg ttaaagataa gggcgttaat    1200
   ttcgaaggag tatattgtaa agtgttggtg gaggttgtgg atgtgaatga taacgtttcg    1260
   gagattatag ttattttcgt gtatagttta gtattcgagg atgttttttt ggggattgtt    1320
   atcgttttgt ttagtgtgat tgatttggat gttggcgaga acgggttggt gatttgcgaa    1380
   gttttatcgg gttttttttt tagttttatt ttttttttta agaattattt tattttgaaa    1440
   attagtgtag atttggatcg ggagattgtg ttagaatata attttagtat tatcgttcga    1500
   gacgtcggaa tttttttttt tttagttttt ataatagtgc gtgtttaagt gttcgatatt    1560
   aatgataatt ttttataatt tttttaattt ttttacgacg tttatattga agaaaataat    1620
   tttttcgggg ttttaatatt aaatttaagt gtttgggatt tcgacgtttc gtagaatgtt    1680
   cggttttttt tttttttttt ggagtaagga gttgaaatcg ggttagtggg tcgttatttt    1740
   ataataaatc gtgataatgg tatagtgtta tttttagtgt ttttagatta tgaggatcgg    1800
   cgggaatttg aattaatagt ttatattagc gatgggggta tttcggtttt agttattaat    1860
   attagcgtga atatatttgt tattgatcgt aatgataatg ttttttaggt tttatatttt   1920
   cggttaggtg ggagttcggt ggagatgttg tttcgaggta ttttagttgg ttatttagtg    1980
   ttacgggtgg taggttggga cgcggatgta gggtataatg tttggttttt ttatagtttt    2040
   ttgggatttt ttaattagag tttttttgtt atagggttgt atattggtta aattagtatt    2100
   gttcgtttag tttaagatat agatttattt aggtagattt ttacggtttt gattaaagat    2160
   aatggggagt tttcgttttt tattattgtt atttttattg tgttagtaat cgaggatttt    2220
   tttgaagttc gagtcgagtt tttttttggt tttgtttttc gggagtagaa aaaaaatttt    2280
   att                                                                  2283
<210> 18
   <211> 2283
   <212> DNA
   <213> Artificial Sequence
<220>
   <223> chemically treated genomic DNA (Homo sapiens)
<400> 18
   ggtgagattt tttttttgtt ttcggggggt agagttagag gggaattcgg ttcgggtttt      60
   aggagagttt tcggttattg atatagtgag ggtagtagtg gtggagagcg aaggtttttt   120
   attgtttttg attaagatcg tgagagtttg tttgggtgaa tttgtgtttt ggattggacg   180
   ggtagtattg atttgattag tgtgtagttt tatggtaaaa aggttttggt taggggattt   240
   taagagattg taggagagtt aggtattgtg ttttgtattc gcgttttagt ttattattcg   300
   tgatattagg tggttagttg aggtatttcg aggtagtatt tttatcgagt ttttatttgg   360
   tcgaggatat aggatttggg gggtattgtt attgcgatta gtgataaata tgtttacgtt   420
   gatgttggtg gttaggatcg gggtgttttt atcgttgata tgagttgtta atttaaattt   480
   tcgtcgattt ttatagttta ggggtattaa ggatgatatt atgttattgt tacgatttat   540
   tgtgaaatag cgatttatta gttcggtttt agttttttgt tttaagagaa agaaagaaag   600
   tcgagtattt tgcggggcgt cggggtttta gatatttagg tttagtattg gagtttcggg   660
   gaggttgttt tttttaatgt aaacgtcgta ggaagattga gaagattgtg gagggttgtt   720
   attgatgtcg gatatttgaa tacgtattat tgtaagggtt gagagggaag gggtttcggc   780
   gtttcgggcg gtgatgttga ggttgtattt tggtatagtt tttcgattta ggtttgtatt   840
   ggtttttaaa gtgaagtaat ttttgaggga agaagtaagg ttgaaaggga gattcggtgg   900
   aatttcgtag gttattagtt cgttttcgtt agtatttagg ttagttatat tgagtaaagc   960
   gatgatagtt tttagagggg tattttcggg tattgggttg tatacggagg tgattgtgat  1020
   tttcggggcg ttgttattta tatttataat ttttattaat attttgtaat gtgttttttc  1080
   gggattggcg tttttgtttt tggtttggat gtaaatttta tggagtttgg tgttttcgaa  1140
   gtttagtcga tttttgattg ttagtatttc ggttataagg tttaaggcga atagttgtcg  1200
   tacgtcggcg cggttgtggt tgtcgaagga gtaaataatt ttatcgttgg ggtttttatt  1260
   tagattcgtt gtaaggattt gtattacgcg cgtgtcggag ggtgtatttt ttaggacgcg  1320
   cgttcggtat aaggattggt tgaagatagg cgtattgtta ttcgcgttta gtattttgat  1380
   gtgaataggt aggttggcgg agagagttgg ggttttttcg tttaacgtcg ttagtattaa  1440
   ttggagatta ggttttcgtt ttcggtttag ggcgcgtttt aatattagtt tcgcgtattt  1500
   ggtgttgttt tttcgcgttt gtacgcgaag cgtaaagtat ttatttcggt ttagtttata  1560
   ggtttgtaaa gagttgtttt ttatatcggg atcgtgcgcg ttttcgagcg gaaagcgcgt  1620
   tttcggagtt acggtttcgt taatttttaa ttttattttt tgggtaggga aagtaggatt  1680
   gttgtcgttg atgttttgga ttattatttt tacgttgaat agttttagcg ggttttttat  1740
   tattaatttt agagttatag tgtaagaggg tagtgtttta tatagttttt ttcgatttag  1800
   acggtcgttt ataaatattt tttcggtttt tcggtttatt ttaaagaatt ttcggttagt  1860
   tttagatatt attcggaatt tgcgggttga gaggttatcg agatttaaat taaggttcgc  1920
   gattacgttg tttatagcga aatttttttt tttttttttc gggattttat agtgaatgat  1980
   cgtggaagtt ttgtttaagg tattaagtag aagtaaaatt tttattattt tttcggtgtt  2040
   tattagttcg tttttttagg tttttgggat tattttattt aaaagtagtt ttttttagcg  2100
   gggtttagat cgtttttgag atttcgttga gaaattttta gcgggttagc gtttgggcgt  2160
   tgggcgtcga gttcgatatg gttttttgga tgtcgttgat ttgttcgtcg gttgtttagt  2220
   ttttgtttta tttcggggcg tagaaagggg acgggtaggg gttcgatttt tagcgttttt  2280
   att                                                                 2283
<210> 19
   <211> 2236
   <212> DNA
   <213> Artificial Sequence
<220>
   <223> chemically treated genomic DNA (Homo sapiens)
<400> 19
   taggtgttaa cgatcgcgtt agattggcgt tggaattttt gggttataag gttttagtgg    60
   gtggcgttat tatttttgcg gtttgtaaaa gcggagtggt tttgttaggc gggaagtttt   120
   tttttttttt ttagattcgc gataggtcgt aggtaagaat tagcgtaatt agggcgcgtt      180
   cgtatagatt tggaggcggt tgtatgttgt tatttgtttt agaaggcgtt cggtggtcgt      240
   ttcgtagttt tagagtttag agataagtag aggaaggaag ataggatatt tgggttagtt      300
   gttcgagtta agtcgttgta aacgttaata tcgttcgtag ttttttcgtt agtatttacg      360
   tttaaaagta atttaagttt gcgcgttagt gggaaatttg gcgtttatga ttcgttagcg      420
   cgggtcgaaa tttttttttg atgttttttt agttgttttt tttttttcgc gtggttagga      480
   ggggtaaata atatgtagtg gggcggggcg ttttggaagg ggtgtgttag ggagggaatg      540
   atgggggttt taggtaaagt ttcgaatttt tttacgtaac gggaggaata tagatacgtt      600
   ttagttaagc gtgcgtggga atcgcggaat taaggtattt agaggttaag ggtttgtgtt      660
   aagttttgta ttttgtttcg taggggaatt tttatatttc gcgattgaat tcgaaagatt      720
   tttttcggcg gatgaaagtc gttatttttt ggttggttga gttatagttt tcgtagcgcg      780
   tttaggagtg cgtcggagat tcggaaattc gtagagattt tttaagttag taggaatttg      840
   gaaatcgttg ttttttttaa acggttttaa atattcgcgt tttttttttt atttaattta      900
   attttaattt gtttttttta taggtttaat ttttattttt ttaaaatttt aaattagtaa      960
   gttaaatcga agttttagaa taaggtttaa ttaatgattt taatttaata ttaagttcga     1020
   atgtttattt ttcggatttg ataaattaga tgtagagcga cgaatggaga ttattttttc     1080
   gggaggcgga atttagttgg gttttagttt gttttgggag aggagtacgt tttttttgag     1140
   ttgtaggcgg gttcggtttt gacgaaacgt tgcgagaatg ttagatagtg tagtttttat     1200
   cgtttgttcg gggtttttgt tgttattaga taagtatttt tatttattta ttttttttta     1260
   ttaattatcg ttagattttt ttcgcgtttt tcgatttcgt tgtaattttt ttttttgggc     1320
   gatgtttgga tagtattagt agtagttgtt cgagggaggg gggtagtttt cgttcgggga     1380
   taggttttaa aaggttgttt atttgttaga gttttacgtt ttttaaatga atttaaatta     1440
   agggtaagtt attacgtatt atgtgatatt cggtagtttt agattagaat aaggttttgt     1500
   attgaacgtg ataattgaat ttttaatgtt aagtgttatt ttttttttag gtaaatgttt     1560
   cgtcgttgtg atatttaagg ttggtttgtg ggcgttgttt tttgttttgt tttattttgt     1620
   tttgaagttt tattttaagt taatattaat tttaatacgg aatagttcga tttttttttt     1680
   ttaggttttt ttattgtttc gagagaggat tataagtatt ttttattttt tttagtatag     1740
   tttttttttt ttttttttgt tatagtatcg gtgttaggta attttaggta tttttatatt     1800
   gtatttatga tagatttgat gtttatcgga gatatgaaaa taattagaaa gttgttatgt     1860
   aaatttaata tgatatttat ttattaggta gagtaatata ataaggtttt tttatttatt     1920
   tttgagtttt atgtttatcg tagagaggaa aataatagcg attggtagtt ttgtttagag     1980
   attgtataaa agaggttagg aagttaggtt tggagatgta ttggagagta ttgtgtcgtt     2040
   ttttaattag taaaattttt attggatttg tggaatttta aatttagaag tatttatttt     2100
   atgtagtagt tttatttaga gataggaggt tcgttttttg ttttatgtta gttaatggaa     2160
   tagatgggat aagaattttg gatttttggt ttttaggtaa tgttttttgg gatagtggaa     2220
   gtagaaaatt aatttt                                                     2236
<210> 20
   <211> 2236
   <212> DNA
   <213> Artificial Sequence
<220>
   <223> chemically treated genomic DNA (Homo sapiens)
<400> 20
   gaaattgatt ttttattttt attgttttag aaagtattgt ttaagagtta aaaatttaag      60
   gtttttgttt tatttatttt attaattaat atgaagtaaa aaacgagttt tttgtttttg     120
   ggtgaaattg ttatatggaa tgggtgtttt tgggtttgaa gttttataga tttagtaggg     180
   attttgttga ttggaggacg gtatagtatt ttttagtgta tttttaagtt tgattttttg     240
   atttttttta tataattttt gggtaaaatt attagtcgtt gttatttttt tttttgcggt     300
   aaatatggag tttaagaatg gatagaaaga ttttattgta ttgttttatt tggtaaataa     360
   atattatgtt gaatttgtat ggtagttttt tgattatttt tatgttttcg ataagtatta     420
   agtttgttat aggtatagta taaaaatgtt tgagattgtt taatatcgat attgtggtag     480
   gaagagagga gaagagttgt attggggaaa atgaggagta tttgtaattt tttttcgggg     540
   tagtgagagg gtttgggaag aaaagatcga gttgtttcgt gttggggtta atgttggttt     600
   gaaatgaaat tttagggtaa aatgaagtag agtaaagagt agcgtttata aattaatttt     660
   aaatattata gcggcggaat atttatttga aagaaaagtg atatttggta ttaaaagttt     720
   aattattacg tttagtgtag agttttgttt tagtttggag ttgtcgggtg ttatatggtg     780
   cgtgataatt tgtttttgat ttgggtttat ttgaagagcg tagaatttta ataaataaat     840
   agttttttgg gatttgtttt cggacgagga ttgttttttt ttttcgggta attattattg     900
   atgttgttta ggtatcgttt aaggggaaag gttgtagcgg ggtcggaagg cgcgggagga     960
   gtttggcggt gattgatggg aagggatgaa tgaataaaag tatttgtttg atggtagtag    1020
   agatttcgag taaacggtgg aggttatatt gtttggtatt ttcgtagcgt ttcgttagag    1080
   tcggattcgt ttgtagttta agggaggcgt gttttttttt tagagtaggt tggaatttag    1140
   ttgggtttcg tttttcggga aggtggtttt tattcgtcgt tttgtatttg gtttgttaga    1200
   ttcgagaggt aaatattcgg gtttggtgtt gaattaaaat tattgattga attttatttt    1260
   ggggtttcgg tttggtttat tagtttggga ttttaaaaaa ataaaaatta agtttataga    1320
   gagggtaaat taaaattagg ttgggtaaag gaaggagcgc gagtgtttga agtcgtttgg    1380
   agggaatagc ggtttttaag tttttgttga tttgagaagt ttttgcgggt tttcgaattt    1440
   tcggcgtatt tttgggcgcg ttgcgggagt tgtagtttag ttagttaggg agtagcggtt    1500
   tttattcgtc gggaggagtt tttcgagttt aatcgcgggg tatagaggtt tttttgcggg    1560
   gtaaaatgta gagtttgata taagtttttg gtttttaggt gttttaattt cgcggttttt    1620
   acgtacgttt aattaagacg tgtttgtatt tttttcgtta cgtgaaagag ttcggagttt    1680
   tgtttgggat ttttattatt ttttttttgg tatatttttt ttagaacgtt tcgttttatt    1740
   gtatattatt tatttttttt ggttacgcgg gggaagaaaa atagttgaga gggtattagg    1800
   aaggagtttc gattcgcgtt ggcgagttat gagcgttaag ttttttattg gcgcgtaaat    1860
   ttgagttatt tttgagcgtg gatattggcg aagaggttgc gggcggtatt agcgtttgta    1920
   gcgatttggt tcgggtagtt gatttaagtg ttttgttttt ttttttttgt ttgtttttag    1980
   gttttgaaat tgcggagcgg ttatcggacg ttttttggag taggtagtag tatgtagtcg    2040
   tttttaagtt tgtgcggacg cgttttggtt gcgttggttt ttgtttgcgg tttgtcgcgg    2100
   atttggggag aggagagagg tttttcgttt gataggttta tttcgttttt gtaaatcgta    2160
   gagataatga cgttatttat taagatttta tggtttaagg gttttaacgt tagtttggcg    2220
   cggtcgttgg tatttg                                                    2236
<210> 21
   <211> 2204
   <212> DNA
   <213> Artificial Sequence
<220>
   <223> chemically treated genomic DNA (Homo sapiens)
<400> 21
   tttttttgta aatcggttta agtttcggga ggtgggattg ggggaggagg gggtttattt      60
   tggggttagg ttagggttgg gtggaggagg cggttttcga gagagtgagt cgggaaagaa    120
   ggggagtggg taaagggtga gatgtggggt ttcggttttc gtttcgttgg ttgttagttt    180
   tttttgcgtt ttttagttcg cgtgcgtgat acgagatagt gtagttttgg tatggggttt    240
   ggtatttggc ggtcgcgaga taattagggt tcgtgcgatg ggaaacgtgt cgttggtagt    300
   tcgttatttt tttatcgtga ttttagtttg ttaaataggg ttcgtgatgt ttagtttatt    360
   agggaggagt cgcggttttg agatttcgtt tagttcgaag tagcggagat tggttaggag    420
   ttcggttgtt agatttcgga gcgtacgttg cgggcggggg ttcggtttac gggggagggt    480
   taggggacgc gggatttatt tttgaaggag tcggggagtc gttgggtttc ggagtttcgt    540
   gtgtgtcgtt tttcggttat gttgttcgag acgtagtcgt attttttcgt tttcgttgtt    600
   aaattcggag tcgtcgggaa tgttttgagg agttagaggt ataagattta gattgtcgtt    660
   tcgtttttaa tcgaggtttc gttttcgtga gagttggtta attaacgttt aggatcgagg    720
   aggatttatt ttttaatttt cgtttttttg tttttttacg gcgcgattgt ttcgtagtta    780
   tttttcgtta gttttgtggg ttgaggggcg tggtttgagg ggcgtggttt gaggggcgag    840
   agtttttaag gttgtgggta tgaggtcggg gttttgtatc ggaagggagg gcggaagtta    900
   cgggacgtgg agtagatggt gaggatagtg gggaggtagt agtagaggat gattcgaggt    960
   aaaagtgtaa gtgaagaaaa gtcgttttgt cgggtagagg tacgggatta aggcgttttt   1020
   tttgggttga ggagttagag ggaagaaaat ggcgttttgt tatgagggcg cgaggttcga   1080
   gttgagggga gaagattgag ggatatagaa gtggtcgaag tgagggcgtt gaggttagtt   1140
   tgagttgaga gggtcggtag attacgtcgt agtcgaagtg aacgaattcg agtgaggtgt   1200
   gttaagaaaa ttaaaatgta taaatttgga aaaggagagg aggtcgggcg cggtggttta   1260
   cgtttgtaat tttagtacgt tggaaggtcg aggcgggtag attatttgag gttagaagtt   1320
   cgagattagt ttggttaata tggtgaaatt tcgtttttgt taaaaatata aaaattagtc   1380
   gggcgtgtgg cgcgcgtttg taattttagt tatttgggag gttgaggtag gagaatcgtt   1440
   tgaatttggg aggtagaggt tgtagtgagt cgagatggtt tgggtgatag agcgagattt   1500
   cgttttaaaa aaataaaata aaaaataaaa aaataggaga ggagatttta ttttttgtaa   1560
   ggggttatag tttgtaaggt ggttatttcg taggttggag aatggttagg attagagata   1620
   ggtatttcgt tggaggaggg gttggagtag cggttttatg ttgaatagtt tgggtagata   1680
   tgtatattta gtaggtgata ggaggagtta tgaatattta tgagggtggt tttgacgtat   1740
   gtttattgag taaatatgta tgtaatatat aatttatgtt tattttggag tgaagagtta   1800
   ttaattaaat gttaaatgtt tggtttgtat ggtgacgttt ttatttttat aaaaagtaaa   1860
   aaaaaaatag ttaggtgggg tggtgtgtgt ttgcggtttt aattagttag gaggttgggg   1920
   taggaggatt atttgagttt aagagttgga ggttgtagtg atgtatgatt agattattgt   1980
   attttagttt gatggatttt aaaaaataat aataatatat gtattatagt tagattttat   2040
   gcgttaaaag gtttttttag gataggaagg tgtgtaagta tgtagtttgt gtaaattagt   2100
   tagagttagt ttatggtcgg tagttttttt atgaggagaa agttattgaa attagttttt   2160
   tgtttaatta aagttgtaat gatggttggt ggaatagggg tttt                     2204
<210> 22
   <211> 2204
   <212> DNA
   <213> Artificial Sequence
<220>
   <223> chemically treated genomic DNA (Homo sapiens)
<400> 22
   ggaattttta ttttattagt tattattata gttttaattg gataagaggt tgattttagt      60
   aatttttttt ttataggaaa attatcgatt atggattggt tttggttagt ttatatagat     120
   tgtatatttg tatatttttt tgttttgaaa agattttttg acgtatgggg tttaattgta     180
   atatatgtat tgttattatt ttttagagtt tattaggttg gagtgtagtg atttgattat     240
   atattattgt agtttttaat ttttgggttt aagtgatttt tttgttttag ttttttgatt     300
   agttgggatc gtaagtatat attattttat ttggttattt tttttttatt ttttgtagag     360
   atggggacgt tattatgtag gttaggtatt taatatttaa ttggtaattt tttattttaa     420
   aatgaatatg ggttgtatgt tatatgtatg tttgtttaat gggtatgcgt taggattatt     480
   tttatgaata tttatagttt tttttgttat ttgttgaata tgtatgttta tttaagttgt     540
   ttagtataaa gtcgttattt taattttttt tttaacgaaa tgtttgtttt tggttttggt     600
   tattttttag tttgcggaat ggttattttg taggttgtaa ttttttataa gaaataaagt       660
   tttttttttt gtttttttgt tttttgtttt gtttttttga gacggagttt cgttttgtta     720
   tttaggttat ttcggtttat tgtaattttt gttttttaag tttaagcgat ttttttgttt       780
   tagtttttta agtagttggg attataggcg cgcgttatac gttcggttaa tttttgtatt     840
   tttagtagag acggggtttt attatgttgg ttaagttggt ttcgaatttt taattttaga     900
   tgatttattc gtttcggttt tttaacgtgt tgggattata ggcgtgagtt atcgcgttcg     960
   gttttttttt tttttttaaa tttatatatt ttgatttttt taatatattt tattcgagtt    1020
   cgtttatttc ggttgcggcg tgatttgtcg gtttttttag tttaggttga ttttagcgtt    1080
   tttatttcgg ttatttttgt gttttttagt tttttttttt taattcggat ttcgcgtttt      1140
   tatgataggg cgttattttt ttttttttaa ttttttagtt tagaggaggc gttttggttt      1200
   cgtatttttg ttcggtaagg cgattttttt ttatttgtat ttttgtttcg ggttattttt     1260
   tgttgttatt tttttattat ttttattatt tgttttacgt ttcgtggttt tcgttttttt     1320
   tttcggtgta gggtttcggt tttatattta tagttttggg ggttttcgtt ttttaggtta    1380
   cgttttttag gttacgtttt ttagtttata gaattagcgg gaagtgattg cgaagtagtc    1440
   gcgtcgtgga gggataggag ggcggggatt gggaggtggg tttttttcga ttttgggcgt      1500
   tgattggtta atttttacga gggcgggatt tcgattgggg gcggggcggt aatttgggtt    1560
   ttgtgttttt ggttttttag ggtattttcg gcggtttcgg gtttggtaac gaggacgggg    1620
   gagtgcgatt gcgtttcggg tagtatggtc gagaagcggt atatacggga tttcgaagtt    1680
   tagcggtttt tcgatttttt taagggtgag tttcgcgttt tttgattttt tttcgtggat    1740
   cgagttttcg ttcgtagcgt gcgtttcgag gtttgatagt cgggtttttg gttagttttc      1800
   gttgtttcgg gttgggcgag attttaaagt cgcggttttt ttttagtaaa ttgagtatta    1860
   cgaattttgt ttggtagatt gaggttacga tggaggggtg gcgggttgtt aacggtacgt      1920
   tttttatcgt acgggttttg gttatttcgc ggtcgttaga tgttaagttt tatgttaggg    1980
   ttgtattatt tcgtgttacg tacgcggatt aggaaacgta gagggaattg gtagttagcg    2040
   aggcggggat cgggatttta tattttattt tttgtttatt tttttttttt ttcgatttat    2100
   tttttcgaag gtcgtttttt ttatttagtt ttggtttggt tttagggtga attttttttt    2160
   tttttaattt tatttttcga aatttagatc gatttgtaga agga                     2204
<210> 23
   <211> 2137
   <212> DNA
   <213> Artificial Sequence
<220>
   <223> chemically treated genomic DNA (Homo sapiens)
<400> 23
   ttttattagt tagtatttaa ttatttaaaa gtgtggttta tgtttggaat tgggttataa      60
   agatggtaat gtgttaaatt agatatttgt tttttgtata tttttataaa ttatattagt     120
   ttattttata attttgtttt tgttagttat aaagaagaaa gtttaatatt ttagacgttt     180
   atattttatt aaattgtggg tgaagagtta gtgagcggta aaaggtggtc gttttgagtt 240
   gtggtgatgg aatttttagg gaatagttag gagtttggaa agttattttt gagttggttt 300
   atttaagtat tcgggataga agtggttagt agaaattttt tatggaattt taaaatatta 360
   ttaggatagt agttttacga tatttttaaa atttggagtt taaaaaataa tgaagtataa 420
   tttatttaga atttaagtat taagggaaaa cgttaagttt tgttttattt taaagatttt 480
   tttttgagga aaatatgtta ttgtttttta gatgtgaatg aaatgatatt ttttttttcg 540
   atatagtttt tttgtttacg attttatatt gttttttttt aatttttttt attattgaaa 600
   tattatatgt aagatttgtt ggtatgtttt ttgatttttt atgtttaagg ggaaaaatgt 660
   ttgtagttga tgtttgagtt ttaggagttc ggtgaagaaa aaaaaaaaaa gtaacgtaag 720
   gaaaattgtt ttaatggtat atttttttta aagtttgaag ttttataggt aaaaggaggg 780
   ataaatattt ttttttgaaa gtaaatagtt aaggtttttt tttatttaat tatttttgaa 840
   ttttttgtaa ttttttcgag gtttaaattt agggtataat taattttaga ttttatttta 900
   aatttaattc gttttttatt tgtaatttgt ttgtaggtgg gttttttttt tttaaattat 960
   aaatttgaat ttgcgttaat tttttataat ataaggtatt taaagttttt tggagttatt 1020
   gatttggtga cgttgtagtg tacgtttgta tttgaaagta agtaagagaa tagaaagcga 1080
   gtaagagaat cgtaattttt attattttgg cgaatttttg tcgggattta tatattaaat 1140
   agtatttgta tatagacgtt ttaagtcgtt ttttttagag tcgttattta gatttgtaag 1200
   gggcggggga ttagtggaaa tttaaataga aatttttatt attataaagg cgagttttta 1260
   ttgattatgg taattgtttt gtttaggttg tacgggtatg gttaattttg tttgaaagtt 1320
   cgtttttttt ttgcgttttt ttgtcgagtt ttttttaggt tttggttttt ttcgacggtg 1380
   gtaagggatg ttttttacgt tttttattat ttttgttcgc gtttgcgttt tttgttcgag 1440
   gttttaggtc gcggcggcgg cgcggtgggt ttattagcgg gcgcgtcggg ttcgcgtagc 1500
   gttgtttgta gtcgcgtcgt cgtatttatt attattatat tagcgcgggg aagtttacgt 1560
   cgagtttttt cggtttcggc gcgttttagt tagcgcgtta gtaaatagaa tatgttggat 1620
   tgtttagaga cggttttggg tgtttggggg tagaaggggt gtttttaggt tcggttttga 1680
   ttcgtatttt tgaagttggt tttgggagat aatgtatttt cgttagttag gtcgcgggaa 1740
   aagtgaaaaa agggttgggg gttatgtggg ggtggggagg tgagttttag cgttagtagc 1800
   gtttggtagt ttttttttag tgtagagaag cggtttgtat taaggcgtat tagagtggtt 1860
   attttgtttt tcggaatatt tacgggtatt tgtaaatttt atagttggtt tagttaaatt 1920
   tttagtaaat attggtaagg gtcggttttg gttgcgttta gagggttttt ttaattggtt 1980
   gaatttaggg agttgtaatt ttttaggtat tttgtaatgt aattattttg agtgtcgtat 2040
   tataataaaa tattggggat tttttgaaaa gtttttataa aattaatagg tgtgtgtttc 2100
   gtttataaaa cgtcgaattt attttttatg aatttgg 2137
<210> 24
   <211> 2137
   <212> DNA
   <213> Artificial Sequence
<220>
   <223> chemically treated genomic DNA (Homo sapiens)
<400> 24
   ttaggtttat agaaggtgaa ttcgacgttt tgtgagcgaa gtatatattt attggttttg      60
   tgaaggtttt ttaagggatt tttaatgttt tattataatg cgatatttag ggtggttgta     120
   ttgtaaagta tttggaaggt tgtagttttt tgaatttagt tagttaaaag agttttttga    180
   acgtaattag ggtcggtttt tgttagtgtt tgttggaaat ttggttgggt taattgtaga     240
   gtttgtaaat gttcgtgaat atttcgagga gtagggtgat tattttgata cgttttgata     300
   tagatcgttt ttttgtattg gagggagatt gttaggcgtt gttggcgttg gagtttattt     360
   ttttattttt atatggtttt tagttttttt tttatttttt tcgcggtttg gttggcggag   420
   atgtattgtt ttttaaagtt agttttagaa atgcggatta aagtcgggtt tgggagtatt     480
   ttttttgttt ttagatattt agagtcgttt ttgagtaatt tagtatgttt tgtttgttgg      540
   cgcgttgatt ggggcgcgtc ggggtcggga gaattcggcg tggatttttt cgcgttgatg     600
   taatgatgat ggatgcggcg acgcggttgt agatagcgtt gcgcgggttc ggcgcgttcg     660
   ttaatgaatt tatcgcgtcg tcgtcgcggt ttgggatttc gggtagggga cgtaggcgcg     720
   ggtaaaagtg gtgaaaggcg tgaaaaatat tttttgttat cgtcggagga aattagaatt     780
   tgaagggaat tcgataggaa agcgtagaga aagagcgagt ttttaaataa aattggttat     840
   attcgtataa tttgagtaaa ataattatta taattaatgg ggattcgttt ttgtaatggt     900
   gaaagttttt gtttgggttt ttattggttt ttcgtttttt gtaggtttaa gtgacgattt     960
   taaagaggac gatttgaggc gtttgtgtgt aagtgttgtt taatatgtag gtttcggtaa    1020
   gaattcgtta aggtggtaaa ggttgcggtt tttttgttcg ttttttgttt ttttgtttat   1080
   ttttaaatgt agacgtgtat tatagcgtta ttaggttagt ggttttagga agttttaaat    1140
   gttttgtgtt gtaagaggtt gacgtaggtt tagatttata atttagaaag aaaagattta    1200
   tttgtaggta ggttgtaaat aggagacgga ttagatttaa aatagggttt ggaattaatt    1260
   gtgttttggg tttaggtttc ggggaaatta taaaaggttt agggatagtt gagtgggaaa    1320
   aagttttgat tgtttgtttt taggaaggaa tgtttatttt tttttttatt tatgaaattt   1380
   taagttttag gaaggatata ttattagagt aatttttttt gcgttgtttt tttttttttt   1440
   ttttatcgag tttttgggat ttaaatatta gttataaata tttttttttt taagtatgaa    1500
   aagttaaggg gtatgttagt aaattttgta tgtaatgttt tagtggtgaa agggattgaa      1560
   ggagaatagt gtggagtcgt aagtaaggaa attatgtcgg gggaaaaagt attattttat    1620
   ttatatttga aaaatagtgg tatgtttttt ttagggaaaa gtttttgaag tgaagtagaa    1680
   tttaacgttt ttttttgatg tttaagtttt aaataaatta tattttatta ttttttaaat   1740
   tttaggtttt aaaagtgtcg tggagttatt gttttagtgg tgttttgaga ttttatgagg    1800
   aatttttatt gattattttt atttcgaatg tttgaatgga ttagtttagg agtgattttt    1860
   taagtttttg attatttttt gaaaatttta ttattataat ttaaagcggt tattttttgt  1920
   cgtttattga ttttttattt atagtttgat gaggtgtggg cgtttagaat gttaaatttt   1980
   tttttttata attgataggg ataaagttgt aaaataagtt gatataattt ataggagtgt   2040
   ataggaagta gatgtttgat ttggtatatt gttattttta tagtttagtt ttaagtataa    2100
   attatatttt taaataatta ggtattaatt aatgaga                             2137
<210> 25
   <211> 2461
   <212> DNA
   <213> Artificial Sequence
<220>
   <223> chemically treated genomic DNA (Homo sapiens)
<400> 25
   tttttttttt tttttttttt tttttttttt tttttttttt tttttttttt tttttttttt 60
   tttttttttt tttttttttt tttcgttttt tttttttttt ttttttttgt tttttatttt 120
   tttagtttga aggttgtatt ttttttattt ttttaagtaa agttgggagt tttgtagtat 180
   tttaggagtg aggataatta tgtttaagaa attttggttt tagtttagtt ttagattaag 240
   aggaaaatat tttaaaattt taaagtaaat tttaaaagtg taatttatat tagagcgatt 300
   gatttggtat ttatgtagat tgaagtttga gaagtaattt aagtggtttt tatttatttt 360
   tttggaagag tttttagaat gtatttttat tggtttgaaa aacgtttgaa aaagagtttt 420
   gatgttaata gtaatggaaa taggagggtt ggtggtttac gattagttta gtggtatttt 480
   ttattttatt tgtatttagg taaagagatt gtggtttgta tgtgattaaa taatttgttg 540
   aagcgttttg ggtttagggt atttaatttt tgtgaagtag gattattagt tgtttgtttt 600
   gtttttgtgt tattttttag aatatattaa agtaggggta atttttgagt ttagattttt 660
   gttatgtgaa gattttttga aaattttgat aaatataggt attgcgtaag tggttagtta 720
   attaaagagg ggaggtggag ttgttttttg tgtatttaat aagtatttat tattttattt 780
   gagtatgaaa agaggttatt gttattattt ttaagaagga aagtaagtag gatagtttat 840
   atttttagaa taatttttta ttaaatggaa taatttcggt gaaaagtggg agtgaggaag 900
   aaagaaaaaa aaaattttta attataatgt ttgggaataa gaaaggaaga agaaatttac 960
   gttaaagtcg attttttttt gtagttgtaa aataaatttt taagattttt tttgttgaaa 1020
   ttttggagag gaaaattgga gtggcgggtg ggttttgttt gtagtttaat tttttttcgc 1080
   ggcgcgggcg cggttgggtt tagtatttcg gaaagcgttt ttcgcggcgt ttcgggatta 1140
   cgtatgtttt ttggggttcg tcgttttggt cgtgtaagtg ttatcgtaat tggtgagagt 1200
   cgttggtaat ttattcggag ttgataatcg cggagagacg tagatattta ttgattttta 1260
   ggaagttgag cgtggtggat ggaattttac gatttttttt tttttaagga cggaaatttt 1320
   atttaagtag ttttagagga aacggataaa ggtatttgaa agggagcgag cggttttaaa 1380
   tcgtataatt gagcggttgg gggagttatg cgttagtgtt ttagtgatcg cgggatacgg 1440
   agaggggaag tttgcgttgt atataaggat ttagggattt cgagtttggt ttgagaattt 1500
   ttggacgtcg agtgtttgtt ttacgggttg tattttttaa ttttgtttta aagtagtcgt 1560
   tgagtttaat ttttgcgttt agggcgttcg ttgcgcgtta ggacgcgttt agtatttagt 1620
   ttttgggttt tttttttagt agttgttttg aaagttttta cgtacgtttc gtaggttagt 1680
   ttggtaataa aattggggta aatcgtgtta ttttaggttt tgttttttag aatatgattt 1740
   agaggtattt gcgtatgtag atggtcgatg tagttacgat agttattatg aataaggtag 1800
   taggcgggga taagttagta gaatttttta gtttggtttc ggattttttg gaggcggtta 1860
   atacgagtgg taacgcgtcg ttgtagtttt cggatttgtg gtgggagttg gggttggagt 1920
   tgtcggacgg cgcgtcgtta ggatattttt cgggtagcgg cggggtagag agcgcggata 1980
   tagaggttcg ggtgcggatt tttattagcg tggtgtattg ggtggtgtgc gttttggggt 2040
   tggcgggtaa tttgttggtt ttttatttga tgaagagtat gtagggttgg cgtaagtttt 2100
   ttattaattt tttcgttatt aatttggcgt tgacggattt ttagtttgtg tttattttgt 2160
   ttttttgggc ggtggagaac gtttttgatt ttaaatggtt tttcggtaag gttatgtgta 2220
   agatcgtgtt tatggtgacg tttatgaata tgtacgttag cgtgtttttt tttattgtta 2280
   tgagtgtgac gcgttattat tcggtggttt cggttttgaa gagttatcgg attcgaggat 2340
   acggtcgggg cgattgttgc ggtcggagtt tgggggatag ttgttgtttt tcggttaagg 2400
   cgttgtgtgt gtggatttgg gttttggtcg cgttggtttc gttgtttagt gttatttttt 2460
   t 2461
<210> 26
   <211> 2461
   <212> DNA
   <213> Artificial Sequence
<220>
   <223> chemically treated genomic DNA (Homo sapiens)
<400> 26
   gagaaaatgg tattgggtag cgaggttagc gcggttaaag tttagattta tatatatagc      60
   gttttggtcg agaagtagta gttgtttttt aggtttcggt cgtagtagtc gtttcggtcg     120
   tgttttcggg ttcggtggtt ttttagagtc gaggttatcg aatggtagcg cgttatattt     180
   atggtagtga ggaagaatac gttggcgtat atgtttatgg acgttattat ggatacgatt     240
   ttatatatgg ttttgtcgaa gggttatttg aagttaagag cgttttttat cgtttagaag     300
   ggtagggtga gtataaattg aaagttcgtt agcgttaggt tggtgacgaa gaggttgata     360
   gaggatttgc gttagttttg tatgtttttt attaggtaga gaattagtag gttgttcgtt     420
   aattttaggg cgtatattat ttagtatatt acgttgatga gaattcgtat tcgggttttt     480
   gtgttcgcgt tttttgtttc gtcgttgttc gggggatgtt ttggcggcgc gtcgttcggt     540
   aattttagtt ttagttttta ttataagttc ggaagttgta gcgacgcgtt attattcgtg     600
   ttggtcgttt ttagaaggtt cgggattaga ttgaagagtt ttgttagttt gttttcgttt     660
   gttgttttat ttatggtggt tatcgtggtt gtatcggtta tttgtatgcg taggtatttt     720
   taggttatgt tttgggggat aagatttaag ataatacggt ttattttagt tttgttgtta     780
   ggttagtttg cgggacgtgc gtgggagttt ttaaagtagt tattgaagag agagtttagg     840
   aattgggtat taagcgcgtt ttggcgcgta gcgaacgttt tggacgtagg agttgagttt     900
   agcggttgtt ttggagtaga gttgaggagt gtagttcgta aggtaagtat tcggcgttta     960
   agggttttta ggttaagttc ggagttttta ggtttttatg tataacgtag attttttttt    1020
   ttcgtgtttc gcggttattg gggtattggc gtataatttt tttagtcgtt taattgtgcg    1080
   atttggggtc gttcgttttt ttttaaatat ttttattcgt tttttttggg attgtttgga    1140
   tgaggttttc gtttttggag agaaagagat cgtagagttt tatttattac gtttagtttt    1200
   ttggaggtta gtgggtgttt gcgttttttc gcggttgtta atttcgggtg ggttgttagc    1260
   ggtttttatt agttacggtg gtatttgtac ggttaaggcg gcgggtttta aggagtatgc    1320
   gtaatttcgg ggcgtcgcga ggggcgtttt tcgaggtgtt gaatttagtc gcgttcgcgt    1380
   cgcgagggag agttgagttg taggtaaagt ttattcgtta ttttagtttt tttttttaga    1440
   gttttagtag gaagggtttt aagagtttat tttatagttg taggagaaag tcggttttga    1500
   cgtgagtttt tttttttttt tttattttta aatattatga ttagaagttt tttttttttt    1560
   ttttttttat ttttattttt tatcggaatt attttatttg gtgaggaatt gttttaaaaa    1620
   tatgagttat tttgtttatt tttttttttg aaagtaataa tagtggtttt tttttatgtt    1680
   taaatgagat aatgggtatt tattggatat ataaaggata gttttatttt ttttttttaa    1740
   ttagttgatt atttacgtag tatttgtgtt tgttaggatt tttaaagggt ttttatatgg    1800
   tagaagtttg ggtttaaggg ttgtttttat tttaatgtat tttgagaggt gatataaaaa    1860
   tagagtaaat aattgatggt tttattttat agaaattaaa tgttttaagt ttaaagcgtt    1920
   ttaataaatt gtttgattat atataggtta tagttttttt atttagatgt aaatagaata    1980
   gaggatatta ttgggttgat cgtgaattat tagttttttt atttttatta ttgttagtat    2040
   taggattttt ttttaggcgt tttttagatt aatggaagta tattttaagg atttttttaa    2100
   gggaatgagt ggaaattatt tgaattattt tttaagtttt agtttatata aatgttaggt    2160
   taatcgtttt ggtgtaaatt atatttttag gatttatttt aaaattttga aatgtttttt    2220
   ttttggtttg ggattaaatt gaaattaaag ttttttaagt ataattgttt ttatttttgg    2280
   agtattatag aatttttagt tttatttaag gagataaaga ggatgtaatt tttaagttga    2340
   ggagataaga aatagaaaaa gaaagaaaga gaaaaacgaa aaagaaagaa agaaagaaag    2400
   aaagaaagaa agaaagaaag aaagaaaaga aaagaaagag aaaaaggaaa aaagaaaaaa    2460
   a                                                                     2461
<210> 27
   <211> 1920
   <212> DNA
   <213> Artificial Sequence
<220>
   <223> chemically treated genomic DNA (Homo sapiens)
<400> 27
   ttaggttgtc gtagatatag tttttgtttt ttcgaaaaat acgttttagg cgtcgggatt      60
   ttagatattt gggaaataga gtgtacgtag ttgttgagag gtttcgcgtt tggttttttt     120
   tattattgag gcgtagaggt gttgtggata gtttagattt atacggcgtt cgaggtgaaa     180
   tagaattttt agttttttta tgaggttatt ggtattttcg gttgttttta gagtttttcg     240
   atttagagtt gaatgtaaag taagcgttcg aaatgtagaa gtagtcgggg tcgtttacgg     300
   tatttgtttc gttcggggcg agagaagacg ttaggttgag gttttagcga ttttaggtat     360
   tagtttcgaa ggagggcggg gagatcgtaa aggggaagtg ttcggagggt taacggtttt     420
   cgcgtatttt gcgttttttt gaagcgcgtc gttttttcgc gtcggggatt gggatttgtt     480
   tttggggaat tcgtttagaa gacggcggcg gattggggtc gggtattttt tagggttgtt     540
   aggttttttt tagttttgta tttgtcgcgt cgttttattt cgttaggaag ttttagagat     600
   ttcggggatg gggtgggagc gtttttttat cgcgggttta aaaagaagga aggacgtttt     660
   taggggtcgt agaaggagga ttagttttaa gttataattt ttttcggatt taaggtaggt     720
   cggttggggt ttcgcgttta tacggttttt ggcgggggtt cgcgcgtttc gggagtttcg     780
   cggttcgggg aggaaagagg agataagaga taggcgagga ttacggggtt gatttagtcg     840
   gggtagggat tatcgtggaa aaattttggc gaggtggggg gacgcggaaa gagagcggtt     900
   cgcgttttgt attttgcgtc gggtatttcg cgttagtgtt tcgtttttag tgtttcgcgt     960
   ttcgcgtttc gcgttttgtt tttatttcgg gttagttgta tcgcgttcgc gtcgtaggaa    1020
   tcgtggagtt ggaaagtggg ggcgtcgcgg ttggggggtt gttttagttg cgtttcggtt    1080
   agcgatcggc gggtcgggtt taaatttagt taggttgggt aggcggtggt cgcgcgattg    1140
   gggatcgggc gtttcgtttt tttcgttttt tttttttttt tttttttttt ttttagtttt    1200
   ttggtttttt ttagttttta tcggatttgt tcgttcgttg tttttttttt tttttcgttt    1260
   tttatattat tttttatttt ttcgttttgt tttcgttttt tttttttttt ttgttttttg    1320
   tttttttttt tttttttttt ttttttaggg gcggagtttt tttttttttt tttagataat    1380
   gttgtggttg cgtttttttt ttcgttagtt cgtttaggtt ttcgtcgtta gcgatttttt    1440
   cgggttgggg gtggggaggt ggggggggag tgtagggttg gggaggatga gttggttttt    1500
   tttatttttt tgttgttgtt ttttttaaga gggatggaga tttggtttaa gtttttcggt    1560
   ttattcggag ttgtgatagt tatttttagg gaatagttac gttgttttat taagtttatt    1620
   tttagcggtt tggatttttt aggtagaggt tgtgggattt tgtttttttt aatattttag    1680
   tttattttta aaagggtttg agtcggatag gggttaaata ggttttttcg atttggcggg    1740
   tcggttagac gtgatagtaa tgttaaggag gttaagtttt tttgtttatt ttttattttt    1800
   ttttttttta tttttggatt ttttggcgtt tttagtatat agaggttttt gagtagttcg    1860
   gttgtaggtt ttttatttat ttagagtttt tttttttacg tgtttatttt taattttgta    1920
<210> 28
   <211> 1920
   <212> DNA
   <213> Artificial Sequence
<220>
   <223> chemically treated genomic DNA (Homo sapiens)
<400> 28
   tgtagggttg gggataggta cgtgaggggg agaattttga gtagataggg aatttgtagt      60
   cgggttgttt aagggttttt gtgtattggg ggcgttagga ggtttaggga tggaaaaggg     120
   ggaggtgaga aatggataaa gaaatttggt ttttttggta ttgttgttac gtttggtcgg     180
   ttcgttaagt cgaaggggtt tgtttagttt ttgttcggtt taaatttttt tgggaataag     240
   ttgggatgtt agaaaaaata aaattttata atttttgttt ggggaattta ggtcgttgga     300
   ggtgggtttg gtagggtagc gtgattgttt tttgggagtg gttgttatag tttcgggtga     360
   atcgaggagt ttgggttaag tttttatttt ttttggagag ggtagtagta aggaggtgag     420
   gggagttagt ttattttttt taattttgta tttttttttt atttttttat ttttagttcg     480
   gaagaatcgt tggcggcggg agtttggacg agttggcggg gaaggggacg tagttatagt     540
   attgtttggg agggagggga gaagtttcgt ttttagaggg gaggggagaa gaggaggggg     600
   taggagataa ggggaggaag aaaggcgaag gtaaggcgaa ggggtggaga gtgatatgaa     660
   gagcgagaga aaagagagga tagcggacga gtagattcgg taggggttga aaaaggttaa     720
   ggggttggag ggagggagag gaaggaggag gggagcgagg agggcggggc gttcggtttt     780
   tagtcgcgcg gttatcgttt gtttagtttg gttggatttg agttcggttc gtcgatcgtt     840
   ggtcgaggcg tagttgaagt agttttttag tcgcggcgtt tttatttttt aattttacgg     900
   tttttgcggc gcgggcgcga tgtagttggt tcggggtgaa ggtaaggcgc ggggcgcggg     960
   gcgcggggta ttgggagcga ggtattggcg cgggatgttc ggcgtaaggt gtagggcgcg    1020
   ggtcgttttt ttttcgcgtt tttttatttc gttaaagttt ttttacgatg gtttttattt    1080
   cggttgggtt agtttcgtaa ttttcgtttg ttttttgttt tttttttttt tttcgagtcg    1140
   cggggttttc ggggcgcgcg gattttcgtt aggggtcgtg taggcgcgga gttttagtcg    1200
   gtttgttttg ggttcgaaga aagttgtggt ttggagttag tttttttttt acgatttttg    1260
   ggggcgtttt tttttttttt tgagttcgcg atgggaaggc gtttttattt tattttcggg    1320
   gtttttgaga ttttttggcg aggtggggcg gcgcggtagg tgtagggttg gggagggttt    1380
   gatagttttg gagagtgttc gattttagtt cgtcgtcgtt ttttaggcgg attttttaga    1440
   ggtaggtttt agttttcggc gcggggaggc ggcgcgtttt agaggggcgt agggtgcgcg    1500
   ggggtcgttg gtttttcggg tatttttttt ttgcggtttt ttcgtttttt ttcggagttg    1560
   gtgtttgagg tcgttgggat tttagtttgg cgtttttttt cgtttcgagc gaggtaggtg    1620
   tcgtgggcgg tttcggttat ttttgtattt cgagcgttta ttttgtattt agttttaagt    1680
   cggagagttt tggggatagt cgagagtgtt agtggtttta tagggagatt gagggttttg    1740
   ttttatttcg ggcgtcgtgt gggtttgggt tgtttatagt atttttgcgt tttagtgata    1800
   ggagaagtta agcgcgaggt tttttaatag ttgcgtgtat tttatttttt aggtatttgg    1860
   aatttcggcg tttagaacgt gtttttcggg agagtaaagg ttgtgtttac ggtagtttgg    1920
<210> 29
   <211> 2225
   <212> DNA
   <213> Artificial Sequence
<220>
   <223> chemically treated genomic DNA (Homo sapiens)
<400> 29
   atttttttgt aaattaaatt tttgttgttt agtttagtgt ttggtgtata gtgggggtta      60
   gatagatatt tgttgagtgt atgattaggg cgttgtaggt ggaggaggtg gtggtggggt     120
   gggggagggg aatttttttg ttgtttttcg ttttgtggga attagtttta gaaagaaatt     180
   ttttgtcgtt tttttatttt tattttaatt ttggatttgt tttttgattt tttttatttg     240
   gttttagttt tttatttaga gttaattttg tttgaaaggt attatgtttt gttattaagg     300
   ttttaaaata gggaaaatag tttttgaagt ttttagttcg taggagatag ggtattttag     360
   ggtataaggt tttatcgagg ggttggaaaa agagtttaga ttttaatatt tatagaaagg     420
   ggttttggtt gtttagttgt gagtggtttt tttttcgttt tttagtatat ttggaaacga     480
   ttttatatgt gggcgtcgcg ggatgttggg tagtaaggag atgatttagt tgaataatgt     540
   atttggttta tttttgcgag gggaggagaa cgtcgaggga taggaattgc gcgttagtaa     600
   aagatatttt aatttggggt gcgttcgtta gttgtttttt taaggtgaaa atttttaaga     660
   ggcgagatgt aaattttagt ggcgtttata ttcggaggtg tttttttttt aggtcgtttt     720
   ttcgttttat gcgatatttt aattttcgta gtttttgttt agcgtgggat taagaatggg     780
   gagttttatt ttttttcggt tttttagttt tttaatttaa gataagaggg cgcgcgttcg     840
   ggttgggata ggttttagtc gtattttcgt agattgtatt tttcgttcgg gagcgattcg     900
   ttttagtagt ttttttttcg ttttattaac gcggtcgtat tgtcggcgtt tagaggcgat     960
   attttggtaa agataggaat taagttagaa atttaggttt tagaagtaaa ggttgcggtg    1020
   ttttttggtt ggaagaaaac ggcgggtggg tggaggatgc ggtgcgcggg aattttttta    1080
   cggacgtttg ttgttttttg tttatttttt aggaaggtgt ttttttattt gtattttttt    1140
   ttgtaatttg gagttcgttc gcgagtttag ttattcgttg gttttttttg ttcgcgttcg    1200
   tatattatag atttcgttta tcgtatttta ttcggtttta gagggtttag aggggatttt    1260
   ttgaaattaa ttcgtttgtt tggagttagt ttcgacgtat atttcgagag tggcgggagg    1320
   taaagtagtc ggaatgttcg agaaaaaagt gggcgttaga ggtttttgtt ttagcgattt    1380
   ttttatcgta tcgattgagt ttcgggattg tagggattta agtttttatt tgtttcggcg    1440
   tttttttttt gtgcgttttg ttgtttcgga agtcggatcg agagttatag tgtggttggg    1500
   cgtaggcggg tcgttcggtt tttatttcga ggattcggat ttgtatttgt agagtcgttg    1560
   cgtgggggat gttttttttt ttttttcgtt tcggcgggtt ttttcggcgt gttagggttt    1620
   ttgggtttta gttggagttg gcgtttcggt tttatagttt tttttagcgt ttcggatttg    1680
   tgcgtttcgt ttcgcgttgg atttcgtttc ggggttttcg tttgtttttt agtttaggtt    1740
   aagggtggat gtttagtttt tttattttat cgcgtgtttt cgggttggtg cgtttagggc    1800
   gcgggtatcg tagtttcggt cgcgttgacg cgagttttcg gtagcggggg tagtttttcg    1860
   tacggatttt agcgttttcg tatttcgttt gaatcggatt atatagtagt cgggttttta    1920
   tcgtgcgttt gggaatagtg agtatcgttt tttgtcgtta gtcgtggtat cgtattcggg    1980
   tcgcgcgcgt aggaggggag agttttgttt ttcggaggtg tttagtgtta gcgttcgcgt    2040
   ttattagaag gtgatttttt taaggaaagt tcggtttcga cgagtgcggt gtgggttttt    2100
   ggagatggcg gtgaaggagg aggtttttag gaggaagttg gggatgggga tagtttgttt    2160
   tgtttaaagt ttttgaagtt tttatttaaa ttttgcgatg attattatta ttagatttta    2220
   tggtt                                                                  2225
<210> 30
   <211> 2225
   <212> DNA
   <213> Artificial Sequence
<220>
   <223> chemically treated genomic DNA (Homo sapiens)
<400> 30
   ggttatggaa tttgatagtg gtggttatcg taaagtttgg gtgaggattt taggggtttt      60
   gagtagagta aattgttttt atttttagtt tttttttggg ggtttttttt tttatcgtta      120
   tttttaaagg tttatatcgt attcgtcgga gtcgggtttt ttttgaaagg gttatttttt      180
   agtgggcgcg gacgttggta ttgggtattt tcggaagata ggattttttt tttttgcgcg     240
   cgcgattcgg gtgcggtgtt acgattggcg gtagagggcg gtgtttattg tttttaagcg     300
   tacggtagaa attcggttat tgtgtggttc gatttagacg ggatgcgaag gcgttgaaat     360
   tcgtgcgaga ggttgttttc gttgtcgggg attcgcgtta gcgcggtcgg agttacggtg     420
   ttcgcgtttt ggacgtatta gttcgagagt acgcggtggg gtgggggagt tgggtattta     480
   tttttggttt ggattaggag gtaggcgggg atttcggggc ggagtttagc gcggggcggg     540
   gcgtataggt tcgagacgtt ggaaagagtt gtggggtcgg agcgttaatt ttagttggaa     600
   tttagagatt ttgatacgtc ggaggagttc gtcggggcga ggaaaaaaga gggatatttt     660
   ttacgtagcg gttttgtaag tgtaagttcg ggttttcgag gtgaaaatcg agcggttcgt     720
   ttgcgtttaa ttatattgtg attttcgatt cggttttcgg agtagtagag cgtatagaaa     780
   ggaggcgtcg agataggtaa gggtttagat ttttgtagtt tcgggattta gtcggtgcgg     840
   tgggggagtc gttaggataa gggtttttga cgtttatttt tttttcgggt atttcggttg     900
   ttttgttttt cgttattttc gagatatgcg tcggggttgg ttttaagtag gcgagttggt     960
   tttaggagat tttttttggg ttttttagga tcgggtaggg tacggtaagc ggagtttgtg   1020
   gtgtgcgggc gcgggtagag ggagttagcg ggtagttgag ttcgcggacg agttttaaat    1080
   tgtaggggag ggtgtaggta gaggggtatt tttttgaaag gtgagtagga aatagtaagc    1140
   gttcgtgggg aaattttcgc gtatcgtatt ttttatttat tcgtcgtttt tttttagtta   1200
   ggaggtatcg tagtttttgt ttttagagtt tgaatttttg atttgatttt tgtttttgtt    1260
   aggatgtcgt ttttagacgt cgatagtgcg gtcgcgttga tggggcggga agggggttgt    1320
   tggaacgggt cgttttcggg cggggagtgt aatttacgaa aatgcggttg ggatttgttt    1380
   tagttcgggc gcgcgttttt ttgttttggg ttagggggtt ggaaagtcga ggaggggtag   1440
   gattttttat ttttggtttt acgttgagta gaggttgcgg ggattggggt gtcgtatgag    1500
   acggaggggc gatttggaga gaagatattt tcgggtgtgg acgttattgg gatttgtatt    1560
   tcgttttttg gaggttttta ttttgggaaa gtagttggcg ggcgtatttt aggttagggt    1620
   gttttttgtt ggcgcgtagt ttttgttttt cggcgttttt tttttttcgt aggagtggat   1680
   taaatgtatt gtttagttgg gttatttttt tgttatttag tatttcgcgg cgtttatatg     1740
   tggagtcgtt tttaggtgtg ttgagaggcg aggggaaggt tatttatagt tggatagtta    1800
   gggttttttt ttataggtgt tgggatttgg gttttttttt tagtttttcg gtgaagtttt     1860
   gtattttggg gtgttttatt ttttgcgggt tgagggtttt agagattatt ttttttattt   1920
   tgagattttg gtgataggat atggtgtttt ttaggtaggg ttgattttgg gtggagaatt    1980
   agggttaagt gaaggaggtt agagagtaga tttaaagttg gagtagggtg ggaagggcgg    2040
   tagaggattt ttttttggag ttggttttta tagaacgggg agtagtaggg gaattttttt     2100
   tttttatttt attattattt tttttattta tagcgttttg gttatgtatt taatagatgt    2160
   ttgtttagtt tttattatgt attaggtatt ggattgggta gtaggaattt ggtttatagg    2220
   aagat                                                                 2225
<210> 31
   <211> 2251
   <212> DNA
   <213> Artificial Sequence
<220>
   <223> chemically treated genomic DNA (Homo sapiens)
<400> 31
   ttttttagcg tgattgatcg cgatttagag gagaatgggt aggtgtagtg cgagttattg     60
   ggagacgtgt tttttcgttt taagtttttt tttaagaatt attatattat cgttatcgaa     120
   gtttttttgg atcgagaggc gggggatttt tatattttga ttgtagtggt tcgggatcgg    180
   ggcgagtttg cgttttttat tagtaagtcg atttaggtat aagtgtcgga tgtgaacgat     240
   aacgcgtcgc gttttagtta gtcggtttac gacgtgtatg tgattgaaaa taacgtgttt     300
   ggcgtttata tttacgcggt gagcgttatc gatcgggatg agggcgttaa cgtttagttt     360
   gtttatttta ttttcgagtg ttagatttag ggtatgagcg tttttattta cgtttttatt     420
   aattttgaga acggttattt gtacgttttg cgttttttcg attatgagta gttgaaggat     480
   tttagttttt aggtggaagt tcgggacgtt ggtagttttt aggcgttggt tggtaacgtt     540
   attgttaata tttttatagt ggattaaaat gataacgttt ttgttatcgt ggcgttttta      600
   ttagggcgta acgggatttt agcgcgtgag gtgttgtttc gttcggcgga gtcgggttat      660
   ttgtttattc gcgtggtcgt cgtggacgcg gacgacggcg agaacgttcg gtttatttat      720
   agtatcgtgc gtggtaacga aatgaatttt tttcgtatgg attggcgtat cggggagttg      780
   cgtatagtac gtcgagtttc ggttaagcgc gatttttagc ggttttatga gttggtgatc      840
   gaggtgcgcg attatgggta gtcgtttttt ttttttatcg ttattttggt ggtttagttg      900
   gtggatggcg tcgtggagtt ttagggcggg ggcgggagcg gaggcggagg gttaggagag      960
   tattagcgtt ttagtcgttt tggcggcggg gaaatttcgt tagattttat ttttattttt     1020
   attatcgcgt tgggttcggt gttttttatt tttttgttgg ttatgatcgt gttggtcgtg     1080
   cgttgttaaa aagagaagaa gtttaatatt tatatttgtt tggttagcga ttgttgtttt     1140
   tgttgttgtt gttgcggtgg cggaggttcg atttgttgtg gtcgttaagt tcgggcgcgt     1200
   aagaagaaat ttagtaagtt agatattatg ttggtgtaga gttttaatgt atttagtaat     1260
   tcggtttagg tgtcgataga ggagttcggg ggttttggtt tttattatta taattagaat     1320
   tattgttatt aggtatgttt gatttttgag ttcgttaaga tcgatttgat gttttttaag    1380
   ttttgtagtt tttcgcggag tacggatatt gagtataatt tttgcggggt tatcgttatc     1440
   ggttatatcg attagtagtt tgatattatt tttaacggaa gtattttgtt taacgaggta     1500
   aggttgaagc gaaaggatta ttatttttta ttttttttat tagaaagttt tttttagttc    1560
   ggtttttgta tttttggtgt attgtattta tttttaggat attagtttat gtgtatcgtt     1620
   gtgggagtag agatgggcgg ttattttttt ttattttttc gtgtgtaatt taattttcgc     1680
   gttgttttat ttttttatat ttatttttat ttcgtttttt tggtattttg ttattttgga      1740
   gttttttttt ttgttttttt attttgttag tttttttttt ttttagtaat ttgggtatga     1800
   agggaaattg cgtgaaggga gagggaaatg tggaggaggg atttattttt tagtattggt     1860
   aaaggttttt tttttttgcg tttgttttag gtattaataa agttggtttt attttgtttt     1920
   gtttaacgat gtttttagtc gcgtgtataa gtaagttata gattgtttaa ttttatatag     1980
   ttgtttaatt tcgaatttat gttttagtga ataagttatt agattttttt tttttgaatt     2040
   cgggttgtaa aaaaagtttt tagttcggtt ttggatagta tttatagacg tttttgaagt     2100
   cgagcgttta tatagtgtga atttgaatga agttgcgttg gtataaattt ttgttaagag     2160
   taaattaacg aaaggtttaa ataattgttg ttttaaatat atttttttag ttaataatta     2220
   tttttttgtt attgttttgg ataaattacg t                                    2251
<210> 32
   <211> 2251
   <212> DNA
   <213> Artificial Sequence
<220>
   <223> chemically treated genomic DNA (Homo sapiens)
<400> 32
   gcgtgattta tttaaaatag tggtaagaag gtgattatta gttaaaaaga tatatttaag      60
   ataataattg tttaagtttt tcgttagttt atttttaata ggggtttgtg ttaacgtagt     120
   tttatttaaa tttatattgt gtgggcgttc ggttttaaga gcgtttgtaa atgttgttta     180
   gagtcgaatt gaaggttttt tttgtaattc gagtttagag gagaaggatt tggtaatttg     240
   tttattaaaa tatgaattcg aggttagata attgtgtaaa gttaaataat ttatagttta     300
   tttgtatacg cgattaaaag tatcgttaaa taaagtaaaa tagagttaat tttattaatg     360
   tttgggatag acgtaaagaa aaaagatttt tgttagtgtt agaaagtaag tttttttttt     420
   atattttttt ttttttttac gtagtttttt tttatgttta ggttattgag aagggaaagg     480
   attgatagga tggaagagta aaggagggag ttttaaggtg gtaaagtatt aaggggacgg     540
   aatgaaaata aatgtgaaag ggtggaataa cgcgaaagtt aggttatata cgaaggagtg     600
   ggagaaggtg atcgtttatt tttgttttta taacgatata tataagttaa tattttaaaa     660
   atagatatag tgtattagag atataagggt cgggttagag gaggtttttt gatggaggag     720
   atgagagatg gtggtttttt cgttttagtt ttatttcgtt ggataaaatg ttttcgttgg     780
   agatgatatt aggttgttgg tcggtgtaat cggtgacgat ggtttcgtag gggttgtgtt      840
   tagtgttcgt atttcgcgaa gggttgtagg gtttaagaaa tattaggtcg gttttggcgg     900
   atttaggggt taggtatatt tgatagtagt aattttggtt gtggtggtgg gagttaaagt     960
   tttcggattt ttttatcggt atttgggtcg ggttattggg tatattggag ttttgtatta    1020
   gtatgatgtt tgatttgttg agtttttttt tgcgcgttcg ggtttggcgg ttatagtagg    1080
   tcgaattttc gttatcgtag tagtagtagt agaggtagta atcgttggtt agataagtat    1140
   agatgttgag tttttttttt ttttggtaac gtacggttag tacgattatg gttagtagga    1200
   agatgaagga tatcgagttt aacgcgatga tgaggatgag ggtgaggttt agcgaggttt    1260
   tttcgtcgtt agagcgattg gggcgttggt gtttttttga tttttcgttt tcgttttcgt    1320
   tttcgttttg gggttttacg gcgttattta ttagttgaat tattagggtg gcggtggagg    1380
   aaaggggcgg ttgtttatgg tcgcgtattt cgattattag tttataaggt cgttgggggt    1440
   cgcgtttggt cgggattcgg cgtgttgtgc gtagtttttc ggtgcgttag tttatgcgaa    1500
   agaggtttat ttcgttgtta cgtacgatgt tgtaagtgag tcgggcgttt tcgtcgtcgt    1560
   tcgcgtttac ggcggttacg cgggtgagta ggtaattcgg tttcgtcgag cggggtagta    1620
   ttttacgcgt tggagtttcg ttgcgttttg gtagaggcgt tacgatggta ggggcgttgt    1680
   tattttgatt tattatgagg atgttgatag tggcgttatt agttagcgtt tgggggttgt    1740
   tagcgtttcg ggtttttatt tgaaaattga agttttttag ttgtttatag tcgaaggagc    1800
   gtagggcgta taagtagtcg tttttagagt tgatagaaac gtaggtgaag acgtttatgt    1860
   tttggatttg gtattcgagg atagagtagg taagttgggc gttggcgttt ttatttcggt    1920
   cggtggcgtt tatcgcgtag atgtaggcgt taggtacgtt gtttttagtt atatatacgt    1980
   cgtagatcgg ttggttgaaa cgcggcgcgt tgtcgtttat attcgatatt tgtatttgga    2040
   tcgatttatt ggtggagagc gtaggttcgt ttcggtttcg agttattata gttagggtgt    2100
   aggagttttt cgtttttcgg tttagggggg tttcggtaac gatggtgtag taatttttaa    2160
   aggaagattt gaggcggaaa ggtacgtttt ttagtagttc gtattgtatt tgtttatttt    2220
   tttttgagtc gcggttagtt acgttgaaaa g                                   2251
<210> 33
   <211> 2553
   <212> DNA
   <213> Artificial Sequence
<220>
   <223> chemically treated genomic DNA (Homo sapiens)
<400> 33
   ttggttattt ttgttttttt ttagattttt agagttttta gtgtagttat agaggatgtt 60
   gttggtttta gttttaagaa gacgtcgttt tttttagagg gttaagtaag tgggaatttt 120
   tttttttatt tgttttgggt tttaggtagg gtttttggtg taaggtttgg ggttggaagt 180
   cgatttattt aggtttaggt tttggggtag aattgaaatt ttttggttat tgtcggttgt 240
   agtttgggag taggttattg ttaaagttgt gggttttttt aggatagttt ttttatgagg 300
   tcggtttttt atttgttgtt tttttatatt tggtggttag ggatgtggtt ttgggtagaa 360
   cgatgatttt ttatttttgt tattatggaa gttatcgttg ttttttagtt tagttagtta 420
   tttgggttgt agagtatttt ttttatgttt ttcgggtgtt tttttttttt tttgttttag 480
   tttggttttg ttttattttg tttttaggga ggggtatttt ggagtggggt tagggtatgg 540
   ttttttttcg agggagtttt tttttggttg tttttagggt agttttgtat agttttagta 600
   tttggcgtat tttttttgat atttttttta gggatagtta ggtattttgt gtggggtatt 660
   taagagagtt aggttcgtta gtttttagtt tttgttagaa tgtaggtttg aggggtgagg 720
   ggcggggtag gggtagggat aggaatttcg gcgtgttttt tattcgtaaa ggtttattga 780
   ggtttcgagt tttagttatt gagttattaa gttagtttgg gttaggtttg ggtgttttgt 840
   ttgtaatgga ggtagagacg gggtttcggg gtagttttga ggatgttggg tgtatagcgg 900
   gggtttcgtc ggtaggaatt atttatgttt ttttttgggt taagttttgt ggatgtttag 960
   tttggggtcg cggggagttg gtaggttagt ggtagatatt ggtgggtaga tttagtgttt 1020
   ggtagaatag gtattaagga agtggtgatc ggagggaagt taagtgtatt taaattttcg 1080
   ggtgagttat tatcgtcggg ttttttatag ttgttgaaag tgagtaatag tgatgaaggt 1140
   ttgtgagttt ttgcgtgagc gagtgaatgg attagtagta gtttttaggt tgtggaagag 1200
   cgtttttttt tcgggatggg gatatttggt tatagtaatt tttaattttt tatttattta 1260
   tcgtttattg tagaggtatg cgggggtttt gttttttgta ggtaggagtg aggggtattt 1320
   ttgtgatgtg gtatttttgt gatcgaggtt atgtgtgatc ggtgtaaggg taggaagcga 1380
   gttattggtt tgtattaggc gtgggggttt ttgcgagggt aggatttaaa gtcggtttgg 1440
   tttttcggtt gtagtatttt tttttttttc gaattaggtt agagttttgg gacgggaggt 1500
   gttttgtaga ttattttttt tattaatttt cgtttttcgt tttattttcg cggtgattcg 1560
   gtgaattgtc ggttttttgt tgtgtatcga gtggggtagt gattttgacg tggcgttttt 1620
   tgtcgttttt gttatcgtta ttattttcgg tggtttagtt ttcgtatttt ttatttttat 1680
   ggaggaatgt attaggtttt tttttttgga tgtatttttt atttatatgt ttttaaattt 1740
   tggtattttt tgtttttttt ttatttttat tttttttttt aggtttttag ataaagggga 1800
   agtggttgga tttttttaaa gggatagtgt tttattagtt tattgttgaa tttttttttt 1860
   taattttagt tttttagtta tagttaatta gtattagtag atagtttatg agtgatattt 1920
   atgtaggttt taggttgtgg agagtttttt gggtaggaaa tagtttttaa ggttttttat 1980
   tttatttagg ttttagtttt ttttatttgt ttttttttta gattgtggtt ttttggagtt 2040
   tggttttttt gtttttgtgt gatcgatata tagtatttaa atagtggtag agcgggacgg 2100
   attttttagt ttgttttttg tgtgggtttg tattttgatt tagatatgtt tttttatagt 2160
   aggatttagg ggggtatatg tgtgtttgcg ggtttattgg ggtattcgta tttggtttat 2220
   tttatttttt agagagaggg ttttgttgtg ttatttagtt ggagtgtagt ggtgtaatta 2280
   tagtatattg tagtttttaa tttttgggtt taagcgattt ttttttttta gtttttttag 2340
   tagttgggag tataggattt attgtatttt ggttaatttt ttaataattt tttaagagat 2400
   ggggttttat tgtgttgttt aggttggttt taaatttttg gttttaagtg atttttttat 2460
   tttcgttttt tgaagtgttg agattatagg tatgagttat tatgtttatt ttagattgat 2520
   atttttatat ttgtttattt tggttgggta ggg 2553
<210> 34
   <211> 2553
   <212> DNA
   <213> Artificial Sequence
<220>
   <223> chemically treated genomic DNA (Homo sapiens)
<400> 34
   ttttgtttag ttaggatgaa taaatataga aatgttagtt tgggatgggt atggtggttt      60
   atgtttgtaa ttttagtatt ttaggaggcg aaggtgggag gattatttga ggttagaggt     120
   ttgaggttag tttgggtaat atagtaagat tttatttttt aaaaaattat taaaaaatta     180
   gttaggatat agtgggtttt gtatttttag ttattaggga ggttggggag ggaggatcgt     240
   ttgagtttag gagttgaagg ttgtagtgtg ttatgattat attattgtat tttagttggg     300
   tgatatagta agattttttt tttaaaaaat aaaataaatt aaatgcgggt gttttagtga     360
   attcgtaggt atatatgtgt ttttttgggt tttgttgtgg gggggtatgt ttgggttagg     420
   gtatagattt atatagagaa taggttaggg ggttcgtttc gttttgttat tgtttgggtg     480
   ttatgtgtcg gttatatagg gatagaagaa ttaggtttta aagggttata atttaggaga     540
   gaggtaggta ggaaagattg ggatttggat gagatgaggg attttaaggg ttgtttttta     600
   tttaggaaat tttttatagt ttggggtttg tatgggtatt atttatgggt tgtttgttaa     660
   tgttaattaa ttgtaattag ggaattgggg ttgaggaggg gagtttagta gtaagttggt     720
   gggatattgt ttttttaaga ggatttagtt attttttttt tgtttgggag tttaggggaa     780
   ggggtgggag taaaggggga gtagaaaatg ttagggtttg gaagtatgtg ggtgaggggt     840
   gtatttagga agggaggttt ggtgtatttt tttatggggg tggggaatgc ggaggttggg     900
   ttatcggagg tggtggcggt ggtaggggcg gtaggagacg ttacgttagg gttattgttt     960
   tattcggtgt atagtagggg gtcggtagtt tatcggatta tcgcgggggt ggggcggagg    1020
   acggaagttg gtgagggggg tggtttatag ggtatttttc gttttagggt tttaatttaa    1080
   ttcgaaaggg aaaggagtgt tgtagtcggg aggttaggtc gattttgggt tttgttttcg    1140
   tagaagtttt tacgtttggt gtagattaat gattcgtttt ttgtttttat atcgattata    1200
   tatgatttcg gttatagggg tgttatatta taggagtgtt ttttattttt gtttgtagga    1260
   agtagggttt tcgtatattt ttgtagtggg cggtgggtgg gtgggggatt aggaattgtt    1320
   gtaattaagt gtttttattt cggggaggga acgttttttt ataatttgga aattgttatt    1380
   ggtttattta ttcgtttacg tagaaattta taaattttta ttattgttgt ttatttttag    1440
   tagttgtgaa agattcggcg gtgatgattt attcgagggt ttgagtgtat ttggtttttt    1500
   ttcggttatt atttttttga tgtttgtttt attagatatt aggtttgttt attagtgttt    1560
   gttattgatt tgttagtttt tcgcggtttt aggttgggta tttataaagt ttggtttagg    1620
   agagagtata agtgattttt gtcggcgagg ttttcgttgt gtatttagta tttttagaat    1680
   tgtttcgaga tttcgttttt gtttttattg tagatagggt atttaggttt ggtttaggtt    1740
   gatttggtgg tttagtggtt ggggttcggg gttttagtga atttttgcgg atggagagta    1800
   cgtcggagtt tttgtttttg tttttgtttc gttttttatt ttttaggttt gtattttggt    1860
   aggagttaga ggttgacggg tttggttttt ttgagtgttt tatatagagt gtttgattgt    1920
   ttttaagaag gatgttaagg gaggtgcgtt aggtattgag gttgtgtaga gttgttttgg    1980
   ggatagttag agaggaattt tttcggggga gagttatgtt ttggttttat tttagggtat    2040
   ttttttttga gagtagggta ggataaagtt aggttggggt aggagaaggg ggaggtattc    2100
   ggagggtatg aaaggggtgt tttgtagttt aggtggttgg ttgggttggg agatagcggt    2160
   ggtttttata atgataggag tggagaatta tcgttttatt tagggttata tttttggtta    2220
   ttaggtgtga agaaatagta ggtggaggat cggttttatg gggagattgt tttggaagga    2280
   tttatagttt tggtagtggt ttgtttttag gttgtagtcg atagtaatta aggagtttta    2340
   gttttgtttt agagtttgga tttaggtggg tcggttttta gttttaggtt ttatattagg    2400
   ggttttgttt ggagtttagg ataagtaggg agggggattt ttatttattt agttttttgg   2460
   aggaagcggc gtttttttgg ggttgaagtt aatagtattt tttgtggtta tattgggggt   2520
   tttggaggtt taagggaggg taggggtggt tag                                 2553
<210> 35
   <211> 2001
   <212> DNA
   <213> Artificial Sequence
<220>
   <223> chemically treated genomic DNA (Homo sapiens)
<400> 35
   ggtaaaagtt tgtttggatt ttttggttat tagaaatatg agtatggtgg tggtttttag       60
   ttttttattt atgtttgggt ttaagagatt gggagtttag gtttattgat tttttgagaa      120
   agattaagat tttgtatttt agaaagaggt ttggggattt ttgttttgcg taagggtaga      180
   aggattagtt gtttttttga gtattttaat tcggaatttc ggttcgaagt cgagatagga      240
   gattggatgc gaggtttttt tagagttggt tttttttaaa taatttttaa aatttttaga      300
   ttttaggggt acgtcgaaat tttttaaagt agtttaaaga atataacgag agttttaata      360
   ttttaggtgg cggcgcgttg gttttttgga gcggggcggg acgcggtcgc gcggatttac      420
   gtgtataatc gcgcgggacg gggttacgcg gatttacgtg tataatcgcg ggattttagc      480
   gttagcggga ttttagcgtt agcgggattt tagcgttagc gggattttag cgttagcggg      540
   attttagcgt tagcgggatt ttagcgttag cgggatttta gcgttagcgg gtttgtggtt      600
   tagtggagcg agtggagcgt tggcgatttg agcggagatt gcgttttgga cgttttagtt      660
   tagacgttaa gttatagttc gcgtagtagt agtaaagggg aaggggtagg agtcgggtat      720
   agttggattc ggaggtcgtg atttagggga aagcgtgggc ggtcgattta gggtagttgc      780
   ggcggcgagg taggtgggtt ttttgttttt tggagtcgtt tttttttata tttgttttcg      840
   gcgtttttag tagtttttat tttggttttt cgcggttatt gcgggattcg gcgttgtcgt      900
   tagtttagtg gggagtgaat tagcgttttt tttcgttttc ggttttttcg acggtacgag      960
   gaatttttgt tttgttttat agattttcgg ttttcgtcga gtgcggtatt ggagtttgtt     1020
   tcgttagggt tttggaatta gagaaagtcg ttttttggtt atttgaagcg tcggattttt     1080
   atagtgtttt ttagtttggg cgggagcggc ggttgcgtcg ttgaaggttg gggtttttgg     1140
   tgcgaaaggg aggtagttgt agttttagtt ttattttaga agcggttttc gtatcgttgc     1200
   ggtgggcgtt ttcgggtttc gatttcgtta gcgtcgcggg gtagaggtat ttggagttcg     1260
   tagggtttag atttgggttg gaaaagtttc gttgattgta ggtaagcgtt cgggaggggc     1320
   ggttaggcga agtttcggcg ttttattata tattttcggg ttttatgtta gttgtattcg     1380
   cggtattggg taggaaatgg tagggttgag gtcgatttta ggagtataag ggagtttttt     1440
   attttttgtt tatatttgtt atttttagtt ttgtaattta ttttagatat atagaaagta     1500
   agtaggattg gtggggagac ggagtttaat aggaatattt tttagtagtg agtaggggtt     1560
   gtatgggacg cgggaggagt ttagaggagg cgcggagagt gttcgaggtt gggtgagtgt     1620
   ttagagggga gatagttgaa tcgggtttaa gaggtgttta gtgggtgttt gttgaatgaa     1680
   tgagtgatgg gttttgaagt ttgagtgtat tgaaagaggg ggtgtgtaaa aagggttttt     1740
   tttattatat aggatatagt atatgtaaat tttttttttg tggaaaagtt agataggtta     1800
   aaaaggttat aaataaatta gtcgggtatg gtggtgcgcg tttgtagttt tagttattag     1860
   ggaggttgag ttaggggaat cgtttgaatt cgggaggcgg agattgtagt gagttaagat     1920
   cgcgttattg tattttagtt tggaaataga gcgagatttc gtttcggaaa aaaaaaaaaa     1980
   aagttataaa tcgtgtgtgg g                                               2001
<210> 36
   <211> 2001
   <212> DNA
   <213> Artificial Sequence
<220>
   <223> chemically treated genomic DNA (Homo sapiens)
<400> 36
   tttatatacg gtttgtaatt tttttttttt tttttcgaga cggagtttcg ttttgttttt      60
   aggttggagt gtagtggcgc gattttggtt tattgtaatt ttcgtttttc gggtttaagc      120
   gatttttttg gtttagtttt tttagtagtt gggattatag acgcgtatta ttatgttcgg     180
   ttaatttgtt tgtaattttt ttaatttgtt tggttttttt atagggagag gatttgtata      240
   tgttgtgttt tgtgtgatga aaggagtttt ttttatatat tttttttttt aatgtattta      300
   gattttaaag tttattattt atttatttaa taaatattta ttaagtattt tttgaattcg     360
   gtttaattat ttttttttta ggtatttatt taatttcggg tatttttcgc gttttttttg       420
   agtttttttc gcgttttata tagtttttgt ttattgttgg aaaatatttt tgttaagttt     480
   cgttttttta ttagttttgt ttgttttttg tgtgtttggg ataggttgta aaattggagg      540
   tgataaatgt gggtaggaaa tggagggttt ttttatattt ttagggtcgg ttttagtttt     600
   gttatttttt gtttaatatc gcggatgtaa ttggtatggg attcggaagt gtgtggtaaa     660
   gcgtcggggt ttcgtttggt cgtttttttc ggacgtttgt ttgtagttag cgaagttttt      720
   ttaatttagg tttgggtttt gcgagtttta ggtgtttttg tttcgcggcg ttggcgaagt     780
   cgaagttcga gaacgtttat cgtagcgatg cgaaggtcgt ttttggggtg gggttgaggt     840
   tgtagttgtt tttttttcgt attaaggatt ttaattttta gcgacgtagt cgtcgttttc      900
   gtttaggttg ggaggtattg tagggattcg acgttttagg tggttaaaga gcgatttttt     960
   ttgattttag ggttttggcg gggtaggttt tagtatcgta ttcggcggag gtcgaaggtt    1020
   tgtggggtag gataggagtt tttcgtgtcg tcggaagggt cgaggacgaa ggagggcgtt    1080
   aatttatttt ttattgggtt ggcggtaacg tcgaatttcg tagtgatcgc ggagggttaa    1140
   ggtgaaaatt gttgggggcg tcgagggtag gtgtggggag gggcggtttt agggagtaag    1200
   gagtttattt gtttcgtcgt cgtagttgtt ttgggtcgat cgtttacgtt tttttttggg    1260
   ttacgatttt cggatttaat tgtgttcggt ttttgttttt ttttttttgt tgttgttgcg     1320
   cgggttgtaa tttgacgttt aggttggggc gtttagggcg tagttttcgt ttaggtcgtt    1380
   agcgttttat tcgttttatt gggttataga ttcgttggcg ttggggtttc gttggcgttg    1440
   gggtttcgtt ggcgttgggg tttcgttggc gttggggttt cgttggcgtt ggggtttcgt    1500
   tggcgttggg gtttcgttgg cgttggggtt tcgcggttgt gtacgtgagt tcgcgtggtt    1560
   tcgtttcgcg cggttgtgta cgtgagttcg cgcggtcgcg tttcgtttcg ttttagggag    1620
   ttagcgcgtc gttatttggg atgttaggat tttcgttgtg ttttttggat tgttttgggg     1680
   gatttcggcg tatttttagg atttaggagt tttggaagtt gtttgagaga aattagtttt    1740
   gggagggttt cgtatttagt tttttgtttc ggtttcggat cggggtttcg ggttaaggtg    1800
   tttagaggaa tagttgattt ttttattttt gcgtagggta gagattttta aatttttttt     1860
   taaaatgtag ggttttagtt ttttttaggg agttagtgaa tttagatttt tagttttttg    1920
   agtttaagta tgaataggga attggggatt attattatgt ttatattttt ggtggttagg    1980
   aagtttaggt aggtttttgt t                                              2001
<210> 37
   <211> 219909
   <212> DNA
   <213> Artificial Sequence
<220>
   <223> chemically treated genomic DNA (Homo sapiens)
<400> 37
<210> 38
   <211> 219909
   <212> DNA
   <213> Artificial Sequence
<220>
   <223> chemically treated genomic DNA (Homo sapiens)
<400> 38
<210> 39
   <211> 1405
   <212> DNA
   <213> Artificial Sequence
<220>
   <223> chemically treated genomic DNA (Homo sapiens)
<400> 39
   cgttattcga gttgtaaagg gcggtttttt gtgtttgttt cgttgtatcg atatatcgag    60
   ttgcgtacgg tgtttagcgt agggagaata aatgattatt tgtttaacgc gtttatttat   120
   aggtgaggaa attaaggttt taatttaatc gacgtatttt gtttttttga ttattagaaa   180
   agtagtagga taggtgtttt gtttcgtttt atttcggttt attaagtcgg tatttcggtt   240
   tcgattttcg gttgtgttcg gcgtcgtcgc ggtgttcggc gtcgtcgttt cgttcggcgg   300
   ggtcgttcgg agcgttcgta ttttcgttcg tttttatttg gtcgggttcg tttcgttcgg   360
   attcgggatt gggaagtgcg gcgattttcg gaattagtta ttggcgttag cgcggggagt   420
   tgggggtgta gagttgcggg cgcggcgggt tacgtaggcg gtttttattt tcggtttggt   480
   ttggtttggt ttggtttgcg ttgtcgcgcg ggggcgtttt tttttaggtt cggcgttcgt   540
   tagtttcgtt tcgttaggtg tagcgtagcg taggggtggg cgggggtggg gttcggcgcg   600
   tacgtttacg gggcggggag ggggcgggtt aggggcggga ttatagtcgg ttgggtcggg   660
   gttttatgcg tattttcggg gaggggcggg gcgggggcgg ggtcggggcg gggttcggtc   720
   ggtgtatttt agacggcggg tcgttttttt ttttcgtttt tttattatcg gtttaggatt   780
   agcgttttgg gagcgcgcgt ttcgttgttt cgtcgttata tttttttggg agcggcggtt   840
   acggaggtat tatgaagaag ttttatttag gtgggtttcg cgttcggggt ggggaggggt   900
   cggtgtttcg ggattagcgt tgtttatttg agtgtttgcg gtcgggagtg gcgaggcgtt   960
   ttcggagttg agcgagtttt cgcggcgggt atattgtagg tcgagttttt tttaggatag  1020
   ggtcgttgtc gggtcgtttt cgatttgagt cgatcgtttt ttgcgttgtt tttagttttt  1080
   gttcgagtgt cggaggggtt gttttggggg acgttttttt tttttcgttt tttgtatttt  1140
   cgtaggaatc gttgattttt taggtcggtc gggtgttttg ggtttttgtg cgtttgtgtg  1200
   ggtgaatggg gtcggggtta ggtggagggg tgtttttggg tttagttttt agggttggtg  1260
   gtttaggtcg tagtattttt ttttcggatt tttttcggtt ttttttttat ttagtggggt  1320
   acgggacggt ttttagatgg gatcgtttag tagcgtttaa atttggcgat tcgggtttac  1380
   gttttgcgtt taggacgtcg ttcgt                                       1405
<210> 40
   <211> 1405
   <212> DNA
   <213> Artificial Sequence
<220>
   <223> chemically treated genomic DNA (Homo sapiens)
<400> 40
   gcgggcggcg ttttgagcgt aaaacgtgaa ttcgagtcgt taagtttggg cgttgttgga      60
   cggttttatt tggaggtcgt ttcgtgtttt attaagtaga ggagaaatcg aagagaattc     120
   gaagaaagga tgttgcggtt tggattatta gttttagagg ttgaatttaa ggatattttt     180
   ttatttagtt tcgattttat ttatttatat agacgtatag ggatttaaag tattcggtcg     240
   atttggaaag ttagcgattt ttgcgagggt gtaaggggcg aggaagaggg agcgtttttt     300
   agggtagttt tttcgatatt cgagtaaggg ttggagatag cgtaggggac ggtcggttta     360
   ggtcgaaagc ggttcgatag cggttttgtt ttgggaggaa ttcgatttgt agtgtgttcg     420
   tcgcggggat tcgtttagtt tcgggggcgt ttcgttattt tcggtcgtag gtatttaggt     480
   gagtagcgtt ggtttcggga tatcgatttt tttttatttc gggcgcgaag tttatttgag     540
   taagattttt ttatggtgtt ttcgtggtcg tcgtttttag gaaagtgtgg cggcgaggta     600
   gcggggcgcg cgtttttagg gcgttaattt tgggtcggta gtaggaaggc gggaagaggg     660
   ggcggttcgt cgtttggagt gtatcgatcg ggtttcgttt cggtttcgtt ttcgtttcgt     720
   tttttttcgg agatgcgtat agaatttcgg tttagtcggt tgtggtttcg tttttgattc     780
   gttttttttt cgtttcgtga acgtgcgcgt cgagttttat tttcgtttat ttttgcgttg     840
   cgttgtattt ggcggagcgg ggttggcggg cgtcgggttt gggagggggc gttttcgcgc     900
   ggtagcgtag attagattag attaggttag gtcgggggtg ggggtcgttt gcgtggttcg     960
   tcgcgttcgt agttttgtat ttttagtttt tcgcgttggc gttaatggtt ggtttcggga    1020
   gtcgtcgtat tttttagttt cgagttcggg cggggcgggt tcggttaggt ggaagcgggc    1080
   ggaggtgcgg gcgtttcggg cggtttcgtc gggcgaggcg gcggcgtcgg gtatcgcggc    1140
   ggcgtcgggt atagtcgggg gtcggagtcg gggtgtcggt ttagtgggtc ggggtagggc    1200
   gggataggat atttgttttg ttattttttt ggtaattaaa agggtagagt gcgtcgattg    1260
   agttggagtt ttagtttttt tatttgtaaa tgggcgcgtt ggatagatga ttatttgttt     1320
   tttttgcgtt gggtatcgtg cgtagttcgg tgtatcggtg tagcggggta gatataggga    1380
   gtcgtttttt ataattcggg taacg                                          1405
<210> 41
   <211> 1818
   <212> DNA
   <213> Artificial Sequence
<220>
   <223> chemically treated genomic DNA (Homo sapiens)
<400> 41
   cgtcgttcgt ttttggtttt tttgatagtc gtgttttatt ttcgttttgt gttttttttt      60
   tcggatagtg ttttttttag ggtttattta ggagggtgta gcggtggttt tcggggcggt      120
   ggtcgtggtg ggggtgttag ttgtaggggt gttttcggtg ggtgggagtt ggtggttttt      180
   cgttggtgtt atgggattcg tatgttcgtt ttgcgttttt cggtttttga gtttataggt      240
   cgggattttg tttgttagtc gcgtgcgttg tcgtttaatt tttgtaggcg tagagcgcgc      300
   ggcggcggtg atagagaatt ttgtttggtt gtttaaatat agttttttgt agaaggattt      360
   tgcgttcggg gaaggggagg aatttttttt tttttgggcg ttcgtttttt tcgttatggt      420
   tcggttttta tattcgttta tatttggtcg tagcggggcg ttcgggggga ggggttgagg      480
   tcgcgttttt cgtcgttttt tgggcgcggg ttaggcgggg aggagggggg cgtttcggtc      540
   gtgtgtttag gattgttttt tagcggttat tcgggtttta gttttttagg tttggttttg      600
   ataggcgggc ggagtagtta gtgcgagata gggaggtcgg tgcgggtgcg ggaatttgat      660
   tcgttcggga ggcgggggcg gggcgggggc gtagcgcgcg gggaggggtc ggcgttcgtt      720
   tttttttttt atttatttag ttgagttagg gggtttaggg gttttttcgg cggttagttt      780
   tgtattgtag gagcgcgggc gcggcgtttt agttagcgcg tagggttcgg gtttcgtcgg      840
   gggcgttttt tcgtcgttgt ttttcgcgcg attcgttgtt tattagttat tatgtcggat      900
   ttcgcggtta acgcgtagtt ggatgggatt atttcggatt tcgaaggtgg gtgttgggtt      960
   ggttgttgcg gtcgcggacg tgttggagag gattttgcgg gtgggtttgg cgcgggacgg     1020
   gggtgcgttg aggggagacg ggagtgcgtt gaggggagac gggattttta atttaggcgt     1080
   tttttcgttg agagcgtcgc gcgttttcgg tttcgtgttc gcgtcgttta cgtgggggat     1140
   tttgttaggg gtattcgcgt agattttgcg cgtttttata ggattttgtg ttcgttttgc     1200
   gtattgtcgt ttgggttttt ttttttttat tgttgtttgt gtttgttaag cgatagcgat     1260
   tttttcgagg gttcgcgagg ttgtttcgga attttttagg acgtatagtt ttattttggg     1320
   aaatttatcg gttttttttt tttggttttt ttcggcggtt ttcgggtttc gtttggattc     1380
   ggtaacggga tagggaggtc gttttttatt ttcgattgag tggatagtcg cgttttgttc     1440
   gggtggatag tttttttttt ttttacgtta gtttcggggt cgttaagttg tgtagttcgt     1500
   gggtcgggag tatcgaacgg atatagttta ggtcgtggta gggtttagag tgggatgttt     1560
   tatggttttt atttaggttt ggggatattt ttattcgttt tttagaatcg ggtcgtgggg     1620
   gatagaaggg gtttgcgtgc gggtagggag agtattttgg tttttttttg ttttcggggt     1680
   ttataaagtg tgttgggatt tgcggggttg ttttgtttaa gtttgggttt ggcgttcgcg     1740
   tttttgagtt tgtgagtgcg tgcgtttttt tgcgtttttt tgattgtcgg tgttggggtt     1800
   ttgcgttttg cgttcgcg                                                   1818
<210> 42
   <211> 1818
   <212> DNA
   <213> Artificial Sequence
<220>
   <223> chemically treated genomic DNA (Homo sapiens)
<400> 42
   cgcgaacgta aaacgtaaaa ttttaatatc gataattaaa aaaacgtaaa aaaacgtacg      60
   tatttataaa tttaaaaacg cgaacgttaa atttaaattt aaataaaata atttcgtaaa     120
   ttttaatata ttttataaat ttcgaaaata aaaaaaaatt aaaatatttt ttttattcgt     180
   acgtaaattt tttttatttt ttacgattcg attttaaaaa acgaataaaa atatttttaa      240
   atttaaataa aaattataaa atattttatt ttaaatttta ttacgattta aattatattc     300
   gttcgatatt ttcgatttac gaattatata atttaacgat ttcgaaatta acgtaaaaaa     360
   aaaaaaaatt atttattcga ataaaacgcg attatttatt taatcgaaaa taaaaaacga     420
   tttttttatt tcgttatcga atttaaacga aattcgaaaa tcgtcgaaaa aaattaaaaa     480
   aaaaaaatcg ataaattttt taaaataaaa ttatacgttt taaaaaattt cgaaataatt     540
   tcgcgaattt tcgaaaaaat cgttatcgtt taataaatat aaataataat aaaaaaaaaa     600
   aaatttaaac gataatacgt aaaacgaata taaaatttta taaaaacgcg taaaatttac     660
   gcgaatattt ttaataaaat tttttacgta aacgacgcga atacgaaatc gaaaacgcgc     720
   gacgttttta acgaaaaaac gtttaaatta aaaatttcgt ttttttttaa cgtattttcg     780
   ttttttttta acgtattttc gtttcgcgtt aaatttattc gtaaaatttt ttttaatacg     840
   ttcgcgatcg taataattaa tttaatattt attttcgaaa ttcgaaataa ttttatttaa     900
   ttacgcgtta atcgcgaaat tcgatataat aattaataaa taacgaatcg cgcgaaaaat     960
   aacgacgaaa aaacgttttc gacgaaattc gaattttacg cgttaattaa aacgtcgcgt    1020
   tcgcgttttt ataatataaa attaatcgtc gaaaaaattt ttaaattttt taatttaatt    1080
   aaataaataa aaaaaaaaaa cgaacgtcga ttttttttcg cgcgttacgt tttcgtttcg    1140
   ttttcgtttt tcgaacgaat taaattttcg tattcgtatc gattttttta tttcgtatta     1200
   attatttcgt tcgtttatta aaattaaatt taaaaaatta aaattcgaat aatcgttaaa    1260
   aaataatttt aaatatacga tcgaaacgtt tttttttttt tcgtttaatt cgcgtttaaa     1320
   aaacgacgaa aaacgcgatt ttaatttttt ttttcgaacg tttcgttacg attaaatata    1380
   aacgaatata aaaatcgaat tataacgaaa aaaacgaacg tttaaaaaaa aaaaaatttt    1440
   tttttttttt cgaacgtaaa atttttttat aaaaaattat atttaaataa ttaaataaaa     1500
   ttttttatta tcgtcgtcgc gcgttttacg tttataaaaa ttaaacgata acgtacgcga    1560
   ttaataaata aaatttcgat ttataaattt aaaaatcgaa aaacgtaaaa cgaatatacg    1620
   aattttataa tattaacgaa aaattattaa tttttattta tcgaaaatat ttttataatt    1680
   aatattttta ttacgattat cgtttcgaaa attatcgtta tattttttta aataaatttt     1740
   aaaaaaaata ttattcgaaa aaaaaaatat aaaacgaaaa taaaatacga ttattaaaaa    1800
   aattaaaaac gaacgacg                                                  1818
<210> 43
   <211> 2519
   <212> DNA
   <213> Artificial Sequence
<220>
   <223> chemically treated genomic DNA (Homo sapiens)
<400> 43
   gatttatgag tgaatgatta aaagtgtagt tgagttttgg tagagggtat ggggttttat      60
   ttagagatag gtagagaaag ttgaagtttt aggattggag gttgtttttt tttatttttt     120
   tattaggttt aggtagggtt tgatttgaat ggaggtttgg gaatttgtgg ttagttttta     180
   tttgttggaa aagagtagtt tttaagttta attgttttag gttgatgttt ttttattttt     240
   ttttatttat ttatttttat tattattttt tttttattga gatggagttt tattttgttg     300
   ttaggttgga gtgtagtggt gtgattttgg tttattgtta tttttgtttg ttgagtttaa     360
   gttttagttt tttgagtagt tttttgggta gttgggatta taggtgtgtg ttattatgtt     420
   aggttaattt tttgtatttt agtatagatg gagttttatt attttggtta ggatggtttt     480
   gattttttga ttttgtgata tttttgtttt ggtattttaa agtgttggga ttataggtat      540
   gagttatagt gtttggtttt tagttttttt taaaaaatgt tagatttgtt tgttgtgttg      600
   agtggaggtg gggtaggttt ttgtttttta attggtttta tttattgagt tttttttttg      660
   tgttgggttt tgtgttaagt atattatatg ttgtattttg tggttaggtt gttgtggttt     720
   agggttgtat tttggtttaa ggttgtaaat tgaggtggga ttttgatttg gtaattatgt     780
   ttgtggtttg gaaatggtgt tttttgaggt ttaggagagg ttgggtggtg agtggttgtg     840
   gagttgagtg tgggtagtgt ggatgttgtt tatgggggag gtagttaagg atggagggtg     900
   agaggtggtt tttttaaggt tatttttttt tgggttgtaa gtaaaggtta ggggattttg     960
   gaatggttag tgtaggagtt tttttgtgtt ttttatgttt tagattttta gagttttaga    1020
   aatggagatt attgttttta tttttatttt atagatgaag aaattgagtt gaggaaagga    1080
   agtgatttgg ttaaggttgg agagtttatt ttttgtaggg tggggtttgg aatttggggt    1140
   ttggtttttg gtaatgtgtt tttggtttgt agttttttgt tttttgtgtt ttgttttggt   1200
   ttttagtgta gttgtagagg ggtttttttt gatgtatata tttaagagtt tgattgtgtg    1260
   gttgaaattg tggagtttgg agttgtggtt ggttgagtga ttgtggtttt tgggttgtgt    1320
   gtgtgttttt tggagttgaa aggagtgtta ggattggggg tgttgtattg ggttgggttt    1380
   tttaatgttt gtagattggg ttgggttgta gttggagatg gtagtaattt tgggaggttt    1440
   ttgggttttt ttagggtgtg tttaggaggt gggttttgtg tgatgtggtg tagtttattt    1500
   ttttttttga gattgtttaa ttttgtgggg gtagttttgg gtgtttggag atgtggaggt    1560
   ttagattgta gtttttggat gttggaagtt tagatttttt ggggttattg gtttttttgt   1620
   tattttagtt gtagagagtt ttattgtttt attgtttgga gtttagtttt tttgtttttt    1680
   tattagtttt tttttttgta ggtttttggg gatttttgat tgtttgtttt tatttttttt   1740
   ttgtttttat attttttgtt tttgttttag gaatggtgat atagttattg gattgttttt   1800
   tattttttgt tagtttatat tgtatataat ttaataattt gtgttttttt atttgggtga   1860
   tagagatata gataatttga gttagtggtg tttaaagtat tggttttaga atagtagtat    1920
   tagtattttt tgggaatttg ttaaaaatga gaatttgggt tgggtgtggt ggtttatgtt    1980
   tgtaatttta gtattttggg aggttgaggt gggtggatta tgaggttagg agattgagat    2040
   tattttggtt aaaatggtga aattttgttt ttattaaaaa tataaaaaat tagttgggta    2100
   tagtggtggg tgtttgtagt tttagttatt tgggaggttg aggtaggaga atggtgtgaa    2160
   tttgggaggt ggagtttgta gtgagttgag attttgttat tgtattttag tttgggtgat    2220
   agagtgagat tttgttttaa aaaaaaaaaa aatgtgaatt tgggggtttt attttagatt   2280
   tattgaatag aaattttgtg gagtttagta gatgattttt atgtatatta aagtttgtga    2340
   gttattgatt taaatatttt tttatttatt tattttttat ttggtttatt tagtattgtt   2400
   agtgggagag atatttgtaa agtattaggt tgtgagtttt attgttgtgt attttgagat    2460
   attgtttatt agttttggga tggtaggtag aggtatttta gtttggttta gtgtagatt     2519
<210> 44
   <211> 2519
   <212> DNA
   <213> Artificial Sequence
<220>
   <223> chemically treated genomic DNA (Homo sapiens)
<400> 44
   ggtttgtatt agattaagtt ggagtatttt tgtttgttat tttaaagtta gtggatagtg      60
   ttttagagtg tatggtggtg gggtttatag tttggtgttt tgtgggtgtt ttttttattg     120
   gtaatgttgg gtgggttagg tgaagaatga atggatgaaa gaatgtttag attagtggtt     180
   tgtaaatttt agtgtgtatg ggaattattt gttgggtttt atagagtttt tgtttagtag     240
   atttggggtg gggtttttaa atttgtattt tttttttttt tgagatggag ttttgttttg     300
   ttgtttaggt tggagtgtag tggtgggatt ttggtttatt gtaagttttg ttttttgggt     360
   ttatgttatt tttttgtttt agttttttaa gtagttggga ttataggtgt ttgttattat     420
   gtttggttaa ttttttgtat ttttagtaga gatggggttt tattgtttta gttgggatgg     480
   ttttgatttt ttgattttgt gatttgtttg ttttggtttt ttaaagtgtt gggattatag     540
   gtgtgagtta ttgtgtttgg tttaaatttt tatttttaat aagtttttaa gagatgttga     600
   tgttgttgtt ttgggattgg tattttgagt attattagtt tagattattt gtgtttttgt     660
   tatttggatg gaagggtgtg gattattgag ttgtgtgtag tgtagattaa taggagatgg     720
   agagtaattt ggtgattgta ttattgtttt tgagataagg gtgggaagta tgggggtagg     780
   gggagagtaa gaataggtgg ttagaagttt ttagagattt gtggagggag ggattggtag     840
   aggaataagg agattaagtt ttggatggtg aagtaatggg attttttgta gttggggtgg      900
   tgggagagtt ggtgatttta gggagtttgg gtttttagta tttggaggtt gtagtttggg     960
   tttttgtgtt tttgagtgtt tgaggttgtt tttgtgaagt taagtggttt tggggggggg     1020
   ggtgggttgt attgtgttgt atggaatttg ttttttggat gtattttgaa gaggtttgga    1080
   gattttttgg gattgttgtt atttttagtt gtagtttggt ttggtttgtg agtgttgagg    1140
   ggtttagttt ggtgtagtgt ttttgatttt ggtgtttttt ttagttttaa ggggtgtgta    1200
   tgtagtttag gaattgtgat tgtttagtta gttgtggttt tgagttttgt gattttagtt    1260
   gtgtggttgg gtttttgagt gtatgtgttg aggggggttt ttttgtagtt gtgttggggg    1320
   ttgaagtggg gtataggggg taggaggttg tgggttgagg gtgtgttgtt gagagttaga     1380
   ttttgggttt taaattttgt tttgtaaaga atgagttttt tgattttggt taagttgttt    1440
   tttttttttg gtttagtttt tttatttgta aaatgggggt ggggatgatg atttttgttt    1500
   ttgaggtttt gaggatttag gatgtggaag gtatagagaa gtttttgtat taattatttt    1560
   gggatttttt gatttttgtt tgtaatttgg aagagggtga ttttggaaga attgtttttt    1620
   gttttttgtt tttggttgtt ttttttatag gtagtatttg tattgtttgt gtttggtttt   1680
   atagttgttt attgtttggt tttttttggg ttttagggga tgttgttttt gggttatggg      1740
   tgtggttgtt ggattggagt tttattttgg tttgtgattt tggattaggg tatgattttg    1800
   gattatagta atttggttgt aggatatagt gtgtgatgtg tttggtatag agtttggtat    1860
   aagggaagag tttggtggat aaggttaatt ggagaatgga ggtttgtttt gtttttattt    1920
   agtgtagtgg atggatttag tgttttttaa aaagaattag gggttgggtg ttgtggttta    1980
   tgtttgtaat tttggtattt tgggatattg aggtgggagt attatgaggt taggagattg    2040
   agattatttt ggttaaagtg gtgaaatttt gtttatatta aaatataaaa aattagtttg    2100
   gtgtggtggt gtatgtttgt agttttagtt atttaggagg ttatttagga ggttgaggtt    2160
   tgaatttagt aggtggaggt ggtagtgagt tgagattgtg ttattgtatt ttaatttggt    2220
   aatagagtaa gattttgttt taataaaaaa aaaataataa taaaaataaa taaataaaaa    2280
   aaagtaggga agtattaatt tggagtaatt gagtttaagg attgtttttt tttaataagt    2340
   aaaggttggt tataggtttt taggtttttg tttagattaa gttttgtttg ggtttggtgg     2400
   ggaggtgagg aagaatggtt tttaattttg ggattttaat tttttttgtt tgtttttggg     2460
   tgggatttta tgttttttgt taggatttag ttgtattttt agttatttat ttataaatt     2519
<210> 45
   <211> 2283
   <212> DNA
   <213> Artificial Sequence
<220>
   <223> chemically treated genomic DNA (Homo sapiens)
<400> 45
   aatgaagatg ttggagattg ggtttttgtt tgtttttttt ttgtgttttg ggatgaggta      60
   gagattgaat agttggtgag taaattaatg gtatttagaa agttatgttg gatttggtgt     120
   ttagtgttta agtgttaatt tgttgaaagt tttttagtga aattttaggg atgatttgga     180
   ttttgttgag aggaattgtt tttgagtgag atggttttag aggtttggag gagtggattg     240
   gtaagtattg ggagggtagt gggagttttg tttttgtttg gtgttttgaa taaggttttt     300
   atggttattt attatgagat tttggaggaa agagagaagg gttttgttgt gggtaatgtg     360
   gttgtgaatt ttggtttgga ttttggtagt tttttagttt gtaggttttg ggtggtgttt     420
   ggagttagtt gaagattttt tgaggtgaat tgggagattg gagagatgtt tgtgaatgat     480
   tgtttggatt gagaggagtt gtgtgggata ttgttttttt gtattgtaat tttggagttg     540
   gtagtggaga atttgttgga gttgtttagt gtggaagtgg tgatttagga tattaatgat     600
   aataattttg ttttttttat ttaggaaatg aaattggaga ttagtgaggt tgtggttttg     660
   gggatgtgtt ttttgtttga gagtgtgtat gattttgatg tgggaagtaa ttttttataa     720
   atttatgagt tgagttgaaa tgaatatttt gtgttttgtg tgtagatgtg ggaggatagt     780
   attaagtatg tggagttggt gttggagtgt gttttggatt gagaatggga gtttagtttt     840
   tagttagtgt tgatggtgtt ggatggaggg attttagttt tttttgttag tttgtttatt     900
   tatattaagg tgttggatgt gaatgataat gtgtttgttt ttaattagtt tttgtattgg     960
   gtgtgtgttt tggaggatgt attttttggt atgtgtgtgg tataagtttt tgtaatggat    1020
   ttggatgaag gttttaatgg tgaaattatt tatttttttg gtagttataa ttgtgttggt    1080
   gtgtggtaat tatttgtttt agattttgta attgggatgt tgataattaa gggttggttg    1140
   gattttgagg atattaaatt ttatgagatt tatatttagg ttaaagataa gggtgttaat    1200
   tttgaaggag tatattgtaa agtgttggtg gaggttgtgg atgtgaatga taatgttttg    1260
   gagattatag ttatttttgt gtatagttta gtatttgagg atgttttttt ggggattgtt    1320
   attgttttgt ttagtgtgat tgatttggat gttggtgaga atgggttggt gatttgtgaa    1380
   gttttattgg gttttttttt tagttttatt ttttttttta agaattattt tattttgaaa    1440
   attagtgtag atttggattg ggagattgtg ttagaatata attttagtat tattgtttga    1500
   gatgttggaa tttttttttt tttagttttt ataatagtgt gtgtttaagt gtttgatatt    1560
   aatgataatt ttttataatt tttttaattt ttttatgatg tttatattga agaaaataat    1620
   ttttttgggg ttttaatatt aaatttaagt gtttgggatt ttgatgtttt gtagaatgtt    1680
   tggttttttt tttttttttt ggagtaagga gttgaaattg ggttagtggg ttgttatttt    1740
   ataataaatt gtgataatgg tatagtgtta tttttagtgt ttttagatta tgaggattgg    1800
   tgggaatttg aattaatagt ttatattagt gatgggggta ttttggtttt agttattaat    1860
   attagtgtga atatatttgt tattgattgt aatgataatg ttttttaggt tttatatttt    1920
   tggttaggtg ggagtttggt ggagatgttg ttttgaggta ttttagttgg ttatttagtg    1980
   ttatgggtgg taggttggga tgtggatgta gggtataatg tttggttttt ttatagtttt    2040
   ttgggatttt ttaattagag tttttttgtt atagggttgt atattggtta aattagtatt    2100
   gtttgtttag tttaagatat agatttattt aggtagattt ttatggtttt gattaaagat    2160
   aatggggagt ttttgttttt tattattgtt atttttattg tgttagtaat tgaggatttt    2220
   tttgaagttt gagttgagtt tttttttggt tttgtttttt gggagtagaa aaaaaatttt    2280
   att                                                                  2283
<210> 46
   <211> 2283
   <212> DNA
   <213> Artificial Sequence
<220>
   <223> chemically treated genomic DNA (Homo sapiens)
<400> 46
   ggtgagattt tttttttgtt tttggggggt agagttagag gggaatttgg tttgggtttt      60
   aggagagttt ttggttattg atatagtgag ggtagtagtg gtggagagtg aaggtttttt   120
   attgtttttg attaagattg tgagagtttg tttgggtgaa tttgtgtttt ggattggatg   180
   ggtagtattg atttgattag tgtgtagttt tatggtaaaa aggttttggt taggggattt   240
   taagagattg taggagagtt aggtattgtg ttttgtattt gtgttttagt ttattatttg   300
   tgatattagg tggttagttg aggtattttg aggtagtatt tttattgagt ttttatttgg   360
   ttgaggatat aggatttggg gggtattgtt attgtgatta gtgataaata tgtttatgtt   420
   gatgttggtg gttaggattg gggtgttttt attgttgata tgagttgtta atttaaattt   480
   ttgttgattt ttatagttta ggggtattaa ggatgatatt atgttattgt tatgatttat   540
   tgtgaaatag tgatttatta gtttggtttt agttttttgt tttaagagaa agaaagaaag   600
   ttgagtattt tgtggggtgt tggggtttta gatatttagg tttagtattg gagttttggg   660
   gaggttgttt tttttaatgt aaatgttgta ggaagattga gaagattgtg gagggttgtt   720
   attgatgttg gatatttgaa tatgtattat tgtaagggtt gagagggaag gggttttggt   780
   gttttgggtg gtgatgttga ggttgtattt tggtatagtt ttttgattta ggtttgtatt   840
   ggtttttaaa gtgaagtaat ttttgaggga agaagtaagg ttgaaaggga gatttggtgg   900
   aattttgtag gttattagtt tgtttttgtt agtatttagg ttagttatat tgagtaaagt   960
   gatgatagtt tttagagggg tatttttggg tattgggttg tatatggagg tgattgtgat  1020
   ttttggggtg ttgttattta tatttataat ttttattaat attttgtaat gtgttttttt  1080
   gggattggtg tttttgtttt tggtttggat gtaaatttta tggagtttgg tgtttttgaa  1140
   gtttagttga tttttgattg ttagtatttt ggttataagg tttaaggtga atagttgttg  1200
   tatgttggtg tggttgtggt tgttgaagga gtaaataatt ttattgttgg ggtttttatt  1260
   tagatttgtt gtaaggattt gtattatgtg tgtgttggag ggtgtatttt ttaggatgtg  1320
   tgtttggtat aaggattggt tgaagatagg tgtattgtta tttgtgttta gtattttgat  1380
   gtgaataggt aggttggtgg agagagttgg ggtttttttg tttaatgttg ttagtattaa  1440
   ttggagatta ggtttttgtt tttggtttag ggtgtgtttt aatattagtt ttgtgtattt  1500
   ggtgttgttt ttttgtgttt gtatgtgaag tgtaaagtat ttattttggt ttagtttata  1560
   ggtttgtaaa gagttgtttt ttatattggg attgtgtgtg tttttgagtg gaaagtgtgt  1620
   ttttggagtt atggttttgt taatttttaa ttttattttt tgggtaggga aagtaggatt  1680
   gttgttgttg atgttttgga ttattatttt tatgttgaat agttttagtg ggttttttat  1740
   tattaatttt agagttatag tgtaagaggg tagtgtttta tatagttttt tttgatttag  1800
   atggttgttt ataaatattt ttttggtttt ttggtttatt ttaaagaatt tttggttagt  1860
   tttagatatt atttggaatt tgtgggttga gaggttattg agatttaaat taaggtttgt  1920
   gattatgttg tttatagtga aatttttttt tttttttttt gggattttat agtgaatgat  1980
   tgtggaagtt ttgtttaagg tattaagtag aagtaaaatt tttattattt ttttggtgtt  2040
   tattagtttg tttttttagg tttttgggat tattttattt aaaagtagtt ttttttagtg  2100
   gggtttagat tgtttttgag attttgttga gaaattttta gtgggttagt gtttgggtgt  2160
   tgggtgttga gtttgatatg gttttttgga tgttgttgat ttgtttgttg gttgtttagt  2220
   ttttgtttta ttttggggtg tagaaagggg atgggtaggg gtttgatttt tagtgttttt  2280
   att                                                                2283
<210> 47
   <211> 2236
   <212> DNA
   <213> Artificial Sequence
<220>
   <223> chemically treated genomic DNA (Homo sapiens)
<400> 47
   taggtgttaa tgattgtgtt agattggtgt tggaattttt gggttataag gttttagtgg      60
   gtggtgttat tatttttgtg gtttgtaaaa gtggagtggt tttgttaggt gggaagtttt     120
   tttttttttt ttagatttgt gataggttgt aggtaagaat tagtgtaatt agggtgtgtt     180
   tgtatagatt tggaggtggt tgtatgttgt tatttgtttt agaaggtgtt tggtggttgt     240
   tttgtagttt tagagtttag agataagtag aggaaggaag ataggatatt tgggttagtt     300
   gtttgagtta agttgttgta aatgttaata ttgtttgtag tttttttgtt agtatttatg     360
   tttaaaagta atttaagttt gtgtgttagt gggaaatttg gtgtttatga tttgttagtg     420
   tgggttgaaa tttttttttg atgttttttt agttgttttt ttttttttgt gtggttagga     480
   ggggtaaata atatgtagtg gggtggggtg ttttggaagg ggtgtgttag ggagggaatg     540
   atgggggttt taggtaaagt tttgaatttt tttatgtaat gggaggaata tagatatgtt     600
   ttagttaagt gtgtgtggga attgtggaat taaggtattt agaggttaag ggtttgtgtt     660
   aagttttgta ttttgttttg taggggaatt tttatatttt gtgattgaat ttgaaagatt     720
   ttttttggtg gatgaaagtt gttatttttt ggttggttga gttatagttt ttgtagtgtg     780
   tttaggagtg tgttggagat ttggaaattt gtagagattt tttaagttag taggaatttg     840
   gaaattgttg ttttttttaa atggttttaa atatttgtgt tttttttttt atttaattta     900
   attttaattt gtttttttta taggtttaat ttttattttt ttaaaatttt aaattagtaa     960
   gttaaattga agttttagaa taaggtttaa ttaatgattt taatttaata ttaagtttga    1020
   atgtttattt tttggatttg ataaattaga tgtagagtga tgaatggaga ttattttttt    1080
   gggaggtgga atttagttgg gttttagttt gttttgggag aggagtatgt tttttttgag    1140
   ttgtaggtgg gtttggtttt gatgaaatgt tgtgagaatg ttagatagtg tagtttttat    1200
   tgtttgtttg gggtttttgt tgttattaga taagtatttt tatttattta ttttttttta    1260
   ttaattattg ttagattttt tttgtgtttt ttgattttgt tgtaattttt ttttttgggt     1320
   gatgtttgga tagtattagt agtagttgtt tgagggaggg gggtagtttt tgtttgggga    1380
   taggttttaa aaggttgttt atttgttaga gttttatgtt ttttaaatga atttaaatta    1440
   agggtaagtt attatgtatt atgtgatatt tggtagtttt agattagaat aaggttttgt     1500
   attgaatgtg ataattgaat ttttaatgtt aagtgttatt ttttttttag gtaaatgttt    1560
   tgttgttgtg atatttaagg ttggtttgtg ggtgttgttt tttgttttgt tttattttgt    1620
   tttgaagttt tattttaagt taatattaat tttaatatgg aatagtttga tttttttttt    1680
   ttaggttttt ttattgtttt gagagaggat tataagtatt ttttattttt tttagtatag    1740
   tttttttttt ttttttttgt tatagtattg gtgttaggta attttaggta tttttatatt    1800
   gtatttatga tagatttgat gtttattgga gatatgaaaa taattagaaa gttgttatgt    1860
   aaatttaata tgatatttat ttattaggta gagtaatata ataaggtttt tttatttatt    1920
   tttgagtttt atgtttattg tagagaggaa aataatagtg attggtagtt ttgtttagag    1980
   attgtataaa agaggttagg aagttaggtt tggagatgta ttggagagta ttgtgttgtt    2040
   ttttaattag taaaattttt attggatttg tggaatttta aatttagaag tatttatttt    2100
   atgtagtagt tttatttaga gataggaggt ttgttttttg ttttatgtta gttaatggaa    2160
   tagatgggat aagaattttg gatttttggt ttttaggtaa tgttttttgg gatagtggaa    2220
   gtagaaaatt aatttt                                                    2236
<210> 48
   <211> 2236
   <212> DNA
   <213> Artificial Sequence
<220>
   <223> chemically treated genomic DNA (Homo sapiens)
<400> 48
   gaaattgatt ttttattttt attgttttag aaagtattgt ttaagagtta aaaatttaag      60
   gtttttgttt tatttatttt attaattaat atgaagtaaa aaatgagttt tttgtttttg     120
   ggtgaaattg ttatatggaa tgggtgtttt tgggtttgaa gttttataga tttagtaggg     180
   attttgttga ttggaggatg gtatagtatt ttttagtgta tttttaagtt tgattttttg     240
   atttttttta tataattttt gggtaaaatt attagttgtt gttatttttt tttttgtggt     300
   aaatatggag tttaagaatg gatagaaaga ttttattgta ttgttttatt tggtaaataa     360
   atattatgtt gaatttgtat ggtagttttt tgattatttt tatgtttttg ataagtatta     420
   agtttgttat aggtatagta taaaaatgtt tgagattgtt taatattgat attgtggtag     480
   gaagagagga gaagagttgt attggggaaa atgaggagta tttgtaattt ttttttgggg     540
   tagtgagagg gtttgggaag aaaagattga gttgttttgt gttggggtta atgttggttt     600
   gaaatgaaat tttagggtaa aatgaagtag agtaaagagt agtgtttata aattaatttt     660
   aaatattata gtggtggaat atttatttga aagaaaagtg atatttggta ttaaaagttt     720
   aattattatg tttagtgtag agttttgttt tagtttggag ttgttgggtg ttatatggtg     780
   tgtgataatt tgtttttgat ttgggtttat ttgaagagtg tagaatttta ataaataaat     840
   agttttttgg gatttgtttt tggatgagga ttgttttttt tttttgggta attattattg     900
   atgttgttta ggtattgttt aaggggaaag gttgtagtgg ggttggaagg tgtgggagga     960
   gtttggtggt gattgatggg aagggatgaa tgaataaaag tatttgtttg atggtagtag    1020
   agattttgag taaatggtgg aggttatatt gtttggtatt tttgtagtgt tttgttagag    1080
   ttggatttgt ttgtagttta agggaggtgt gttttttttt tagagtaggt tggaatttag    1140
   ttgggttttg ttttttggga aggtggtttt tatttgttgt tttgtatttg gtttgttaga    1200
   tttgagaggt aaatatttgg gtttggtgtt gaattaaaat tattgattga attttatttt    1260
   ggggttttgg tttggtttat tagtttggga ttttaaaaaa ataaaaatta agtttataga    1320
   gagggtaaat taaaattagg ttgggtaaag gaaggagtgt gagtgtttga agttgtttgg    1380
   agggaatagt ggtttttaag tttttgttga tttgagaagt ttttgtgggt ttttgaattt    1440
   ttggtgtatt tttgggtgtg ttgtgggagt tgtagtttag ttagttaggg agtagtggtt    1500
   tttatttgtt gggaggagtt ttttgagttt aattgtgggg tatagaggtt tttttgtggg    1560
   gtaaaatgta gagtttgata taagtttttg gtttttaggt gttttaattt tgtggttttt    1620
   atgtatgttt aattaagatg tgtttgtatt ttttttgtta tgtgaaagag tttggagttt    1680
   tgtttgggat ttttattatt ttttttttgg tatatttttt ttagaatgtt ttgttttatt    1740
   gtatattatt tatttttttt ggttatgtgg gggaagaaaa atagttgaga gggtattagg    1800
   aaggagtttt gatttgtgtt ggtgagttat gagtgttaag ttttttattg gtgtgtaaat    1860
   ttgagttatt tttgagtgtg gatattggtg aagaggttgt gggtggtatt agtgtttgta    1920
   gtgatttggt ttgggtagtt gatttaagtg ttttgttttt ttttttttgt ttgtttttag    1980
   gttttgaaat tgtggagtgg ttattggatg ttttttggag taggtagtag tatgtagttg    2040
   tttttaagtt tgtgtggatg tgttttggtt gtgttggttt ttgtttgtgg tttgttgtgg    2100
   atttggggag aggagagagg ttttttgttt gatagggtta ttttgttttt gtaaattgta    2160
   gagataatga tgttatttat taagatttta tggtttaagg gttttaatgt tagtttggtg    2220
   tggttgttgg tatttg                                                    2236
<210> 49
   <211> 2204
   <212> DNA
   <213> Artificial Sequence
<220>
   <223> chemically treated genomic DNA (Homo sapiens)
<400> 49
   tttttttgta aattggttta agttttggga ggtgggattg ggggaggagg gggtttattt      60
   tggggttagg ttagggttgg gtggaggagg tggtttttga gagagtgagt tgggaaagaa     120
   ggggagtggg taaagggtga gatgtggggt tttggttttt gttttgttgg ttgttagttt     180
   tttttgtgtt ttttagtttg tgtgtgtgat atgagatagt gtagttttgg tatggggttt     240
   ggtatttggt ggttgtgaga taattagggt ttgtgtgatg ggaaatgtgt tgttggtagt     300
   ttgttatttt tttattgtga ttttagtttg ttaaataggg tttgtgatgt ttagtttatt     360
   agggaggagt tgtggttttg agattttgtt tagtttgaag tagtggagat tggttaggag     420
   tttggttgtt agattttgga gtgtatgttg tgggtggggg tttggtttat gggggagggt     480
   taggggatgt gggatttatt tttgaaggag ttggggagtt gttgggtttt ggagttttgt     540
   gtgtgttgtt ttttggttat gttgtttgag atgtagttgt atttttttgt ttttgttgtt     600
   aaatttggag ttgttgggaa tgttttgagg agttagaggt ataagattta gattgttgtt     660
   ttgtttttaa ttgaggtttt gtttttgtga gagttggtta attaatgttt aggattgagg     720
   aggatttatt ttttaatttt tgtttttttg tttttttatg gtgtgattgt tttgtagtta     780
   ttttttgtta gttttgtggg ttgaggggtg tggtttgagg ggtgtggttt gaggggtgag     840
   agtttttaag gttgtgggta tgaggttggg gttttgtatt ggaagggagg gtggaagtta     900
   tgggatgtgg agtagatggt gaggatagtg gggaggtagt agtagaggat gatttgaggt     960
   aaaagtgtaa gtgaagaaaa gttgttttgt tgggtagagg tatgggatta aggtgttttt    1020
   tttgggttga ggagttagag ggaagaaaat ggtgttttgt tatgagggtg tgaggtttga    1080
   gttgagggga gaagattgag ggatatagaa gtggttgaag tgagggtgtt gaggttagtt    1140
   tgagttgaga gggttggtag attatgttgt agttgaagtg aatgaatttg agtgaggtgt    1200
   gttaagaaaa ttaaaatgta taaatttgga aaaggagagg aggttgggtg tggtggttta    1260
   tgtttgtaat tttagtatgt tggaaggttg aggtgggtag attatttgag gttagaagtt    1320
   tgagattagt ttggttaata tggtgaaatt ttgtttttgt taaaaatata aaaattagtt    1380
   gggtgtgtgg tgtgtgtttg taattttagt tatttgggag gttgaggtag gagaattgtt    1440
   tgaatttggg aggtagaggt tgtagtgagt tgagatggtt tgggtgatag agtgagattt    1500
   tgttttaaaa aaataaaata aaaaataaaa aaataggaga ggagatttta ttttttgtaa    1560
   ggggttatag tttgtaaggt ggttattttg taggttggag aatggttagg attagagata    1620
   ggtattttgt tggaggaggg gttggagtag tggttttatg ttgaatagtt tgggtagata    1680
   tgtatattta gtaggtgata ggaggagtta tgaatattta tgagggtggt tttgatgtat    1740
   gtttattgag taaatatgta tgtaatatat aatttatgtt tattttggag tgaagagtta    1800
   ttaattaaat gttaaatgtt tggtttgtat ggtgatgttt ttatttttat aaaaagtaaa    1860
   aaaaaaatag ttaggtgggg tggtgtgtgt ttgtggtttt aattagttag gaggttgggg    1920
   taggaggatt atttgagttt aagagttgga ggttgtagtg atgtatgatt agattattgt    1980
   attttagttt gatggatttt aaaaaataat aataatatat gtattatagt tagattttat    2040
   gtgttaaaag gtttttttag gataggaagg tgtgtaagta tgtagtttgt gtaaattagt    2100
   tagagttagt ttatggttgg tagttttttt atgaggagaa agttattgaa attagttttt    2160
   tgtttaatta aagttgtaat gatggttggt ggaatagggg tttt                      2204
<210> 50
   <211> 2204
   <212> DNA
   <213> Artificial Sequence
<220>
   <223> chemically treated genomic DNA (Homo sapiens)
<400> 50
   ggaattttta ttttattagt tattattata gttttaattg gataagaggt tgattttagt      60
   aatttttttt ttataggaaa attattgatt atggattggt tttggttagt ttatatagat     120
   tgtatatttg tatatttttt tgttttgaaa agattttttg atgtatgggg tttaattgta     180
   atatatgtat tgttattatt ttttagagtt tattaggttg gagtgtagtg atttgattat     240
   atattattgt agtttttaat ttttgggttt aagtgatttt tttgttttag ttttttgatt     300
   agttgggatt gtaagtatat attattttat ttggttattt tttttttatt ttttgtagag     360
   atggggatgt tattatgtag gttaggtatt taatatttaa ttggtaattt tttattttaa     420
   aatgaatatg ggttgtatgt tatatgtatg tttgtttaat gggtatgtgt taggattatt     480
   tttatgaata tttatagttt tttttgttat ttgttgaata tgtatgttta tttaagttgt     540
   ttagtataaa gttgttattt taattttttt tttaatgaaa tgtttgtttt tggttttggt     600
   tattttttag tttgtggaat ggttattttg taggttgtaa ttttttataa gaaataaagt     660
   tttttttttt gtttttttgt tttttgtttt gtttttttga gatggagttt tgttttgtta     720
   tttaggttat tttggtttat tgtaattttt gttttttaag tttaagtgat ttttttgttt     780
   tagtttttta agtagttggg attataggtg tgtgttatat gtttggttaa tttttgtatt     840
   tttagtagag atggggtttt attatgttgg ttaagttggt tttgaatttt taattttaga     900
   tgatttattt gttttggttt tttaatgtgt tgggattata ggtgtgagtt attgtgtttg     960
   gttttttttt tttttttaaa tttatatatt ttgatttttt taatatattt tatttgagtt    1020
   tgtttatttt ggttgtggtg tgatttgttg gtttttttag tttaggttga ttttagtgtt    1080
   tttattttgg ttatttttgt gttttttagt tttttttttt taatttggat tttgtgtttt    1140
   tatgataggg tgttattttt ttttttttaa ttttttagtt tagaggaggt gttttggttt    1200
   tgtatttttg tttggtaagg tgattttttt ttatttgtat ttttgttttg ggttattttt    1260
   tgttgttatt tttttattat ttttattatt tgttttatgt tttgtggttt ttgttttttt    1320
   ttttggtgta gggttttggt tttatattta tagttttggg ggtttttgtt ttttaggtta    1380
   tgttttttag gttatgtttt ttagtttata gaattagtgg gaagtgattg tgaagtagtt    1440
   gtgttgtgga gggataggag ggtggggatt gggaggtggg ttttttttga ttttgggtgt    1500
   tgattggtta atttttatga gggtgggatt ttgattgggg gtggggtggt aatttgggtt    1560
   ttgtgttttt ggttttttag ggtatttttg gtggttttgg gtttggtaat gaggatgggg    1620
   gagtgtgatt gtgttttggg tagtatggtt gagaagtggt atatatggga ttttgaagtt    1680
   tagtggtttt ttgatttttt taagggtgag ttttgtgttt tttgattttt ttttgtggat    1740
   tgagtttttg tttgtagtgt gtgttttgag gtttgatagt tgggtttttg gttagttttt    1800
   gttgttttgg gttgggtgag attttaaagt tgtggttttt ttttagtaaa ttgagtatta    1860
   tgaattttgt ttggtagatt gaggttatga tggaggggtg gtgggttgtt aatggtatgt     1920
   tttttattgt atgggttttg gttattttgt ggttgttaga tgttaagttt tatgttaggg    1980
   ttgtattatt ttgtgttatg tatgtggatt aggaaatgta gagggaattg gtagttagtg    2040
   aggtggggat tgggatttta tattttattt tttgtttatt tttttttttt tttgatttat    2100
   ttttttgaag gttgtttttt ttatttagtt ttggtttggt tttagggtga attttttttt     2160
   tttttaattt tattttttga aatttagatt gatttgtaga agga                     2204
<210> 51
   <211> 2137
   <212> DNA
   <213> Artificial Sequence
<220>
   <223> chemically treated genomic DNA (Homo sapiens)
<400> 51
   ttttattagt tagtatttaa ttatttaaaa gtgtggttta tgtttggaat tgggttataa      60
   agatggtaat gtgttaaatt agatatttgt tttttgtata tttttataaa ttatattagt     120
   ttattttata attttgtttt tgttagttat aaagaagaaa gtttaatatt ttagatgttt     180
   atattttatt aaattgtggg tgaagagtta gtgagtggta aaaggtggtt gttttgagtt     240
   gtggtgatgg aatttttagg gaatagttag gagtttggaa agttattttt gagttggttt     300
   atttaagtat ttgggataga agtggttagt agaaattttt tatggaattt taaaatatta     360
   ttaggatagt agttttatga tatttttaaa atttggagtt taaaaaataa tgaagtataa     420
   tttatttaga atttaagtat taagggaaaa tgttaagttt tgttttattt taaagatttt     480
   tttttgagga aaatatgtta ttgtttttta gatgtgaatg aaatgatatt tttttttttg     540
   atatagtttt tttgtttatg attttatatt gttttttttt aatttttttt attattgaaa     600
   tattatatgt aagatttgtt ggtatgtttt ttgatttttt atgtttaagg ggaaaaatgt     660
   ttgtagttga tgtttgagtt ttaggagttt ggtgaagaaa aaaaaaaaaa gtaatgtaag     720
   gaaaattgtt ttaatggtat atttttttta aagtttgaag ttttataggt aaaaggaggg     780
   ataaatattt ttttttgaaa gtaaatagtt aaggtttttt tttatttaat tatttttgaa     840
   ttttttgtaa tttttttgag gtttaaattt agggtataat taattttaga ttttatttta     900
   aatttaattt gttttttatt tgtaatttgt ttgtaggtgg gttttttttt tttaaattat     960
   aaatttgaat ttgtgttaat tttttataat ataaggtatt taaagttttt tggagttatt    1020
   gatttggtga tgttgtagtg tatgtttgta tttgaaagta agtaagagaa tagaaagtga    1080
   gtaagagaat tgtaattttt attattttgg tgaatttttg ttgggattta tatattaaat    1140
   agtatttgta tatagatgtt ttaagttgtt ttttttagag ttgttattta gatttgtaag    1200
   gggtggggga ttagtggaaa tttaaataga aatttttatt attataaagg tgagttttta    1260
   ttgattatgg taattgtttt gtttaggttg tatgggtatg gttaattttg tttgaaagtt    1320
   tgtttttttt ttgtgttttt ttgttgagtt ttttttaggt tttggttttt tttgatggtg    1380
   gtaagggatg ttttttatgt tttttattat ttttgtttgt gtttgtgttt tttgtttgag    1440
   gttttaggtt gtggtggtgg tgtggtgggt ttattagtgg gtgtgttggg tttgtgtagt    1500
   gttgtttgta gttgtgttgt tgtatttatt attattatat tagtgtgggg aagtttatgt    1560
   tgagtttttt tggttttggt gtgttttagt tagtgtgtta gtaaatagaa tatgttggat    1620
   tgtttagaga tggttttggg tgtttggggg tagaaggggt gtttttaggt ttggttttga    1680
   tttgtatttt tgaagttggt tttgggagat aatgtatttt tgttagttag gttgtgggaa    1740
   aagtgaaaaa agggttgggg gttatgtggg ggtggggagg tgagttttag tgttagtagt    1800
   gtttggtagt ttttttttag tgtagagaag tggtttgtat taaggtgtat tagagtggtt    1860
   attttgtttt ttggaatatt tatgggtatt tgtaaatttt atagttggtt tagttaaatt    1920
   tttagtaaat attggtaagg gttggttttg gttgtgttta gagggttttt ttaattggtt    1980
   gaatttaggg agttgtaatt ttttaggtat tttgtaatgt aattattttg agtgttgtat    2040
   tataataaaa tattggggat tttttgaaaa gtttttataa aattaatagg tgtgtgtttt    2100
   gtttataaaa tgttgaattt attttttatg aatttgg                             2137
<210> 52
   <211> 2137
   <212> DNA
   <213> Artificial Sequence
<220>
   <223> chemically treated genomic DNA (Homo sapiens)
<400> 52
   ttaggtttat agaaggtgaa tttgatgttt tgtgagtgaa gtatatattt attggttttg      60
   tgaaggtttt ttaagggatt tttaatgttt tattataatg tgatatttag ggtggttgta     120
   ttgtaaagta tttggaaggt tgtagttttt tgaatttagt tagttaaaag agttttttga     180
   atgtaattag ggttggtttt tgttagtgtt tgttggaaat ttggttgggt taattgtaga     240
   gtttgtaaat gtttgtgaat attttgagga gtagggtgat tattttgata tgttttgata     300
   tagattgttt ttttgtattg gagggagatt gttaggtgtt gttggtgttg gagtttattt     360
   ttttattttt atatggtttt tagttttttt tttatttttt ttgtggtttg gttggtggag     420
   atgtattgtt ttttaaagtt agttttagaa atgtggatta aagttgggtt tgggagtatt     480
   ttttttgttt ttagatattt agagttgttt ttgagtaatt tagtatgttt tgtttgttgg     540
   tgtgttgatt ggggtgtgtt ggggttggga gaatttggtg tggatttttt tgtgttgatg     600
   taatgatgat ggatgtggtg atgtggttgt agatagtgtt gtgtgggttt ggtgtgtttg     660
   ttaatgaatt tattgtgttg ttgttgtggt ttgggatttt gggtagggga tgtaggtgtg     720
   ggtaaaagtg gtgaaaggtg tgaaaaatat tttttgttat tgttggagga aattagaatt     780
   tgaagggaat ttgataggaa agtgtagaga aagagtgagt ttttaaataa aattggttat      840
   atttgtataa tttgagtaaa ataattatta taattaatgg ggatttgttt ttgtaatggt      900
   gaaagttttt gtttgggttt ttattggttt tttgtttttt gtaggtttaa gtgatgattt      960
   taaagaggat gatttgaggt gtttgtgtgt aagtgttgtt taatatgtag gttttggtaa     1020
   gaatttgtta aggtggtaaa ggttgtggtt tttttgtttg ttttttgttt ttttgtttat    1080
   ttttaaatgt agatgtgtat tatagtgtta ttaggttagt ggttttagga agttttaaat     1140
   gttttgtgtt gtaagaggtt gatgtaggtt tagatttata atttagaaag aaaagattta     1200
   tttgtaggta ggttgtaaat aggagatgga ttagatttaa aatagggttt ggaattaatt     1260
   gtgttttggg tttaggtttt ggggaaatta taaaaggttt agggatagtt gagtgggaaa     1320
   aagttttgat tgtttgtttt taggaaggaa tgtttatttt tttttttatt tatgaaattt     1380
   taagttttag gaaggatata ttattagagt aatttttttt gtgttgtttt tttttttttt     1440
   ttttattgag tttttgggat ttaaatatta gttataaata tttttttttt taagtatgaa     1500
   aagttaaggg gtatgttagt aaattttgta tgtaatgttt tagtggtgaa agggattgaa     1560
   ggagaatagt gtggagttgt aagtaaggaa attatgttgg gggaaaaagt attattttat     1620
   ttatatttga aaaatagtgg tatgtttttt ttagggaaaa gtttttgaag tgaagtagaa     1680
   tttaatgttt ttttttgatg tttaagtttt aaataaatta tattttatta ttttttaaat     1740
   tttaggtttt aaaagtgttg tggagttatt gttttagtgg tgttttgaga ttttatgagg     1800
   aatttttatt gattattttt attttgaatg tttgaatgga ttagtttagg agtgattttt     1860
   taagtttttg attatttttt gaaaatttta ttattataat ttaaagtggt tattttttgt    1920
   tgtttattga ttttttattt atagtttgat gaggtgtggg tgtttagaat gttaaatttt    1980
   tttttttata attgataggg ataaagttgt aaaataagtt gatataattt ataggagtgt    2040
   ataggaagta gatgtttgat ttggtatatt gttattttta tagtttagtt ttaagtataa     2100
   attatatttt taaataatta ggtattaatt aatgaga                              2137
<210> 53
   <211> 2461
   <212> DNA
   <213> Artificial Sequence
<220>
   <223> chemically treated genomic DNA (Homo sapiens)
<400> 53
   tttttttttt tttttttttt tttttttttt tttttttttt tttttttttt tttttttttt      60
   tttttttttt tttttttttt ttttgttttt tttttttttt ttttttttgt tttttatttt     120
   tttagtttga aggttgtatt ttttttattt ttttaagtaa agttgggagt tttgtagtat     180
   tttaggagtg aggataatta tgtttaagaa attttggttt tagtttagtt ttagattaag     240
   aggaaaatat tttaaaattt taaagtaaat tttaaaagtg taatttatat tagagtgatt     300
   gatttggtat ttatgtagat tgaagtttga gaagtaattt aagtggtttt tatttatttt     360
   tttggaagag tttttagaat gtatttttat tggtttgaaa aatgtttgaa aaagagtttt     420
   gatgttaata gtaatggaaa taggagggtt ggtggtttat gattagttta gtggtatttt     480
   ttattttatt tgtatttagg taaagagatt gtggtttgta tgtgattaaa taatttgttg     540
   aagtgttttg ggtttagggt atttaatttt tgtgaagtag gattattagt tgtttgtttt     600
   gtttttgtgt tattttttag aatatattaa agtaggggta atttttgagt ttagattttt     660
   gttatgtgaa gattttttga aaattttgat aaatataggt attgtgtaag tggttagtta     720
   attaaagagg ggaggtggag ttgttttttg tgtatttaat aagtatttat tattttattt     780
   gagtatgaaa agaggttatt gttattattt ttaagaagga aagtaagtag gatagtttat     840
   atttttagaa taatttttta ttaaatggaa taattttggt gaaaagtggg agtgaggaag     900
   aaagaaaaaa aaaattttta attataatgt ttgggaataa gaaaggaaga agaaatttat     960
   gttaaagttg attttttttt gtagttgtaa aataaatttt taagattttt tttgttgaaa    1020
   ttttggagag gaaaattgga gtggtgggtg ggttttgttt gtagtttaat ttttttttgt    1080
   ggtgtgggtg tggttgggtt tagtattttg gaaagtgttt tttgtggtgt tttgggatta    1140
   tgtatgtttt ttggggtttg ttgttttggt tgtgtaagtg ttattgtaat tggtgagagt    1200
   tgttggtaat ttatttggag ttgataattg tggagagatg tagatattta ttgattttta    1260
   ggaagttgag tgtggtggat ggaattttat gatttttttt tttttaagga tggaaatttt    1320
   atttaagtag ttttagagga aatggataaa ggtatttgaa agggagtgag tggttttaaa    1380
   ttgtataatt gagtggttgg gggagttatg tgttagtgtt ttagtgattg tgggatatgg    1440
   agaggggaag tttgtgttgt atataaggat ttagggattt tgagtttggt ttgagaattt    1500
   ttggatgttg agtgtttgtt ttatgggttg tattttttaa ttttgtttta aagtagttgt    1560
   tgagtttaat ttttgtgttt agggtgtttg ttgtgtgtta ggatgtgttt agtatttagt    1620
   ttttgggttt tttttttagt agttgttttg aaagttttta tgtatgtttt gtaggttagt    1680
   ttggtaataa aattggggta aattgtgtta ttttaggttt tgttttttag aatatgattt    1740
   agaggtattt gtgtatgtag atggttgatg tagttatgat agttattatg aataaggtag    1800
   taggtgggga taagttagta gaatttttta gtttggtttt ggattttttg gaggtggtta    1860
   atatgagtgg taatgtgttg ttgtagtttt tggatttgtg gtgggagttg gggttggagt    1920
   tgttggatgg tgtgttgtta ggatattttt tgggtagtgg tggggtagag agtgtggata    1980
   tagaggtttg ggtgtggatt tttattagtg tggtgtattg ggtggtgtgt gttttggggt    2040
   tggtgggtaa tttgttggtt ttttatttga tgaagagtat gtagggttgg tgtaagtttt    2100
   ttattaattt ttttgttatt aatttggtgt tgatggattt ttagtttgtg tttattttgt    2160
   ttttttgggt ggtggagaat gtttttgatt ttaaatggtt ttttggtaag gttatgtgta    2220
   agattgtgtt tatggtgatg tttatgaata tgtatgttag tgtgtttttt tttattgtta    2280
   tgagtgtgat gtgttattat ttggtggttt tggttttgaa gagttattgg atttgaggat    2340
   atggttgggg tgattgttgt ggttggagtt tgggggatag ttgttgtttt ttggttaagg    2400
   tgttgtgtgt gtggatttgg gttttggttg tgttggtttt gttgtttagt gttatttttt    2460
   t                                                                    2461
<210> 54
   <211> 2461
   <212> DNA
   <213> Artificial Sequence
<220>
   <223> chemically treated genomic DNA (Homo sapiens)
<400> 54
   gagaaaatgg tattgggtag tgaggttagt gtggttaaag tttagattta tatatatagt      60
   gttttggttg agaagtagta gttgtttttt aggttttggt tgtagtagtt gttttggttg     120
   tgtttttggg tttggtggtt ttttagagtt gaggttattg aatggtagtg tgttatattt     180
   atggtagtga ggaagaatat gttggtgtat atgtttatgg atgttattat ggatatgatt     240
   ttatatatgg ttttgttgaa gggttatttg aagttaagag tgttttttat tgtttagaag     300
   ggtagggtga gtataaattg aaagtttgtt agtgttaggt tggtgatgaa gaggttgata     360
   gaggatttgt gttagttttg tatgtttttt attaggtaga gaattagtag gttgtttgtt     420
   aattttaggg tgtatattat ttagtatatt atgttgatga gaatttgtat ttgggttttt     480
   gtgtttgtgt tttttgtttt gttgttgttt gggggatgtt ttggtggtgt gttgtttggt     540
   aattttagtt ttagttttta ttataagttt ggaagttgta gtgatgtgtt attatttgtg     600
   ttggttgttt ttagaaggtt tgggattaga ttgaagagtt ttgttagttt gtttttgttt     660
   gttgttttat ttatggtggt tattgtggtt gtattggtta tttgtatgtg taggtatttt     720
   taggttatgt tttgggggat aagatttaag ataatatggt ttattttagt tttgttgtta     780
   ggttagtttg tgggatgtgt gtgggagttt ttaaagtagt tattgaagag agagtttagg     840
   aattgggtat taagtgtgtt ttggtgtgta gtgaatgttt tggatgtagg agttgagttt     900
   agtggttgtt ttggagtaga gttgaggagt gtagtttgta aggtaagtat ttggtgttta     960
   agggttttta ggttaagttt ggagttttta ggtttttatg tataatgtag attttttttt    1020
   tttgtgtttt gtggttattg gggtattggt gtataatttt tttagttgtt taattgtgtg    1080
   atttggggtt gtttgttttt ttttaaatat ttttatttgt tttttttggg attgtttgga    1140
   tgaggttttt gtttttggag agaaagagat tgtagagttt tatttattat gtttagtttt    1200
   ttggaggtta gtgggtgttt gtgttttttt gtggttgtta attttgggtg ggttgttagt    1260
   ggtttttatt agttatggtg gtatttgtat ggttaaggtg gtgggtttta aggagtatgt    1320
   gtaattttgg ggtgttgtga ggggtgtttt ttgaggtgtt gaatttagtt gtgtttgtgt    1380
   tgtgagggag agttgagttg taggtaaagt ttatttgtta ttttagtttt tttttttaga    1440
   gttttagtag gaagggtttt aagagtttat tttatagttg taggagaaag ttggttttga    1500
   tgtgagtttt tttttttttt tttattttta aatattatga ttagaagttt tttttttttt    1560
   ttttttttat ttttattttt tattggaatt attttatttg gtgaggaatt gttttaaaaa    1620
   tatgagttat tttgtttatt tttttttttg aaagtaataa tagtggtttt tttttatgtt    1680
   taaatgagat aatgggtatt tattggatat ataaaggata gttttatttt ttttttttaa    1740
   ttagttgatt atttatgtag tatttgtgtt tgttaggatt tttaaagggt ttttatatgg    1800
   tagaagtttg ggtttaaggg ttgtttttat tttaatgtat tttgagaggt gatataaaaa    1860
   tagagtaaat aattgatggt tttattttat agaaattaaa tgttttaagt ttaaagtgtt    1920
   ttaataaatt gtttgattat atataggtta tagttttttt atttagatgt aaatagaata    1980
   gaggatatta ttgggttgat tgtgaattat tagttttttt atttttatta ttgttagtat    2040
   taggattttt ttttaggtgt tttttagatt aatggaagta tattttaagg atttttttaa    2100
   gggaatgagt ggaaattatt tgaattattt tttaagtttt agtttatata aatgttaggt    2160
   taattgtttt ggtgtaaatt atatttttag gatttatttt aaaattttga aatgtttttt    2220
   ttttggtttg ggattaaatt gaaattaaag ttttttaagt ataattgttt ttatttttgg    2280
   agtattatag aatttttagt tttatttaag gagataaaga ggatgtaatt tttaagttga    2340
   ggagataaga aatagaaaaa gaaagaaaga gaaaaatgaa aaagaaagaa agaaagaaag    2400
   aaagaaagaa agaaagaaag aaagaaaaga aaagaaagag aaaaaggaaa aaagaaaaaa    2460
   a                                                                    2461
<210> 55
   <211> 1920
   <212> DNA
   <213> Artificial Sequence
<220>
   <223> chemically treated genomic DNA (Homo sapiens)
<400> 55
   ttaggttgtt gtagatatag tttttgtttt tttgaaaaat atgttttagg tgttgggatt      60
   ttagatattt gggaaataga gtgtatgtag ttgttgagag gttttgtgtt tggttttttt     120
   tattattgag gtgtagaggt gttgtggata gtttagattt atatggtgtt tgaggtgaaa     180
   tagaattttt agttttttta tgaggttatt ggtatttttg gttgttttta gagttttttg    240
   atttagagtt gaatgtaaag taagtgtttg aaatgtagaa gtagttgggg ttgtttatgg     300
   tatttgtttt gtttggggtg agagaagatg ttaggttgag gttttagtga ttttaggtat     360
   tagttttgaa ggagggtggg gagattgtaa aggggaagtg tttggagggt taatggtttt     420
   tgtgtatttt gtgttttttt gaagtgtgtt gtttttttgt gttggggatt gggatttgtt     480
   tttggggaat ttgtttagaa gatggtggtg gattggggtt gggtattttt tagggttgtt     540
   aggttttttt tagttttgta tttgttgtgt tgttttattt tgttaggaag ttttagagat     600
   tttggggatg gggtgggagt gtttttttat tgtgggttta aaaagaagga aggatgtttt     660
   taggggttgt agaaggagga ttagttttaa gttataattt tttttggatt taaggtaggt     720
   tggttggggt tttgtgttta tatggttttt ggtgggggtt tgtgtgtttt gggagttttg     780
   tggtttgggg aggaaagagg agataagaga taggtgagga ttatggggtt gatttagttg     840
   gggtagggat tattgtggaa aaattttggt gaggtggggg gatgtggaaa gagagtggtt     900
   tgtgttttgt attttgtgtt gggtattttg tgttagtgtt ttgtttttag tgttttgtgt     960
   tttgtgtttt gtgttttgtt tttattttgg gttagttgta ttgtgtttgt gttgtaggaa    1020
   ttgtggagtt ggaaagtggg ggtgttgtgg ttggggggtt gttttagttg tgttttggtt   1080
   agtgattggt gggttgggtt taaatttagt taggttgggt aggtggtggt tgtgtgattg    1140
   gggattgggt gttttgtttt ttttgttttt tttttttttt tttttttttt ttttagtttt    1200
   ttggtttttt ttagttttta ttggatttgt ttgtttgttg tttttttttt ttttttgttt   1260
   tttatattat tttttatttt tttgttttgt ttttgttttt tttttttttt ttgttttttg   1320
   tttttttttt tttttttttt ttttttaggg gtggagtttt tttttttttt tttagataat    1380
   gttgtggttg tgtttttttt tttgttagtt tgtttaggtt tttgttgtta gtgatttttt    1440
   tgggttgggg gtggggaggt ggggggggag tgtagggttg gggaggatga gttggttttt    1500
   tttatttttt tgttgttgtt ttttttaaga gggatggaga tttggtttaa gttttttggt   1560
   ttatttggag ttgtgatagt tatttttagg gaatagttat gttgttttat taagtttatt    1620
   tttagtggtt tggatttttt aggtagaggt tgtgggattt tgtttttttt aatattttag    1680
   tttattttta aaagggtttg agttggatag gggttaaata ggtttttttg atttggtggg    1740
   ttggttagat gtgatagtaa tgttaaggag gttaagtttt tttgtttatt ttttattttt    1800
   ttttttttta tttttggatt ttttggtgtt tttagtatat agaggttttt gagtagtttg    1860
   gttgtaggtt ttttatttat ttagagtttt tttttttatg tgtttatttt taattttgta     1920
<210> 56
   <211> 1920
   <212> DNA
   <213> Artificial Sequence
<220>
   <223> chemically treated genomic DNA (Homo sapiens)
<400> 56
   tgtagggttg gggataggta tgtgaggggg agaattttga gtagataggg aatttgtagt      60
   tgggttgttt aagggttttt gtgtattggg ggtgttagga ggtttaggga tggaaaaggg     120
   ggaggtgaga aatggataaa gaaatttggt ttttttggta ttgttgttat gtttggttgg     180
   tttgttaagt tgaaggggtt tgtttagttt ttgtttggtt taaatttttt tgggaataag     240
   ttgggatgtt agaaaaaata aaattttata atttttgttt ggggaattta ggttgttgga     300
   ggtgggtttg gtagggtagt gtgattgttt tttgggagtg gttgttatag ttttgggtga     360
   attgaggagt ttgggttaag tttttatttt ttttggagag ggtagtagta aggaggtgag     420
   gggagttagt ttattttttt taattttgta tttttttttt atttttttat ttttagtttg      480
   gaagaattgt tggtggtggg agtttggatg agttggtggg gaaggggatg tagttatagt     540
   attgtttggg agggagggga gaagttttgt ttttagaggg gaggggagaa gaggaggggg     600
   taggagataa ggggaggaag aaaggtgaag gtaaggtgaa ggggtggaga gtgatatgaa     660
   gagtgagaga aaagagagga tagtggatga gtagatttgg taggggttga aaaaggttaa     720
   ggggttggag ggagggagag gaaggaggag gggagtgagg agggtggggt gtttggtttt      780
   tagttgtgtg gttattgttt gtttagtttg gttggatttg agtttggttt gttgattgtt      840
   ggttgaggtg tagttgaagt agttttttag ttgtggtgtt tttatttttt aattttatgg      900
   tttttgtggt gtgggtgtga tgtagttggt ttggggtgaa ggtaaggtgt ggggtgtggg      960
   gtgtggggta ttgggagtga ggtattggtg tgggatgttt ggtgtaaggt gtagggtgtg     1020
   ggttgttttt tttttgtgtt tttttatttt gttaaagttt ttttatgatg gtttttattt     1080
   tggttgggtt agttttgtaa tttttgtttg ttttttgttt tttttttttt ttttgagttg     1140
   tggggttttt ggggtgtgtg gatttttgtt aggggttgtg taggtgtgga gttttagttg     1200
   gtttgttttg ggtttgaaga aagttgtggt ttggagttag tttttttttt atgatttttg     1260
   ggggtgtttt tttttttttt tgagtttgtg atgggaaggt gtttttattt tatttttggg     1320
   gtttttgaga ttttttggtg aggtggggtg gtgtggtagg tgtagggttg gggagggttt     1380
   gatagttttg gagagtgttt gattttagtt tgttgttgtt ttttaggtgg attttttaga     1440
   ggtaggtttt agtttttggt gtggggaggt ggtgtgtttt agaggggtgt agggtgtgtg     1500
   ggggttgttg gttttttggg tatttttttt ttgtggtttt tttgtttttt tttggagttg     1560
   gtgtttgagg ttgttgggat tttagtttgg tgtttttttt tgttttgagt gaggtaggtg     1620
   ttgtgggtgg ttttggttat ttttgtattt tgagtgttta ttttgtattt agttttaagt     1680
   tggagagttt tggggatagt tgagagtgtt agtggtttta tagggagatt gagggttttg     1740
   ttttattttg ggtgttgtgt gggtttgggt tgtttatagt atttttgtgt tttagtgata     1800
   ggagaagtta agtgtgaggt tttttaatag ttgtgtgtat tttatttttt aggtatttgg     1860
   aattttggtg tttagaatgt gttttttggg agagtaaagg ttgtgtttat ggtagtttgg     1920
<210> 57
   <211> 2225
   <212> DNA
   <213> Artificial Sequence
<220>
   <223> chemically treated genomic DNA (Homo sapiens)
<400> 57
   atttttttgt aaattaaatt tttgttgttt agtttagtgt ttggtgtata gtgggggtta 60
   gatagatatt tgttgagtgt atgattaggg tgttgtaggt ggaggaggtg gtggtggggt 120
   gggggagggg aatttttttg ttgttttttg ttttgtggga attagtttta gaaagaaatt 180
   ttttgttgtt ttttttattt tattttaatt ttggatttgt tttttgattt tttttatttg 240
   gttttagttt tttatttaga gttaattttg tttgaaaggt attatgtttt gttattaagg 300
   ttttaaaata gggaaaatag tttttgaagt ttttagtttg taggagatag ggtattttag 360
   ggtataaggt tttattgagg ggttggaaaa agagtttaga ttttaatatt tatagaaagg 420
   ggttttggtt gtttagttgt gagtggtttt ttttttgttt tttagtatat ttggaaatga 480
   ttttatatgt gggtgttgtg ggatgttggg tagtaaggag atgatttagt tgaataatgt 540
   atttggttta tttttgtgag gggaggagaa tgttgaggga taggaattgt gtgttagtaa 600
   aagatatttt aatttggggt gtgtttgtta gttgtttttt taaggtgaaa atttttaaga 660
   ggtgagatgt aaattttagt ggtgtttata tttggaggtg tttttttttt aggttgtttt 720
   tttgttttat gtgatatttt aatttttgta gtttttgttt agtgtgggat taagaatggg 780
   gagttttatt tttttttggt tttttagttt tttaatttaa gataagaggg tgtgtgtttg 840
   ggttgggata ggttttagtt gtatttttgt agattgtatt ttttgtttgg gagtgatttg 900
   ttttagtagt tttttttttg ttttattaat gtggttgtat tgttggtgtt tagaggtgat 960
   attttggtaa agataggaat taagttagaa atttaggttt tagaagtaaa ggttgtggtg 1020
   ttttttggtt ggaagaaaat ggtgggtggg tggaggatgt ggtgtgtggg aattttttta 1080
   tggatgtttg ttgttttttg tttatttttt aggaaggtgt ttttttattt gtattttttt 1140
   ttgtaatttg gagtttgttt gtgagtttag ttatttgttg gttttttttg tttgtgtttg 1200
   tatattatag attttgttta ttgtatttta tttggtttta gagggtttag aggggatttt 1260
   ttgaaattaa tttgtttgtt tggagttagt tttgatgtat attttgagag tggtgggagg 1320
   taaagtagtt ggaatgtttg agaaaaaagt gggtgttaga ggtttttgtt ttagtgattt 1380
   ttttattgta ttgattgagt tttgggattg tagggattta agtttttatt tgttttggtg 1440
   tttttttttt gtgtgttttg ttgttttgga agttggattg agagttatag tgtggttggg 1500
   tgtaggtggg ttgtttggtt tttattttga ggatttggat ttgtatttgt agagttgttg 1560
   tgtgggggat gttttttttt tttttttgtt ttggtgggtt tttttggtgt gttagggttt 1620
   ttgggtttta gttggagttg gtgttttggt tttatagttt tttttagtgt tttggatttg 1680
   tgtgttttgt tttgtgttgg attttgtttt ggggtttttg tttgtttttt agtttaggtt 1740
   aagggtggat gtttagtttt tttattttat tgtgtgtttt tgggttggtg tgtttagggt 1800
   gtgggtattg tagttttggt tgtgttgatg tgagtttttg gtagtggggg tagttttttg 1860
   tatggatttt agtgtttttg tattttgttt gaattggatt atatagtagt tgggttttta 1920
   ttgtgtgttt gggaatagtg agtattgttt tttgttgtta gttgtggtat tgtatttggg 1980
   ttgtgtgtgt aggaggggag agttttgttt tttggaggtg tttagtgtta gtgtttgtgt 2040
   ttattagaag gtgatttttt taaggaaagt ttggttttga tgagtgtggt gtgggttttt 2100
   ggagatggtg gtgaaggagg aggtttttag gaggaagttg gggatgggga tagtttgttt 2160
   tgtttaaagt ttttgaagtt tttatttaaa ttttgtgatg attattatta ttagatttta 2220
   tggtt 2225
<210> 58
   <211> 2225
   <212> DNA
   <213> Artificial Sequence
<220>
   <223> chemically treated genomic DNA (Homo sapiens)
<400> 58
   ggttatggaa tttgatagtg gtggttattg taaagtttgg gtgaggattt taggggtttt      60
   gagtagagta aattgttttt atttttagtt tttttttggg ggtttttttt tttattgtta     120
   tttttaaagg tttatattgt atttgttgga gttgggtttt ttttgaaagg gttatttttt     180
   agtgggtgtg gatgttggta ttgggtattt ttggaagata ggattttttt tttttgtgtg     240
   tgtgatttgg gtgtggtgtt atgattggtg gtagagggtg gtgtttattg tttttaagtg     300
   tatggtagaa atttggttat tgtgtggttt gatttagatg ggatgtgaag gtgttgaaat     360
   ttgtgtgaga ggttgttttt gttgttgggg atttgtgtta gtgtggttgg agttatggtg     420
   tttgtgtttt ggatgtatta gtttgagagt atgtggtggg gtgggggagt tgggtattta     480
   tttttggttt ggattaggag gtaggtgggg attttggggt ggagtttagt gtggggtggg     540
   gtgtataggt ttgagatgtt ggaaagagtt gtggggttgg agtgttaatt ttagttggaa     600
   tttagagatt ttgatatgtt ggaggagttt gttggggtga ggaaaaaaga gggatatttt     660
   ttatgtagtg gttttgtaag tgtaagtttg ggtttttgag gtgaaaattg agtggtttgt     720
   ttgtgtttaa ttatattgtg atttttgatt tggtttttgg agtagtagag tgtatagaaa     780
   ggaggtgttg agataggtaa gggtttagat ttttgtagtt ttgggattta gttggtgtgg     840
   tgggggagtt gttaggataa gggtttttga tgtttatttt ttttttgggt attttggttg     900
   ttttgttttt tgttattttt gagatatgtg ttggggttgg ttttaagtag gtgagttggt     960
   tttaggagat tttttttggg ttttttagga ttgggtaggg tatggtaagt ggagtttgtg    1020
   gtgtgtgggt gtgggtagag ggagttagtg ggtagttgag tttgtggatg agttttaaat    1080
   tgtaggggag ggtgtaggta gaggggtatt tttttgaaag gtgagtagga aatagtaagt    1140
   gtttgtgggg aaatttttgt gtattgtatt ttttatttat ttgttgtttt tttttagtta    1200
   ggaggtattg tagtttttgt ttttagagtt tgaatttttg atttgatttt tgtttttgtt    1260
   aggatgttgt ttttagatgt tgatagtgtg gttgtgttga tggggtggga agggggttgt    1320
   tggaatgggt tgtttttggg tggggagtgt aatttatgaa aatgtggttg ggatttgttt    1380
   tagtttgggt gtgtgttttt ttgttttggg ttagggggtt ggaaagttga ggaggggtag    1440
   gattttttat ttttggtttt atgttgagta gaggttgtgg ggattggggt gttgtatgag    1500
   atggaggggt gatttggaga gaagatattt ttgggtgtgg atgttattgg gatttgtatt    1560
   ttgttttttg gaggttttta ttttgggaaa gtagttggtg ggtgtatttt aggttagggt    1620
   gttttttgtt ggtgtgtagt ttttgttttt tggtgttttt ttttttttgt aggagtggat   1680
   taaatgtatt gtttagttgg gttatttttt tgttatttag tattttgtgg tgtttatatg    1740
   tggagttgtt tttaggtgtg ttgagaggtg aggggaaggt tatttatagt tggatagtta    1800
   gggttttttt ttataggtgt tgggatttgg gttttttttt tagttttttg gtgaagtttt   1860
   gtattttggg gtgttttatt ttttgtgggt tgagggtttt agagattatt ttttttattt    1920
   tgagattttg gtgataggat atggtgtttt ttaggtaggg ttgattttgg gtggagaatt    1980
   agggttaagt gaaggaggtt agagagtaga tttaaagttg gagtagggtg ggaagggtgg    2040
   tagaggattt ttttttggag ttggttttta tagaatgggg agtagtaggg gaattttttt    2100
   tttttatttt attattattt tttttattta tagtgttttg gttatgtatt taatagatgt    2160
   ttgtttagtt tttattatgt attaggtatt ggattgggta gtaggaattt ggtttatagg    2220
   aagat                                                                2225
<210> 59
   <211> 2251
   <212> DNA
   <213> Artificial Sequence
<220>
   <223> chemically treated genomic DNA (Homo sapiens)
<400> 59
   ttttttagtg tgattgattg tgatttagag gagaatgggt aggtgtagtg tgagttattg      60
   ggagatgtgt ttttttgttt taagtttttt tttaagaatt attatattat tgttattgaa     120
   gtttttttgg attgagaggt gggggatttt tatattttga ttgtagtggt ttgggattgg    180
   ggtgagtttg tgttttttat tagtaagttg atttaggtat aagtgttgga tgtgaatgat     240
   aatgtgttgt gttttagtta gttggtttat gatgtgtatg tgattgaaaa taatgtgttt     300
   ggtgtttata tttatgtggt gagtgttatt gattgggatg agggtgttaa tgtttagttt     360
   gtttatttta tttttgagtg ttagatttag ggtatgagtg tttttattta tgtttttatt     420
   aattttgaga atggttattt gtatgttttg tgtttttttg attatgagta gttgaaggat    480
   tttagttttt aggtggaagt ttgggatgtt ggtagttttt aggtgttggt tggtaatgtt     540
   attgttaata tttttatagt ggattaaaat gataatgttt ttgttattgt ggtgttttta     600
   ttagggtgta atgggatttt agtgtgtgag gtgttgtttt gtttggtgga gttgggttat     660
   ttgtttattt gtgtggttgt tgtggatgtg gatgatggtg agaatgtttg gtttatttat     720
   agtattgtgt gtggtaatga aatgaatttt ttttgtatgg attggtgtat tggggagttg     780
   tgtatagtat gttgagtttt ggttaagtgt gatttttagt ggttttatga gttggtgatt     840
   gaggtgtgtg attatgggta gttgtttttt ttttttattg ttattttggt ggtttagttg     900
   gtggatggtg ttgtggagtt ttagggtggg ggtgggagtg gaggtggagg gttaggagag     960
   tattagtgtt ttagttgttt tggtggtggg gaaattttgt tagattttat ttttattttt    1020
   attattgtgt tgggtttggt gttttttatt tttttgttgg ttatgattgt gttggttgtg   1080
   tgttgttaaa aagagaagaa gtttaatatt tatatttgtt tggttagtga ttgttgtttt    1140
   tgttgttgtt gttgtggtgg tggaggtttg atttgttgtg gttgttaagt ttgggtgtgt    1200
   aagaagaaat ttagtaagtt agatattatg ttggtgtaga gttttaatgt atttagtaat    1260
   ttggtttagg tgttgataga ggagtttggg ggttttggtt tttattatta taattagaat    1320
   tattgttatt aggtatgttt gatttttgag tttgttaaga ttgatttgat gttttttaag   1380
   ttttgtagtt ttttgtggag tatggatatt gagtataatt tttgtggggt tattgttatt    1440
   ggttatattg attagtagtt tgatattatt tttaatggaa gtattttgtt taatgaggta    1500
   aggttgaagt gaaaggatta ttatttttta ttttttttat tagaaagttt tttttagttt    1560
   ggtttttgta tttttggtgt attgtattta tttttaggat attagtttat gtgtattgtt    1620
   gtgggagtag agatgggtgg ttattttttt ttattttttt gtgtgtaatt taatttttgt    1680
   gttgttttat ttttttatat ttatttttat tttgtttttt tggtattttg ttattttgga    1740
   gttttttttt ttgttttttt attttgttag tttttttttt ttttagtaat ttgggtatga    1800
   agggaaattg tgtgaaggga gagggaaatg tggaggaggg atttattttt tagtattggt    1860
   aaaggttttt tttttttgtg tttgttttag gtattaataa agttggtttt attttgtttt    1920
   gtttaatgat gtttttagtt gtgtgtataa gtaagttata gattgtttaa ttttatatag    1980
   ttgtttaatt ttgaatttat gttttagtga ataagttatt agattttttt tttttgaatt    2040
   tgggttgtaa aaaaagtttt tagtttggtt ttggatagta tttatagatg tttttgaagt    2100
   tgagtgttta tatagtgtga atttgaatga agttgtgttg gtataaattt ttgttaagag    2160
   taaattaatg aaaggtttaa ataattgttg ttttaaatat atttttttag ttaataatta   2220
   tttttttgtt attgttttgg ataaattatg t                                  2251
<210> 60
   <211> 2251
   <212> DNA
   <213> Artificial Sequence
<220>
   <223> chemically treated genomic DNA (Homo sapiens)
<400> 60
<210> 61
   <211> 2553
   <212> DNA
   <213> Artificial Sequence
<220>
   <223> chemically treated genomic DNA (Homo sapiens)
<400> 61
   ttggttattt ttgttttttt ttagattttt agagttttta gtgtagttat agaggatgtt      60
   gttggtttta gttttaagaa gatgttgttt tttttagagg gttaagtaag tgggaatttt     120
   tttttttatt tgttttgggt tttaggtagg gtttttggtg taaggtttgg ggttggaagt     180
   tgatttattt aggtttaggt tttggggtag aattgaaatt ttttggttat tgttggttgt     240
   agtttgggag taggttattg ttaaagttgt gggttttttt aggatagttt ttttatgagg     300
   ttggtttttt atttgttgtt tttttatatt tggtggttag ggatgtggtt ttgggtagaa     360
   tgatgatttt ttatttttgt tattatggaa gttattgttg ttttttagtt tagttagtta     420
   tttgggttgt agagtatttt ttttatgttt tttgggtgtt tttttttttt tttgttttag     480
   tttggttttg ttttattttg tttttaggga ggggtatttt ggagtggggt tagggtatgg     540
   tttttttttg agggagtttt tttttggttg tttttagggt agttttgtat agttttagta     600
   tttggtgtat tttttttgat atttttttta gggatagtta ggtattttgt gtggggtatt     660
   taagagagtt aggtttgtta gtttttagtt tttgttagaa tgtaggtttg aggggtgagg     720
   ggtggggtag gggtagggat aggaattttg gtgtgttttt tatttgtaaa ggtttattga     780
   ggttttgagt tttagttatt gagttattaa gttagtttgg gttaggtttg ggtgttttgt     840
   ttgtaatgga ggtagagatg gggttttggg gtagttttga ggatgttggg tgtatagtgg     900
   gggttttgtt ggtaggaatt atttatgttt ttttttgggt taagttttgt ggatgtttag     960
   tttggggttg tggggagttg gtaggttagt ggtagatatt ggtgggtaga tttagtgttt    1020
   ggtagaatag gtattaagga agtggtgatt ggagggaagt taagtgtatt taaatttttg    1080
   ggtgagttat tattgttggg ttttttatag ttgttgaaag tgagtaatag tgatgaaggt    1140
   ttgtgagttt ttgtgtgagt gagtgaatgg attagtagta gtttttaggt tgtggaagag    1200
   tgtttttttt ttgggatggg gatatttggt tatagtaatt tttaattttt tatttattta    1260
   ttgtttattg tagaggtatg tgggggtttt gttttttgta ggtaggagtg aggggtattt    1320
   ttgtgatgtg gtatttttgt gattgaggtt atgtgtgatt ggtgtaaggg taggaagtga    1380
   gttattggtt tgtattaggt gtgggggttt ttgtgagggt aggatttaaa gttggtttgg    1440
   ttttttggtt gtagtatttt tttttttttt gaattaggtt agagttttgg gatgggaggt    1500
   gttttgtaga ttattttttt tattaatttt tgttttttgt tttatttttg tggtgatttg    1560
   gtgaattgtt ggttttttgt tgtgtattga gtggggtagt gattttgatg tggtgttttt    1620
   tgttgttttt gttattgtta ttatttttgg tggtttagtt tttgtatttt ttatttttat    1680
   ggaggaatgt attaggtttt tttttttgga tgtatttttt atttatatgt ttttaaattt    1740
   tggtattttt tgtttttttt ttatttttat tttttttttt aggtttttag ataaagggga    1800
   agtggttgga tttttttaaa gggatagtgt tttattagtt tattgttgaa tttttttttt    1860
   taattttagt tttttagtta tagttaatta gtattagtag atagtttatg agtgatattt    1920
   atgtaggttt taggttgtgg agagtttttt gggtaggaaa tagtttttaa ggttttttat    1980
   tttatttagg ttttagtttt ttttatttgt ttttttttta gattgtggtt ttttggagtt    2040
   tggttttttt gtttttgtgt gattgatata tagtatttaa atagtggtag agtgggatgg    2100
   attttttagt ttgttttttg tgtgggtttg tattttgatt tagatatgtt tttttatagt    2160
   aggatttagg ggggtatatg tgtgtttgtg ggtttattgg ggtatttgta tttggtttat    2220
   tttatttttt agagagaggg ttttgttgtg ttatttagtt ggagtgtagt ggtgtaatta    2280
   tagtatattg tagtttttaa tttttgggtt taagtgattt ttttttttta gtttttttag    2340
   tagttgggag tataggattt attgtatttt ggttaatttt ttaataattt tttaagagat    2400
   ggggttttat tgtgttgttt aggttggttt taaatttttg gttttaagtg atttttttat    2460
   ttttgttttt tgaagtgttg agattatagg tatgagttat tatgtttatt ttagattgat    2520
   atttttatat ttgtttattt tggttgggta ggg                                 2553
<210> 62
   <211> 2553
   <212> DNA
   <213> Artificial Sequence
<220>
   <223> chemically treated genomic DNA (Homo sapiens)
<400> 62
   ttttgtttag ttaggatgaa taaatataga aatgttagtt tgggatgggt atggtggttt 60
   atgtttgtaa ttttagtatt ttaggaggtg aaggtgggag gattatttga ggttagaggt 120
   ttgaggttag tttgggtaat atagtaagat tttatttttt aaaaaattat taaaaaatta 180
   gttaggatat agtgggtttt gtatttttag ttattaggga ggttggggag ggaggattgt 240
   ttgagtttag gagttgaagg ttgtagtgtg ttatgattat attattgtat tttagttggg 300
   tgatatagta agattttttt tttaaaaaat aaaataaatt aaatgtgggt gttttagtga 360
   atttgtaggt atatatgtgt ttttttgggt tttgttgtgg gggggtatgt ttgggttagg 420
   gtatagattt atatagagaa taggttaggg ggtttgtttt gttttgttat tgtttgggtg 480
   ttatgtgttg gttatatagg gatagaagaa ttaggtttta aagggttata atttaggaga 540
   gaggtaggta ggaaagattg ggatttggat gagatgaggg attttaaggg ttgtttttta 600
   tttaggaaat tttttatagt ttggggtttg tatgggtatt atttatgggt tgtttgttaa 660
   tgttaattaa ttgtaattag ggaattgggg ttgaggaggg gagtttagta gtaagttggt 720
   gggatattgt ttttttaaga ggatttagtt attttttttt tgtttgggag tttaggggaa 780
   ggggtgggag taaaggggga gtagaaaatg ttagggtttg gaagtatgtg ggtgaggggt 840
   gtatttagga agggaggttt ggtgtatttt tttatggggg tggggaatgt ggaggttggg 900
   ttattggagg tggtggtggt ggtaggggtg gtaggagatg ttatgttagg gttattgttt 960
   tatttggtgt atagtagggg gttggtagtt tattggatta ttgtgggggt ggggtggagg 1020
   atggaagttg gtgagggggg tggtttatag ggtatttttt gttttagggt tttaatttaa 1080
   tttgaaaggg aaaggagtgt tgtagttggg aggttaggtt gattttgggt tttgtttttg 1140
   tagaagtttt tatgtttggt gtagattaat gatttgtttt ttgtttttat attgattata 1200
   tatgattttg gttatagggg tgttatatta taggagtgtt ttttattttt gtttgtagga 1260
   agtagggttt ttgtatattt ttgtagtggg tggtgggtgg gtgggggatt aggaattgtt 1320
   gtaattaagt gtttttattt tggggaggga atgttttttt ataatttgga aattgttatt 1380
   ggtttattta tttgtttatg tagaaattta taaattttta ttattgttgt ttatttttag 1440
   tagttgtgaa agatttggtg gtgatgattt atttgagggt ttgagtgtat ttggtttttt 1500
   tttggttatt atttttttga tgtttgtttt attagatatt aggtttgttt attagtgttt 1560
   gttattgatt tgttagtttt ttgtggtttt aggttgggta tttataaagt ttggtttagg 1620
   agagagtata agtgattttt gttggtgagg tttttgttgt gtatttagta tttttagaat 1680
   tgttttgaga ttttgttttt gtttttattg tagatagggt atttaggttt ggtttaggtt 1740
   gatttggtgg tttagtggtt ggggtttggg gttttagtga atttttgtgg atggagagta 1800
   tgttggagtt tttgtttttg tttttgtttt gttttttatt ttttaggttt gtattttggt 1860
   aggagttaga ggttgatggg tttggttttt ttgagtgttt tatatagagt gtttgattgt 1920
   ttttaagaag gatgttaagg gaggtgtgtt aggtattgag gttgtgtaga gttgttttgg    1980
   ggatagttag agaggaattt ttttggggga gagttatgtt ttggttttat tttagggtat    2040
   ttttttttga gagtagggta ggataaagtt aggttggggt aggagaaggg ggaggtattt 2100
   ggagggtatg aaaggggtgt tttgtagttt aggtggttgg ttgggttggg agatagtggt 2160
   ggtttttata atgataggag tggagaatta ttgttttatt tagggttata tttttggtta 2220
   ttaggtgtga agaaatagta ggtggaggat tggttttatg gggagattgt tttggaagga 2280
   tttatagttt tggtagtggt ttgtttttag gttgtagttg atagtaatta aggagtttta 2340
   gttttgtttt agagtttgga tttaggtggg ttggttttta gttttaggtt ttatattagg 2400
   ggttttgttt ggagtttagg ataagtaggg agggggattt ttatttattt agttttttgg 2460
   aggaagtggt gtttttttgg ggttgaagtt aatagtattt tttgtggtta tattgggggt 2520
   tttggaggtt taagggaggg taggggtggt tag 2553
<210> 63
   <211> 2001
   <212> DNA
   <213> Artificial Sequence
<220>
   <223> chemically treated genomic DNA (Homo sapiens)
<400> 63
   ggtaaaagtt tgtttggatt ttttggttat tagaaatatg agtatggtgg tggtttttag      60
   ttttttattt atgtttgggt ttaagagatt gggagtttag gtttattgat tttttgagaa     120
   agattaagat tttgtatttt agaaagaggt ttggggattt ttgttttgtg taagggtaga     180
   aggattagtt gtttttttga gtattttaat ttggaatttt ggtttgaagt tgagatagga     240
   gattggatgt gaggtttttt tagagttggt tttttttaaa taatttttaa aatttttaga     300
   ttttaggggt atgttgaaat tttttaaagt agtttaaaga atataatgag agttttaata     360
   ttttaggtgg tggtgtgttg gttttttgga gtggggtggg atgtggttgt gtggatttat     420
   gtgtataatt gtgtgggatg gggttatgtg gatttatgtg tataattgtg ggattttagt     480
   gttagtggga ttttagtgtt agtgggattt tagtgttagt gggattttag tgttagtggg     540
   attttagtgt tagtgggatt ttagtgttag tgggatttta gtgttagtgg gtttgtggtt     600
   tagtggagtg agtggagtgt tggtgatttg agtggagatt gtgttttgga tgttttagtt     660
   tagatgttaa gttatagttt gtgtagtagt agtaaagggg aaggggtagg agttgggtat     720
   agttggattt ggaggttgtg atttagggga aagtgtgggt ggttgattta gggtagttgt     780
   ggtggtgagg taggtgggtt ttttgttttt tggagttgtt tttttttata tttgtttttg     840
   gtgtttttag tagtttttat tttggttttt tgtggttatt gtgggatttg gtgttgttgt     900
   tagtttagtg gggagtgaat tagtgttttt ttttgttttt ggtttttttg atggtatgag     960
   gaatttttgt tttgttttat agatttttgg tttttgttga gtgtggtatt ggagtttgtt    1020
   ttgttagggt tttggaatta gagaaagttg ttttttggtt atttgaagtg ttggattttt    1080
   atagtgtttt ttagtttggg tgggagtggt ggttgtgttg ttgaaggttg gggtttttgg    1140
   tgtgaaaggg aggtagttgt agttttagtt ttattttaga agtggttttt gtattgttgt    1200
   ggtgggtgtt tttgggtttt gattttgtta gtgttgtggg gtagaggtat ttggagtttg    1260
   tagggtttag atttgggttg gaaaagtttt gttgattgta ggtaagtgtt tgggaggggt    1320
   ggttaggtga agttttggtg ttttattata tatttttggg ttttatgtta gttgtatttg    1380
   tggtattggg taggaaatgg tagggttgag gttgatttta ggagtataag ggagtttttt    1440
   attttttgtt tatatttgtt atttttagtt ttgtaattta ttttagatat atagaaagta    1500
   agtaggattg gtggggagat ggagtttaat aggaatattt tttagtagtg agtaggggtt    1560
   gtatgggatg tgggaggagt ttagaggagg tgtggagagt gtttgaggtt gggtgagtgt    1620
   ttagagggga gatagttgaa ttgggtttaa gaggtgttta gtgggtgttt gttgaatgaa    1680
   tgagtgatgg gttttgaagt ttgagtgtat tgaaagaggg ggtgtgtaaa aagggttttt    1740
   tttattatat aggatatagt atatgtaaat tttttttttg tggaaaagtt agataggtta    1800
   aaaaggttat aaataaatta gttgggtatg gtggtgtgtg tttgtagttt tagttattag    1860
   ggaggttgag ttaggggaat tgtttgaatt tgggaggtgg agattgtagt gagttaagat    1920
   tgtgttattg tattttagtt tggaaataga gtgagatttt gttttggaaa aaaaaaaaaa    1980
   aagttataaa ttgtgtgtgg g                                              2001
<210> 64
   <211> 2001
   <212> DNA
   <213> Artificial Sequence
<220>
   <223> chemically treated genomic DNA (Homo sapiens)
<400> 64
   tttatatatg gtttgtaatt tttttttttt ttttttgaga tggagttttg ttttgttttt      60
   aggttggagt gtagtggtgt gattttggtt tattgtaatt tttgtttttt gggtttaagt     120
   gatttttttg gtttagtttt tttagtagtt gggattatag atgtgtatta ttatgtttgg     180
   ttaatttgtt tgtaattttt ttaatttgtt tggttttttt atagggagag gatttgtata     240
   tgttgtgttt tgtgtgatga aaggagtttt ttttatatat tttttttttt aatgtattta     300
   gattttaaag tttattattt atttatttaa taaatattta ttaagtattt tttgaatttg     360
   gtttaattat ttttttttta ggtatttatt taattttggg tattttttgt gttttttttg     420
   agtttttttt gtgttttata tagtttttgt ttattgttgg aaaatatttt tgttaagttt     480
   tgttttttta ttagttttgt ttgttttttg tgtgtttggg ataggttgta aaattggagg     540
   tgataaatgt gggtaggaaa tggagggttt ttttatattt ttagggttgg ttttagtttt     600
   gttatttttt gtttaatatt gtggatgtaa ttggtatggg atttggaagt gtgtggtaaa     660
   gtgttggggt tttgtttggt tgtttttttt ggatgtttgt ttgtagttag tgaagttttt     720
   ttaatttagg tttgggtttt gtgagtttta ggtgtttttg ttttgtggtg ttggtgaagt     780
   tgaagtttga gaatgtttat tgtagtgatg tgaaggttgt ttttggggtg gggttgaggt     840
   tgtagttgtt ttttttttgt attaaggatt ttaattttta gtgatgtagt tgttgttttt     900
   gtttaggttg ggaggtattg tagggatttg atgttttagg tggttaaaga gtgatttttt     960
   ttgattttag ggttttggtg gggtaggttt tagtattgta tttggtggag gttgaaggtt    1020
   tgtggggtag gataggagtt ttttgtgttg ttggaagggt tgaggatgaa ggagggtgtt    1080
   aatttatttt ttattgggtt ggtggtaatg ttgaattttg tagtgattgt ggagggttaa    1140
   ggtgaaaatt gttgggggtg ttgagggtag gtgtggggag gggtggtttt agggagtaag    1200
   gagtttattt gttttgttgt tgtagttgtt ttgggttgat tgtttatgtt tttttttggg    1260
   ttatgatttt tggatttaat tgtgtttggt ttttgttttt ttttttttgt tgttgttgtg    1320
   tgggttgtaa tttgatgttt aggttggggt gtttagggtg tagtttttgt ttaggttgtt    1380
   agtgttttat ttgttttatt gggttataga tttgttggtg ttggggtttt gttggtgttg    1440
   gggttttgtt ggtgttgggg ttttgttggt gttggggttt tgttggtgtt ggggttttgt    1500
   tggtgttggg gttttgttgg tgttggggtt ttgtggttgt gtatgtgagt ttgtgtggtt    1560
   ttgttttgtg tggttgtgta tgtgagtttg tgtggttgtg ttttgttttg ttttagggag    1620
   ttagtgtgtt gttatttggg atgttaggat ttttgttgtg ttttttggat tgttttgggg    1680
   gattttggtg tatttttagg atttaggagt tttggaagtt gtttgagaga aattagtttt    1740
   gggagggttt tgtatttagt tttttgtttt ggttttggat tggggttttg ggttaaggtg    1800
   tttagaggaa tagttgattt ttttattttt gtgtagggta gagattttta aatttttttt    1860
   taaaatgtag ggttttagtt ttttttaggg agttagtgaa tttagatttt tagttttttg    1920
   agtttaagta tgaataggga attggggatt attattatgt ttatattttt ggtggttagg    1980
   aagtttaggt aggtttttgt t                                              2001
<210> 65
   <211> 219909
   <212> DNA
   <213> Artificial Sequence
<220>
   <223> chemically treated genomic DNA (Homo sapiens)
<400> 65
<210> 66
   <211> 219909
   <212> DNA
   <213> Artificial Sequence
<220>
   <223> chemically treated genomic DNA (Homo sapiens)
<400> 66
<210> 67
   <211> 1405
   <212> DNA
   <213> Artificial Sequence
<220>
   <223> chemically treated genomic DNA (Homo sapiens)
<400> 67
   tgttatttga gttgtaaagg gtggtttttt gtgtttgttt tgttgtattg atatattgag      60
   ttgtgtatgg tgtttagtgt agggagaata aatgattatt tgtttaatgt gtttatttat     120
   aggtgaggaa attaaggttt taatttaatt gatgtatttt gtttttttga ttattagaaa     180
   agtagtagga taggtgtttt gttttgtttt attttggttt attaagttgg tattttggtt     240
   ttgatttttg gttgtgtttg gtgttgttgt ggtgtttggt gttgttgttt tgtttggtgg    300
   ggttgtttgg agtgtttgta tttttgtttg tttttatttg gttgggtttg ttttgtttgg     360
   atttgggatt gggaagtgtg gtgatttttg gaattagtta ttggtgttag tgtggggagt     420
   tgggggtgta gagttgtggg tgtggtgggt tatgtaggtg gtttttattt ttggtttggt     480
   ttggtttggt ttggtttgtg ttgttgtgtg ggggtgtttt tttttaggtt tggtgtttgt     540
   tagttttgtt ttgttaggtg tagtgtagtg taggggtggg tgggggtggg gtttggtgtg     600
   tatgtttatg gggtggggag ggggtgggtt aggggtggga ttatagttgg ttgggttggg     660
   gttttatgtg tatttttggg gaggggtggg gtgggggtgg ggttggggtg gggtttggtt     720
   ggtgtatttt agatggtggg ttgttttttt tttttgtttt tttattattg gtttaggatt     780
   agtgttttgg gagtgtgtgt tttgttgttt tgttgttata tttttttggg agtggtggtt     840
   atggaggtat tatgaagaag ttttatttag gtgggttttg tgtttggggt ggggaggggt     900
   tggtgttttg ggattagtgt tgtttatttg agtgtttgtg gttgggagtg gtgaggtgtt     960
   tttggagttg agtgagtttt tgtggtgggt atattgtagg ttgagttttt tttaggatag    1020
   ggttgttgtt gggttgtttt tgatttgagt tgattgtttt ttgtgttgtt tttagttttt    1080
   gtttgagtgt tggaggggtt gttttggggg atgttttttt ttttttgttt tttgtatttt    1140
   tgtaggaatt gttgattttt taggttggtt gggtgttttg ggtttttgtg tgtttgtgtg    1200
   ggtgaatggg gttggggtta ggtggagggg tgtttttggg tttagttttt agggttggtg    1260
   gtttaggttg tagtattttt tttttggatt ttttttggtt ttttttttat ttagtggggt    1320
   atgggatggt ttttagatgg gattgtttag tagtgtttaa atttggtgat ttgggtttat    1380
   gttttgtgtt taggatgttg tttgt                                          1405
<210> 68
   <211> 1405
   <212> DNA
   <213> Artificial Sequence
<220>
   <223> chemically treated genomic DNA (Homo sapiens)
<400> 68
   gtgggtggtg ttttgagtgt aaaatgtgaa tttgagttgt taagtttggg tgttgttgga      60
   tggttttatt tggaggttgt tttgtgtttt attaagtaga ggagaaattg aagagaattt     120
   gaagaaagga tgttgtggtt tggattatta gttttagagg ttgaatttaa ggatattttt     180
   ttatttagtt ttgattttat ttatttatat agatgtatag ggatttaaag tatttggttg     240
   atttggaaag ttagtgattt ttgtgagggt gtaaggggtg aggaagaggg agtgtttttt     300
   agggtagttt ttttgatatt tgagtaaggg ttggagatag tgtaggggat ggttggttta     360
   ggttgaaagt ggtttgatag tggttttgtt ttgggaggaa tttgatttgt agtgtgtttg     420
   ttgtggggat ttgtttagtt ttgggggtgt tttgttattt ttggttgtag gtatttaggt     480
   gagtagtgtt ggttttggga tattgatttt tttttatttt gggtgtgaag tttatttgag     540
   taagattttt ttatggtgtt tttgtggttg ttgtttttag gaaagtgtgg tggtgaggta     600
   gtggggtgtg tgtttttagg gtgttaattt tgggttggta gtaggaaggt gggaagaggg     660
   ggtggtttgt tgtttggagt gtattgattg ggttttgttt tggttttgtt tttgttttgt     720
   ttttttttgg agatgtgtat agaattttgg tttagttggt tgtggttttg tttttgattt     780
   gttttttttt tgttttgtga atgtgtgtgt tgagttttat ttttgtttat ttttgtgttg     840
   tgttgtattt ggtggagtgg ggttggtggg tgttgggttt gggagggggt gtttttgtgt     900
   ggtagtgtag attagattag attaggttag gttggggtgg ggggttgttt gtgtggtttg     960
   ttgtgtttgt agttttgtat ttttagtttt ttgtgttggt gttaatggtt ggttttggga     1020
   gttgttgtat tttttagttt tgagtttggg tggggtgggt ttggttaggt ggaagtgggt    1080
   ggaggtgtgg gtgttttggg tggttttgtt gggtgaggtg gtggtgttgg gtattgtggt    1140
   ggtgttgggt atagttgggg gttggagttg gggtgttggt ttagtgggtt ggggtagggt    1200
   gggataggat atttgttttg ttattttttt ggtaattaaa agggtagagt gtgttgattg    1260
   agttggagtt ttagtttttt tatttgtaaa tgggtgtgtt ggatagatga ttatttgttt    1320
   tttttgtgtt gggtattgtg tgtagtttgg tgtattggtg tagtggggta gatataggga    1380
   gttgtttttt ataatttggg taatg                                          1405
<210> 69
   <211> 1818
   <212> DNA
   <213> Artificial Sequence
<220>
   <223> chemically treated genomic DNA (Homo sapiens)
<400> 69
   tgttgtttgt ttttggtttt tttgatagtt gtgttttatt tttgttttgt gttttttttt      60
   ttggatagtg ttttttttag ggtttattta ggagggtgta gtggtggttt ttggggtggt    120
   ggttgtggtg ggggtgttag ttgtaggggt gtttttggtg ggtgggagtt ggtggttttt     180
   tgttggtgtt atgggatttg tatgtttgtt ttgtgttttt tggtttttga gtttataggt     240
   tgggattttg tttgttagtt gtgtgtgttg ttgtttaatt tttgtaggtg tagagtgtgt     300
   ggtggtggtg atagagaatt ttgtttggtt gtttaaatat agttttttgt agaaggattt    360
   tgtgtttggg gaaggggagg aatttttttt tttttgggtg tttgtttttt ttgttatggt   420
   ttggttttta tatttgttta tatttggttg tagtggggtg tttgggggga ggggttgagg     480
   ttgtgttttt tgttgttttt tgggtgtggg ttaggtgggg aggagggggg tgttttggtt     540
   gtgtgtttag gattgttttt tagtggttat ttgggtttta gttttttagg tttggttttg   600
   ataggtgggt ggagtagtta gtgtgagata gggaggttgg tgtgggtgtg ggaatttgat     660
   ttgtttggga ggtgggggtg gggtgggggt gtagtgtgtg gggaggggtt ggtgtttgtt     720
   tttttttttt atttatttag ttgagttagg gggtttaggg gtttttttgg tggttagttt   780
   tgtattgtag gagtgtgggt gtggtgtttt agttagtgtg tagggtttgg gttttgttgg     840
   gggtgttttt ttgttgttgt tttttgtgtg atttgttgtt tattagttat tatgttggat     900
   tttgtggtta atgtgtagtt ggatgggatt attttggatt ttgaaggtgg gtgttgggtt     960
   ggttgttgtg gttgtggatg tgttggagag gattttgtgg gtgggtttgg tgtgggatgg    1020
   gggtgtgttg aggggagatg ggagtgtgtt gaggggagat gggattttta atttaggtgt    1080
   ttttttgttg agagtgttgt gtgtttttgg ttttgtgttt gtgttgttta tgtgggggat    1140
   tttgttaggg gtatttgtgt agattttgtg tgtttttata ggattttgtg tttgttttgt    1200
   gtattgttgt ttgggttttt ttttttttat tgttgtttgt gtttgttaag tgatagtgat    1260
   ttttttgagg gtttgtgagg ttgttttgga attttttagg atgtatagtt ttattttggg    1320
   aaatttattg gttttttttt tttggttttt tttggtggtt tttgggtttt gtttggattt    1380
   ggtaatggga tagggaggtt gttttttatt tttgattgag tggatagttg tgttttgttt    1440
   gggtggatag tttttttttt ttttatgtta gttttggggt tgttaagttg tgtagtttgt    1500
   gggttgggag tattgaatgg atatagttta ggttgtggta gggtttagag tgggatgttt    1560
   tatggttttt atttaggttt ggggatattt ttatttgttt tttagaattg ggttgtgggg    1620
   gatagaaggg gtttgtgtgt gggtagggag agtattttgg tttttttttg tttttggggt    1680
   ttataaagtg tgttgggatt tgtggggttg ttttgtttaa gtttgggttt ggtgtttgtg    1740
   tttttgagtt tgtgagtgtg tgtgtttttt tgtgtttttt tgattgttgg tgttggggtt    1800
   ttgtgttttg tgtttgtg                                                  1818
<210> 70
   <211> 1818
   <212> DNA
   <213> Artificial Sequence
<220>
   <223> chemically treated genomic DNA (Homo sapiens)
<400> 70
   tgtgaatgta aaatgtaaaa ttttaatatt gataattaaa aaaatgtaaa aaaatgtatg      60
   tatttataaa tttaaaaatg tgaatgttaa atttaaattt aaataaaata attttgtaaa     120
   ttttaatata ttttataaat tttgaaaata aaaaaaaatt aaaatatttt ttttatttgt     180
   atgtaaattt tttttatttt ttatgatttg attttaaaaa atgaataaaa atatttttaa     240
   atttaaataa aaattataaa atattttatt ttaaatttta ttatgattta aattatattt     300
   gtttgatatt tttgatttat gaattatata atttaatgat tttgaaatta atgtaaaaaa     360
   aaaaaaaatt atttatttga ataaaatgtg attatttatt taattgaaaa taaaaaatga     420
   tttttttatt ttgttattga atttaaatga aatttgaaaa ttgttgaaaa aaattaaaaa     480
   aaaaaaattg ataaattttt taaaataaaa ttatatgttt taaaaaattt tgaaataatt     540
   ttgtgaattt ttgaaaaaat tgttattgtt taataaatat aaataataat aaaaaaaaaa     600
   aaatttaaat gataatatgt aaaatgaata taaaatttta taaaaatgtg taaaatttat     660
   gtgaatattt ttaataaaat tttttatgta aatgatgtga atatgaaatt gaaaatgtgt     720
   gatgttttta atgaaaaaat gtttaaatta aaaattttgt ttttttttaa tgtatttttg     780
   ttttttttta atgtattttt gttttgtgtt aaatttattt gtaaaatttt ttttaatatg     840
   tttgtgattg taataattaa tttaatattt atttttgaaa tttgaaataa ttttatttaa     900
   ttatgtgtta attgtgaaat ttgatataat aattaataaa taatgaattg tgtgaaaaat     960
   aatgatgaaa aaatgttttt gatgaaattt gaattttatg tgttaattaa aatgttgtgt    1020
   ttgtgttttt ataatataaa attaattgtt gaaaaaattt ttaaattttt taatttaatt    1080
   aaataaataa aaaaaaaaaa tgaatgttga tttttttttg tgtgttatgt ttttgttttg    1140
   tttttgtttt ttgaatgaat taaatttttg tatttgtatt gattttttta ttttgtatta    1200
   attattttgt ttgtttatta aaattaaatt taaaaaatta aaatttgaat aattgttaaa    1260
   aaataatttt aaatatatga ttgaaatgtt tttttttttt ttgtttaatt tgtgtttaaa    1320
   aaatgatgaa aaatgtgatt ttaatttttt tttttgaatg ttttgttatg attaaatata    1380
   aatgaatata aaaattgaat tataatgaaa aaaatgaatg tttaaaaaaa aaaaaatttt    1440
   tttttttttt tgaatgtaaa atttttttat aaaaaattat atttaaataa ttaaataaaa    1500
   ttttttatta ttgttgttgt gtgttttatg tttataaaaa ttaaatgata atgtatgtga    1560
   ttaataaata aaattttgat ttataaattt aaaaattgaa aaatgtaaaa tgaatatatg    1620
   aattttataa tattaatgaa aaattattaa tttttattta ttgaaaatat ttttataatt    1680
   aatattttta ttatgattat tgttttgaaa attattgtta tattttttta aataaatttt    1740
   aaaaaaaata ttatttgaaa aaaaaaatat aaaatgaaaa taaaatatga ttattaaaaa    1800
   aattaaaaat gaatgatg                                                  1818
<210> 71
   <211> 20
   <212> DNA
   <213> Artificial Sequence
<220>
   <223> chemically treated genomic DNA (Homo sapiens)
<400> 71
   ggagtggagg aaattgagat 20
<210> 72
   <211> 22
   <212> DNA
   <213> Artificial Sequence
<220>
   <223> chemically treated genomic DNA (Homo sapiens)
<400> 72
   ccacacaaca aatactcaaa ac 22
<210> 73
   <211> 33
   <212> DNA
   <213> Artificial Sequence
<220>
   <223> chemically treated genomic DNA (Homo sapiens)
<400> 73
   tgggtgtttg taatttttgt tttgtgttag gtt 33
<210> 74
   <211> 23
   <212> DNA
   <213> Artificial Sequence
<220>
   <223> chemically treated genomic DNA (Homo sapiens)
<400> 74
   tttcgtttcg gtttttagcg tag 23
<210> 75
   <211> 17
   <212> DNA
   <213> Artificial Sequence
<220>
   <223> chemically treated genomic DNA (Homo sapiens)
<400> 75
   aaccaaccgc gactccg 17
<210> 76
   <211> 22
   <212> DNA
   <213> Artificial Sequence
<220>
   <223> chemically treated genomic DNA (Homo sapiens)
<400> 76
   cgatttcaac cgcgcgatcg aa 22
<210> 77
   <211> 16
   <212> DNA
   <213> Artificial Sequence
<220>
   <223> chemically treated genomic DNA (Homo sapiens)
<400> 77
   cgtggtttcg gggacg 16
<210> 78
   <211> 18
   <212> DNA
   <213> Artificial Sequence
<220>
   <223> chemically treated genomic DNA (Homo sapiens)
<400> 78
   cgtctacacg cgaaacgc 18
<210> 79
   <211> 25
   <212> DNA
   <213> Artificial Sequence
<220>
   <223> chemically treated genomic DNA (Homo sapiens)
<400> 79
   cacatcgaaa tcgtacgcgc tctcg 25
<210> 80
   <211> 19
   <212> DNA
   <213> Artificial Sequence
<220>
   <223> chemically treated genomic DNA (Homo sapiens)
<400> 80
   caccttcccg aaaaacgaa 19
<210> 81
   <211> 27
   <212> DNA
   <213> Artificial Sequence
<220>
   <223> chemically treated genomic DNA (Homo sapiens)
<400> 81
   atggtagtag agatttcgag taaacgg 27
<210> 82
   <211> 28
   <212> DNA
   <213> Artificial Sequence
<220>
   <223> chemically treated genomic DNA (Homo sapiens)
<400> 82
   cgaatccgac tctaacgaaa cgctacga 28
<210> 83
   <211> 22
   <212> DNA
   <213> Artificial Sequence
<220>
   <223> chemically treated genomic DNA (Homo sapiens)
<400> 83
   gaagtagtcg gggtcgttta cg 22
<210> 84
   <211> 20
   <212> DNA
   <213> Artificial Sequence
<220>
   <223> chemically treated genomic DNA (Homo sapiens)
<400> 84
   gcaaaatacg cgaaaaccgt 20
<210> 85
   <211> 23
   <212> DNA
   <213> Artificial Sequence
<220>
   <223> chemically treated genomic DNA (Homo sapiens)
<400> 85
   acgtcttctc tcgccccgaa cga 23
<210> 86
   <211> 20
   <212> DNA
   <213> Artificial Sequence
<220>
   <223> chemically treated genomic DNA (Homo sapiens)
<400> 86
   tcgtgtgtgt cgtttttcgg 20
<210> 87
   <211> 19
   <212> DNA
   <213> Artificial Sequence
<220>
   <223> chemically treated genomic DNA (Homo sapiens)
<400> 87
   attcccgacg actccgaat 19
<210> 88
   <211> 33
   <212> DNA
   <213> Artificial Sequence
<220>
   <223> chemically treated genomic DNA (Homo sapiens)
<400> 88
   caacgaaaac gaaaaaatac gactacgtct cga 33
<210> 89
   <211> 20
   <212> DNA
   <213> Artificial Sequence
<220>
   <223> chemically treated genomic DNA (Homo sapiens)
<400> 89
   tttgaagcgg ttttcgtatc 20
<210> 90
   <211> 20
   <212> DNA
   <213> Artificial Sequence
<220>
   <223> chemically treated genomic DNA (Homo sapiens)
<400> 90
   ccgaacgctt acctacaatc 20
<210> 91
   <211> 22
   <212> DNA
   <213> Artificial Sequence
<220>
   <223> chemically treated genomic DNA (Homo sapiens)
<400> 91
   ttgcggtggg cgttttcggg tt 22
<210> 92
   <211> 19
   <212> DNA
   <213> Artificial Sequence
<220>
   <223> chemically treated genomic DNA (Homo sapiens)
<400> 92
   tttgtagtcg cgtcgtcgt 19
<210> 93
   <211> 20
   <212> DNA
   <213> Artificial Sequence
<220>
   <223> chemically treated genomic DNA (Homo sapiens)
<400> 93
   ctaactaaaa cgcgccgaaa 20
<210> 94
   <211> 22
   <212> DNA
   <213> Artificial Sequence
<220>
   <223> chemically treated genomic DNA (Homo sapiens)
<400> 94
   tcgacgtaaa cttccccgcg ct 22
<210> 95
   <211> 20
   <212> DNA
   <213> Artificial Sequence
<220>
   <223> chemically treated genomic DNA (Homo sapiens)
<400> 95
   atttcggaaa gcgtttttcg 20
<210> 96
   <211> 25
   <212> DNA
   <213> Artificial Sequence
<220>
   <223> chemically treated genomic DNA (Homo sapiens)
<400> 96
   caactccgaa taaattacca acgac 25
<210> 97
   <211> 33
   <212> DNA
   <213> Artificial Sequence
<220>
   <223> chemically treated genomic DNA (Homo sapiens)
<400> 97
   ttacgataac acttacacga ccaaaacgac gaa 33
<210> 98
   <211> 21
   <212> DNA
   <213> Artificial Sequence
<220>
   <223> chemically treated genomic DNA (Homo sapiens)
<400> 98
   cgtttttcgt tttattttcg c 21
<210> 99
   <211> 18
   <212> DNA
   <213> Artificial Sequence
<220>
   <223> chemically treated genomic DNA (Homo sapiens)
<400> 99
   gacaaaaaac gccacgtc 18
<210> 100
   <211> 22
   <212> DNA
   <213> Artificial Sequence
<220>
   <223> chemically treated genomic DNA (Homo sapiens)
<400> 100
   ccgacaattc accgaatcac cg 22
<210> 101
   <211> 22
   <212> DNA
   <213> Artificial Sequence
<220>
   <223> chemically treated genomic DNA (Homo sapiens)
<400> 101
   tcgcgagttt agttattcgt tg 22
<210> 102
   <211> 22
   <212> DNA
   <213> Artificial Sequence
<220>
   <223> chemically treated genomic DNA (Homo sapiens)
<400> 102
   ccactctcga aatatacgtc ga 22
<210> 103
   <211> 35
   <212> DNA
   <213> Artificial Sequence
<220>
   <223> chemically treated genomic DNA (Homo sapiens)
<400> 103
   cgataaacga aatctataat atacgaacgc gaaca 35
<210> 104
   <211> 17
   <212> DNA
   <213> Artificial Sequence
<220>
   <223> chemically treated genomic DNA (Homo sapiens)
<400> 104
   tagtcgtttt ggcggcg 17
<210> 105
   <211> 18
   <212> DNA
   <213> Artificial Sequence
<220>
   <223> chemically treated genomic DNA (Homo sapiens)
<400> 105
   aacgcacgac caacacga 18
<210> 106
   <211> 30
   <212> DNA
   <213> Artificial Sequence
<220>
   <223> chemically treated genomic DNA (Homo sapiens)
<400> 106
   aaaaaacacc gaacccaacg cgataataaa 30
<210> 107
   <211> 26
   <212> DNA
   <213> Artificial Sequence
<220>
   <223> chemically treated genomic DNA (Homo sapiens)
<400> 107
   gtagtagtta gtttagtatt tatttt 26
<210> 108
   <211> 24
   <212> DNA
   <213> Artificial Sequence
<220>
   <223> chemically treated genomic DNA (Homo sapiens)
<400> 108
   gatttagagt tgaatgtaaa gtaa 24
<210> 109
   <211> 18
   <212> DNA
   <213> Artificial Sequence
<220>
   <223> chemically treated genomic DNA (Homo sapiens)
<400> 109
   ttttttggag ttgaaagg 18
<210> 110
   <211> 20
   <212> DNA
   <213> Artificial Sequence
<220>
   <223> chemically treated genomic DNA (Homo sapiens)
<400> 110
   ttaggaaatg aaattggaga 20
<210> 111
   <211> 20
   <212> DNA
   <213> Artificial Sequence
<220>
   <223> chemically treated genomic DNA (Homo sapiens)
<400> 111
   aaacccaaac ctaaattaaa 20
<210> 112
   <211> 17
   <212> DNA
   <213> Artificial Sequence
<220>
   <223> chemically treated genomic DNA (Homo sapiens)
<400> 112
   cccaccaacc atcatat 17
<210> 113
   <211> 18
   <212> DNA
   <213> Artificial Sequence
<220>
   <223> chemically treated genomic DNA (Homo sapiens)
<400> 113
   ctaaaacctc aacctaac 18
<210> 114
   <211> 23
   <212> DNA
   <213> Artificial Sequence
<220>
   <223> chemically treated genomic DNA (Homo sapiens)
<400> 114
   aaattactac catctccaac tac 23
<210> 115
   <211> 23
   <212> DNA
   <213> Artificial Sequence
<220>
   <223> chemically treated genomic DNA (Homo sapiens)
<400> 115
   tataaaaaat tacttcccac atc 23
<210> 116
   <211> 17
   <212> DNA
   <213> Artificial Sequence
<220>
   <223> chemically treated genomic DNA (Homo sapiens)
<400> 116
   ggaagtgtgt ggtaaag 17
<210> 117
   <211> 32
   <212> DNA
   <213> Artificial Sequence
<220>
   <223> chemically treated genomic DNA (Homo sapiens)
<400> 117
   accatcatat caaaccccac aatcaacaca ca 32
<210> 118
   <211> 31
   <212> DNA
   <213> Artificial Sequence
<220>
   <223> chemically treated genomic DNA (Homo sapiens)
<400> 118
   cctaacatct tctctcaccc caaacaaaac a 31
<210> 119
   <211> 31
   <212> DNA
   <213> Artificial Sequence
<220>
   <223> chemically treated genomic DNA (Homo sapiens)
<400> 119
   caactacaac ccaacccaat ctacaaacat t 31
<210> 120
   <211> 37
   <212> DNA
   <213> Artificial Sequence
<220>
   <223> chemically treated genomic DNA (Homo sapiens)
<400> 120
   ccacatcaaa atcatacaca ctctcaaaca aaaaaca 37
<210> 121
   <211> 28
   <212> DNA
   <213> Artificial Sequence
<220>
   <223> chemically treated genomic DNA (Homo sapiens)
<400> 121
   taaagtgttg gggttttgtt tggttgtt 28
<210> 122
   <211> 25
   <212> DNA
   <213> Artificial Sequence
<220>
   <223> chemically treated genomic DNA (Homo sapiens)
<400> 122
   gttcgaaatg attttattta gttgc 25
<210> 123
   <211> 23
   <212> DNA
   <213> Artificial Sequence
<220>
   <223> chemically treated genomic DNA (Homo sapiens)
<400> 123
   aacgaaacaa ataccgtaaa cga 23
<210> 124
   <211> 22
   <212> DNA
   <213> Artificial Sequence
<220>
   <223> chemically treated genomic DNA (Homo sapiens)
<400> 124
   ggttgggttt tttaacgttc gt 22
<210> 125
   <211> 16
   <212> DNA
   <213> Artificial Sequence
<220>
   <223> chemically treated genomic DNA (Homo sapiens)
<400> 125
   ggtttcgggg acgcgt 16
<210> 126
   <211> 23
   <212> DNA
   <213> Artificial Sequence
<220>
   <223> chemically treated genomic DNA (Homo sapiens)
<400> 126
   aaacaaacgt ccgaaaaaaa cga 23
<210> 127
   <211> 17
   <212> DNA
   <213> Artificial Sequence
<220>
   <223> chemically treated genomic DNA (Homo sapiens)
<400> 127
   cgttgatcgc ggggttc 17
<210> 128
   <211> 24
   <212> DNA
   <213> Artificial Sequence
<220>
   <223> chemically treated genomic DNA (Homo sapiens)
<400> 128
   ccgactactt ctacatttcg aacg 24
<210> 129
   <211> 20
   <212> DNA
   <213> Artificial Sequence
<220>
   <223> chemically treated genomic DNA (Homo sapiens)
<400> 129
   atcgggtcgg gttgtagttg 20
<210> 130
   <211> 17
   <212> DNA
   <213> Artificial Sequence
<220>
   <223> chemically treated genomic DNA (Homo sapiens)
<400> 130
   ttcgttcgag agcgcgt 17
<210> 131
   <211> 18
   <212> DNA
   <213> Artificial Sequence
<220>
   <223> chemically treated genomic DNA (Homo sapiens)
<400> 131
   caaacgaaac cccgacgc 18
<210> 132
   <211> 4333
   <212> DNA
   <213> Homo Sapiens
<400> 132
   atgtatatct taatgaatta tagaaagcta tctaaatgaa ctaattcatg ctagggtaca 60
   gatacacttc attcatttta aaagatgagc ttcttatcaa aaaggaattt ctaattagtg 120
   gcattacaac tttgaagcca acatggtatg tatattcaac caatgtaata aaacactcca 180
   gttctgatat gagacaaatg tttctaatca ttttcgggct gaaattctac cctagagaag 240
   cttgctttat gaaacaatca ggaatgagat ttcaggaaat tatgagaact tgacatgatc 300
   ctttgaattg atcttcgaat gagttgtctt tcatttattt tctatgggga attttaggat 360
   aatgaaggag caaatatttc tccttttgaa atgtatgttt ttgttcatta ctgttcttgg 420
   gaggggtgaa gaataaggtg gttcgtgtct aggagtctgc aagtacataa gcctactaca 480
   tacagtgctt tgggtaaaaa ctgacatcaa atttgctttg gcatacacct gtattgtcct 540
   caacaactgc cttaaaattt ggctttccca gtcattaccc tcactcagca tctccttgat 600
   ctctcctctc catattcaaa cacattttga aggcttcctg tcttctctct ctctctctct 660
   ctctctctct gtatacacac cccattctca gggaagaaaa ctatcttgtt aacctgacaa 720
   ccttacctaa acttattgga gagagcagaa ttcaaaccct atggcaatct tttggcaatg 780
   tcctggagga tttgtgtccc caagacaggg atttcagaaa ggggaatata caaatctagc 840
   agtaagatac tttttctcag caggagtttc tagtctcttt caaatacctt gcaatagagg 900
   atggcttgct ccaatacaca cccgcggttc tatgagtctg tactgaagtc ggatatccat 960
   aatattttcc ccaacatcct atcatttttc tcccttgaca accatgtttt gagaacttga 1020
   agcaacagat atgaagagtc ttaagggatt gcatcaatgt acgctaagat tagtctctac 1080
   cacatagtcc aatgacacgg ttttaggttc agaagggatg ctctacaccc acagaatccc 1140
   accgagttca ggtgccagct cagagaactc atcaccttcc tgtttccctt caagagttaa 1200
   ctgatgggat agtatgcaaa gcacatgcgt tcttgctgcc aaaatagcag ccaaaactgc 1260
   ctgcctagat gtgtaagatc ccatgaaatg tcttggcaaa agctagttta ggagtgttgg 1320
   ccaaggtgct gaagcgaagc aaggggagtc aggaccacct ttgccactga atctcccctc 1380
   ccccacctct ttttaaagac agcgagctta ttcattttga caagataccc taggatttca 1440
   aatctgacat aattttggag aaagcagtgc gtgtgtgtgt gtgtgtgtgt gtgtgtgtgt 1500
   gtggtgtaga agggtatctg ggatacataa tctttataga aattctagtg ccacaaaaat 1560
   tctaggagga tctacattta aaaaaacaca caacagttta agctgtttat tccaattgaa 1620
   cgctgtgtgt ttccatcaga tcctcagtga gattcagtcc aatgagtcaa ttgcaaaata 1680
   aagtttttgt caagggaatg gcagaaaatg acaaaaccac aattctgaat gatcattcaa 1740
   aagtaaaaag tactttctac tactgtgtgg agattttctt atctctactt ttactcgaat 1800
   tgaatctttt cttctaatgt tcttttgaac agtttgtctt tggtgtcctg aaaatgtgca 1860
   cgctgatagc agtaccacat gctgtccctg gcacagagtg agctaacagt tgaaccacct 1920
   ctgggtaact gcggactgca gctagtgtaa gtgcaagaga aggcaccctg gccaccaatt 1980
   cgtttcacac tccaatcacc cccgcccgct ctacaccccc tgggcacccg atgcctcttc 2040
   cctctgccag gcaatttgct tccgttctgt tcaactttcc gacctctggc tttagagaat 2100
   agtcctggct gtgggaactg gctctgggaa gcttttggcg ccctcctcat tggtgactgg 2160
   caagagtaac actttcacac tcttaaaaag agccgtttag ttcctaatga tattttctgg 2220
   caaaaaagaa aatgaaacac acaacccgga ttccggtcca ccaaacgcag gccaagagcg 2280
   cggctgctga gccccggtct tgctgtgagg ctgttgttcc cactcgcttc ccgtttccac 2340
   tagagttgat ttgtctcttg ccacctctcc ccggcagtgt ggacctactg caacgcagga 2400
   acatctgggt tggtatggat aggatcctag catcagatgc tggctgctgc cccagggact 2460
   tcccgcggaa atcacccagc acttagaagc cgtttcaata aggaagtaac cagtaaagca 2520
   ttgctggtgg gagaaccccg cgaaacatct tagaaaaata tcagaaaccg ctggagacct 2580
   aatgacccac gtctcctcca gcctgtgttc ctcagccctt tctggaggag tattaagtgg 2640
   tttgtgggtt tttaccctgc ggtgggaggg ctgttccaag tacatctatt tctgcgatag 2700
   ttaacgtcgg ggggccccag ctctaaccag ggcgaggacc acggactgca tccctctctc 2760
   tcagcgcact ggtctgttta ctggagggtg agagggacgc gcagacctcc ggactcaggg 2820
   gttctgttct gtagatcccc ccaccccacc cggggtctct ggcactcgta tcacttagca    2880
   ccagtgtgac tggcggtgca ttccggcccc ctcgcgcccc attaaagccc ctgcacatcc    2940
   tctcccacgc tgcgcttctg ctacccacgc agctgctcct ctgagcgtcc cgcgtgcaca    3000
   aacgcgcccc cggtccttac gcgtcggtgg cgctgcctcg ctgaggtggc gccgggggag    3060
   cccgcagagg cggcggggga gggagtgcgg aggagaggac ccgtgagctg cggggacctg    3120
   ggcccgccga gcttttgggg aagctgggct ccgttccccc cgggcgcctg gggcgcgcgg    3180
   gaaggcgcgg aggacgcgcc gcactcaccc gctgctgctg ccggctgctg actgccaccg    3240
   cggcggcgga cgcggcgctg tgccggagct cggccgcctg cccaggtctc aacaggctcc    3300
   gggtgaacct gcgggtccgc tcaccggcca tcgccggtca cgccaatcgc gccgcgggcc    3360
   cggggcgcgc ctcccgccgg tcttccccgc gccaaccttc tctatccacc cgccgttcag    3420
   gaagcggaac tcggagcctt cccccccctc ttcctcgctc aactcgcaag aggcgggttt    3480
   ttctctcttg cacccccact cttcccaccc ccttccctcc tctgaagctt ctcgaagact    3540
   ttccaaactc tgcgctcccc cgggcgcgcg cggccaactc ggctggcggc cgggccctgc    3600
   agcggcgcat ctggagcggg cgtcgcggga cgcgtgtggg gcggctcctg cccgcgccgc    3660
   ccacacgggc tcagctggcc ggccggggct gtccacgccg cccgggggaa ctgcaggctg    3720
   cgggggaggg cggggggcgg ccgccgaggg gctccgggcg gggcctgtcc aaggccgtcg    3780
   cctgtccaag gccgtcagcc tcatttggag gttgctggga gagttggagc cctggatgca    3840
   tttgccgttt agacttctat tttctgggtc cggctagtgg gtgggggcca agctcggttc    3900
   gcccttccac cctcgggaag gggtcagagg cttattaaag atctggtggc tgggatccca    3960
   gcagcatcgc ccaagccagc gtttggaagg ctcccttgcc agaagtccat gttcagggcc    4020
   ttggaataag cttccccatt cattcactcc ttcaatgagt atttattaag cgcctactat    4080
   atgccagctc acactgttct ggaaactgag gatacggaga ggaacttagc aactacactt    4140
   taaggctgtg tagagctgat aggttttttc ggggtagggg tgggagacag caagtaacca    4200
   agcgaacaaa taaacaaata cagagaaatc aaggagacag tttaagctac taacacgaac    4260
   tcaaaaccaa taaatcagcg ttctgggggt aagagacttt gggtggcatt aggatgagta    4320
   ggcaagaacc aac                                                       4333
<210> 133
   <211> 2333
   <212> DNA
   <213> Homo Sapiens
<400> 133
   accatgtttt gagaacttga agcaacagat atgaagagtc ttaagggatt gcatcaatgt      60
   acgctaagat tagtctctac cacatagtcc aatgacacgg ttttaggttc agaagggatg     120
   ctctacaccc acagaatccc accgagttca ggtgccagct cagagaactc atcaccttcc     180
   tgtttccctt caagagttaa ctgatgggat agtatgcaaa gcacatgcgt tcttgctgcc     240
   aaaatagcag ccaaaactgc ctgcctagat gtgtaagatc ccatgaaatg tcttggcaaa     300
   agctagttta ggagtgttgg ccaaggtgct gaagcgaagc aaggggagtc aggaccacct     360
   ttgccactga atctcccctc ccccacctct ttttaaagac agcgagctta ttcattttga     420
   caagataccc taggatttca aatctgacat aattttggag aaagcagtgc gtgtgtgtgt     480
   gtgtgtgtgt gtgtgtgtgt gtggtgtaga agggtatctg ggatacataa tctttataga     540
   aattctagtg ccacaaaaat tctaggagga tctacattta aaaaaacaca caacagttta     600
   agctgtttat tccaattgaa cgctgtgtgt ttccatcaga tcctcagtga gattcagtcc     660
   aatgagtcaa ttgcaaaata aagtttttgt caagggaatg gcagaaaatg acaaaaccac     720
   aattctgaat gatcattcaa aagtaaaaag tactttctac tactgtgtgg agattttctt     780
   atctctactt ttactcgaat tgaatctttt cttctaatgt tcttttgaac agtttgtctt     840
   tggtgtcctg aaaatgtgca cgctgatagc agtaccacat gctgtccctg gcacagagtg     900
   agctaacagt tgaaccacct ctgggtaact gcggactgca gctagtgtaa gtgcaagaga     960
   aggcaccctg gccaccaatt cgtttcacac tccaatcacc cccgcccgct ctacaccccc    1020
   tgggcacccg atgcctcttc cctctgccag gcaatttgct tccgttctgt tcaactttcc    1080
   gacctctggc tttagagaat agtcctggct gtgggaactg gctctgggaa gcttttggcg    1140
   ccctcctcat tggtgactgg caagagtaac actttcacac tcttaaaaag agccgtttag    1200
   ttcctaatga tattttctgg caaaaaagaa aatgaaacac acaacccgga ttccggtcca    1260
   ccaaacgcag gccaagagcg cggctgctga gccccggtct tgctgtgagg ctgttgttcc    1320
   cactcgcttc ccgtttccac tagagttgat ttgtctcttg ccacctctcc ccggcagtgt    1380
   ggacctactg caacgcagga acatctgggt tggtatggat aggatcctag catcagatgc    1440
   tggctgctgc cccagggact tcccgcggaa atcacccagc acttagaagc cgtttcaata    1500
   aggaagtaac cagtaaagca ttgctggtgg gagaaccccg cgaaacatct tagaaaaata    1560
   tcagaaaccg ctggagacct aatgacccac gtctcctcca gcctgtgttc ctcagccctt    1620
   tctggaggag tattaagtgg tttgtgggtt tttaccctgc ggtgggaggg ctgttccaag    1680
   tacatctatt tctgcgatag ttaacgtcgg ggggccccag ctctaaccag ggcgaggacc    1740
   acggactgca tccctctctc tcagcgcact ggtctgttta ctggagggtg agagggacgc    1800
   gcagacctcc ggactcaggg gttctgttct gtagatcccc ccaccccacc cggggtctct    1860
   ggcactcgta tcacttagca ccagtgtgac tggcggtgca ttccggcccc ctcgcgcccc    1920
   attaaagccc ctgcacatcc tctcccacgc tgcgcttctg ctacccacgc agctgctcct    1980
   ctgagcgtcc cgcgtgcaca aacgcgcccc cggtccttac gcgtcggtgg cgctgcctcg    2040
   ctgaggtggc gccgggggag cccgcagagg cggcggggga gggagtgcgg aggagaggac    2100
   ccgtgagctg cggggacctg ggcccgccga gcttttgggg aagctgggct ccgttccccc    2160
   cgggcgcctg gggcgcgcgg gaaggcgcgg aggacgcgcc gcactcaccc gctgctgctg    2220
   ccggctgctg actgccaccg cggcggcgga cgcggcgctg tgccggagct cggccgcctg    2280
   cccaggtctc aacaggctcc gggtgaacct gcgggtccgc tcaccggcca tcg           2333
<210> 134
   <211> 4333
   <212> DNA
   <213> Artificial Sequence
<220>
   <223> chemically treated genomic DNA (Homo sapiens)
<400> 134
   atgtatattt taatgaatta tagaaagtta tttaaatgaa ttaatttatg ttagggtata      60
   gatatatttt atttatttta aaagatgagt tttttattaa aaaggaattt ttaattagtg     120
   gtattataat tttgaagtta atatggtatg tatatttaat taatgtaata aaatatttta     180
   gttttgatat gagataaatg tttttaatta ttttcgggtt gaaattttat tttagagaag     240
   tttgttttat gaaataatta ggaatgagat tttaggaaat tatgagaatt tgatatgatt     300
   ttttgaattg attttcgaat gagttgtttt ttatttattt tttatgggga attttaggat     360
   aatgaaggag taaatatttt tttttttgaa atgtatgttt ttgtttatta ttgtttttgg     420
   gaggggtgaa gaataaggtg gttcgtgttt aggagtttgt aagtatataa gtttattata     480
   tatagtgttt tgggtaaaaa ttgatattaa atttgttttg gtatatattt gtattgtttt     540
   taataattgt tttaaaattt ggttttttta gttattattt ttatttagta tttttttgat     600
   tttttttttt tatatttaaa tatattttga aggttttttg tttttttttt tttttttttt     660
   tttttttttt gtatatatat tttattttta gggaagaaaa ttattttgtt aatttgataa     720
   ttttatttaa atttattgga gagagtagaa tttaaatttt atggtaattt tttggtaatg     780
   ttttggagga tttgtgtttt taagataggg attttagaaa ggggaatata taaatttagt     840
   agtaagatat ttttttttag taggagtttt tagttttttt taaatatttt gtaatagagg     900
   atggtttgtt ttaatatata ttcgcggttt tatgagtttg tattgaagtc ggatatttat     960
   aatatttttt ttaatatttt attatttttt ttttttgata attatgtttt gagaatttga    1020
   agtaatagat atgaagagtt ttaagggatt gtattaatgt acgttaagat tagtttttat    1080
   tatatagttt aatgatacgg ttttaggttt agaagggatg ttttatattt atagaatttt    1140
   atcgagttta ggtgttagtt tagagaattt attatttttt tgtttttttt taagagttaa    1200
   ttgatgggat agtatgtaaa gtatatgcgt ttttgttgtt aaaatagtag ttaaaattgt    1260
   ttgtttagat gtgtaagatt ttatgaaatg ttttggtaaa agttagttta ggagtgttgg    1320
   ttaaggtgtt gaagcgaagt aaggggagtt aggattattt ttgttattga attttttttt    1380
   ttttattttt ttttaaagat agcgagttta tttattttga taagatattt taggatttta    1440
   aatttgatat aattttggag aaagtagtgc gtgtgtgtgt gtgtgtgtgt gtgtgtgtgt    1500
   gtggtgtaga agggtatttg ggatatataa tttttataga aattttagtg ttataaaaat    1560
   tttaggagga tttatattta aaaaaatata taatagttta agttgtttat tttaattgaa    1620
   cgttgtgtgt ttttattaga tttttagtga gatttagttt aatgagttaa ttgtaaaata    1680
   aagtttttgt taagggaatg gtagaaaatg ataaaattat aattttgaat gattatttaa    1740
   aagtaaaaag tattttttat tattgtgtgg agattttttt atttttattt ttattcgaat    1800
   tgaatttttt tttttaatgt ttttttgaat agtttgtttt tggtgttttg aaaatgtgta    1860
   cgttgatagt agtattatat gttgtttttg gtatagagtg agttaatagt tgaattattt    1920
   ttgggtaatt gcggattgta gttagtgtaa gtgtaagaga aggtattttg gttattaatt    1980
   cgttttatat tttaattatt ttcgttcgtt ttatattttt tgggtattcg atgttttttt    2040
   tttttgttag gtaatttgtt ttcgttttgt ttaatttttc gatttttggt tttagagaat    2100
   agttttggtt gtgggaattg gttttgggaa gtttttggcg ttttttttat tggtgattgg    2160
   taagagtaat atttttatat ttttaaaaag agtcgtttag tttttaatga tattttttgg    2220
   taaaaaagaa aatgaaatat ataattcgga tttcggttta ttaaacgtag gttaagagcg    2280
   cggttgttga gtttcggttt tgttgtgagg ttgttgtttt tattcgtttt tcgtttttat    2340
   tagagttgat ttgttttttg ttattttttt tcggtagtgt ggatttattg taacgtagga    2400
   atatttgggt tggtatggat aggattttag tattagatgt tggttgttgt tttagggatt    2460
   tttcgcggaa attatttagt atttagaagt cgttttaata aggaagtaat tagtaaagta    2520
   ttgttggtgg gagaatttcg cgaaatattt tagaaaaata ttagaaatcg ttggagattt    2580
   aatgatttac gtttttttta gtttgtgttt tttagttttt tttggaggag tattaagtgg    2640
   tttgtgggtt tttattttgc ggtgggaggg ttgttttaag tatatttatt tttgcgatag    2700
   ttaacgtcgg ggggttttag ttttaattag ggcgaggatt acggattgta tttttttttt    2760
   ttagcgtatt ggtttgttta ttggagggtg agagggacgc gtagattttc ggatttaggg    2820
   gttttgtttt gtagattttt ttattttatt cggggttttt ggtattcgta ttatttagta    2880
   ttagtgtgat tggcggtgta tttcggtttt ttcgcgtttt attaaagttt ttgtatattt    2940
   ttttttacgt tgcgtttttg ttatttacgt agttgttttt ttgagcgttt cgcgtgtata    3000
   aacgcgtttt cggtttttac gcgtcggtgg cgttgtttcg ttgaggtggc gtcgggggag    3060
   ttcgtagagg cggcggggga gggagtgcgg aggagaggat tcgtgagttg cggggatttg    3120
   ggttcgtcga gtttttgggg aagttgggtt tcgttttttt cgggcgtttg gggcgcgcgg    3180
   gaaggcgcgg aggacgcgtc gtatttattc gttgttgttg tcggttgttg attgttatcg    3240
   cggcggcgga cgcggcgttg tgtcggagtt cggtcgtttg tttaggtttt aataggtttc    3300
   gggtgaattt gcgggttcgt ttatcggtta tcgtcggtta cgttaatcgc gtcgcgggtt    3360
   cggggcgcgt ttttcgtcgg tttttttcgc gttaattttt tttatttatt cgtcgtttag    3420
   gaagcggaat tcggagtttt tttttttttt tttttcgttt aattcgtaag aggcgggttt    3480
   tttttttttg tatttttatt ttttttattt tttttttttt tttgaagttt ttcgaagatt    3540
   ttttaaattt tgcgtttttt cgggcgcgcg cggttaattc ggttggcggt cgggttttgt    3600
   agcggcgtat ttggagcggg cgtcgcggga cgcgtgtggg gcggtttttg ttcgcgtcgt   3660
   ttatacgggt ttagttggtc ggtcggggtt gtttacgtcg ttcgggggaa ttgtaggttg    3720
   cgggggaggg cggggggcgg tcgtcgaggg gtttcgggcg gggtttgttt aaggtcgtcg    3780
   tttgtttaag gtcgttagtt ttatttggag gttgttggga gagttggagt tttggatgta    3840
   tttgtcgttt agatttttat tttttgggtt cggttagtgg gtgggggtta agttcggttc    3900
   gtttttttat tttcgggaag gggttagagg tttattaaag atttggtggt tgggatttta   3960
   gtagtatcgt ttaagttagc gtttggaagg tttttttgtt agaagtttat gtttagggtt   4020
   ttggaataag tttttttatt tatttatttt tttaatgagt atttattaag cgtttattat   4080
   atgttagttt atattgtttt ggaaattgag gatacggaga ggaatttagt aattatattt    4140
   taaggttgtg tagagttgat aggttttttc ggggtagggg tgggagatag taagtaatta   4200
   agcgaataaa taaataaata tagagaaatt aaggagatag tttaagttat taatacgaat    4260
   ttaaaattaa taaattagcg ttttgggggt aagagatttt gggtggtatt aggatgagta    4320
   ggtaagaatt aat                                                       4333
<210> 135
   <211> 4333
   <212> DNA
   <213> Artificial Sequence
<220>
   <223> chemically treated genomic DNA (Homo sapiens)
<400> 135
   gttggttttt gtttatttat tttaatgtta tttaaagttt tttattttta gaacgttgat      60
   ttattggttt tgagttcgtg ttagtagttt aaattgtttt tttgattttt ttgtatttgt     120
   ttatttgttc gtttggttat ttgttgtttt ttatttttat ttcgaaaaaa tttattagtt     180
   ttatatagtt ttaaagtgta gttgttaagt tttttttcgt atttttagtt tttagaatag     240
   tgtgagttgg tatatagtag gcgtttaata aatatttatt gaaggagtga atgaatgggg     300
   aagtttattt taaggttttg aatatggatt tttggtaagg gagtttttta aacgttggtt     360
   tgggcgatgt tgttgggatt ttagttatta gatttttaat aagtttttga ttttttttcg    420
   agggtggaag ggcgaatcga gtttggtttt tatttattag tcggatttag aaaatagaag     480
   tttaaacggt aaatgtattt agggttttaa tttttttagt aatttttaaa tgaggttgac     540
   ggttttggat aggcgacggt tttggatagg tttcgttcgg agtttttcgg cggtcgtttt     600
   tcgttttttt tcgtagtttg tagttttttc gggcggcgtg gatagtttcg gtcggttagt    660
   tgagttcgtg tgggcggcgc gggtaggagt cgttttatac gcgtttcgcg acgttcgttt     720
   tagatgcgtc gttgtagggt tcggtcgtta gtcgagttgg tcgcgcgcgt tcgggggagc     780
   gtagagtttg gaaagttttc gagaagtttt agaggaggga agggggtggg aagagtgggg     840
   gtgtaagaga gaaaaattcg ttttttgcga gttgagcgag gaagaggggg gggaaggttt    900
   cgagtttcgt tttttgaacg gcgggtggat agagaaggtt ggcgcgggga agatcggcgg     960
   gaggcgcgtt tcgggttcgc ggcgcgattg gcgtgatcgg cgatggtcgg tgagcggatt    1020
   cgtaggttta ttcggagttt gttgagattt gggtaggcgg tcgagtttcg gtatagcgtc    1080
   gcgttcgtcg tcgcggtggt agttagtagt cggtagtagt agcgggtgag tgcggcgcgt    1140
   ttttcgcgtt ttttcgcgcg ttttaggcgt tcggggggaa cggagtttag tttttttaaa  1200
   agttcggcgg gtttaggttt tcgtagttta cgggtttttt ttttcgtatt ttttttttcg    1260
   tcgtttttgc gggttttttc ggcgttattt tagcgaggta gcgttatcga cgcgtaagga   1320
   tcgggggcgc gtttgtgtac gcgggacgtt tagaggagta gttgcgtggg tagtagaagc    1380
   gtagcgtggg agaggatgtg taggggtttt aatggggcgc gagggggtcg gaatgtatcg    1440
   ttagttatat tggtgttaag tgatacgagt gttagagatt tcgggtgggg tggggggatt   1500
   tatagaatag aatttttgag ttcggaggtt tgcgcgtttt ttttattttt tagtaaatag    1560
   attagtgcgt tgagagagag ggatgtagtt cgtggttttc gttttggtta gagttggggt    1620
   ttttcgacgt taattatcgt agaaatagat gtatttggaa tagttttttt atcgtagggt    1680
   aaaaatttat aaattattta atattttttt agaaagggtt gaggaatata ggttggagga    1740
   gacgtgggtt attaggtttt tagcggtttt tgatattttt ttaagatgtt tcgcggggtt    1800
   tttttattag taatgtttta ttggttattt ttttattgaa acggttttta agtgttgggt    1860
   gattttcgcg ggaagttttt ggggtagtag ttagtatttg atgttaggat tttatttata    1920
   ttaatttaga tgtttttgcg ttgtagtagg tttatattgt cggggagagg tggtaagaga    1980
   taaattaatt ttagtggaaa cgggaagcga gtgggaataa tagttttata gtaagatcgg    2040
   ggtttagtag tcgcgttttt ggtttgcgtt tggtggatcg gaattcgggt tgtgtgtttt    2100
   attttttttt ttgttagaaa atattattag gaattaaacg gtttttttta agagtgtgaa    2160
   agtgttattt ttgttagtta ttaatgagga gggcgttaaa agttttttag agttagtttt   2220
   tatagttagg attatttttt aaagttagag gtcggaaagt tgaatagaac ggaagtaaat    2280
   tgtttggtag agggaagagg tatcgggtgt ttagggggtg tagagcgggc gggggtgatt    2340
   ggagtgtgaa acgaattggt ggttagggtg tttttttttg tatttatatt agttgtagtt    2400
   cgtagttatt tagaggtggt ttaattgtta gtttattttg tgttagggat agtatgtggt    2460
   attgttatta gcgtgtatat ttttaggata ttaaagataa attgtttaaa agaatattag    2520
   aagaaaagat ttaattcgag taaaagtaga gataagaaaa tttttatata gtagtagaaa    2580
   gtatttttta tttttgaatg attatttaga attgtggttt tgttattttt tgttattttt    2640
   ttgataaaaa ttttattttg taattgattt attggattga attttattga ggatttgatg    2700
   gaaatatata gcgtttaatt ggaataaata gtttaaattg ttgtgtgttt ttttaaatgt    2760
   agattttttt agaatttttg tggtattaga atttttataa agattatgta ttttagatat     2820
   ttttttatat tatatatata tatatatata tatatatata tacgtattgt tttttttaaa    2880
   attatgttag atttgaaatt ttagggtatt ttgttaaaat gaataagttc gttgttttta     2940
   aaaagaggtg ggggagggga gatttagtgg taaaggtggt tttgattttt tttgtttcgt     3000
   tttagtattt tggttaatat ttttaaatta gtttttgtta agatatttta tgggatttta    3060
   tatatttagg taggtagttt tggttgttat tttggtagta agaacgtatg tgttttgtat    3120
   attattttat tagttaattt ttgaagggaa ataggaaggt gatgagtttt ttgagttggt    3180
   atttgaattc ggtgggattt tgtgggtgta gagtattttt tttgaattta aaatcgtgtt    3240
   attggattat gtggtagaga ttaattttag cgtatattga tgtaattttt taagattttt    3300
   tatatttgtt gttttaagtt tttaaaatat ggttgttaag ggagaaaaat gataggatgt    3360
   tggggaaaat attatggata ttcgatttta gtatagattt atagaatcgc gggtgtgtat    3420
   tggagtaagt tattttttat tgtaaggtat ttgaaagaga ttagaaattt ttgttgagaa     3480
   aaagtatttt attgttagat ttgtatattt ttttttttga aatttttgtt ttggggatat   3540
   aaatttttta ggatattgtt aaaagattgt tatagggttt gaattttgtt ttttttaata   3600
   agtttaggta aggttgttag gttaataaga tagttttttt ttttgagaat ggggtgtgta    3660
   tatagagaga gagagagaga gagagagaga agataggaag tttttaaaat gtgtttgaat    3720
   atggagagga gagattaagg agatgttgag tgagggtaat gattgggaaa gttaaatttt    3780
   aaggtagttg ttgaggataa tataggtgta tgttaaagta aatttgatgt tagtttttat    3840
   ttaaagtatt gtatgtagta ggtttatgta tttgtagatt tttagatacg aattatttta    3900
   ttttttattt tttttaagaa tagtaatgaa taaaaatata tattttaaaa ggagaaatat   3960
   ttgttttttt attattttaa aattttttat agaaaataaa tgaaagataa tttattcgaa   4020
   gattaattta aaggattatg ttaagttttt ataatttttt gaaattttat ttttgattgt    4080
   tttataaagt aagttttttt agggtagaat tttagttcga aaatgattag aaatatttgt   4140
   tttatattag aattggagtg ttttattata ttggttgaat atatatatta tgttggtttt    4200
   aaagttgtaa tgttattaat tagaaatttt tttttgataa gaagtttatt ttttaaaatg    4260
   aatgaagtgt atttgtattt tagtatgaat tagtttattt agatagtttt ttataattta    4320
   ttaagatata tat                                                       4333
<210> 136
   <211> 2333
   <212> DNA
   <213> Artificial Sequence
<220>
   <223> chemically treated genomic DNA (Homo sapiens)
<400> 136
   attatgtttt gagaatttga agtaatagat atgaagagtt ttaagggatt gtattaatgt      60
   acgttaagat tagtttttat tatatagttt aatgatacgg ttttaggttt agaagggatg     120
   ttttatattt atagaatttt atcgagttta ggtgttagtt tagagaattt attatttttt     180
   tgtttttttt taagagttaa ttgatgggat agtatgtaaa gtatatgcgt ttttgttgtt      240
   aaaatagtag ttaaaattgt ttgtttagat gtgtaagatt ttatgaaatg ttttggtaaa     300
   agttagttta ggagtgttgg ttaaggtgtt gaagcgaagt aaggggagtt aggattattt     360
   ttgttattga attttttttt ttttattttt ttttaaagat agcgagttta tttattttga      420
   taagatattt taggatttta aatttgatat aattttggag aaagtagtgc gtgtgtgtgt     480
   gtgtgtgtgt gtgtgtgtgt gtggtgtaga agggtatttg ggatatataa tttttataga     540
   aattttagtg ttataaaaat tttaggagga tttatattta aaaaaatata taatagttta     600
   agttgtttat tttaattgaa cgttgtgtgt ttttattaga tttttagtga gatttagttt     660
   aatgagttaa ttgtaaaata aagtttttgt taagggaatg gtagaaaatg ataaaattat     720
   aattttgaat gattatttaa aagtaaaaag tattttttat tattgtgtgg agattttttt     780
   atttttattt ttattcgaat tgaatttttt tttttaatgt ttttttgaat agtttgtttt     840
   tggtgttttg aaaatgtgta cgttgatagt agtattatat gttgtttttg gtatagagtg      900
   agttaatagt tgaattattt ttgggtaatt gcggattgta gttagtgtaa gtgtaagaga     960
   aggtattttg gttattaatt cgttttatat tttaattatt ttcgttcgtt ttatattttt    1020
   tgggtattcg atgttttttt tttttgttag gtaatttgtt ttcgttttgt ttaatttttc    1080
   gatttttggt tttagagaat agttttggtt gtgggaattg gttttgggaa gtttttggcg    1140
   ttttttttat tggtgattgg taagagtaat atttttatat ttttaaaaag agtcgtttag    1200
   tttttaatga tattttttgg taaaaaagaa aatgaaatat ataattcgga tttcggttta    1260
   ttaaacgtag gttaagagcg cggttgttga gtttcggttt tgttgtgagg ttgttgtttt    1320
   tattcgtttt tcgtttttat tagagttgat ttgttttttg ttattttttt tcggtagtgt     1380
   ggatttattg taacgtagga atatttgggt tggtatggat aggattttag tattagatgt    1440
   tggttgttgt tttagggatt tttcgcggaa attatttagt atttagaagt cgttttaata    1500
   aggaagtaat tagtaaagta ttgttggtgg gagaatttcg cgaaatattt tagaaaaata    1560
   ttagaaatcg ttggagattt aatgatttac gtttttttta gtttgtgttt tttagttttt    1620
   tttggaggag tattaagtgg tttgtgggtt tttattttgc ggtgggaggg ttgttttaag    1680
   tatatttatt tttgcgatag ttaacgtcgg ggggttttag ttttaattag ggcgaggatt    1740
   acggattgta tttttttttt ttagcgtatt ggtttgttta ttggagggtg agagggacgc    1800
   gtagattttc ggatttaggg gttttgtttt gtagattttt ttattttatt cggggttttt     1860
   ggtattcgta ttatttagta ttagtgtgat tggcggtgta tttcggtttt ttcgcgtttt    1920
   attaaagttt ttgtatattt ttttttacgt tgcgtttttg ttatttacgt agttgttttt    1980
   ttgagcgttt cgcgtgtata aacgcgtttt cggtttttac gcgtcggtgg cgttgtttcg    2040
   ttgaggtggc gtcgggggag ttcgtagagg cggcggggga gggagtgcgg aggagaggat    2100
   tcgtgagttg cggggatttg ggttcgtcga gtttttgggg aagttgggtt tcgttttttt    2160
   cgggcgtttg gggcgcgcgg gaaggcgcgg aggacgcgtc gtatttattc gttgttgttg    2220
   tcggttgttg attgttatcg cggcggcgga cgcggcgttg tgtcggagtt cggtcgtttg    2280
   tttaggtttt aataggtttc gggtgaattt gcgggttcgt ttatcggtta tcg           2333
<210> 137
   <211> 2333
   <212> DNA
   <213> Artificial Sequence
<220>
   <223> chemically treated genomic DNA (Homo sapiens)
<400> 137
   cgatggtcgg tgagcggatt cgtaggttta ttcggagttt gttgagattt gggtaggcgg 60
   tcgagtttcg gtatagcgtc gcgttcgtcg tcgcggtggt agttagtagt cggtagtagt 120
   agcgggtgag tgcggcgcgt ttttcgcgtt ttttcgcgcg ttttaggcgt tcggggggaa 180
   cggagtttag tttttttaaa agttcggcgg gtttaggttt tcgtagttta cgggtttttt 240
   ttttcgtatt ttttttttcg tcgtttttgc gggttttttc ggcgttattt tagcgaggta 300
   gcgttatcga cgcgtaagga tcgggggcgc gtttgtgtac gcgggacgtt tagaggagta 360
   gttgcgtggg tagtagaagc gtagcgtggg agaggatgtg taggggtttt aatggggcgc 420
   gagggggtcg gaatgtatcg ttagttatat tggtgttaag tgatacgagt gttagagatt 480
   tcgggtgggg tggggggatt tatagaatag aatttttgag ttcggaggtt tgcgcgtttt 540
   ttttattttt tagtaaatag attagtgcgt tgagagagag ggatgtagtt cgtggttttc 600
   gttttggtta gagttggggt ttttcgacgt taattatcgt agaaatagat gtatttggaa 660
   tagttttttt atcgtagggt aaaaatttat aaattattta atattttttt agaaagggtt 720
   gaggaatata ggttggagga gacgtgggtt attaggtttt tagcggtttt tgatattttt 780
   ttaagatgtt tcgcggggtt tttttattag taatgtttta ttggttattt ttttattgaa 840
   acggttttta agtgttgggt gattttcgcg ggaagttttt ggggtagtag ttagtatttg 900
   atgttaggat tttatttata ttaatttaga tgtttttgcg ttgtagtagg tttatattgt 960
   cggggagagg tggtaagaga taaattaatt ttagtggaaa cgggaagcga gtgggaataa 1020
   tagttttata gtaagatcgg ggtttagtag tcgcgttttt ggtttgcgtt tggtggatcg 1080
   gaattcgggt tgtgtgtttt attttttttt ttgttagaaa atattattag gaattaaacg 1140
   gtttttttta agagtgtgaa agtgttattt ttgttagtta ttaatgagga gggcgttaaa 1200
   agttttttag agttagtttt tatagttagg attatttttt aaagttagag gtcggaaagt 1260
   tgaatagaac ggaagtaaat tgtttggtag agggaagagg tatcgggtgt ttagggggtg 1320
   tagagcgggc gggggtgatt ggagtgtgaa acgaattggt ggttagggtg tttttttttg 1380
   tatttatatt agttgtagtt cgtagttatt tagaggtggt ttaattgtta gtttattttg 1440
   tgttagggat agtatgtggt attgttatta gcgtgtatat ttttaggata ttaaagataa 1500
   attgtttaaa agaatattag aagaaaagat ttaattcgag taaaagtaga gataagaaaa 1560
   tttttatata gtagtagaaa gtatttttta tttttgaatg attatttaga attgtggttt 1620
   tgttattttt tgttattttt ttgataaaaa ttttattttg taattgattt attggattga 1680
   attttattga ggatttgatg gaaatatata gcgtttaatt ggaataaata gtttaaattg 1740
   ttgtgtgttt ttttaaatgt agattttttt agaatttttg tggtattaga atttttataa 1800
   agattatgta ttttagatat ttttttatat tatatatata tatatatata tatatatata 1860
   tacgtattgt tttttttaaa attatgttag atttgaaatt ttagggtatt ttgttaaaat 1920
   gaataagttc gttgttttta aaaagaggtg ggggagggga gatttagtgg taaaggtggt 1980
   tttgattttt tttgtttcgt tttagtattt tggttaatat ttttaaatta gtttttgtta 2040
   agatatttta tgggatttta tatatttagg taggtagttt tggttgttat tttggtagta 2100
   agaacgtatg tgttttgtat attattttat tagttaattt ttgaagggaa ataggaaggt 2160
   gatgagtttt ttgagttggt atttgaattc ggtgggattt tgtgggtgta gagtattttt 2220
   tttgaattta aaatcgtgtt attggattat gtggtagaga ttaattttag cgtatattga 2280
   tgtaattttt taagattttt tatatttgtt gttttaagtt tttaaaatat ggt 2333
<210> 138
   <211> 4333
   <212> DNA
   <213> Artificial Sequence
<220>
   <223> chemically treated genomic DNA (Homo sapiens)
<400> 138
   atgtatattt taatgaatta tagaaagtta tttaaatgaa ttaatttatg ttagggtata      60
   gatatatttt atttatttta aaagatgagt tttttattaa aaaggaattt ttaattagtg     120
   gtattataat tttgaagtta atatggtatg tatatttaat taatgtaata aaatatttta     180
   gttttgatat gagataaatg tttttaatta tttttgggtt gaaattttat tttagagaag     240
   tttgttttat gaaataatta ggaatgagat tttaggaaat tatgagaatt tgatatgatt     300
   ttttgaattg atttttgaat gagttgtttt ttatttattt tttatgggga attttaggat     360
   aatgaaggag taaatatttt tttttttgaa atgtatgttt ttgtttatta ttgtttttgg     420
   gaggggtgaa gaataaggtg gtttgtgttt aggagtttgt aagtatataa gtttattata     480
   tatagtgttt tgggtaaaaa ttgatattaa atttgttttg gtatatattt gtattgtttt     540
   taataattgt tttaaaattt ggttttttta gttattattt ttatttagta tttttttgat     600
   tttttttttt tatatttaaa tatattttga aggttttttg tttttttttt tttttttttt     660
   tttttttttt gtatatatat tttattttta gggaagaaaa ttattttgtt aatttgataa     720
   ttttatttaa atttattgga gagagtagaa tttaaatttt atggtaattt tttggtaatg     780
   ttttggagga tttgtgtttt taagataggg attttagaaa ggggaatata taaatttagt     840
   agtaagatat ttttttttag taggagtttt tagttttttt taaatatttt gtaatagagg     900
   atggtttgtt ttaatatata tttgtggttt tatgagtttg tattgaagtt ggatatttat     960
   aatatttttt ttaatatttt attatttttt ttttttgata attatgtttt gagaatttga    1020
   agtaatagat atgaagagtt ttaagggatt gtattaatgt atgttaagat tagtttttat    1080
   tatatagttt aatgatatgg ttttaggttt agaagggatg ttttatattt atagaatttt    1140
   attgagttta ggtgttagtt tagagaattt attatttttt tgtttttttt taagagttaa    1200
   ttgatgggat agtatgtaaa gtatatgtgt ttttgttgtt aaaatagtag ttaaaattgt    1260
   ttgtttagat gtgtaagatt ttatgaaatg ttttggtaaa agttagttta ggagtgttgg    1320
   ttaaggtgtt gaagtgaagt aaggggagtt aggattattt ttgttattga attttttttt    1380
   ttttattttt ttttaaagat agtgagttta tttattttga taagatattt taggatttta    1440
   aatttgatat aattttggag aaagtagtgt gtgtgtgtgt gtgtgtgtgt gtgtgtgtgt    1500
   gtggtgtaga agggtatttg ggatatataa tttttataga aattttagtg ttataaaaat    1560
   tttaggagga tttatattta aaaaaatata taatagttta agttgtttat tttaattgaa    1620
   tgttgtgtgt ttttattaga tttttagtga gatttagttt aatgagttaa ttgtaaaata    1680
   aagtttttgt taagggaatg gtagaaaatg ataaaattat aattttgaat gattatttaa    1740
   aagtaaaaag tattttttat tattgtgtgg agattttttt atttttattt ttatttgaat    1800
   tgaatttttt tttttaatgt ttttttgaat agtttgtttt tggtgttttg aaaatgtgta    1860
   tgttgatagt agtattatat gttgtttttg gtatagagtg agttaatagt tgaattattt    1920
   ttgggtaatt gtggattgta gttagtgtaa gtgtaagaga aggtattttg gttattaatt    1980
   tgttttatat tttaattatt tttgtttgtt ttatattttt tgggtatttg atgttttttt    2040
   tttttgttag gtaatttgtt tttgttttgt ttaatttttt gatttttggt tttagagaat    2100
   agttttggtt gtgggaattg gttttgggaa gtttttggtg ttttttttat tggtgattgg    2160
   taagagtaat atttttatat ttttaaaaag agttgtttag tttttaatga tattttttgg    2220
   taaaaaagaa aatgaaatat ataatttgga ttttggttta ttaaatgtag gttaagagtg    2280
   tggttgttga gttttggttt tgttgtgagg ttgttgtttt tatttgtttt ttgtttttat    2340
   tagagttgat ttgttttttg ttattttttt ttggtagtgt ggatttattg taatgtagga    2400
   atatttgggt tggtatggat aggattttag tattagatgt tggttgttgt tttagggatt    2460
   ttttgtggaa attatttagt atttagaagt tgttttaata aggaagtaat tagtaaagta    2520
   ttgttggtgg gagaattttg tgaaatattt tagaaaaata ttagaaattg ttggagattt    2580
   aatgatttat gtttttttta gtttgtgttt tttagttttt tttggaggag tattaagtgg    2640
   tttgtgggtt tttattttgt ggtgggaggg ttgttttaag tatatttatt tttgtgatag    2700
   ttaatgttgg ggggttttag ttttaattag ggtgaggatt atggattgta tttttttttt    2760
   ttagtgtatt ggtttgttta ttggagggtg agagggatgt gtagattttt ggatttaggg    2820
   gttttgtttt gtagattttt ttattttatt tggggttttt ggtatttgta ttatttagta    2880
   ttagtgtgat tggtggtgta ttttggtttt tttgtgtttt attaaagttt ttgtatattt    2940
   ttttttatgt tgtgtttttg ttatttatgt agttgttttt ttgagtgttt tgtgtgtata    3000
   aatgtgtttt tggtttttat gtgttggtgg tgttgttttg ttgaggtggt gttgggggag    3060
   tttgtagagg tggtggggga gggagtgtgg aggagaggat ttgtgagttg tggggatttg    3120
   ggtttgttga gtttttgggg aagttgggtt ttgttttttt tgggtgtttg gggtgtgtgg    3180
   gaaggtgtgg aggatgtgtt gtatttattt gttgttgttg ttggttgttg attgttattg    3240
   tggtggtgga tgtggtgttg tgttggagtt tggttgtttg tttaggtttt aataggtttt    3300
   gggtgaattt gtgggtttgt ttattggtta ttgttggtta tgttaattgt gttgtgggtt    3360
   tggggtgtgt tttttgttgg ttttttttgt gttaattttt tttatttatt tgttgtttag    3420
   gaagtggaat ttggagtttt tttttttttt ttttttgttt aatttgtaag aggtgggttt    3480
   tttttttttg tatttttatt ttttttattt tttttttttt tttgaagttt tttgaagatt    3540
   ttttaaattt tgtgtttttt tgggtgtgtg tggttaattt ggttggtggt tgggttttgt    3600
   agtggtgtat ttggagtggg tgttgtggga tgtgtgtggg gtggtttttg tttgtgttgt    3660
   ttatatgggt ttagttggtt ggttggggtt gtttatgttg tttgggggaa ttgtaggttg    3720
   tgggggaggg tggggggtgg ttgttgaggg gttttgggtg gggtttgttt aaggttgttg    3780
   tttgtttaag gttgttagtt ttatttggag gttgttggga gagttggagt tttggatgta    3840
   tttgttgttt agatttttat tttttgggtt tggttagtgg gtgggggtta agtttggttt    3900
   gtttttttat ttttgggaag gggttagagg tttattaaag atttggtggt tgggatttta    3960
   gtagtattgt ttaagttagt gtttggaagg tttttttgtt agaagtttat gtttagggtt    4020
   ttggaataag tttttttatt tatttatttt tttaatgagt atttattaag tgtttattat    4080
   atgttagttt atattgtttt ggaaattgag gatatggaga ggaatttagt aattatattt    4140
   taaggttgtg tagagttgat aggttttttt ggggtagggg tgggagatag taagtaatta    4200
   agtgaataaa taaataaata tagagaaatt aaggagatag tttaagttat taatatgaat    4260
   ttaaaattaa taaattagtg ttttgggggt aagagatttt gggtggtatt aggatgagta    4320
   ggtaagaatt aat                                                        4333
<210> 139
   <211> 4333
   <212> DNA
   <213> Artificial Sequence
<220>
   <223> chemically treated genomic DNA (Homo sapiens)
<400> 139
   gttggttttt gtttatttat tttaatgtta tttaaagttt tttattttta gaatgttgat      60
   ttattggttt tgagtttgtg ttagtagttt aaattgtttt tttgattttt ttgtatttgt     120
   ttatttgttt gtttggttat ttgttgtttt ttatttttat tttgaaaaaa tttattagtt     180
   ttatatagtt ttaaagtgta gttgttaagt ttttttttgt atttttagtt tttagaatag     240
   tgtgagttgg tatatagtag gtgtttaata aatatttatt gaaggagtga atgaatgggg     300
   aagtttattt taaggttttg aatatggatt tttggtaagg gagtttttta aatgttggtt     360
   tgggtgatgt tgttgggatt ttagttatta gatttttaat aagtttttga tttttttttg     420
   agggtggaag ggtgaattga gtttggtttt tatttattag ttggatttag aaaatagaag     480
   tttaaatggt aaatgtattt agggttttaa tttttttagt aatttttaaa tgaggttgat     540
   ggttttggat aggtgatggt tttggatagg ttttgtttgg agttttttgg tggttgtttt     600
   ttgttttttt ttgtagtttg tagttttttt gggtggtgtg gatagttttg gttggttagt     660
   tgagtttgtg tgggtggtgt gggtaggagt tgttttatat gtgttttgtg atgtttgttt     720
   tagatgtgtt gttgtagggt ttggttgtta gttgagttgg ttgtgtgtgt ttgggggagt     780
   gtagagtttg gaaagttttt gagaagtttt agaggaggga agggggtggg aagagtgggg     840
   gtgtaagaga gaaaaatttg ttttttgtga gttgagtgag gaagaggggg gggaaggttt     900
   tgagttttgt tttttgaatg gtgggtggat agagaaggtt ggtgtgggga agattggtgg     960
   gaggtgtgtt ttgggtttgt ggtgtgattg gtgtgattgg tgatggttgg tgagtggatt    1020
   tgtaggttta tttggagttt gttgagattt gggtaggtgg ttgagttttg gtatagtgtt    1080
   gtgtttgttg ttgtggtggt agttagtagt tggtagtagt agtgggtgag tgtggtgtgt    1140
   tttttgtgtt tttttgtgtg ttttaggtgt ttggggggaa tggagtttag tttttttaaa 1200
   agtttggtgg gtttaggttt ttgtagttta tgggtttttt tttttgtatt tttttttttg 1260
   ttgtttttgt gggttttttt ggtgttattt tagtgaggta gtgttattga tgtgtaagga 1320
   ttgggggtgt gtttgtgtat gtgggatgtt tagaggagta gttgtgtggg tagtagaagt 1380
   gtagtgtggg agaggatgtg taggggtttt aatggggtgt gagggggttg gaatgtattg 1440
   ttagttatat tggtgttaag tgatatgagt gttagagatt ttgggtgggg tggggggatt 1500
   tatagaatag aatttttgag tttggaggtt tgtgtgtttt ttttattttt tagtaaatag 1560
   attagtgtgt tgagagagag ggatgtagtt tgtggttttt gttttggtta gagttggggt 1620
   tttttgatgt taattattgt agaaatagat gtatttggaa tagttttttt attgtagggt 1680
   aaaaatttat aaattattta atattttttt agaaagggtt gaggaatata ggttggagga 1740
   gatgtgggtt attaggtttt tagtggtttt tgatattttt ttaagatgtt ttgtggggtt 1800
   tttttattag taatgtttta ttggttattt ttttattgaa atggttttta agtgttgggt 1860
   gatttttgtg ggaagttttt ggggtagtag ttagtatttg atgttaggat tttatttata 1920
   ttaatttaga tgtttttgtg ttgtagtagg tttatattgt tggggagagg tggtaagaga 1980
   taaattaatt ttagtggaaa tgggaagtga gtgggaataa tagttttata gtaagattgg 2040
   ggtttagtag ttgtgttttt ggtttgtgtt tggtggattg gaatttgggt tgtgtgtttt 2100
   attttttttt ttgttagaaa atattattag gaattaaatg gtttttttta agagtgtgaa 2160
   agtgttattt ttgttagtta ttaatgagga gggtgttaaa agttttttag agttagtttt 2220
   tatagttagg attatttttt aaagttagag gttggaaagt tgaatagaat ggaagtaaat 2280
   tgtttggtag agggaagagg tattgggtgt ttagggggtg tagagtgggt gggggtgatt 2340
   ggagtgtgaa atgaattggt ggttagggtg tttttttttg tatttatatt agttgtagtt 2400
   tgtagttatt tagaggtggt ttaattgtta gtttattttg tgttagggat agtatgtggt 2460
   attgttatta gtgtgtatat ttttaggata ttaaagataa attgtttaaa agaatattag 2520
   aagaaaagat ttaatttgag taaaagtaga gataagaaaa tttttatata gtagtagaaa 2580
   gtatttttta tttttgaatg attatttaga attgtggttt tgttattttt tgttattttt 2640
   ttgataaaaa ttttattttg taattgattt attggattga attttattga ggatttgatg 2700
   gaaatatata gtgtttaatt ggaataaata gtttaaattg ttgtgtgttt ttttaaatgt 2760
   agattttttt agaatttttg tggtattaga atttttataa agattatgta ttttagatat 2820
   ttttttatat tatatatata tatatatata tatatatata tatgtattgt tttttttaaa 2880
   attatgttag atttgaaatt ttagggtatt ttgttaaaat gaataagttt gttgttttta 2940
   aaaagaggtg ggggagggga gatttagtgg taaaggtggt tttgattttt tttgttttgt 3000
   tttagtattt tggttaatat ttttaaatta gtttttgtta agatatttta tgggatttta 3060
   tatatttagg taggtagttt tggttgttat tttggtagta agaatgtatg tgttttgtat 3120
   attattttat tagttaattt ttgaagggaa ataggaaggt gatgagtttt ttgagttggt 3180
   atttgaattt ggtgggattt tgtgggtgta gagtattttt tttgaattta aaattgtgtt 3240
   attggattat gtggtagaga ttaattttag tgtatattga tgtaattttt taagattttt 3300
   tatatttgtt gttttaagtt tttaaaatat ggttgttaag ggagaaaaat gataggatgt 3360
   tggggaaaat attatggata tttgatttta gtatagattt atagaattgt gggtgtgtat 3420
   tggagtaagt tattttttat tgtaaggtat ttgaaagaga ttagaaattt ttgttgagaa 3480
   aaagtatttt attgttagat ttgtatattt ttttttttga aatttttgtt ttggggatat 3540
   aaatttttta ggatattgtt aaaagattgt tatagggttt gaattttgtt ttttttaata 3600
   agtttaggta aggttgttag gttaataaga tagttttttt ttttgagaat ggggtgtgta 3660
   tatagagaga gagagagaga gagagagaga agataggaag tttttaaaat gtgtttgaat 3720
   atggagagga gagattaagg agatgttgag tgagggtaat gattgggaaa gttaaatttt 3780
   aaggtagttg ttgaggataa tataggtgta tgttaaagta aatttgatgt tagtttttat 3840
   ttaaagtatt gtatgtagta ggtttatgta tttgtagatt tttagatatg aattatttta 3900
   tttttatttt tttttaagaa tagtaatgaa taaaaatata tattttaaaa ggagaaatat 3960
   ttgttttttt attattttaa aattttttat agaaaataaa tgaaagataa tttatttgaa 4020
   gattaattta aaggattatg ttaagttttt ataatttttt gaaattttat ttttgattgt 4080
   tttataaagt aagttttttt agggtagaat tttagtttga aaatgattag aaatatttgt 4140
   tttatattag aattggagtg ttttattata ttggttgaat atatatatta tgttggtttt 4200
   aaagttgtaa tgttattaat tagaaatttt tttttgataa gaagtttatt ttttaaaatg 4260
   aatgaagtgt atttgtattt tagtatgaat tagtttattt agatagtttt ttataattta 4320
   ttaagatata tat 4333
<210> 140
   <211> 2333
   <212> DNA
   <213> Artificial Sequence
<220>
   <223> chemically treated genomic DNA (Homo sapiens)
<400> 140
   attatgtttt gagaatttga agtaatagat atgaagagtt ttaagggatt gtattaatgt      60
   atgttaagat tagtttttat tatatagttt aatgatatgg ttttaggttt agaagggatg     120
   ttttatattt atagaatttt attgagttta ggtgttagtt tagagaattt attatttttt    180
   tgtttttttt taagagttaa ttgatgggat agtatgtaaa gtatatgtgt ttttgttgtt     240
   aaaatagtag ttaaaattgt ttgtttagat gtgtaagatt ttatgaaatg ttttggtaaa     300
   agttagttta ggagtgttgg ttaaggtgtt gaagtgaagt aaggggagtt aggattattt     360
   ttgttattga attttttttt ttttattttt ttttaaagat agtgagttta tttattttga    420
   taagatattt taggatttta aatttgatat aattttggag aaagtagtgt gtgtgtgtgt     480
   gtgtgtgtgt gtgtgtgtgt gtggtgtaga agggtatttg ggatatataa tttttataga     540
   aattttagtg ttataaaaat tttaggagga tttatattta aaaaaatata taatagttta     600
   agttgtttat tttaattgaa tgttgtgtgt ttttattaga tttttagtga gatttagttt     660
   aatgagttaa ttgtaaaata aagtttttgt taagggaatg gtagaaaatg ataaaattat     720
   aattttgaat gattatttaa aagtaaaaag tattttttat tattgtgtgg agattttttt     780
   atttttattt ttatttgaat tgaatttttt tttttaatgt ttttttgaat agtttgtttt      840
   tggtgttttg aaaatgtgta tgttgatagt agtattatat gttgtttttg gtatagagtg    900
   agttaatagt tgaattattt ttgggtaatt gtggattgta gttagtgtaa gtgtaagaga     960
   aggtattttg gttattaatt tgttttatat tttaattatt tttgtttgtt ttatattttt    1020
   tgggtatttg atgttttttt tttttgttag gtaatttgtt tttgttttgt ttaatttttt    1080
   gatttttggt tttagagaat agttttggtt gtgggaattg gttttgggaa gtttttggtg   1140
   ttttttttat tggtgattgg taagagtaat atttttatat ttttaaaaag agttgtttag   1200
   tttttaatga tattttttgg taaaaaagaa aatgaaatat ataatttgga ttttggttta   1260
   ttaaatgtag gttaagagtg tggttgttga gttttggttt tgttgtgagg ttgttgtttt    1320
   tatttgtttt ttgtttttat tagagttgat ttgttttttg ttattttttt ttggtagtgt   1380
   ggatttattg taatgtagga atatttgggt tggtatggat aggattttag tattagatgt    1440
   tggttgttgt tttagggatt ttttgtggaa attatttagt atttagaagt tgttttaata    1500
   aggaagtaat tagtaaagta ttgttggtgg gagaattttg tgaaatattt tagaaaaata    1560
   ttagaaattg ttggagattt aatgatttat gtttttttta gtttgtgttt tttagttttt    1620
   tttggaggag tattaagtgg tttgtgggtt tttattttgt ggtgggaggg ttgttttaag    1680
   tatatttatt tttgtgatag ttaatgttgg ggggttttag ttttaattag ggtgaggatt    1740
   atggattgta tttttttttt ttagtgtatt ggtttgttta ttggagggtg agagggatgt    1800
   gtagattttt ggatttaggg gttttgtttt gtagattttt ttattttatt tggggttttt    1860
   ggtatttgta ttatttagta ttagtgtgat tggtggtgta ttttggtttt tttgtgtttt    1920
   attaaagttt ttgtatattt ttttttatgt tgtgtttttg ttatttatgt agttgttttt    1980
   ttgagtgttt tgtgtgtata aatgtgtttt tggtttttat gtgttggtgg tgttgttttg    2040
   ttgaggtggt gttgggggag tttgtagagg tggtggggga gggagtgtgg aggagaggat    2100
   ttgtgagttg tggggatttg ggtttgttga gtttttgggg aagttgggtt ttgttttttt    2160
   tgggtgtttg gggtgtgtgg gaaggtgtgg aggatgtgtt gtatttattt gttgttgttg    2220
   ttggttgttg attgttattg tggtggtgga tgtggtgttg tgttggagtt tggttgtttg    2280
   tttaggtttt aataggtttt gggtgaattt gtgggtttgt ttattggtta ttg           2333
<210> 141
   <211> 2333
   <212> DNA
   <213> Artificial Sequence
<220>
   <223> chemically treated genomic DNA (Homo sapiens)
<400> 141
   tgatggttgg tgagtggatt tgtaggttta tttggagttt gttgagattt gggtaggtgg      60
   ttgagttttg gtatagtgtt gtgtttgttg ttgtggtggt agttagtagt tggtagtagt     120
   agtgggtgag tgtggtgtgt tttttgtgtt tttttgtgtg ttttaggtgt ttggggggaa     180
   tggagtttag tttttttaaa agtttggtgg gtttaggttt ttgtagttta tgggtttttt     240
   tttttgtatt tttttttttg ttgtttttgt gggttttttt ggtgttattt tagtgaggta     300
   gtgttattga tgtgtaagga ttgggggtgt gtttgtgtat gtgggatgtt tagaggagta      360
   gttgtgtggg tagtagaagt gtagtgtggg agaggatgtg taggggtttt aatggggtgt     420
   gagggggttg gaatgtattg ttagttatat tggtgttaag tgatatgagt gttagagatt     480
   ttgggtgggg tggggggatt tatagaatag aatttttgag tttggaggtt tgtgtgtttt    540
   ttttattttt tagtaaatag attagtgtgt tgagagagag ggatgtagtt tgtggttttt     600
   gttttggtta gagttggggt tttttgatgt taattattgt agaaatagat gtatttggaa     660
   tagttttttt attgtagggt aaaaatttat aaattattta atattttttt agaaagggtt     720
   gaggaatata ggttggagga gatgtgggtt attaggtttt tagtggtttt tgatattttt     780
   ttaagatgtt ttgtggggtt tttttattag taatgtttta ttggttattt ttttattgaa     840
   atggttttta agtgttgggt gatttttgtg ggaagttttt ggggtagtag ttagtatttg     900
   atgttaggat tttatttata ttaatttaga tgtttttgtg ttgtagtagg tttatattgt     960
   tggggagagg tggtaagaga taaattaatt ttagtggaaa tgggaagtga gtgggaataa    1020
   tagttttata gtaagattgg ggtttagtag ttgtgttttt ggtttgtgtt tggtggattg    1080
   gaatttgggt tgtgtgtttt attttttttt ttgttagaaa atattattag gaattaaatg    1140
   gtttttttta agagtgtgaa agtgttattt ttgttagtta ttaatgagga gggtgttaaa     1200
   agttttttag agttagtttt tatagttagg attatttttt aaagttagag gttggaaagt    1260
   tgaatagaat ggaagtaaat tgtttggtag agggaagagg tattgggtgt ttagggggtg    1320
   tagagtgggt gggggtgatt ggagtgtgaa atgaattggt ggttagggtg tttttttttg   1380
   tatttatatt agttgtagtt tgtagttatt tagaggtggt ttaattgtta gtttattttg    1440
   tgttagggat agtatgtggt attgttatta gtgtgtatat ttttaggata ttaaagataa    1500
   attgtttaaa agaatattag aagaaaagat ttaatttgag taaaagtaga gataagaaaa    1560
   tttttatata gtagtagaaa gtatttttta tttttgaatg attatttaga attgtggttt    1620
   tgttattttt tgttattttt ttgataaaaa ttttattttg taattgattt attggattga    1680
   attttattga ggatttgatg gaaatatata gtgtttaatt ggaataaata gtttaaattg    1740
   ttgtgtgttt ttttaaatgt agattttttt agaatttttg tggtattaga atttttataa    1800
   agattatgta ttttagatat ttttttatat tatatatata tatatatata tatatatata   1860
   tatgtattgt tttttttaaa attatgttag atttgaaatt ttagggtatt ttgttaaaat    1920
   gaataagttt gttgttttta aaaagaggtg ggggagggga gatttagtgg taaaggtggt    1980
   tttgattttt tttgttttgt tttagtattt tggttaatat ttttaaatta gtttttgtta    2040
   agatatttta tgggatttta tatatttagg taggtagttt tggttgttat tttggtagta    2100
   agaatgtatg tgttttgtat attattttat tagttaattt ttgaagggaa ataggaaggt    2160
   gatgagtttt ttgagttggt atttgaattt ggtgggattt tgtgggtgta gagtattttt    2220
   tttgaattta aaattgtgtt attggattat gtggtagaga ttaattttag tgtatattga    2280
   tgtaattttt taagattttt tatatttgtt gttttaagtt tttaaaatat ggt           2333
<210> 142
   <211> 26
   <212> DNA
   <213> Artificial Sequence
<220>
   <223> chemically treated genomic DNA (Homo sapiens)
<400> 142
   ggtttttacg tacgttgttt ttttcg 26
<210> 143
   <211> 25
   <212> DNA
   <213> Artificial Sequence
<220>
   <223> chemically treated genomic DNA (Homo sapiens)
<400> 143
   tcactaccga aaacgaaaat tacga 25
<210> 144
   <211> 19
   <212> DNA
   <213> Artificial Sequence
<220>
   <223> chemically treated genomic DNA (Homo sapiens)
<400> 144
   atcgccaacg ccgcgcaaa 19
<210> 145
   <211> 20
   <212> DNA
   <213> Artificial Sequence
<220>
   <223> chemically treated genomic DNA (Homo sapiens)
<400> 145
   tcctctaaac gtcccgcgta 20
<210> 146
   <211> 20
   <212> DNA
   <213> Artificial Sequence
<220>
   <223> chemically treated genomic DNA (Homo sapiens)
<400> 146
   caaacgcgcc cccgatcctt 20
<210> 147
   <211> 20
   <212> DNA
   <213> Artificial Sequence
<220>
   <223> chemically treated genomic DNA (Homo sapiens)
<400> 147
   gcgaggtagc gttatcgacg 20
<210> 148
   <211> 21
   <212> DNA
   <213> Artificial Sequence
<220>
   <223> chemically treated genomic DNA (Homo sapiens)
<400> 148
   ttttttttcg tcgtttttgc g 21

## Description

### FIELD OF THE INVENTION

The present invention relates to genomic DNA sequences that exhibit altered expression patterns in disease states relative to normal.

### BACKGROUND

### Incidence and diagnosis of cancer.

Cancer is the second leading cause of death of the United States. Mortality rates could be significantly improved if current screening methods would be improved in terms of patient compliance, sensitivity and ease of screening. Current recommended methods for diagnosis of cancer are often expensive and are not suitable for application as population wide screening tests.

In the United States the annual incidence of colorectal cancer is approximately 150,000, with 56,600 individuals dying from colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscople examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure™', with an improved sensitivity of 77 % (adenomas) and 88.9% (colorectal carcinoma.

*Molecular disease markers.* Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (e.g., sample collection), and, most importantly, the large amount of mRNA needed for analysis (Lipshutz, R. J. et al., Nature Genetics 21:20-24, 1999; Bowtell, D. D. L. Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisting TPEF, HIC1, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from β-catenin mutations. Furthermore, alterations in the TGF-β signalling pathway together with its signal transducers SMAD4 and SMAD2 have been linked to the development of colon cancer.

Despite recent progress in the understanding of the pathogenesis of adertomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported.

*CpG island methylation.* Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

Recently several groups have also analyzed the methylation of various genes in colorectal cancer and reported the transcriptional silencing by promoter methylation for p16INK4, p14ARF, p15INK4b, MGMT, hMLH1, GSTP 1, DAPK, CDH1, TIMP-3 and APC among others. Thus apart from mutational inactivation of certain genes, the hypermethylation of these genes also contributes significantly to the pathogenesis of this disease. See also "Reverse transcirption primer used in cDNA analysis technique", EBI accession no. GSN:AAQ75611, 4 August 1995 and "Oligonucleotide for detecting cytosine methylation SEQ ID NO: 2679.", EBI accession no. GSN:ABQ16088.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, e.g., Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Sci USA 98:265-270, 2001). For the identification of genes in pancreatic cancer see Pogue-Geile et al., Cancer Genomics & Proteomics, Vol.1 1 Jan 2004, p 371-386.

*Multifactorial approach.* Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states. The present invention describes a plurality of particularly efficient and unique panels of genes, the methylation analysis of one or a combination of the members of the panel enabling the detection of colon cell proliferative disorders with a particularly high sensitivity, specificity and/or predictive value.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e.,* individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e.,* individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene-based test that can detect a p53 mutation. Specificity is important when the cost or risk associated with further diagnostic procedures or further medical intervention are very high.

*Pronounced need in the art.* It is generally accepted that there is a pronounced need in the art for improved screening and early detection of cancers. As an example, if colon cancer screening specificity can be increased, the problem of false positive test results leading to unnecessary colonoscopic examination would be reduced leading to cost savings and improved safety.

In view of the incidence of cancers in general and more particularly the disadvantages associated with current colorectal cell proliferative disorder screening methods there is a substantial need in the art for improved methods for the early detection of cancer, in particular colon cancer, to be used in addition to or as a substitute for currently available tests.

*Background of the genes of the present invention.* The human Septin 9 gene (also known as MLL septin-like fusion protein, MLL septin-like fusion protein MSF-A, Slpa, Eseptin, Msf, septin-like protein Ovarian/Breast septin (Ov/Br septin) and Septin D1) is located on chromosome 17q25 within contig AC068594.15.1.168501 and is a member of the Septin gene family. SEQ ID NO: 12 provides the sequence of said gene, comprising regions of both the Septin 9 and Q9HC74 transcripts and promoter regions. SEQ ID NO: 13 and SEQ ID NO: 14 provide particularly preferred CpG rich regions of said gene according to the present invention.

It has been postulated that members of the Septin gene family are associated with multiple cellular functions ranging from vesicle transport to cytokinesis. Disruption of the action of *Septin* 9 results in incomplete cell division, see Surka, M.C., Tsang, C.W., and Trimble, W.S. Mol Biol Cell, 13: 3532-45 (2002). *Septin* 9 and other proteins have been shown to be fusion partners of the proto-oncogene MLL suggesting a role in tumorogenesis, see Osaka, M, Rowley, J.D. and Zeleznik-Le, N.J. PNAS, 96:6428-6433 (1999). Burrows et al. reported an in depth study of expression of the multiple isoforms of the *Septin* 9 gene in ovarian cancer and showed tissue specific expression of various transcripts, see Burrows, J. F., Chanduloy, et al. S.E.H. Journal of Pathology, 201:581-588 (2003).

A recent study (post-priority date published prior art) of over 7000 normal and tumor tissues indicates that there is consistent over-expression of *Septin* 9 isoforms in a number of tumor tissues, see Scott, M., Hyland, P.L., et al. Oncogene, 24: 4688-4700 (2005). The authors speculate that the gene is likely a type II cancer gene where changes in RNA transcript processing control regulation of different protein products, and the levels of these altered protein isoforms may provide answers to the gene's role in malignancy.

KAY L. POGUE-GEILE ET AL: CANCER GENOMICS & PROTEOMICS, vol. 1, 1 January 2004 (2004-01-01), pages 371-386 discloses that mRNA of PCDHGC3, as analysed on a cDNA microarray, is underexpressed in pancreatic cancer.

DATABASE Geneseq [Online] 4 August 1995, retrieved from EBI accession no. GSN:AAQ75611 discloses a sequence which has 100 % identity to SEQ ID NO:17 of the present application.

WO2007/073220, published on 28.07.2007 (i.e. after the present application's priority date) discloses that a large number of genes, e.g. PCDHGC3, are differentially expressed between relapsing and non-relapsing colorectal cancer

### BRIEF DESCRIPTION OF THE DRAWINGS

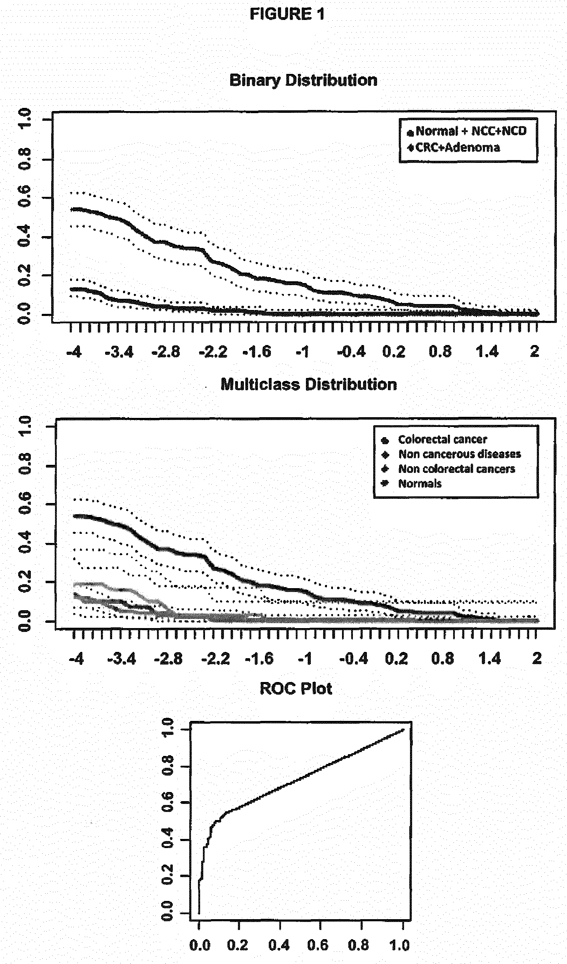

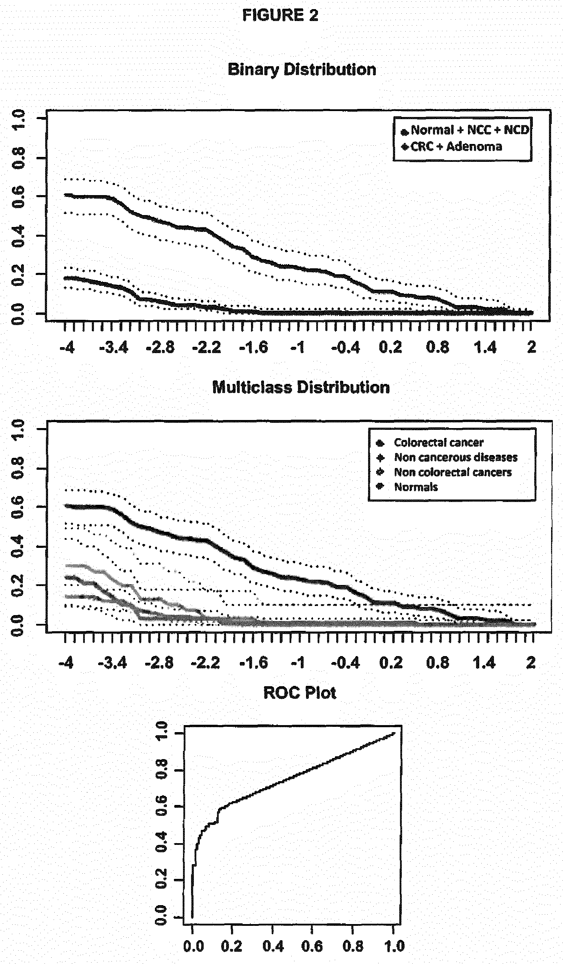

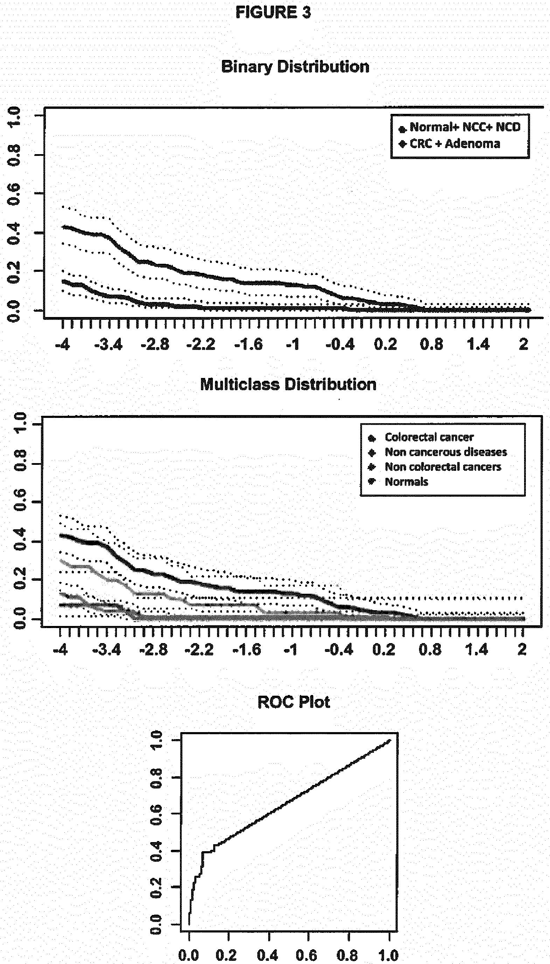

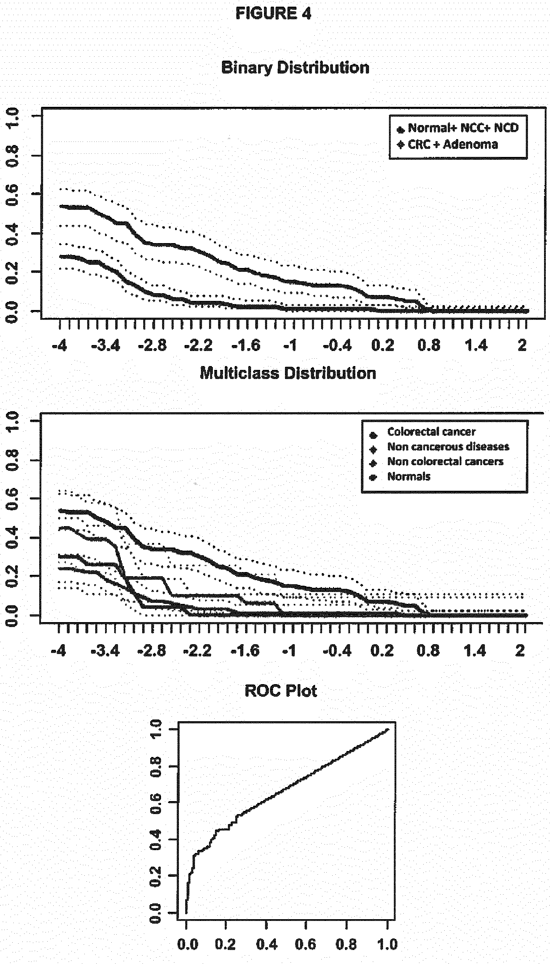

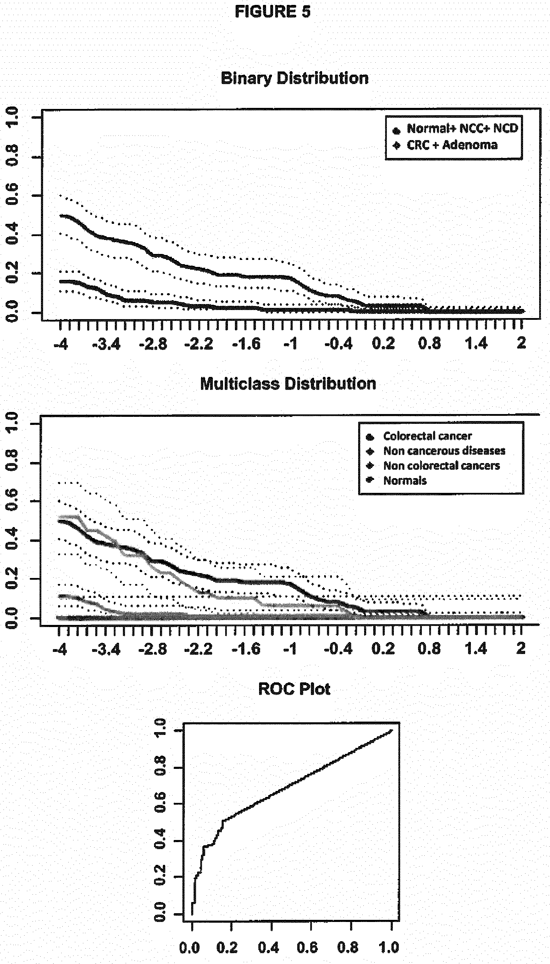

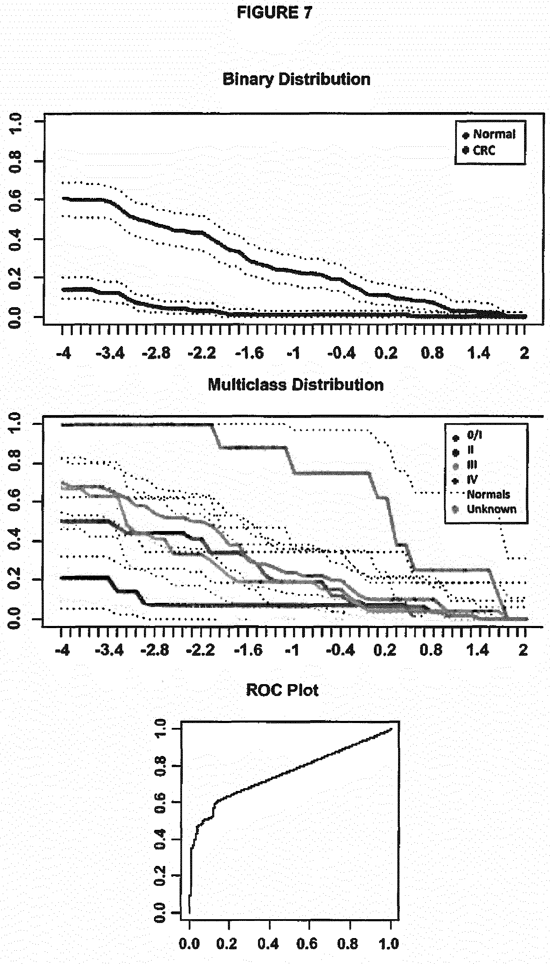

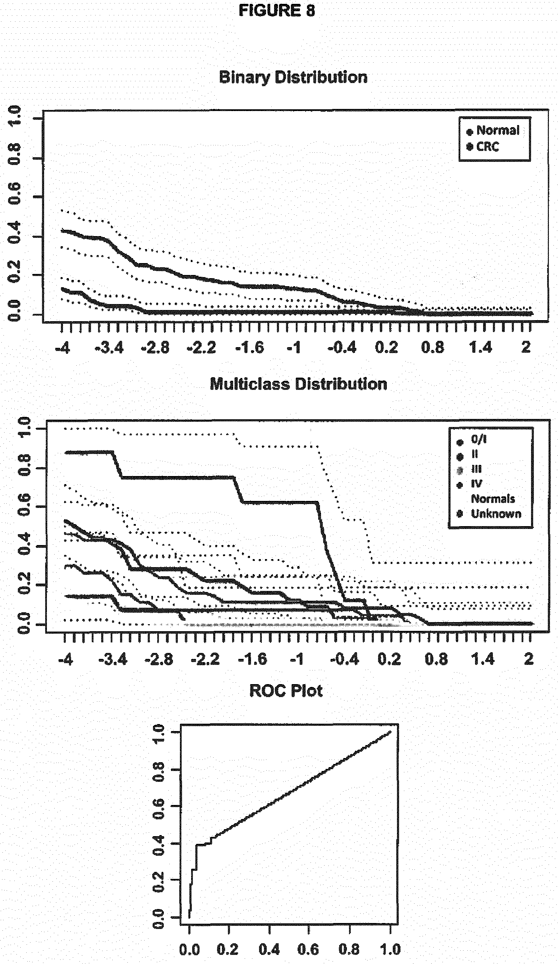

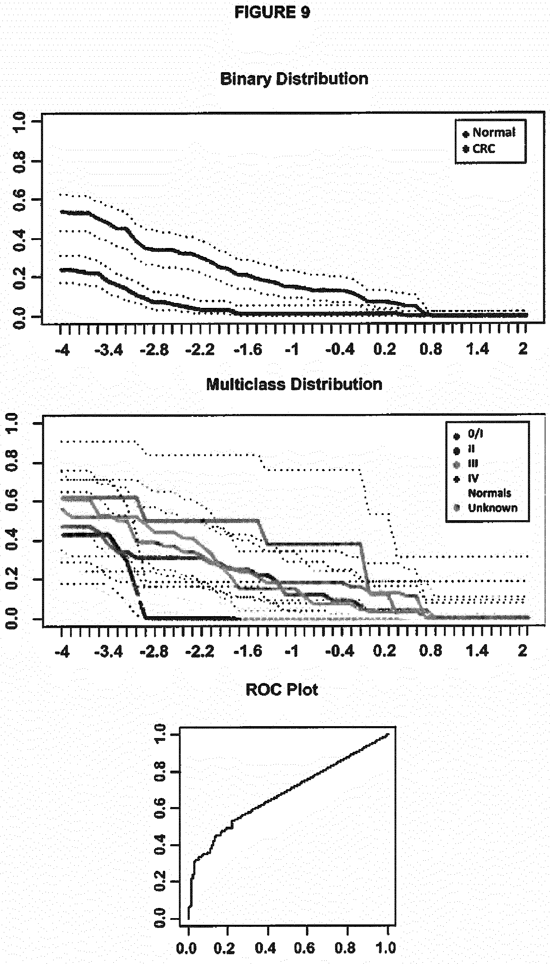

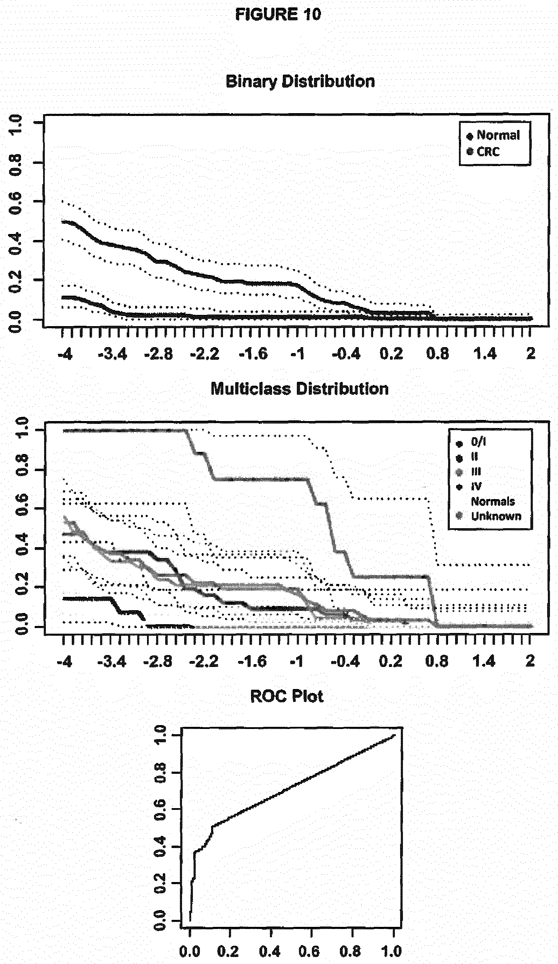

Figures 1 to 10 provide an overview of the log mean methylation measured by means of the HM assay according to Example 2. Each figures consists of three plots, the plots at the top and in the middle provide the binary and multiclass analysis respectively, sensitivity is shown on the Y-axis, DNA methylation measured in (log 10 ng/mL) is shown on the X-axis.

In each figure the plot at the bottom provides an ROC wherein sensitivity is shown on the Y-axis and 1-specificity is shown on the X-axis.
Figure 1 provides an overview of the performance of the RASSF2 HM assay according to Example 2, in all samples.
Figure 2 provides an overview of the performance of the Septin 9 HM assay according to Example 2, in all samples.
Figure 3 provides an overview of the performance of the SND1 HM assay according to Example 2, in all samples.
Figure 4 provides an overview of the performance of the PCDHGC3 HM assay according to Example 2, in all samples.
Figure 5 provides an overview of the performance of the TFAP2E HM assay according to Example 2, in all samples.
Figure 6 provides an overview of the performance of the RASSF2 HM assay according to Example 2, in all colorectal carcinoma and normal colorectal tissue samples.
Figure 7 provides an overview of the performance of the Septin 9 HM assay according to Example 2, in all colorectal carcinoma and normal colorectal tissue samples.
Figure 8 provides an overview of the performance of the SND1 HM assay according to Example 2, in all colorectal carcinoma and normal colorectal tissue samples.
Figure 9 provides an overview of the performance of the PCDHGC3 HM assay according to Example 2, in all colorectal carcinoma and normal colorectal tissue samples.
Figure 10 provides an overview of the performance of the TFAP2E HM assay according to Example 2, in all colorectal carcinoma and normal colorectal tissue samples.
Figure 11 provides an overview of the predictive power of the logistic regression model of combinations of markers. Se is sensitivitiy, sp is specificity, AUC is area under the curve.
Figures 12 to 21 provide an overview of the log majority mean methylation measured by means of the HM assay according to Example 2. Each figures consists of three plots, the plots at the top and in the middle provide the binary and multiclass analysis respectively, sensitivity is shown on the Y-axis, DNA methylation measured in (log 10 ng/mL) is shown on the X-axis.

In each figure the plot at the bottom provides an ROC wherein sensitivity is shown on the Y-axis and 1-specificity is shown on the X-axis.

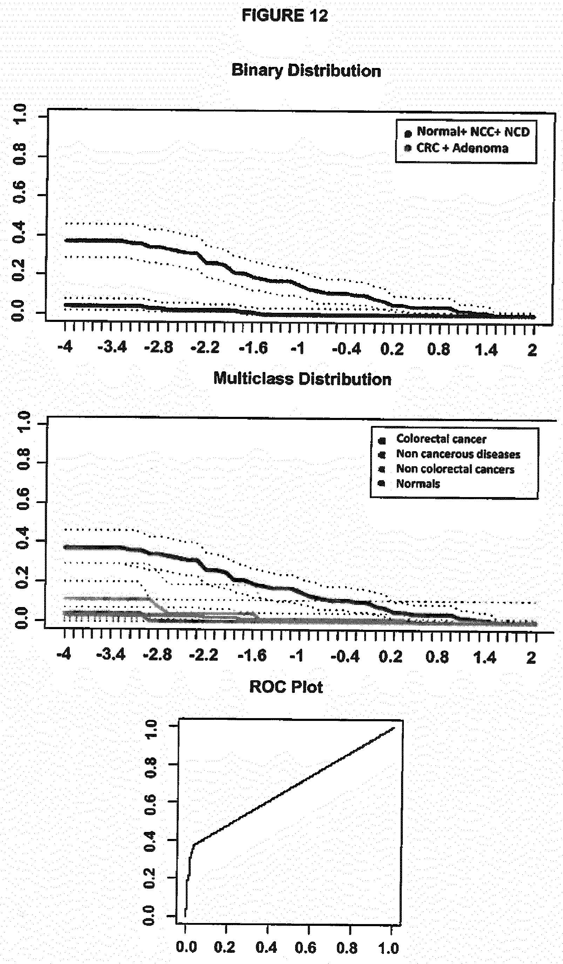

Figure 12 provides an overview of the performance of the RASSF2 HM assay according to Example 2, in all samples.

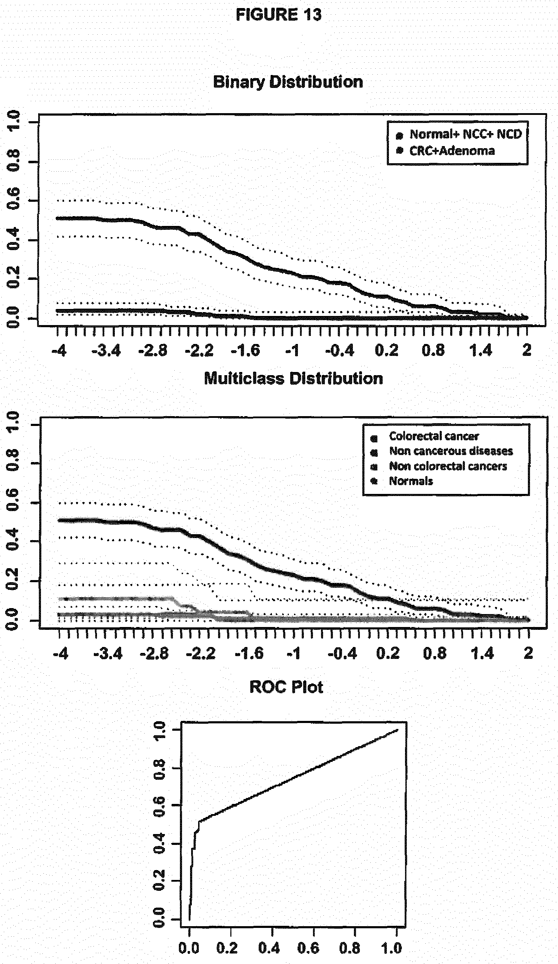

Figure 13 provides an overview of the performance of the Septin 9 HM assay according to Example 2, in all samples.

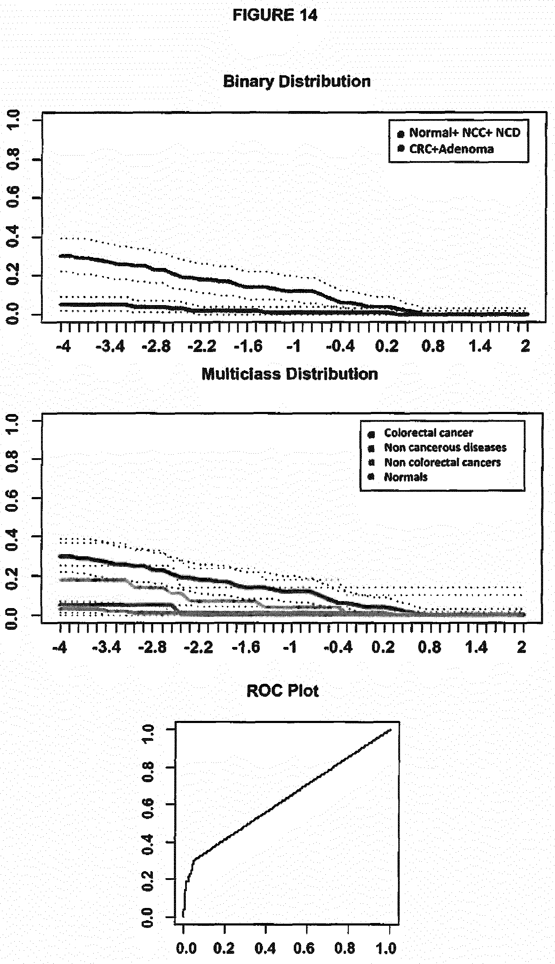

Figure 14 provides an overview of the performance of the SND1 HM assay according to Example 2, in all samples.

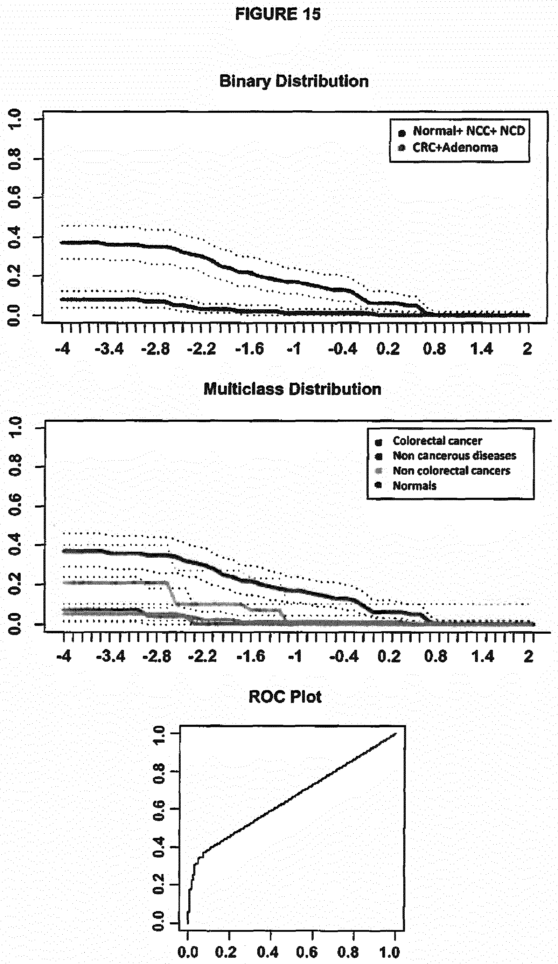

Figure 15 provides an overview of the performance of the PCDHGC3 HM assay according to Example 2, in all samples.

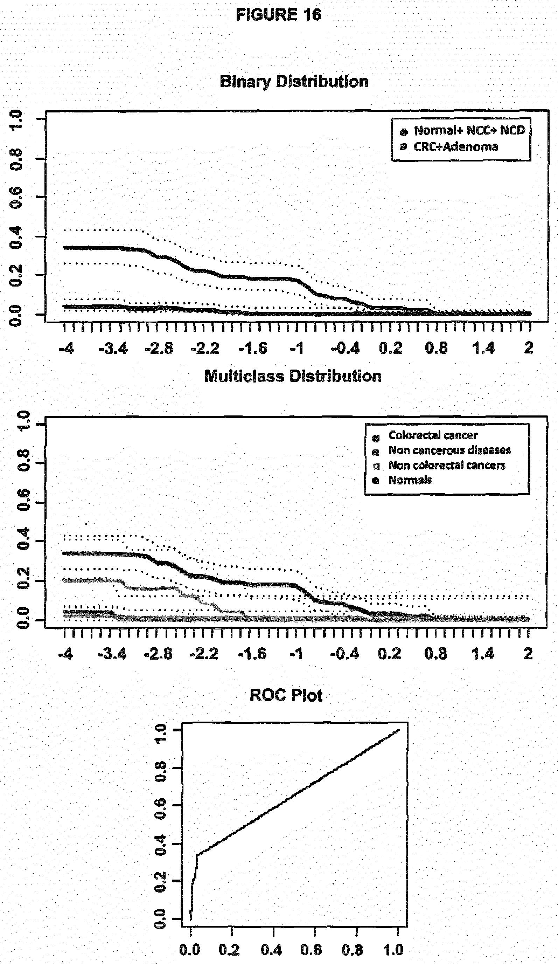

Figure 16 provides an overview of the performance of the TFAP2E HM assay according to Example 2, in all samples.

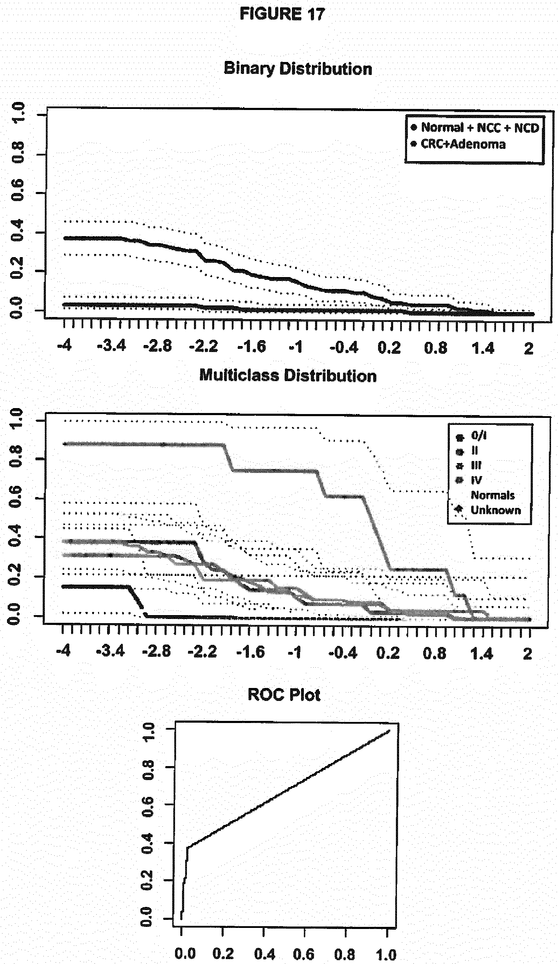

Figure 17 provides an overview of the performance of the RASSF2 HM assay according to Example 2, in all colorectal carcinoma and normal colorectal tissue samples.

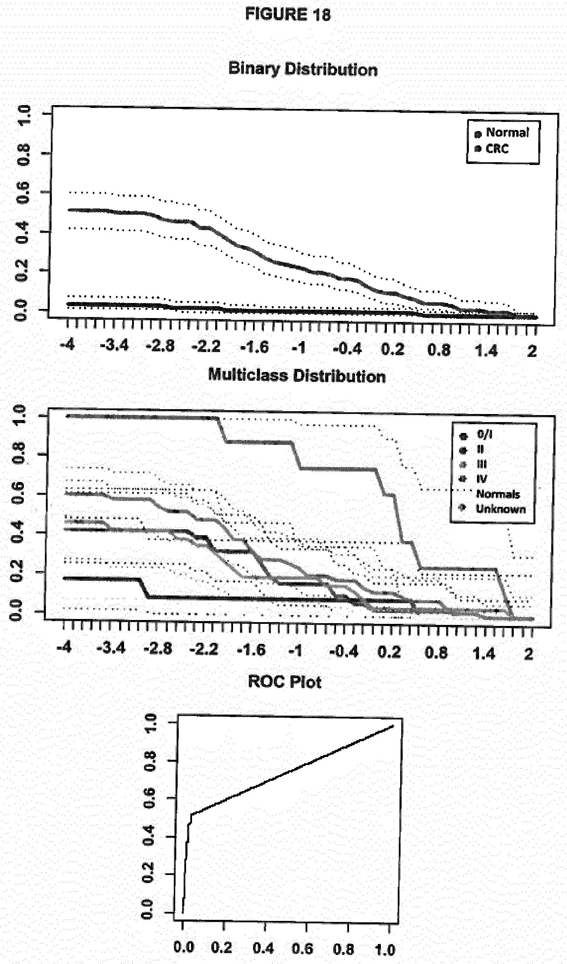

Figure 18 provides an overview of the performance of the Septin 9 HM assay according to Example 2, in all colorectal carcinoma and normal colorectal tissue samples.

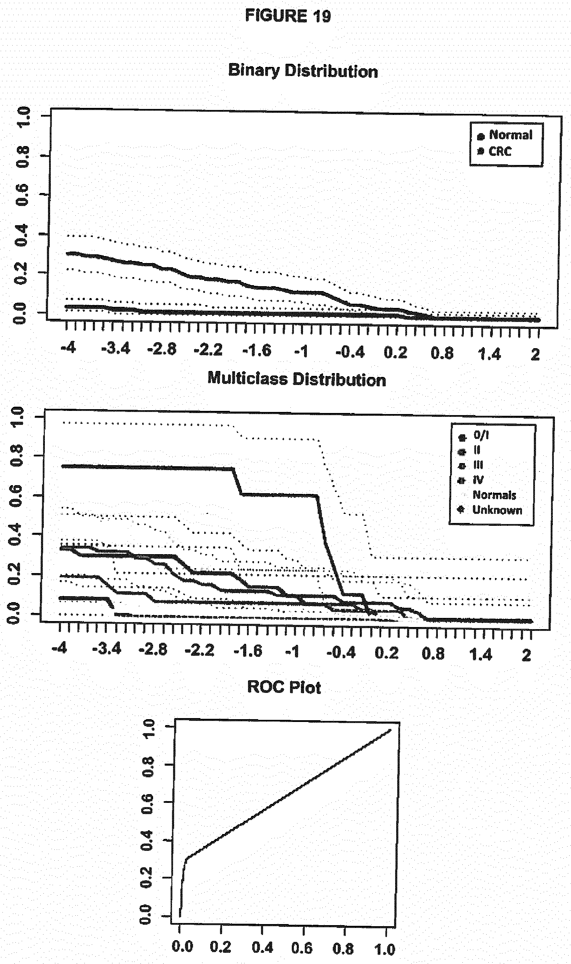

Figure 19 provides an overview of the performance of the SND1 HM assay according to Example 2, in all colorectal carcinoma and normal colorectal tissue samples.

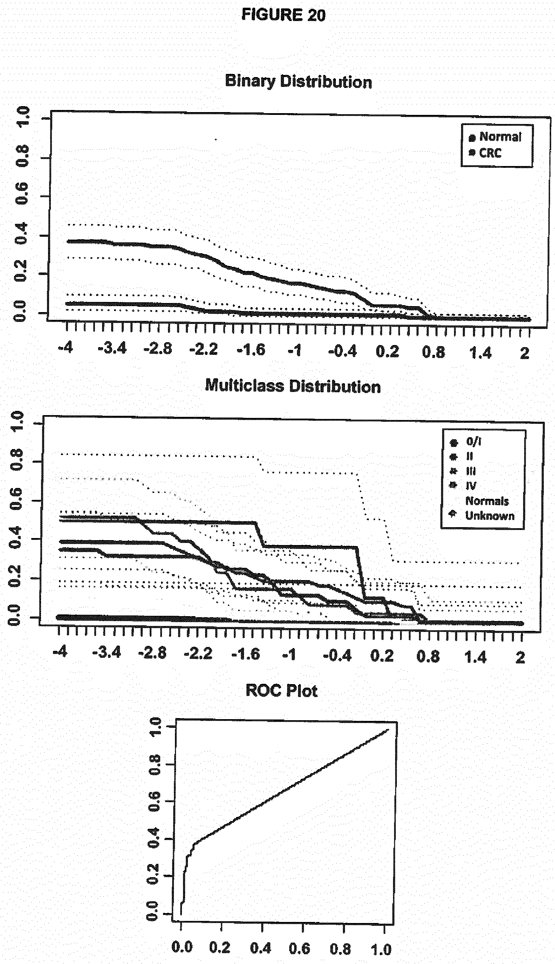

Figure 20 provides an overview of the performance of the PCDHGC3 HM assay according to Example 2, in all colorectal carcinoma and normal colorectal tissue samples.

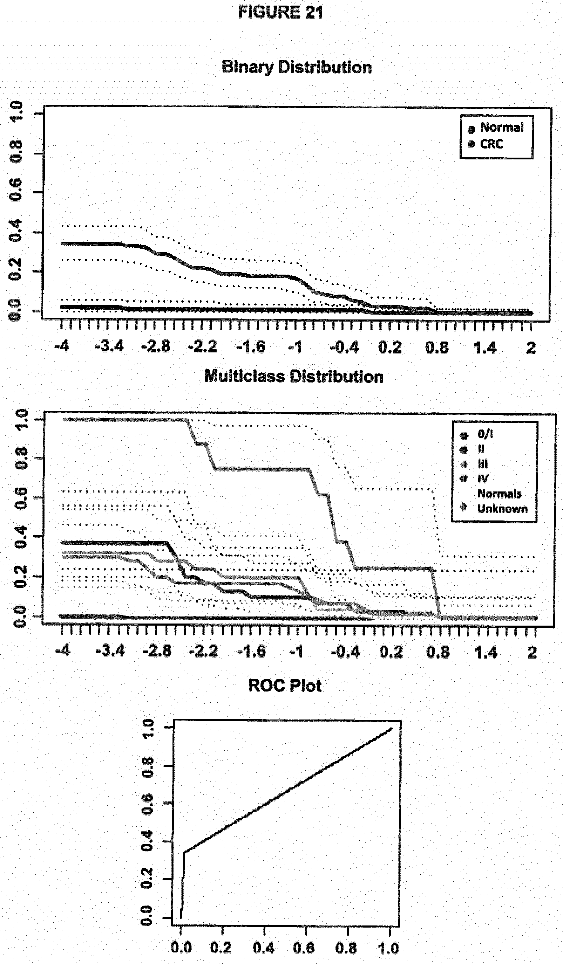

Figure 21 provides an overview of the performance of the TFAP2E HM assay according to Example 2, in all colorectal carcinoma and normal colorectal tissue samples.

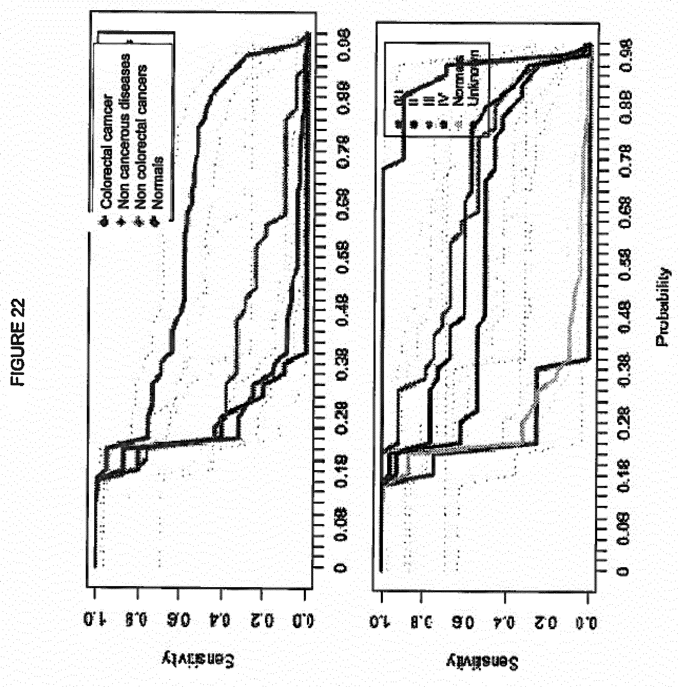

Figures 22 to 26 provide an overview of the log mean methylation measured by means of combinations HM assays (gene panels) according to Example 2. Each figures consists of two plots, The upper plot shows all samples (Normals, Non Colorectal Disease, Non-Coloretal Cancers and all CRC stages), the lower plot shows only Normaland CRC samples.
sensitivity is shown on the Y-axis, DNA methylation measured in (log 10 ng/mL) is shown on the X-axis.
Figure 22 provides an overview of the performance of the Septin 9+TFAP2E+RASSF2+PCDHGC3+SND1 assays.

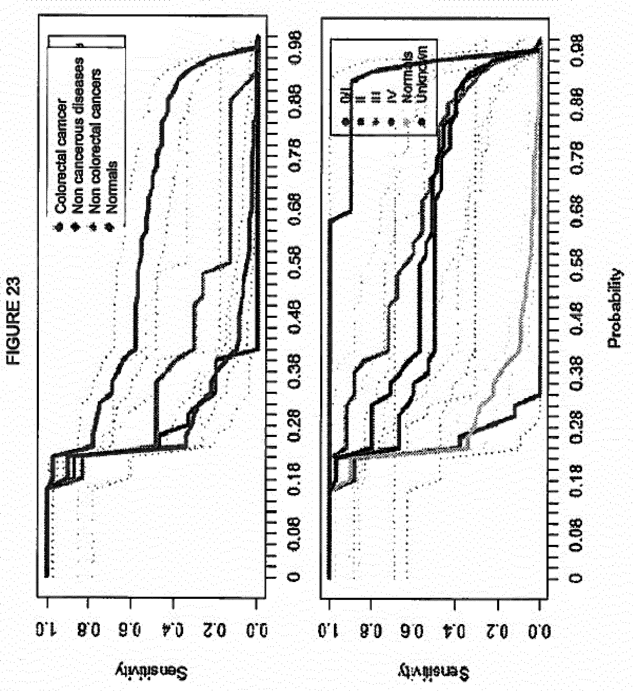

Figure 23 provides an overview of the performance of the Septin 9+TFAP2E+RASSF2+PCDHGC3 assays.

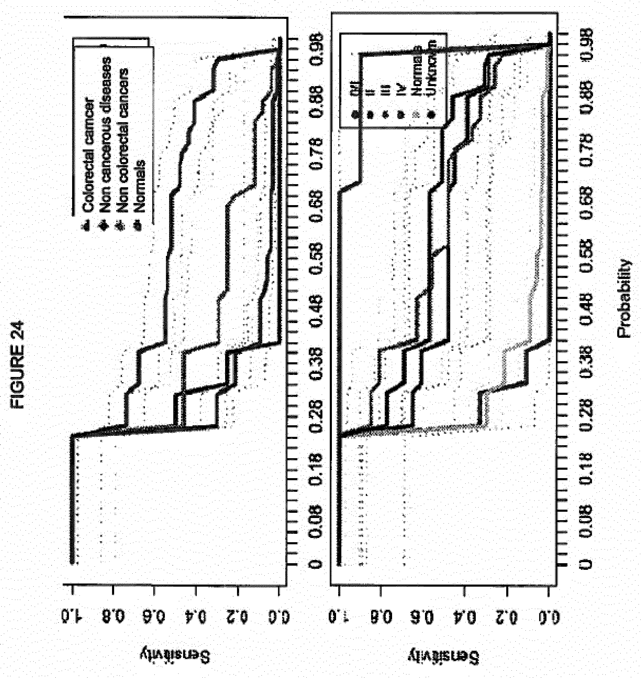

Figure 24 provides an overview of the performance of the Septin 9+TFAP2E+RASSF2 assays.

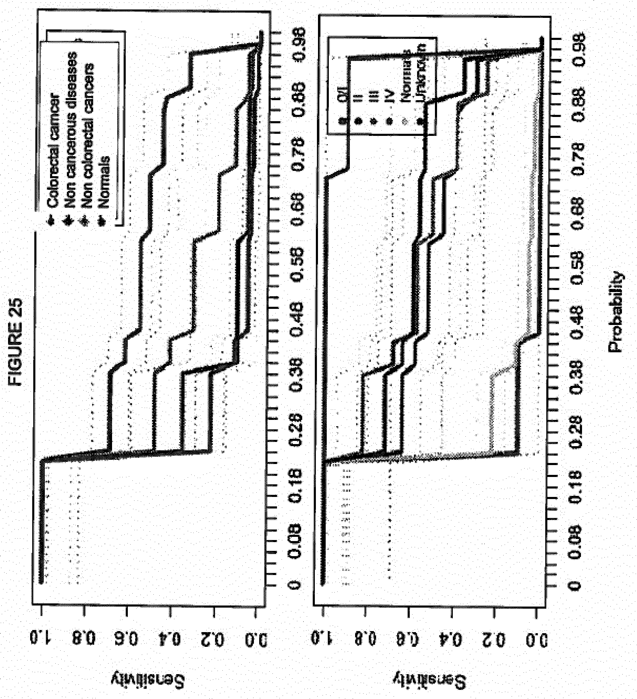

Figure 25 provides an overview of the performance of the Septin 9+TFAP2E assays.

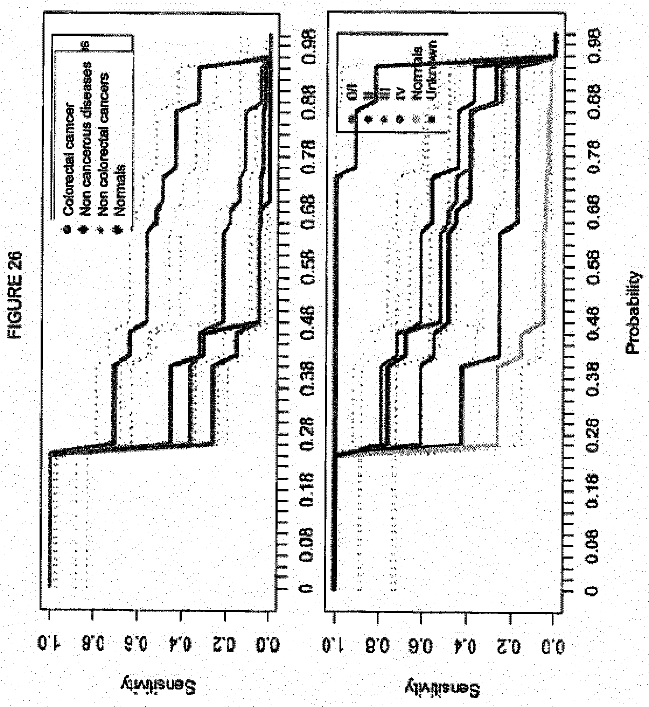

Figure 26 provides an overview of the performance of the Septin 9+RASSF2 assays.

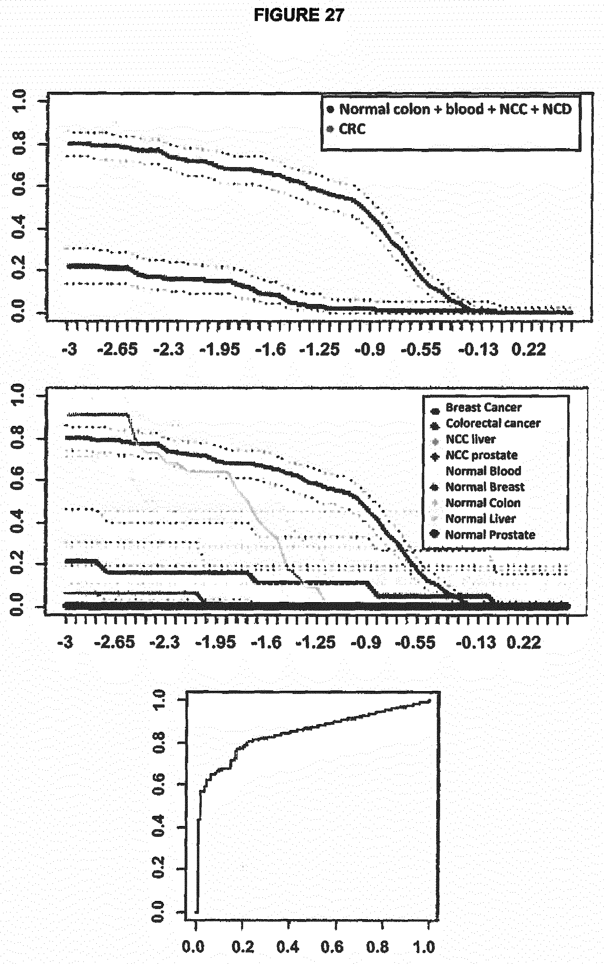

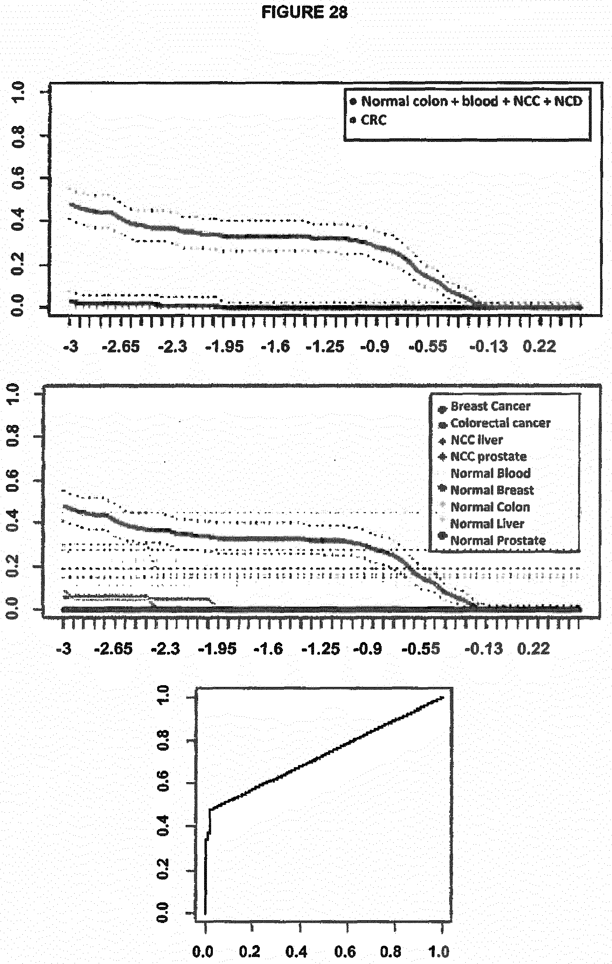

Figures 27 and 28 provide an overview of the methylation measured by means of the MSP assay according to Example 1. Each figure consists of three plots, the plots at the top and in the middle provide the binary and multiclass analysis respectively, sensitivity is shown on the Y-axis, DNA methylation is shown on the X-axis.

In each figure the plot at the bottom provides an ROC wherein sensitivity is shown on the Y-axis and 1-specificity is shown on the X-axis. Figure 27 provides an overview of the MRPS21 assay according to Example 1. Figure 28 provides an overview of the DOCK10 assay according to Example 1.

### SUMMARY OF THE INVENTION

The present invention provides a method for detecting cell proliferative disorders in a subject comprising determining the expression levels of PCDHGC3 in a biological sample isolated from said subject wherein underexpression and/or CpG methylation is indicative of the presence or class of said disorder.

Aspects of the invention provide a method for detecting cell proliferative disorders in a subject comprising determining the expression levels of the PCDHGC3 gene or the PCDHGC3 gene and Septin 9 or the PCDHGC3 gene and Septin 9 and at least one gene selected from the group consisting of RASSF2, TFAP2E, SND1, EDNRB, STOM, GLI3, RXFP3, LimK1, GPR73L1. PCDH10, DOCK10 and MRPS21 . Preferably said group consists of the genes RASSF2, TFAP2E , SND1.

Preferably the expression of a plurality of said genes is determined. Preferably the expression of 2, 3 or 4 of said genes is determined. Preferably the expression levels of one the following combinations of genes is determined:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

Various aspects of the present invention provide an efficient and unique genetic marker, whereby expression analysis of said marker enables the detection of cellular proliferative disorders with a particularly high sensitivity, specificity and/or predictive value. In the context of colorectal carcinoma the inventive testing methods have particular utility for the screening of at-risk populations. The inventive methods have advantages over prior art methods (including the industry standard FOBT), because of improved sensitivity, specificity and likely patient compliance.

The methods and kits of the present invention are utilised
for detecting colorectal carcinoma.

In one embodiment the invention provides a method for detecting colorectal carcinomas in a subject comprising determining the expression levels of at least the PCDHGC3 gene or the PCDHGC3 gene and Septin 9 or the PCDHGC3 gene and Septin 9 and one gene selected from the group consisting of RASSF2, TFAP2E , SND1, EDNRB, STOM, GLI3, RXFP3, LimK1, GPR73L1, PCDH10, DOCK10 and MRPS21 in a biological sample isolated from said subject wherein underexpression and/or CpG methylation is indicative of the presence or class of said disorder. Preferably said group consists of the genes RASSF2, TFAP2E , SND1 & PCDHGC3.

Preferably the presence, absence or level of mRNA expression of a plurality of said genes is determined. Preferably the expression of 2, 3 or 4 of said genes is determined.

In one embodiment said expression level is determined by detecting the presence, absence or level of mRNA transcribed from said gene. It is particularly preferred that said method comprises determining the expression levels of the PCDHGC3 gene or the PCDHGC3 gene and Septin 9 or the PCDHGC3 gene and Septin 9 and of at least one gene selected from the group consisting of RASSF2, TFAP2E, SND1 EDNRB, STOM, GLI3, RXFP3, LimK1, GPR73L1, PCDH10, DOCK10 and MRPS21 . Preferably said group consists of the genes RASSF2, TFAP2E, SND1 & PCDHGC3. Preferably the expression of 2, 3 or 4 of said genes is determined. Preferably the expression levels of one the following combinations of genes is determined:
- Septin 9+RASSF2+TFAP2E+PCDHGC3+SND1
   Septin 9+RASSF2+TFAP2E+PCDHGC3
- Septin 9+TFAP2E+PCDHGC3
- Septin 9+PCDHGC3

In a further embodiment said expression level is determined by detecting the presence, absence or level of a polypeptide encoded by said gene or sequence thereof

In a further preferred embodiment said expression is determined by detecting the presence or absence of CpG methylation within said gene, wherein the presence of methylation indicates the presence of a cell proliferative disorder. Said method comprises the following steps: i) contacting genomic DNA isolated from a biological sample (preferably selected from the group consisting of blood plasma, blood serum, whole blood, isolated blood cells, cells isolated from the blood) obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence of the PCDHGC3 gene or the PCDHGC3 gene and Septin 9 or the PCDHGC3 gene and Septin 9 and at least one gene selected from the group consisting of RASSF2, TFAP2E, SND1" EDNRB, STOM, GLI3, RXFP3, LimK1, GPR73L1, PCDH10, DOCK10 and MRPS21 ; and ii) detecting colorectal carcinoma, at least in part.

Preferably said group consists of the genes RASSF2, TFAP2E , SND1 & PCDHGC3 and/or their promotor or regulatory regions. Preferably a plurality of target regions are analyzed. Preferably 2, 3 or 4 of target regions are analyzed.

Preferably the target region comprises, or hybridizes under stringent conditions to a sequence of at least 16 contiguous nucleotides of at least one sequence selected from the group consisting of SEQ ID NO: 1 TO SEQ ID NO: 11 AND SEQ ID NO: 132 TO SEQ ID NO: 133. Preferably said group consists of SEQ ID NO: 1; SEQ ID NO: 2; SEQ ID NO: 1 & SEQ ID NO: 11.

In i) it is particularly preferred that the nucleotide sequence of said target region comprises at least one CpG dinucleotide sequence of the PCDHGC3 gene or the PCDHGC3 gene and Septin 9 or the PCDHGC3 gene and Septin 9 and at least one gene selected from the group consisting of RASSF2, TFAP2E, SND1,,EDNRB, STOM, GLI3, RXFP3, LimK1, GPR73L1, PCDH10, DOCK10 and MRPS21 . Preferably said group consists of the genes RASSF2, TFAP2E , SND1 & PCDHGC3 and/or their promotor or regulatory regions.

Preferably the target regions consist of one the following combinations of genes:
- Septin 9+RASSF2+TFAP2E+PCDHGC3+SND1
- Septin 9+RASSF2+TFAP2E+PCDHGC3
-
- Septin 9+TFAP2E+PCDHGC3
- Septin 9+PCDHGC3

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%.

The method is novel as no methods currently exist that enable the detection of cancer by analysis of body fluids, with a sensitivity and specificity high enough for use in a commercially available and regulatory body approved assay. For example, current methods used to detect and diagnose colorectal carcinoma include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a clear technical advantage over current methods known in the art in that it is anticipated that at least for colorectal carcinoma screening patient compliance for a single body fluid based test will be higher than the triplicate analysis of stool currently recommended for FOBT.

A particular embodiment the method comprises the use of at least the PCDHGC3 gene or the PCDHGC3 gene and the Septin 9 gene or the PCDHGC3 gene and the Septin 9 gene and one gene selected from the group consisting of RASSF2, TFAP2E , SND1, PCDHGC3, EDNRB, STOM, GLI3, RXFP3, LimK1, GPR73L1, PCDH10, DOCK10 and MRPS21 as a marker for the detection and distinguishing of cellular proliferative disorders. Preferably said group consists of the genes RASSF2, TFAP2E andSND1. and/or their promotor or regulatory regions.

A preferred embodiment the method comprises the use of at least least the PCDHGC3 gene or the PCDHGC3 gene and the Septin 9 gene or the PCDHGC3 gene and the Septin 9 gene andone gene selected from the group consisting of RASSF2, TFAP2E , SND1, EDNRB, STOM, GLI3, RXFP3, LimK1, GPR73L1, PCDH10, DOCK10 and MRPS21 as a marker for the detection and distinguishing of cellular proliferative disorders.. Preferably said group consists of the genes RASSF2, TFAP2E and SND1 and/or their promotor or regulatory regions.

Preferably a plurality of genes are analyzed. Preferably 2, 3 or 4 genes are analyzed. Particularly preferred are the following combinations of genes:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

The present invention is particularly suited for the detection of colorectal cancer.

The methods and kits of the present invention are particularly
effective in the detection of colorectal cancers. Said use of the gene may be enabled by means of any analysis of the expression of the gene, by means of mRNA expression analysis or protein expression analysis. However, in the most preferred embodiment of the invention, the detection of colorectal cell proliferative disorders is enabled by means of analysis of the *methylation status* of at least least the PCDHGC3 gene or the PCDHGC3 gene and the Septin 9 gene or the PCDHGC3 gene and the Septin 9 gene and one gene selected from the group consisting of RASSF2, TFAP2E , SND1, PCDHGC3, EDNRB, STOM, GLI3, RXFP3, LimK1, GPR73L1, PCDH10, DOCK10, and MRPS21, and their promoter or regulatory elements. Preferably said group consists of the genes RASSF2, TFAP2E and SND1 and/or their promotor or regulatory regions. Preferably a plurality of genes are analyzed. Preferably 2, 3 or 4 genes are analyzed.

It is further prefered that the detection of colorectal cell proliferative disorders is enabled by means of analysis of the *methylation status* of the gene PCDHGC3 in combination with Septin 9 and at least one gene selected from the group consisting of RASSF2, TFAP2E , SND1,, EDNRB, STOM, GLI3, RXFP3, LimK1, GPR73L1, PCDH10, DOCK10 and MRPS21 , and its promoter or regulatory elements. Preferably said group consists of the genes RASSF2, TFAP2E andSND1 and/or their promotor or regulatory regions. Preferably a plurality of genes are analyzed. Preferably 2, 3 or 4 genes are analyzed. Particularly preferred are the following combinations of genes:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

The disclosure provides a method for the analysis of biological samples for features associated with the development of cellular proliferative disorders, the method characterized in that at least one nucleic acid, or a fragment thereof, from the group consisting of SEQ ID NO: 1 TO SEQ ID NO: 11 AND SEQ ID NO: 132 TO SEQ ID NO: 133 is contacted with a reagent or series of reagents capable of distinguishing between methylated and non methylated CpG dinucleotides within the genomic sequence, or sequences of interest.. In a preferred embodiment said group consists of SEQ ID NO: 1; SEQ ID NO: 2; SEQ ID NO: 7 & SEQ ID NO: 11.

It is further prefered that at least one nucleic acid, or a fragment thereof, of SEQ ID NO: 12 TO SEQ ID NO: 14 and at least one nucleic acid, or a fragment thereof, from the group consisting of SEQ ID NO: 1 TO SEQ ID NO: 11 AND SEQ ID NO: 132 TO SEQ ID NO: 133 (or more preferably SEQ ID NO: 1; SEQ ID NO: 2; SEQ ID NO: 7 & SEQ ID NO: 11) is contacted with a reagent or series of reagents capable of distinguishing between methylated and non-methylated CpG dinucleotides.

The present disclosure provides a method for ascertaining epigenetic parameters of
genomic DNA associated with the development of neoplastic cellular proliferative disorders (e.g. cancers). The method has utility for the improved diagnosis, treatment and monitoring of said diseases.

Preferably, the source of the test sample is selected from the group consisting of cells or cell lines, histological slides, biopsies, paraffin-embedded tissue, body fluids, ejaculate, stool, urine, blood, and combinations thereof. More preferably, the source is selected from the group consisting of stool, blood plasma, blood serum, whole blood, isolated blood cells, cells isolated from the blood obtained from the subject.

Specifically, the present disclosure provides a method for detecting colorectal carcinoma including at early stages, comprising:
obtaining a biological sample comprising genomic nucleic acid(s); contacting the nucleic acid(s), or a fragment thereof, with one reagent or a plurality of reagents sufficient for distinguishing between methylated and non-methylated CpG dinucleotide sequences within at least one target sequence of the subject nucleic acid, wherein the target sequence comprises, or hybridizes under stringent conditions to, a sequence comprising at least 16 contiguous nucleotides of a sequence selected from the group consisting SEQ ID NO: 1 to SEQ ID NO: 11 AND SEQ ID NO: 132 TO SEQ ID NO: 133 (more preferably SEQ ID NO: 1; SEQ ID NO: 2; SEQ ID NO: 7 & SEQ ID NO: 11), said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. It is further preferred that at least two target sequence s are analyzed wherein a first target sequence comprises, or hybridizes under stringent conditions to, a sequence comprising at least 16 contiguous nucleotides of a sequence selected from the group consisting SEQ ID NO: 12 to SEQ ID NO: 14 and a second target region comprises, or hybridizes under stringent conditions to, a sequence comprising at least 16 contiguous nucleotides of a sequence selected from the group consisting SEQ ID NO: 1 TO SEQ ID NO: 11 AND SEQ ID NO: 132 TO SEQ ID NO: 133 (more preferably SEQ ID NO: 1; SEQ ID NO: 2; SEQ ID NO: 7 & SEQ ID NO: 11).

Preferably, distinguishing between methylated and non-methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 15 TO SEQ ID NO: 36 and SEQ ID NO: 43 TO SEQ ID NO: 64 AND SEQ ID NO: 134 TO SEQ ID NO: 141, and contiguous regions thereof corresponding to the target sequence. It is further preferred that said consists of SEQ ID NOS: 15, 16, 43, 44, 17; 18, 45, 46, 27, 28, 55, 56,35 36 63 & 64.

It is further preferred that distinguishing between methylated and non-methylated CpG dinucleotide sequences within the target sequence comprises methylation state-dependent conversion or non-conversion of at least one such CpG dinucleotide sequence to the corresponding converted or non-converted dinucleotide sequence within SEQ ID NO: 37 TO SEQ ID NO: 42 AND SEQ ID NO: 65 TO SEQ ID NO: 70 and at least one such CpG dinucleotide sequence to the corresponding converted or non-converted dinucleotide sequence selected from the group consisting of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the group consisting of SEQ ID NOS: 15,16, 43, 44,17, 18, 45, 46, 27, 28, 55, 56,35 36 63 & 64), and contiguous regions thereof corresponding to the target sequence.

Additional aspects of the disclosure provide a method for the detection of neoplastic cellular proliferative disorders, most preferably colorectal carcinoma, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 15 TO SEQ ID NO: 36 AND SEQ ID NO: 43 TO SEQ ID NO: 64 AND SEQ ID NO: 134 TO SEQ ID NO: 141 (and more preferably the group consisting of SEQ ID NOS: 15,16, 43, 44, 17, 18, 45, 46, 27, 28, 55, 56,35 36 63 & 64), and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one, but more preferably a plurality of CpG dinucleotides of a sequence selected from the group consisting of SEQ ID NO: 1 TO SEQ ID NO: 11 AND SEQ ID NO: 132 TO SEQ ID NO: 133 (and more preferably the group consisting of SEQ ID NO: 1; SEQ ID NO: 2; SEQ ID NO: 7 & SEQ ID NO: 11). It is further preferred that said method additionally comprises contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 37 TO SEQ ID NO: 42 AND SEQ ID NO: 65 TO SEQ ID NO: 70, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of a sequence selected from the group consisting of SEQ ID NO: 12 TO SEQ ID NO: 14.

Preferably, determining comprises use of at least one method selected from the group consisting of: I) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the group consisting of SEQ ID NOS: 15, 16, 43, 44, 17, 18, 45, 46, 27, 28, 55, 56, 35 36 63 & 64), and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the group consisting of SEQ ID NOS: 15, 16, 43, 44,17, 18, 45, 46, 27, 28, 55, 56,35 36 63 & 64), and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the group consisting of SEQ ID NOS: 15, 16, 43, 44,17, 18, 45, 46, 27, 28, 55, 56,35 36 63 & 64), and complements thereof, and extending at least one such hybridized nucleic acid molecule by at least one nucleotide base; and iv) sequencing of the amplificates.

Further aspects of the disclosure provide a method for the analysis (i.e. detection) of cell
proliferative disorders, comprising: obtaining a biological sample having subject genomic DNA; extracting the genomic DNA; contacting the genomic DNA, or a fragment thereof, comprising one or more sequences selected from the group consisting of SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11) or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of at least one genomic sequence selected from the group consisting of SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11), or an average, or a value reflecting an average methylation state of a plurality of CpG dinucleotide sequences thereof. Preferably, the digested or undigested genomic DNA is amplified prior to said determining.

It is further preferred that said method additionally comprises determining, based on a presence or absence of, or on property of at least one such fragment, the methylation state of at least one CpG dinucleotide sequence of at least one genomic sequence selected from the group consisting of SEQ ID NO: 12 TO SEQ ID NO: 14, or an average, or a value reflecting an average methylation state of a plurality of CpG dinucleotide sequences thereof.

Additional aspects of the disclosure provide novel genomic and chemically modified nucleic acid
sequences, as well as oligonucleotides and/or PNA-oligomers for analysis of cytosine methylation patterns within sequences from the group consisting of SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11).

### BRIEF DESCRIPTION OF THE DRAWINGS

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nuclei acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily- Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401 A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specif restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5. ⁴Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The term "gene" shall be taken to include all transcript variants thereof (e.g. the term "Septin 9" shall include for example its truncated transcript Q9HC74) and all promoter and regulatory elements thereof. Furthermore as a plurality of SNPs are known within said gene the term shall be taken to include all sequence variants thereof.

### Overview:

The present disclosure provides a method for detecting cell proliferative disorders (preferably colorectal carcinoma) in a subject comprising determining the expression levels of at least the PCDHGC3 gene or the PCDHGC3 gene and the Septin 9 gene or the PCDHGC3 gene and the Septin 9 gene and one gene selected from the group consisting of RASSF2, TFAP2E , SND1,, EDNRB, STOM, GLI3, RXFP3, LimK1, GPR73L1, PCDH10, DOCK10 and MRPS21 in a biological sample isolated from said subject wherein underexpression and/or CpG methylation is indicative of the presence or class of said disorder. Preferably said group consists of the genes RASSF2, TFAP2E and SND1. and/or their promotor or regulatory regions.

Said markers may be used for the diagnosis of cellular proliferative disorders (preferably cancer), most preferably colorectal carcinoma. Said method is characterized in that underexpression and/or the presence of CpG methylation is indicative of the presence of a neoplastic cell proliferative disorder or colorectal carcioma and the absence thereof is indicative of the absence thereof. It is further preferred that the expression levels of the gene least the PCDHGC3 gene or the PCDHGC3 gene and the Septin 9 gene or the PCDHGC3 gene and the Septin 9 gene and and of at least one gene selected from the group consisting of RASSF2, TFAP2E, SND1, EDNRB, STOM, GLI3, RXFP3, LimK1, GPR73L1, PCDH10, DOCK10 and MRPS21 in a biological sample isolated from said subject is determined wherein underexpression and/or CpG methylation is indicative of the presence or class of said disorder. Preferably said group consists of the genes RASSF2, TFAP2E and SND1and/or their promotor or regulatory regions.

Preferably a plurality of genes are analyzed. Preferably 2, 3 or 4 genes are analyzed, Particularly preferred are the following combinations of genes:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

The markers of the present invention are particularly efficient in detecting or distinguishing between colorectal cell proliferative disorders, thereby providing improved means for the early detection and thus improved treatment of said disorders.

In addition to the embodiments above wherein the methylation analysis of at least the PCDHGC3 gene or the PCDHGC3 gene and the Septin 9 gene or the PCDHGC3 gene and the Septin 9 gene and one gene selected from the group consisting of RASSF2, TFAP2E , SND1, EDNRB, STOM, GLI3, RXFP3, LimK1, GPR73L1, PCDH10, DOCK10 and MRPS21 is analyzed, the invention presents further panels of genes comprising at least one gene selected from the group consisting of RASSF2, TFAP2E , SND1, EDNRB, STOM, GLI3, RXFP3, LimK1, GPR73L1, PCDH10, DOCK10 and MRPS21 with novel utility for the detection of cancers, in particular colorectal cancer.

Preferably said panels of genes comprise at least one gene selected from the group consisting of RASSF2, TFAP2E and SND1 and/or their promotor or regulatory regions.

In a preferred embodiment of the method said panel comprises the gene least the PCDHGC3 gene or the PCDHGC3 gene and the Septin 9 gene or the PCDHGC3 gene and the Septin 9 gene and at least one gene selected from the group consisting of RASSF2, TFAPGE, SND 1, EDNRB, STOM, GLI3, RXFP3, LimK1, GPR73L1, PCDH10, DOCK10 and MRPS21 . Preferably said group consists of the genes RASSF2, TFAP2E and SND1 and/or their promotor or regulatory regions.

Particularly preferred are the following combinations of genes:
- Septin 9+RASSF2+TFAP2E+PCDHGC3+SND1
- Septin 9+RASSF2+TFAP2E+PGDHGC3
- Septin 9+TFAP2E+PCDHGC3
- Septin 9+PCDHGC3

In a first further embodiment the present invention is based upon the analysis of CpG methylaton status of at least least the PCDHGC3 gene or the PCDHGC3 gene and the Septin 9 gene or the PCDHGC3 gene and the Septin 9 gene and one gene selected from the group consisting of RASSF2, TFAP2E , SND1,, EDNRB, STOM, GLI3, RXFP3, LimK1, GPR73L1, PCDH10, DOCK10 and MRPS21 . It is preferred that the sequences of said genes are as according to Table 1. Preferably said group consists of the genes RASSF2, TFAP2E , SND1 and/or their promoter or regulatory regions. In said embodiment it is preferred that the sequences of said genes are as according to Table 7.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6,1994; Zeschnigk M, et al., Hum Mol Genet, 6:387-95, 1997; Feil R, et al., Nucleic Acids Res., 22:695-, 1994; Martin V, et al., Gene, 157:261-4, 1995; WO 9746705 and WO 9515373).

The present disclosure provides for the use of the bisulfite technique, in combination
with one or more methylation assays, for determination of the methylation status of CpG dinucleotide sequences within at least one sequence selected from the group consisting SEQ ID NO: 1 to SEQ ID NO: 11 AND SEQ ID NO: 132 TO SEQ ID NO: 133. Particularly preferred is the sub-group thereof consisting of SEQ ID NO: 1; SEQ ID NO: 2; SEQ ID NO: 7 & SEQ ID NO: 11.

Genomic CpG dinucleotides can be methylated or unmethylated (alternatively known as up- and down- methylated respectively). However the methods of the present disclosure are
suitable for the analysis of biological samples of a heterogeneous nature e.g. a low concentration of tumor cells within a background of blood or stool. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) the genes or genomic sequence selected from the group consisting of RASSF2, TFAP2E , SND1, PCDHGC3, EDNRB, STOM, GLI3, RXFP3, LimK1, GPR73L1, PCDH10; DOCK10 and MRPS21 and the gene SEPTIN 9.

According to the present disclosure, determination of the methylation status of CpG
dinucleotide sequences within SEQ ID NO: 1 TO SEQ ID NO: 11 AND SEQ ID NO: 132 TO SEQ ID NO: 133 (and more preferably the sub-group thereof consisting of SEQ ID NO: 1: SEQ ID NO: 2; SEQ ID NO: 7 & SEQ ID NO: 11) has utility both in the diagnosis and characterization of cellular proliferative disorders. *Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g.,* the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

*COBRA.* COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (e.g., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specif PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents *(e.g.,* as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1 % methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MethyLigth*™. The MethyLight™ assay is a high-throughput quantitative methylation assay that utilizes fluorescence-based real-time PCR (Taqman□) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil), Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight™ assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight™ process can by used with any suitable probes e.g. "TaqMan®", Lightcycler® etc.... For example, double-stranded genomic DNA is treated with sodium bisulfite and subjected to one of two sets of PCR reactions using TaqMan® probes; e.g., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical MethyLight□-based kit) for MethyLight™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM™ (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM™ process can by used with any suitable probes e.g. "TaqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical QM™ -based kit) for QM™ analysis may include, but are not limited to: PCR primers for specific gene (or bisulfite treated DNA sequence or CpG island); Taqman® or Lightcycler® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides.

Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

The Genomic Sequence According to SEQ ID NO: 1 TO SEQ ID NO: 11 AND SEQ ID NO: 132 TO SEQ ID NO: 133 (and more preferably the sub-group thereof consisting_of SEQ ID NO: 1; SEQ ID NO: 2; SEQ ID NO: 7 & SEQ ID NO: 11), and Non-naturally Occurring Treated Variants Thereof According to SEQ ID NO: 15 TO SEQ IQ NO: 36 AND SO ID NO: 43 TO SEQ ID NO: 64 AND SEQ ID NO: 134 TO SEQ ID NO: 141 (and more preferably the sub-group thereof consisting of SEQ ID NOS: 15, 16, 43, 44, 17, 18, 45, 46, 27, 28, 55, 56, 35 36 63 & 64), were Determined to have Novel Utility for the Early Detection of Cellular Proliferative Disorders, in Particular Colorectal Cell Proliferative Disorders

In one aspect, the method comprises the following steps: i) contacting genomic DNA (preferably isolated from body fluids) obtained from the subject with at least one reagent, or series of reagents that distinguishes between methylated and non-methylated CpG dinucleotides within at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 (including their promoter and regulatory regions); and li) detecting, or detecting and distinguishing between or among colon cell proliferative disorders. In a preferred aspect, the accuracy of said method is increased by additionally determining the methylation status of the gne SEPTIN 9 by means of said method steps. Preferably said group consists of the genes RASSF2, TFAP2E , SND1 & PCDHGC3 and/or their promoter or regulatory regions.

Particularly preferred are the following combinations of genes:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

Preferably, the sensitivity is from about 75% to about 96%, or from about 80% to about 90%, or from about 80% to about 85%. Preferably, the specificity is from about 75% to about 96%, or from about 80% to about 90%, or from about 80% to about 85%.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising cellular matter are suitable for us e in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood.

The genomic DNA sample is then treated with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of at least one sequence selected from the group consisting of SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO:11) respectively, wherein said contiguous nucleotides comprise at least one CpG dinucleotide sequence. It is further preferred that said reagent, or series of reagents distinguishes between methylated and non-methylated CpG dinucleotides within at least two target regions of the genomic DNA, wherein a first the target region comprises, or hybridizes under stringent conditions to a sequence of at least 16 contiguous nucleotides of at least one sequence selected from the group consisting of SEQ ID NO: 12 TO SEQ ID NO: 14 and a second target region that comprises, or hybridizes under stringent conditions to a sequence of at least 16 contiguous nucleotides of at least one sequence selected from the group consisting of SEQ ID NO: 1 TO SEQ ID NO: 11 AND SEQ ID NO: 132 TO SEQ ID NO: 133(and more preferably the sub-group thereof consisting of SEQ ID NO:1; SEQ ID NO: 2; SEQ ID NO: 7 & SEQ ID NO:11), wherein said contiguous nucleotides comprise at least one CpG dinucleotide sequence.

It is particularly preferred that said reagent converts cytosine bases which are unmethylated at the 5'-position to uracil, thymine, or another base which is dissimilar to cytosine in terms of hybridization behaviour. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behavior It is preferred that this treatment is carried out with bisulfite (hydrogen sulfite, disulfite) and subsequent alkaline hydrolysis. Such a treatment results in the conversion of SEQ ID NO: 1 - SEQ ID NO: 14 to SEQ ID NO: 43 - SEQ ID NO: 70 wherein said CpG dinucleotides are unmethylated and to SEQ ID NO: 15 - SEQ ID NO: 42 wherein said CpG dinucleotides are methylated.

The treated DNA is then analyzed in order to determine the methylation state of the target gene sequences (at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 prior to the treatment). It is particularly preferred that the target region comprises, or hybridizes under stringent conditions to at least 16 contiguous nucleotides of at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21. It is preferred that the sequence of said genes according to SEQ ID NO: 1 TO SEQ ID NO: 11 AND SEQ ID NO: 132 TO SEQ ID NO: 133 are analyzed.

It is further preferred that said group consists of the genes RASSF2, TFAP2E, SND1 & PCDHGC3 and/or their promotor or regulatory regions. In said embodiment it is preferred that the sequence of said genes according to SEQ ID NO: 1; SEQ ID NO: 2; SEQ ID NO: 7 & SEQ ID NO:11 are analyzed.

The method of analysis may be selected from those known in the art, including those listed herein. Particularly preferred are MethyLightTM, MSP and the use of blocking oligonucleotides (HeavyMethyl™) as described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the sub-group thereof consisting of SEQ ID NOS: 15, 16, 43, 44,17, 18, 45, 46, 27, 28, 55, 56,35 36 63 & 64) and sequences complementary thereto.

Aberrant methylation, more specifically hypermethylation of the genes or genomic sequence selected from the group consisting of RASSF2, TFAP2E , SND1, PCDHGC3, EDNRB, STOM, GLI3; RXFP3, LimK1, GPR73L1, PCDH10, DOCK10 and MRPS21 (including their promoter and/or regulatory regions) is associated with the presence of neoplastic cellular proliferative disorders, and is particularly prevalent in colorectal carcinomas. Accordingly wherein a biological sample presents within any degree of methylation, said sample should be determined as cancerous.

Analysis of one the genes or genomic sequence selected from the group consisting of RASSF2, TFAP2E , SND1, PCDHGC3, EDNRB, STOM, GLI3, RXFP3, LimK1, GPR73L1, PCDH10, DOCK10 and MRPS21 enables for the first time detecting, or detecting and distinguishing between or among colon cell proliferative disorders afforded with a sensitivity of greater than or equal to 80% and a specificity of greater than or equal to 80%. Preferably said group consists of the genes RASSF2, TFAP2E , SND1 *&* PCDHGC3 and/or their promotor or regulatory regions.

Sensitivity is calculated as:{detected neoplasia/all neoplasia) e.g. {detected colon neoplasia/all colon neoplasia); and specificity is calculated as (non-detected negatives/total negatives)

Preferably, the sensitivity is from about 75% to about 96%, or from about 80% to about 90%, or from about 80% to about 85%. Preferably, the specificity is from about 75% to about 96%, or from about 80% to about 90%, or from about 80% to about 85%.

Colon neoplasia is herein defined as all colon malignancies and adenomas greater than 1 cm., or subsets thereof. Negatives can be defined as healthy individuals.

In one aspect, the method discloses the use of at least least the PCDHGC3 gene
or the PCDHGC3 gene and the Septin 9 gene or the PCDHGC3 gene and the Septin 9 gene and one gene selected from the group consisting of RASSF2, TFAP2E, SND1;EDNRB, STOM, GLI3, RXFP3, LimK1, GPR73L1, PCDH10, DOCK10 and MRPS21 (or promoter and/or regulatory regions thereof) as a marker for the detection of cellular proliferative disorders (in particular colon disorders). Preferably said group consists of the genes RASSF2, TFAP2E, SND1 and/or their promoter or regulatory regions. Said method may be enabled by means of any analysis of the expression of an RNA transcribed therefrom or polypeptide or protein translated from said RNA, preferably by means of mRNA expression analysis or polypeptide expression analysis. Accordingly the present invention also provides diagnostic assays and methods, both quantitative and qualitative for detecting the expression of at least least the PCDHGC3 gene or the PCDHGC3 gene and the Septin 9 gene or the PCDHGC3 gene and the Septin 9 gene and one gene selected from the group consisting of RASSF2, TFAP2E , SND1,, EDNRB, STOM, GLI3, RXFP3, LimK1, GPR73L1, PCDH10, DOCK10 and MRPS21 in a subject and determining therefrom upon the presence or absence of cancer in said subject.

The disclosure further provides diagnostic assays and methods, both quantitative and
qualitative for detecting the expression of the gene PCDHGC3 in combination with at least one gene selected from the group consisting of Septin 9, RASSF2, TFAP2E , SND1, PCDHGC3, EDNRB, STOM, GLI3, RXFP3, LimK1, GPR73L1, PCDH10, DOCK10 and MRPS21 in a subject and determining therefrom upon the presence or absence of cancer in said subject.

Particularly preferred are the following combinations of genes:
- Septin 9+RASSF2+TFAP2E+PCDHGC3+SND1
- Septin 9+RASSF2+TFAP2E+PCDHGC3
-
- Septin 9+TFAP2E+PCDHGC3
- Septin 9+PCDHGC3

Aberrant expression of mRNA transcribed from the genes or genomic sequences selected from the group consisting of RASSF2, TFAP2E. SND1; PCDHGC3, EDNRB, STOM, GLI3, RXFP3, LimK1. GPR73L1, PCDH10, DOCK10 and MRPS21 are associated with the presence of cancer in a subject. Preferably said group consists of the genes SND1; PCDHGC3 & RASSF2.

According to the present invention, under expression (and/or presence methylation) is associated with the presence of cancer, and vice versa over-expression (and/or absence of methylation) is associated with the absence of cancer.

To detect the presence of mRNA encoding a gene, a sample is obtained from a patient. The sample may be any suitable sample comprising cellular matter of the tumour. Suitable sample types include cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sample types are stool or body fluids selected from the group consisting colonic effluent, urine, blood plasma, blood serum, whole blood, isolated blood cells, cells isolated from the blood.

The sample may be treated to extract the RNA contained therein. The resulting nucleic acid from the sample is then analyzed. Many techniques are known in the state of the art for determining absolute and relative levels of gene expression, commonly used techniques suitable for use in the present invention include in situ hybridization (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 318-325, 1987 which are incorporated by reference). Further preferred is the "Real-time" variant of RT- PCR, wherein the PCR product is detected by means of hybridization probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide (or other solid phase). After hybridization, arrays are scanned using a fluorescent microarray scanner. Analysing the relative fluorescent intensity of different genes provides a measure of the differences in gene expression.

DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of the genes of interest (in this case the genes or genomic sequences selected from the group consisting of RASSF2, TFAP2E , SND1, PCDHGC3, EDNRB, STOM, GLI3, RXFP3, LimK1, GPR73L1, PCDH10, DOCK10, MRPS21 and Septin 9 and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy^{®}3- or Cy^{®}5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy^{®}3. The resulting labelled cDNA is purified to remove unincorporated nucleotides, free dye and residual RNA. Following purification, the labelled cDNA samples are hybridized to the microarray. The stringency of hybridization is determined by a number of factors during hybridization and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner. The fluorescent intensity of each spot indicates the level of expression of the analyzed gene; bright spots correspond to strongly expressed genes, while dim spots indicate weak expression..

Once the images are obtained, the raw data must be analyzed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridization, array imperfections or variability in the array set-up, cDNA labelling, hybridization or washing. Data normalization allows the results of multiple arrays to be compared.

Another aspect of the disclosure relates to a kit for use in diagnosis of cancer in a
subject according to the methods of the present invention, said kit comprising: a means for measuring the level of transcription of genes or genomic sequences selected from the group consisting of RASSF2, TFAP2E , SND1, PCDHGC3, EDNRB, STOM, GLI3, RXFP3, LimK1, GPR73L1, PCDH10, DOCK10 and MRPS21 . Preferably said group consists of the genes RASSF2, TFAP2E , SND1 & PCDHGC3 and/or their promotor or regulatory regions.

In a preferred aspect, the means for measuring the level of transcription
comprise oligonucleotides or polynucleotides able to hybridize under stringent or moderately stringent conditions to the transcription products of at least the PCDHGC3 gene or the PCDHGC3 gene and the Septin 9 gene or the PCDHGC3 gene and the Septin 9 gene and gene selected from the group consisting of RASSF2, TFAP2E , SND1, EDNRB, STOM, GLI3, RXFP3, LimK1, GPR73L1, PCDH10, DOCK10 and MRPS21. Preferably said group consists of the genes RASSF2, TFAP2E, SND1 and/or their promotor or regulatory regions.

It is further prefered that said kit further comprises means for measuring the level of transcription of the gene Septin 9 comprising oligonucleotides or polynucleotides able to hybridize under stringent or moderately stringent conditions to the transcription products of said gene. In one embodiment said oligonucleotides or polynucleotides comprise at least 9, 18 or 25 bases of a sequence complementary to or hybridizing to said transcription product.

In a most preferred embodiment the level of transcription is determined by techniques selected from the group of Northern Blot analysis, reverse transcriptase PCR, real-time PCR, RNAse protection, and microarray. In another embodiment of the invention the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results.

In a preferred aspect, the kit comprises (a) a plurality of oligonucleotides or
polynucleotides able to hybridize under stringent or moderately stringent conditions to the transcription products of at least the PCDHGC3 gene or the PCDHGC3 gene and the Septin 9 gene or the PCDHGC3 gene and the Septin 9 gene and one gene selected from the group consisting of RASSF2, TFAP2E, SND1, EDNRB, STOM, GLI3, RXFP3, LimK1, GPR73L1, PCDH10, DOCK10 and MRPS21 ; (b) a container, preferably suitable for containing the oligonucleotides or polynucleotides and a biological sample of the patient comprising the transcription products wherein the oligonucleotides or polynucleotides can hybridize under stringent or moderately stringent conditions to the transcription products, (c) means to detect the hybridization of (b); and optionally, (d) instructions for use and interpretation of the kit results. It is further preferred that said oligonucleotides or polynucleotides of (a) comprise in each case at least 9, 18 or 25 bases of a sequence complementary to or hybridizing to the transcription products of a gene selected from the group consisting of RASSF2, TFAP2E, SND1, PCDHGC3, EDNRB, STOM, GLI3, RXFP3, LimK1, GPR73L1, PCDH10, DOCK10 and MRPS21 and sequences complementary thereto. Preferably said group consists of the genes RASSF2, TFAP2E , SND1 & PCDHGC3 and/or their promoter or regulatory regions.

It is further preferred that said kit comprises (e) a plurality of oligonucleotides or polynucleotides able to hybridize under stringent or moderately stringent conditions to the transcription products of the gene Septin 9. Said oligonucleotides or polynucleotides of (e) preferably comprise in each case at least 9, 18 or 25 bases of a sequence complementary to or hybridizing to a Septin 9 transcription products.

The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR. Preferably said polymerase is a reverse transcriptase. It is further preferred that said kit further contains an Rnase reagent.

The present disclosure further provides for methods for the detection of the presence of
the polypeptide encoded by said gene sequences in a sample obtained from a patient.

Aberrant levels of polypeptide expression of the polypeptides encoded by the genes or genomic sequences selected from the group consisting of RASSF2, TFAP2E , SND1, PCDHGC3, EDNRB, STOM, GLI3, RXFP3, LimK1, GPR73L1, PCDH10, DOCK10 and MOPS21 are associated with the presence of cancer. The accuracy of the diagnosis of colorectal cancer is increased when polypeptides of the gene Septin 9 are analyzed in combination with said aforenmentioned polypeptides.

According to the present invention, under expression of said polypeptides is associated with the presence of cancer.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991).

Preferred are binder-ligand immunoassay methods including reacting antibodies with an epitope or epitopes and competitively displacing a labelled polypeptide or derivative thereof.

Certain aspects of the present disclosure, comprise the use of antibodies specific to the polypeptide encoded by a gene selected from the group consisting of RASSF2, TFAP2E, SND1, PCDHGC3, EDNRB, STOM, GLI3, RXFP3, LimK1, GPR73L1, PCDH10, DOCK10 and MRPS21. Preferably said group consists of the genes RASSF2, TFAP2E , SND1 & PCDHGC3. Further embodiments of the present invention comprise the use of a first species of antibodies specific to the polypeptide encoded by the gene PCDHGC3 and a second species of antibodies specific to the polypeptide encoded by a gene selected from the group consisting of RASSF2, TFAP2E , SND1, EDNRB, STOM, GLI3, RXFP3, LimK1, GPR73L1, PCDH10, DOCK10, MRPS21 and Septin 9. Preferably said second species of antibodies is specific to the polypeptide encoded by a gene selected from the group consisting of RASSF2, TFAP2E , SND1 & Septin 9.

Such antibodies are useful for cancer diagnosis. In certain embodiments production of monoclonal or polyclonal antibodies can be induced by the use of an epitope encoded by a polypeptides encoded by a gene selected from the group consisting of RASSF2, TFAP2E, SND1, PCDHGC3, EDNRB, STOM, GLI3, RXFP3, LirnK1, GPR73L1, PCDH10, DOCK10 MRPS21 and Septin 9 as an antigene. Preferably said group consists of the genes RASSF2, TFAP2E, SND1 & Septin 9. Such antibodies may in turn be used to detect expressed polypeptides as markers for cancer diagnosis. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference in its entirety). Methods for preparation of the polypeptides or epitopes thereof include, but are not limited to chemical synthesis, recombinant DNA techniques or isolation from biological samples.

In the final step of the method the diagnosis of the patient is determined, whereby under-expression least the PCDHGC3 gene or the PCDHGC3 gene and the Septin 9 gene or the PCDHGC3 gene and the Septin 9 gene andone gene selected from the group consisting of RASSF2, TFAP2E , SND1,, EDNRB, STOM, GLI3, RXFP3, LimK1, GPR73L1, PCDH10, DOCK10 and MRPS21 is indicative of the presence of cancer. The term under-expression shall be taken to mean expression at a detected level less than a pre-determined cut off which may be selected from the group consisting of the mean, median or an optimized threshold value. Another aspect of the disclosure provides a kit for use in diagnosis of cancer in a subject
according to the methods of the present invention, comprising: a means for detecting polypeptides at least least the PCDHGC3 gene or the PCDHGC3 gene and the Septin 9 gene or the PCDHGC3 gene and the Septin 9 gene andone gene selected from the group consisting of RASSF2, TFAP2E , SND1, PCDHGC3, EDNRB, STOM, GLI3, RXFP3, LimK1, GPR73L1, PCDH10, DOCK10,and MRPS21. Preferably said group consists of the genes RASSF2, TFAP2E, SND1. The means for detecting the polypeptides comprise preferably antibodies, antibody derivatives, or antibody fragments. The polypeptides are most preferably detected by means of Western Blotting utilizing a labelled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit comprises: (a) a means for detecting polypeptides at least the PCDHGC3 gene or the PCDHGC3 gene and the Septin 9 gene or the PCDHGC3 gene and the Septin 9 gene and one gene selected from the group consisting of RASSF2, TFAP2E , SAND1, EDNRB, STOM, GLI3, RXFP3, LimK1, GPR73L1, PCDH10, DOCK10 and MRPS21; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

Preferably (a) is a means for detecting polypeptides at least one gene selected from the group consisting of RASSF2, TFAP2E , SND1 & Septin 9.

The kit may also contain other components such as buffers or solutions suitable for blocking, washing or coating , packaged in a separate container.

Particular aspects of the present disclosure provide a novel application of the analysis of methylation levels and/or patterns within said sequences that enables a precise detection, characterization and/or treatment of colorectal cell proliferative disorders. Early detection of cancer is directly linked with disease prognosis, and the disclosed method thereby enables the physician and patient to make better and more informed treatment decisions.

### FURTHER IMPROVEMENTS

The present disclosure provides novel uses for the genomic sequence SEQ ID NO: 1
TO SEQ ID NO: 11 AND SEQ ID NO: 132 TO SEQ ID NO: 133. Additional embodiments provide modified variants of SEQ ID NO: 1 TO SEQ ID NO:11 AND SEQ ID NO: 132 TO SEQ ID NO: 133, as well as oligonucleotides and/or PNA-oligomers for analysis of cytosine methylation patterns within SEQ ID NO: 1 TO SEQ ID NO: 11 AND SEQ ID NO: 132 TO SEQ ID NO: 133.

An objective of the disclosure comprises analysis of the methylation state of one or
more CpG dinucleotides within at least one sequence selected from the group consisting of SEQ ID NO: 1 to SEQ ID NO: 14 and SEQ ID NO: 132 to SEQ
ID NO: 133 and sequences complementary thereto.

The disclosure provides treated nucleic acids, derived from genomic SEQ ID NO:1 TO SEQ ID NO: 14 AND SEQ ID NO: 132 TO SEQ ID NO: 133, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred aspect, the disclosure provides a non-naturally occurring modified nucleic acid comprising a sequence of at least 16 contiguous nucleotide bases in length of a sequence selected from the group consisting of SEQ ID NO: 15 TO SEQ ID NO: 70 AND SEQ ID NO: 134 TO SEQ ID NO: 141. In further preferred aspect said nucleic acid is at least 50, 100, 150, 200, 250 or 500 base pairs in length of a segment of the nucleic acid sequence disclosed in SEQ ID NO: 15 TO SEQ ID NO: 36 AND SEQ ID NO: 43 TO SEQ ID NO: 64 AND SEQ ID NO: 134 TO SEQ ID NO: 141. Particularly preferred is a nucleic acid molecule that is not identical or complementary to all or a portion of the sequences SEQ ID NO: 15 TO SEQ ID NO: 70 AND SEQ ID NO: 134 TO SEQ ID NO: 141 but not SEQ ID NO: 1 TO SEQ ID NO: 14 AND SEQ ID NO: 132 TO SEQ ID NO: 133 or other naturally occurring DNA.

It is preferred that said sequence comprises at least one CpG, TpA or CpA dinucleotide and sequences complementary thereto. The sequences of SEO ID NO: 15 TO SEQ ID NO: 70 AND SEQ ID NO: 134 TO SEQ ID NO: 141 provide non-naturally occurring modified versions of the nucleic acid according to SEQ ID NO: 1 TO SEQ ID NO: 14 AND SEQ ID NO: 132 TO SEQ ID NO: 133, wherein the modification of each genomic sequence results in the synthesis of a nucleic acid having a sequence that is unique and distinct from said genomic sequence as follows. For each sense strand genomic DNA, *e.g.,* SEQ ID NO: 1, four converted versions are disclosed. A first version wherein "C" is converted to "T," but "CpG" remains "CpG" *(i.e.,* corresponds to case where, for the genomic sequence, all "C" residues of CpG dinucleotide sequences are methylated and are thus not converted); a second version discloses the complement of the disclosed genomic DNA sequence (i.e. *antisense* strand), wherein "C" is converted to "T," but "CpG" remains "CpG" (*i.e.*, corresponds to case where, for all "C" residues of CpG dinucleotide sequences are methylated and are thus not converted). The 'upmethylated' converted sequences of SEQ ID NO: 1 TO SEQ ID NO: 14 AND SEQ ID NO: 132 TO SEQ ID NO: 133 correspond to SEQ ID NO: 15 TO SEQ ID NO: 42. A third chemically converted version of each genomic sequences is provided, wherein "C" is converted to "T" for all "C" residues, including those of "CpG" dinucleotide sequences (*i.e.,* corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" is converted to "T" for all "C" residues, including those of "CpG" dinucleotide sequences (*i.e.,* corresponds to case where, for the complement (*anti sense* strand) of each genomic sequence, all "C" residues of CpG dinucleotide sequences are unmethylated). The 'downmethylated' converted sequences of SEQ ID NO: 1 TO SEQ ID NO: 14 AND SEQ ID NO: 132 TO SEQ ID NO: 133 correspond to SEQ ID NO: 43 TO SEQ ID NO: 70. See Table 1 for further details.

Significantly, heretofore, the nucleic acid sequences and molecules according SEQ ID NO: 15 TO SEQ ID NO: 36 AND SEQ ID NO: 43 TO SEQ ID NO: 64 AND SEQ ID NO: 134 TO SEQ ID NO: 141 were not implicated in or connected with the detection of cellular proliferative disorders.

In an alternative preferred aspect, the disclosure further provides
oligonucleotides or oligomers suitable for use in the methods of the invention for detecting the cytosine methylation state within genomic or treated (chemically modified) DNA, according to SEQ ID NO: 15 TO SEQ ID NO: 70 AND SEQ ID NO: 134 TO SEQ ID NO: 141; SEQ ID NO: 1 TO SEQ ID NO: 14 AND SEQ ID NO: 132 TO SEQ ID NO: 133. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 15 TO SEQ ID NO: 70 AND SEQ ID NO: 134 TO SEQ ID NO: 141 and/or sequences complementary thereto, or to a genomic sequence according to SEQ ID NO: 1 TO SEQ ID NO: 14 AND SEQ ID NO: 132 TO SEQ ID NO: 133 and/or sequences complementary thereto.

Thus, the present disclosure includes nucleic acid molecules (e.g., oligonucleotides
and peptide nucleic acid (PNA) molecules (PNA-oligomers)) that hybridize under moderately stringent and/or stringent hybridization conditions to all or a portion of a sequence selected from the group consisting of SEQ ID NO: 15 TO SEQ ID NO: 70 AND SEQ ID NO: 134 TO SEQ ID NO: 141, SEQ ID NO: 1 TO SEQ ID NO: 14 AND SEQ ID NO: 132 TO SEQ ID NO: 133 or to the complements thereof. Particularly preferred is a nucleic acid molecule that hybridizes under moderately stringent and/or stringent hybridization conditions to all or a portion of the sequences SEQ ID NO: 15 TO SEQ ID NO: 70 AND SEQ ID NO: 134 TO SEQ ID NO: 141 but not SEQ ID NO: 1 TO SEQ ID NO: 14 AND SEQ ID NO: 132 TO SEQ ID NO: 133 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present disclosure.

Preferably, the hybridizing portion of the inventive hybridizing nucleic acids is at least 95%, or at least 98%, or 100% identical to the sequence, or to a portion thereof of a sequence selected from the group consisting of SEQ ID NO: 1 TO SEQ ID NO: 11 AND SEQ ID NO: 132 TO SEQ ID NO: 133, SEQ ID NO: 15 TO SEQ ID NO: 36 AND SEQ ID NO: 43 TO SEQ ID NO: 64 AND SEQ ID NO: 134 TO SEQ ID NO: 141, or to the complements thereof.

Hybridizing nucleic acids of the type described herein can be used, for example, as a primer (*e.g.,* a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 1 TO SEQ ID NO: 14 AND SEQ ID NO: 132 TO SEQ ID NO: 133 (such as allelic variants and SNPs), rather than identical, it is useful to first establish the lowest temperature at which only homologous hybridization occurs with a particular concentration of salt (*e,g.,* SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of oligonucleotides of length X (in nucleotides), as indicated by polynucleotide positions with reference to, *e.g.*, SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
Sln to (n + (X-1));
where n=1, 2, 3,... (Y-(X-1));
where Y equals the length (nucleotides or base pairs) of SEQ ID NO: 1 (2519);
where X equals the common length (in nucleotides) of each oligonucleotide in the set (*e.g.,* X=20 for a set of consecutively overlapping 20-mers); and
where the number (Z) of consecutively overlapping oligomers of length X for a given SEQ ID NO of length Y is equal to Y- (2519-1). For example Z= 2519-19= 2500 for either sense or antisense sets of SEQ ID NO:1, where X=20.

Preferably, the set is limited to those oligomers that comprise at least one CpG, TpG or CpA dinucleotide.

Examples of 20-mer oligonucleotides include the following set of 2.261 oligomers (and the antisense set complementary thereto), indicated by polynucleotide positions with reference to SEQ ID NO: 1:
1-20, 2-21, 3-22, 4-23, 5-24, ............... and 2499-2519.

Preferably, the set is limited to those oligomers that comprise at least one CpG, TpG or CpA dinucleotide.

Likewise, examples of 25-mer oligonucleotides include the following set of 2,256 oligomers (and the antisense set complementary thereto), indicated by polynucleotide positions with reference to SEQ ID NO: 1:
1-25, 2-26, 3-27, 4-28, 5-29, ...... and 2494-2519.

Preferably, the set is limited to those oligomers that comprise at least one CpG, TpG or CpA dinucleotide.

The present disclosure encompasses, for each of SEO ID NO: 1 to SEQ ID NO: 11 and SEQ ID NO: 132 to SEQ ID NO: 133, SEQ ID NO: 15 to SEQ ID NO: 36 and SEQ ID NO: 43 to SEQ ID NO: 64 and SEQ ID NO: 134 to SEQ ID NO: 141 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present disclosure constitute
effective tools useful to ascertain genetic and epigenetic parameters of the genomic sequences selected from the group consisting of SEQ ID NO: 1 TO SEQ ID NO: 14 AND SEQ ID NO: 132 TO SEQ ID NO: 133. Preferred sets of such oligonucleotides or modified oligonucleotides of length X are those consecutively overlapping sets of oligomers corresponding to SEQ ID NO: 1 TO SEQ ID NO: 14 AND SEQ ID NO: 132 TO SEQ ID NO: 133, SEQ ID NO: 15 TO SEQ ID NO: 70 AND SEQ ID NO: 134 TO SEQ ID NO: 141 (and to the complements thereof). Preferably, said oligomers comprise at least one CpG, TpG or CpA dinucleotide.

Particularly preferred oligonucleotides or oligomers according to the present disclosure are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the disclosure can also be modified by chemically linking the
oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc,

The oligonucleotide may also comprise at least one art-recognized modified sugar and/or base moiety, or may comprise a modified backbone or non-natural internucleoside linkage.

The oligonucleotides or oligomers according to particular aspects of the present disclosure are typically used in 'sets,' which contain at least one oligomer for analysis of each of the CpG dinucleotides of a genomic sequence selected from the group consisting SEQ ID NO: 1 TO SEQ ID NO: 11 AND SEQ ID NO: 132 TO SEQ ID NO: 133 and sequences complementary thereto, or to the corresponding CpG, TpG or CpA dinucleotide within a sequence of the treated nucleic acids according to SEQ ID NO: 15 TO SEQ ID NO: 36 AND SEQ ID NO: 43 TO SEQ ID NO: 64 AND SEQ ID NO: 134 TO SEQ ID NO: 141 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular aspects, the present disclosure provides a set of at least
two (2) (oligonucleotides and/or PNA-oligomers) useful for detecting the cytosine methylation state in treated genomic DNA (SEQ ID NO: 15 TO SEQ ID NO: 70 AND SEQ ID NO: 134 TO SEQ ID NO: 141), or in genomic DNA (SEQ ID NO: 1 TO SEQ ID NO: 14 AND SEQ ID NO: 132 TO SEQ ID NO: 133 and sequences complementary thereto). These probes enable diagnosis, classification and/or therapy of genetic and epigenetic parameters of colorectal cell proliferative disorders. The set of oligomers may also be used for detecting single nucleotide polymorphisms (SNPs) in treated genomic DNA (SEQ ID NO: 15 TO SEQ ID NO: 70 AND SEQ ID NO: 134 TO SEQ ID NO: 141), or in genomic DNA (SEQ ID NO:1 TO SEQ ID NO:14 AND SEQ ID NO: 132 TO SEQ ID NO:133 and sequences complementary thereto).

In preferred embodiments, at least one, and more preferably all members of a set of oligonucleotides is bound to a solid phase.

In further aspects, the present disclosure provides a set of at least two (2) oligonucleotides that are used as 'primer' oligonucleotides for amplifying DNA sequences of one of SEQ ID NO:1 TO SEQ ID NO: 14 AND SEQ ID NO: 132 TO SEQ ID NO: 133, SEQ ID NO: 15 TO SEQ ID NO: 70 AND SEQ ID NO: 134 TO SEQ ID NO: 141 and sequences complementary thereto, or segments thereof.

It is anticipated that the oligonucleotides may constitute all or part of an "array" or "DNA chip" (*i.e*., an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available:

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture *(i.e.,* each analyte) can be uniquely bound by a unique label and thus detected (each label is directly counted, resulting in a digital read-out of each molecular species in the mixture).

It is particularly preferred that the oligomers are utilised for at least one of: detection of; diagnosis of; treatment of; monitoring of; and treatment and monitoring of colorectal cell proliferative disorders. This is enabled by use of said sets for the detection of one or more of the following classes of tissues: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon healthy tissue and non-colon cancer tissue.

Particularly preferred are those sets of oligomers according to the Examples.

In the most preferred aspect of the method, the presence or absence of a cellular
proliferative disorder, most preferably colorectal carcinoma is determined. This is achieved by analysis of the methylation status of at least one target sequence comprising at least one CpG position said sequence comprising, or hybridizing under stringent conditions to at least 16 contiguous nucleotides of a sequence selected from the group consisting SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11) and complements thereof. In a further preferred embodiment this is achieved by analysis of the methylation status of a target sequence comprising at least one CpG position said sequence comprising, or hybridizing under stringent conditions to at least 16 contiguous nucleotides of a sequence selected from the group consisting SEQ ID NO: 12 TO SEQ ID NO: 14 and at least one further target sequence comprising at least one CpG position said sequence comprising, or hybridizing under stringent conditions to at least 16 contiguous nucleotides of a sequence selected from the group consisting SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11) and complements thereof.

The present disclosure further provides a method for ascertaining genetic and/or
epigenetic parameters of the genomic sequence according to SEQ ID NO: 1 TO SEQ ID NO: 11 AND SEQ ID NO: 132 TO SEQ ID NO: 133 within a subject by analysing cytosine methylation and single nucleotide polymorphisms. Said method comprising contacting a nucleic acid comprising SEQ ID NO: 1 TO SEQ ID NO: 11 AND SEQ ID NO: 132 TO SEQ ID NO: 133(and more preferably the sub-group thereof consisting of SEQ ID NO: 1; SEQ ID NO: 2; SEQ ID NO: 7 & SEQ ID NO:11) in a biological sample obtained from said subject with at least one reagent or a series of reagents, wherein said reagent or series of reagents, distinguishes between methylated and non-methylated CpG dinucleotides within the target nucleic acid.

In a preferred aspect, said method comprises the following steps: In the *first*
*step,* a sample of the tissue to be analyzed is obtained. The source may be any suitable source, such as cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sources of DNA are stool or body fluids selected from the group consisting colonic effluent, urine, blood plasma, blood serum, whole blood, isolated blood cells, cells isolated from the blood.

The genomic DNA is then isolated from the sample. Genomic DNA may be isolated by any means standard in the art, including the use of commercially available kits, Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration, silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridization behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715 which is published as WO2005/038051). It is preferred
that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1 % and 35% (v/v). It is also preferred that the bisulfite reaction is carried out in the presence of scavengers such as but not limited to chromane derivatives, e.g., 6-hydroxy-2, 5,7,8, -tetramethylchromane 2-carboxylic acid or trihydroxybenzoe acid and derivates thereof, e.g. Gallic acid (see: PCT/EP2004/011715).

The bisulfite conversion is preferably carried out at a reaction temperature between 30°C and 70°C, whereby the temperature is increased to over 85°C for short periods of times during the reaction (see: PCT/EP2004/011715).

The bisulfite treated
DNA is preferably purified priori to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715).

In the *third step* of the method, fragments of the treated DNA are amplified, using sets of primer oligonucleotides according to the present disclosure, and an amplification enzyme.
The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the sub-group thereof consisting of SEQ ID NOS: 15, 16, 43, 44, 17, 18, 45, 46, 27, 28, 55, 56,35 36 63 & 64) and sequences complementary thereto. It is preferred that the set of primer oligonucleotides further comprises at least two oligonucleotides whose sequences are each reverse complementary, identical, or hybridize under stringent or highly stringent conditions to an at least 16-base-pair long segment of the base sequences of one of SEQ ID NO: 37 TO SEQ ID NO: 42 AND SEQ ID NO: 65 TO SEQ ID NO: 70 and sequences complementary thereto.

In an alternate aspect of the method, the methylation status of pre-selected CpG
positions within at least one nucleic acid sequences selected from the group consisting SEQ ID NO: 1 TO SEQ ID NO: 11 AND SEQ ID NO: 132 TO SEQ ID NO: 133 (and more preferably the sub-group thereof consisting of SEQ ID NO:1; SEQ ID NO: 2; (SEQ ID NO: 7 & SEQ ID NO: 11), and preferably additionally SEQ ID NO: 12 TO SEQ ID
NO: 14 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the sub-group thereof consisting of SEQ ID NOS: 15, 16, 43, 44,17, 18, 45, 46, 27, 28, 55, 56,35 36 63 & 64) and sequences complementary thereto, wherein the base sequence of said oligomers comprises at least one CpG dinucleotide .A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivatized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO: 15 TO SEQ ID NO: 36 AND SEQ ID NO: 43 TO SEQ ID NO: 64 AND SEQ ID NO: 134 TO SEQ ID NO: 141 (and more preferably the sub-group thereof consisting of SEQ ID NOS: 15, 16, 43, 44,17, 18, 45, 46, 27, 28, 55, 56,35 36 63 & 64) and sequences complementary thereto, wherein the base sequence of said oligonucleotides comprises at least one CpG, TpG or CpA dinucleotide. Also preferred is a blocking oligonucleotides having a length of at least 9 nucleotides which hybridizes to a treated nucleic acid sequence according to one of SEQ ID NO: 37 TO SEQ ID NO: 42 AND SEQ ID NO: 65 TO SEQ ID NO: 70 and sequences complementary thereto, wherein the base sequence of said oligonucleotides comprises at least one CpG, TpG or CpA dinucleotide

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, *e.g.,* matrix assisted laser desorption/ionization mass spectrometry (MALDI) or using electron spray mass spectrometry (ESI).

Matrix Assisted Laser Desorption/ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analyzed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of based-based methods such as, but not limited to, array technology and probe based technologies as well as by means of techniques such as sequencing and template directed extension.

In one embodiment of the method, the amplificates synthesized in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence selected from the group consisting SEQ ID NO: 1 TO SEQ ID NO: 11 AND SEQ ID NO: 132 TO SEQ ID NO: 133, and the equivalent positions within SEQ ID NO: 15 TO SEQ ID NO: 36 AND SEQ ID NO: 43 TO SEQ ID NO: 64 AND SEQ ID NO: 134 TO SEQ ID NO: 141. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; also see United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridize to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467,1977).

### Best mode

In the most preferred embodiment of the method the genomic nucleic acids are isolated and treated according to the first three steps of the method outlined above, namely:
1. a) obtaining, from a subject, a biological sample having subject genomic DNA;
2. b) extracting or otherwise isolating the genomic DNA;
3. c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
4. d) amplifying subsequent to treatment in c) is carried out in a methylation specific manner, namely by use of methylation specific primers or *blocking oligonucleotides,* and further wherein
5. e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO: 15 TO SEQ ID NO: 36 AND SEQ ID NO: 43 TO SEQ ID NO: 64 AND SEQ ID NO: 134 TO SEQ ID NO: 141 (and more preferably one of the sub-group thereof consisting of SEQ ID NOS: 15, 16, 43, 44,17, 18, 45, 46, 27, 28, 55, 56,35 36 63 & 64) and sequences complementary thereto, wherein the base sequence of said oligomers comprise at least one CpG dinucleotide. It is particularly preferred that the subsequent amplification of d) is additionally carried out by means of methylation specific primers each comprising a sequence having a length of at least 9 nucleotides which hybridizes to a treated nucleic acid sequence according to one of SEQ ID NO: 37 TO SEQ ID NO: 42 AND SEQ ID NO: 65 TO SEQ ID NO: 70 (and more preferably one of the sub-group thereof consisting of SEQ ID NOS: 15, 16, 43, 44, 17, 18, 45, 46, 27, 28, 55, 56,35 36 63 & 64) and sequences complementary thereto, wherein the base sequence of said oligomers comprise at least one CpG dinucleotide.

Step e) of the method, namely the detection of the specific amplificates indicative of the methylation status of one or more CpG positions of at least one sequence of the group comprising SEQ ID NO: 1 TO SEQ ID NO: 11 AND SEQ ID NO: 132 TO SEQ ID NO: 133(and more preferably the sub-group thereof consisting of (SEQ ID NO: 1; SEQ ID NO: 2; SEQ ID NO: 7 & SEQ ID NO: 11) and preferably also SEQ ID NO: 12 TO SEQ ID
NO: 14 is carried out by means of *real-time* detection methods as described above.

Additional aspects of the disclosure provide a method for the analysis of the methylation status of genomic DNA according to the disclosure (SEQ ID NO: 1 TO SEQ ID
NO: 11 AND SEQ ID NO: 132 TO SEQ ID NO: 133 and furthermore SEQ ID NO: 12 TO SEQ ID NO: 14 and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the *frist step* of such additional aspects, the genomic DNA sample is isolated
from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood.

Once the nucleic acids have been extracted, the genomic double-stranded DNA is used in the analysis.

In a preferred aspect, the DNA may be cleaved prior to treatment with
methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of MseI, Bfal, Csp61, Tru1I, Tvu1I, Tru91, Tvu91, Mael and Xspl. Particularly preferred is the use of two or three such enzymes. Particularly preferred is the use of a combination of Msel, Bfal and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of at least the PCDHGC3 gene or the PCDHGC3 gene and the Septin 9 gene or the PCDHGC3 gene and the Septin 9 gene and one gene selected from the group consisting of RASSF2, TFAP2E , SND1, EDNRB, STOM, GLI3, RXFP3, LimK1, GPR73L1; PCDH10, DOCK10 and MRPS21 . Preferably said group consists of the genes RASSF2, TFAP2E and SND1.

It is particularly preferred that the digestion is carried out such that hydrolysis of the DNA at the restriction site is informative of the methylation status of at least one CpG dinucleotide of the gene SEPTIN 9 and furthermore of at least one CpG dinucleotide of at least the PCDHGC3 gene or the PCDHGC3 gene and the Septin 9 gene or the PCDHGC3 gene and the Septin 9 gene and one gene selected from the group consisting of RASSF2, TFAP2E , SND1, PCDHGC3, EDNRB, STOM, GLI3, RXFP3, LimK1, GPR73L1, PCDH10, DOCK10 and MRPS21 . Preferably said group consists of the genes RASSF2, TFAP2E, SND1 & PCDHGC3.

Particularly preferred are the following combinations of genes:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

Preferably, the methylation-specific restriction enzyme is selected from the group consisting of *Bsi E1, Hga I HinPI, Hpy99I*, *Ava I, Bce Al, Bsa HI, Bisl, BstUI, Bsh1236I, Accll, BstFNI, McrBC, Glal, Mvnl, Hpall (HapII), Hhal, Acil, Smal, HinP1I. HpyCH4lV, Eagl* and mixtures of two or more of the above enzymes. Preferred is a mixture containing the restriction enzymes BstUI, Hpall, HpyCH41V and HinP1I.

In the *fourth step,* which is optional but a preferred aspect, the restriction
fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from the group consisting SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11), and complements thereof. Further preferred is amplification by means of an amplification enzyme, at least two primers comprising, in each case a contiguous sequence at least 16 nucleotides in length that is complementary to, or hybridizes under moderately stringent or stringent conditions to a sequence selected from the group consisting SEQ ID NO: 1 TO SEQ ID NO: 11 AND SEQ ID NO: 132 TO SEQ ID NO: 133(and more preferably the sub-group thereof consisting of SEQ ID NO: 1; SEQ ID NO: 2; SEQ ID NO: 7 & SEQ ID NO: 11), and complements thereof and at least two primers comprising, in each case a contiguous sequence at least 16 nucleotides in length that is complementary to, or hybridizes under moderately stringent or stringent conditions to a sequence selected from the group consisting SEQ ID NO: 12 TO SEQ ID NO: 14, and complements thereof. Preferably said contiguous sequence is at least 16, 20 or 25 nucleotides in length. In an alternative embodiment said primers may be complementary to any adaptors linked to the fragments.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis. Preferably said detection is carried out by hybridization to at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 1 TO SEQ ID NO: 11 AND SEQ ID NO: 132 TO SEQ ID NO: 133(and more preferably the sub-group thereof consisting of SEQ ID NO: 1; SEQ ID NO: 2; SEQ ID NO: 7 & SEQ ID NO: 11), and complements thereof. It is further preferred that said detection is carried out by hybridization to at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 1 TO SEQ ID NO: 11 AND SEQ ID NO: 132 TO SEQ ID NO: 133(and more preferably the sub-group thereof consisting of SEQ ID NO: 1; SEQ ID NO: 2; SEQ ID NO: 7 & SEQ ID NO: 11), and complements thereof and additionally at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 12 TO SEQ ID NO: 14 Preferably said contiguous sequence is at least 16, 20 or 25 nucleotides in length.

Subsequent to the determination of the methylation state or level of the genomic nucleic acids the presence or absence of cellular proliferative disorder (most preferably colorectal carcinoma) is deduced based upon the methylation state or level of at least one CpG dinucleotide sequence of at least one sequence selected from the group consisting SEQ ID NO: 1 TO SEQ ID NO: 11 AND SEQ ID NO: 132 TO SEQ ID NO: 133(and more preferably the sub-group thereof consisting of SEQ ID NO: 1; SEQ ID NO: 2; SEQ ID NO: 7 & SEQ ID NO: 11), or an average, or a value reflecting an average methylation state of a plurality of CpG dinucleotide sequences of at least one sequence selected from the group consisting SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11) wherein methylation is associated with the presence of colorectal carcinoma. It is further preferred that the determination of the methylation state or level of the genomic nucleic acids the presence or absence of cellular proliferative disorder (most preferably colorectal carcinoma) is deduced based upon the methylation state or level of at least one CpG dinucleotide sequence of the gene Septin 9 and furthermore of at least one
CpG dinucleotide sequence of at least one sequence selected from the group consisting SEQ ID NO: 12 TO SEQ ID NO: 14, or an average, or a value reflecting an average methylation state of a plurality of CpG dinucleotide sequences of at least one sequence selected from the group consisting SEQ ID NO: 1 TO SEQ ID NO: 11 AND SEQ ID NO: 132 TO SEQ ID NO: 133 (and more preferably the sub-group thereof consisting of SEQ ID NO: 1; SEQ ID NO: 2; SEQ ID NO: 7 & SEQ ID NO: 11) wherein methylation of any of the analyzed CpG positions is associated with the presence of colorectal carcinoma.

Wherein said methylation is determined by quantitative means the cut-off point for determining said the presence of methylation is preferably zero (i.e. wherein a sample displays any degree of methylation it is determined as having a methylated status at the analyzed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected from the group consisting of 0%-5%, 5%-10%, 10%-15%, 15%-20%, 20%-30% and 30%-50%. Particularly preferred are the cut-offs 10%, 15%, 25%, and 30%.

In an alternative aspect of the method wherein a panel of genes comprising the
Septin 9 or its truncated transcript Q9HC74 and at least one gene selected from the group consisting RASSF2, TFAP2E , SND1, PCDHGC3, EDNRB, STOM, GLI3, RXFP3, LimK1, GPR73L1, PCDH10, DOCK10 and MRPS21 (preferably said group consists of the genes RASSF2, TFAP2E , SND1 & PCDHGC3) subsequent to the determination of the methylation state of the genomic nucleic acids the presence or absence of cellular proliferative disorders, in particular colorectal cell proliferative disorder is deduced based upon the methylation state of at least one CpG dinucleotide sequence of SEQ ID NO: 12 to SEQ ID NO: 14 and at least one CpG dinucleotide sequence of SEQ ID NO: 1 TO SEQ ID NO: 11 AND SEQ ID NO: 132 TO SEQ ID NO: 133 , or an average, or a value reflecting an average methylation state of a plurality of CpG dinucleotide sequences thereof wherein hypermethylation is associated with cancers, in particular colorectal cancer.

### Diagnostic and Prognostic Assays for cellular proliferative disorders

The present disclosure enables diagnosis of events which are disadvantageous to
patients or individuals in which important genetic and/or epigenetic parameters within at least the PCDHGC3 gene or the PCDHGC3 gene and the Septin 9 gene or the PCDHGC3 gene and the Septin 9 gene and one gene selected from the group consisting of RASSF2, TFAP2E, SND1, EDNRB, STOM, GLI3, RXFP3, LimK1, GPR73L1, PCDH10, DOCK10 and MRPS21may be used as markers. Said parameters obtained by means of the present disclosure may be compared to another set of genetic and/or epigenetic parameters, the differences serving as the basis for a diagnosis and/or prognosis of events which are disadvantageous to patients or individuals.

More specifically the present invention enables the screening of at-risk populations for the early detection of colorectal carcinomas. Neoplastic colorectal, cellular
proliferative disorders, most particularly carcinomas, present decreased methylation (i.e. decreased expression) within at least the PCDHGC3 gene or the PCDHGC3 gene and the Septin 9 gene or the PCDHGC3 gene and the Septin 9 gene andone gene selected from the group consisting of RASSF2, TFAP2E , SND1, EDNRB, STOM, GLI3, RXFP3, LimK1, GPR73L1, PCDH10, DOCK10 and MRPS21, as opposed to benign disorders and normal
tissues which do not.

Specifically the present disclosure provides for diagnostic cancer assays based on
measurement of differential expression (preferably methylation) of one or more CpG dinucleotide sequences of at least one sequence selected from the group consisting SEQ ID NO: 1 TO SEQ ID NO: 11 AND SEQ ID NO: 132 TO SEQ ID NO: 133 that comprise such a CpG dinucleotide sequence. Typically, such assays involve obtaining a sample from a subject, performing an assay to measure the expression of at least least the PCDHGC3 gene or the PCDHGC3 gene and the Septin 9 gene or the PCDHGC3 gene and the Septin 9 gene andone gene selected from the group consisting of RASSF2, TFAP2E , SND1, EDNRB, STOM, GLI3, RXFP3, LimK1, GPR73L1, PCDH10, DOCK10, and MRPS21, preferably by determining the methylation status of at least one sequence selected from the group consisting SEQ ID NO: 1 TO SEQ ID NO: 11 AND SEQ ID NO: 132 TO SEQ ID NO: 133, derived from the sample, relative to a control sample, or a known standard and making a diagnosis based thereon. Preferably said group consists of the genes RASSF2, TFAP2E and SND1.

Particularly preferred are the following combinations of genes:
- Septin 9+RASSF2+TFAP2E+PCDHGC3+SND1
- Septin 9+RASSF2+TFAP2E+PCDHGC3
- Septin 9+TFAP2E+PCDHGC3
- Septin 9+PCDHGC3

In particular preferred aspects, oligomers are used to assess the CpG
dinucleotide methylation status, such as those based on SEQ ID NO: 1 TO SEQ ID NO: 11 AND SEQ ID NO: 132 TO SEQ ID NO: 133, SEQ ID NO:15 TO SEQ ID NO: 36 AND SEQ ID NO: 43 TO SEQ ID NO: 64 AND SEQ ID NO: 134 TO SEQ ID NO: 141, or arrays thereof, as well as in kits based thereon and useful for the diagnosis of cellular proliferative disorders, most preferably colorectal carcinoma.

### Kits

Moreover, an additional aspect of the present disclosure is a kit comprising: a means
for determining methylation of at least the PCDHGC3 gene or the PCDHGC3 gene and the Septin 9 gene or the PCDHGC3 gene and the Septin 9 gene and one gene selected from the group consisting of RASSF2, TFAP2E , SND1, EDNRB, STOM, GLI3, RXFP3, LimK1, GPR73L1, PCDH10, DOCK10 and MRPS21. Preferably said group consists of the genes RASSF2, TFAP2E and SND1.

The means for determining methylation comprise preferably a bisulfite-containing reagent; one or a plurality of oligonucleotides consisting whose sequences in each case are identical, are complementary, or hybridize under stringent or highly stringent conditions to an at least 9 or more preferably 18 base long segment of a sequence selected from SEQ ID NO: 15 TO SEQ ID NO: 36 AND (SEQ ID NO: 43 TO SEQ ID NO: 64 AND SEQ ID NO: 134 TO SEQ ID NO: 141 **(and more preferably the sub-group thereof consisting of SEQ ID NOS: 15, 16, 43, 44, 17, 18, 45, 46, 27, 28, 55, 56, 35 36 63 & 64);** and optionally instructions for carrying out and evaluating the described method of methylation analysis. In one embodiment the base sequence of said oligonucleotides comprises at least one CpG, CpA or TpG dinucleotide. In a further embodiment said kit may further comprise one or a plurality of oligonucleotides consisting whose sequences in each case are identical, are complementary, or hybridize under stringent or highly stringent conditions to an at least 9 or more preferably 18 base long segment of a sequence selected from SEQ ID NO: 37 TO SEQ ID NO: 42 AND SEQ ID NO: 65 TO SEQ ID NO: 70 **(and more preferably the sub-group thereof consisting of SEQ ID NOS: 15, 16, 43, 44, 17, 18, 45, 46, 27, 28, 55, 56, 35 36 63 & 64).**

In a further aspect, said kit may further comprise standard reagents for
performing a CpG position-specific methylation analysis, wherein said analysis comprises one or more of the following techniques: MS-SNuPE, MSP, MethyLight™, HeavyMethyl, COBRA, and nucleic acid sequencing. However, a kit along the lines of the present invention can also contain only part of the aforementioned components.

In a preferred aspect, the kit may comprise additional bisulfite conversion
reagents selected from the group consisting: DNA denaturation buffer; sulfonation buffer; DNA recovery reagents or kits (e.g., precipitation, ultrafiltration, affinity column); desulfonation buffer; and DNA recovery components.

In a further alternative aspect, the kit may contain, packaged in separate
containers, a polymerase and a reaction buffer optimized for primer extension mediated by the polymerase, such as PCR. In another aspect, the kit further comprising
means for obtaining a biological sample of the patient. Preferred is a kit, which further comprises a container suitable for containing the means for determining methylation of at least least the PCDHGC3 gene or the PCDHGC3 gene and the Septin 9 gene or the PCDHGC3 gene and the Septin 9 gene and one gene selected from the group consisting of
RASSF2, TFAP2E, SND1,, EDNRB, STOM, GLI3, RXFP3, LimK1, GPR73L, PCDH10, DOCK10, and MRPS21 in the biological sample of the patient, and most preferably further comprises instructions for use and interpretation of the kit results. In a preferred aspect, the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridize under stringent or highly stringent conditions to an at least 9 or more preferably 18 base long segment of a sequence selected from SEQ ID NO: 15 TO SEQ ID NO: 36 AND SEQ ID NO: 43 TO SEQ ID NO: 64 AND SEQ ID NO: 134 TO SEQ ID NO: 141 (and more preferably the **sub-group thereof consisting of SEQ ID NOS: 15,16, 43, 44,17,18, 45, 46, 27, 28, 55, 56, 35 36 63 & 64);** and optionally (d) instructions for use and interpretation of the kit results. In an alternative preferred aspect, the kit comprises:
(a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridizes to a pre-treated nucleic acid sequence according to one of SEQ ID NO: 15 TO SEQ ID NO: 36 AND SEQ ID NO: 43 TO SEQ ID NO: 64 AND SEQ ID NO: 134 TO SEQ ID NO: 141 (and more preferably according to one of **the sub-group thereof consisting of SEQ ID NOS: 15, 16, 43,44,17,18, 45, 46, 27, 28, 55, 56, 35 36 63 & 64)** and sequences complementary thereto; and optionally (d) instructions for use and interpretation of the kit results.

In an alternative aspect the kit comprises: (a) a bisulfite reagent; (b) a container
suitable for containing the said bisulfite reagent and the biological sample of the patient; (c) at least one set of primer oligonucleotides containing two oligonucleotides whose sequences in each case are identical, are complementary, or hybridize under stringent or highly stringent conditions to an at least 9 or more preferably 18 base long segment of a sequence selected from SEQ ID NO: 15 TO SEQ ID NO: 36 AND SEQ ID NO: 43 TO SEQ ID NO: 64 AND SEQ ID NO: 134 TO SEQ ID NO:141 (and more preferably selected from the sub-group thereof consisting of SEQ ID NOS: 15, 16, 43, 44, 17, 18, 45, 46, 27, 28, 55, 56,35 36 63 & 64); (d) at least one oligonucleotides and/or PNA-oligomer having a length of at least 9 or 16 nucleotides which is identical to or hybridizes to a pre-treated nucleic acid sequence according to one of SEQ ID NO: 15 TO SEQ ID NO: 36 AND SEQ ID NO: 43 TO SEQ ID NO: 64 AND SEQ ID NO: 134 TO SEQ ID NO: 141 (and more preferably selected from the sub-group thereof consisting of SEQ ID NOS: 15, 16, 43, 44, 17, 18, 45, 46, 27, 28, 55, 56,35 36 63 & 64) and sequences complementary thereto; and optionally (e) instructions for use and interpretation of the kit results.

Said kits may further comprise at least one set of primer oligonucleotides containing two oligonucleotides whose sequences in each case are identical, are complementary, or hybridize under stringent or highly stringent conditions to a 9 or more preferably 18 base long segment of a sequence selected from SEQ ID NO: 37 TO SEQ ID NO: 42 AND SEQ ID NO: 65 TO SEQ ID NO: 70.

The kit may also contain other components such as buffers or solutions suitable for blocking, washing or coating, packaged in a separate container.

Typical reagents (*e.g*., as might be found in a typical COBRA™-based kit) for COBRA™ analysis may include, but are not limited to: PCR primers for at least the PCDHGC3 gene or the PCDHGC3 gene and Septin 9 or the PCDHGC3 gene and Septin 9 and one gene selected from the group consisting of RASSF2, TFAP2E , SND1, EDNRB, STOM, GLI3, RXFP3, LimK1, GPR73L1, PCDH10, DOCK10 and MRPS21; restriction enzyme and appropriate buffer; gene-hybridization oligo; control hybridization oligo; kinase labeling kit for oligo probe; and labeled nucleotides. Typical reagents (e.g., as might be found in a typical MethyLight™ -based kit) for MethyLight™ analysis may include, but are not limited to: PCR primers for the bisulfite converted sequence of at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 ; bisulfite specific probes (e.g. TaqMan™ or Lightcycler™); optimized PCR buffers and deoxynucleotides; and Taq polymerase.

Typical reagents (*e.g*., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for the bisulfite converted sequence of at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 ; reaction buffer (for the Ms-SNuPE reaction); and labelled nucleotides.

Typical reagents (e.g., as might be found in a typical MSP-based kit) for MSP analysis may include, but are not limited to: methylated and unmethylated PCR primers for the bisulfite converted sequence of or genomic sequence selected from the group consisting of RASSF2, TFAP2E , SND1, PCDHGC3, EDNRB, STOM, GLI3, RXFP3, LimK1, GPR73L1, PCDH10, DOCK10 MRPS21 and Septin 9 , optimized PCR buffers and deoxynucleotides, and specific probes.

Moreover, an additional aspect of the present disclosure is an alternative kit
comprising a means for determining methylation of at least the PCDHGC3 gene or the PCDHGC3 gene and Septin 9 or the PCDHGC3 gene and Septin 9 and one gene selected from the group consisting of RASSF2, TFAP2E , SND1,, EDNRB, STOM, GLI3, RXFP3, LimK1, GPR73L1, PCDH10, DOCK10 and MRPS21 , wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 1 TO SEQ ID NO: 11 AND SEQ ID NO: 132 TO SEQ ID NO: 133(and more preferably selected from the sub-group thereof consisting of SEQ ID NO: 1; SEQ ID NO: 2; SEQ ID NO: 7 & SEQ ID NO: 11); and optionally instructions for carrying out and evaluating the described method of methylation analysis. In one aspect, the base sequence of said
oligonucleotides are identical, are complementary, or hybridize under stringent or highly stringent conditions to an at least 18 base long segment of a sequence selected from SEQ ID NO: 1 TO SEQ ID NO: 11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO: 11). Preferably said kit further comprises one or a plurality of primer oligonucleotides (preferably one or a plurality of primer pairs) suitable for the amplification of a sequence comprising at least one CpG dinucleotide of a sequence selected from SEQ ID NO: 12 TO SEQ ID NO: 14.

In a further aspect said kit may comprise one or a plurality of oligonucleotide
probes for the analysis of the digest fragments, preferably said oligonucleotides are identical, are complementary, or hybridize under stringent or highly stringent conditions to an at least 16 base long segment of a sequence selected from SEQ ID NO: 1 TO SEQ ID NO: 11 AND SEQ ID NO: 132 TO SEQ ID NO: **133 (and more preferably selected from the sub-group thereof consisting of SEQ ID NO: 1; SEQ ID NO: 2; SEQ ID NO: 7 & SEQ ID NO: 11).** In a further embodiment said kit may further comprise one or a plurality of oligonucleotide probes for the analysis of the digest fragments, preferably said oligonucleotides are identical, are complementary, or hybridize under stringent or highly stringent conditions to an at least 16 base long segment of a sequence selected from SEQ ID NO:12 TO SEQ ID NO: 14.

In a preferred aspect, the kit may comprise additional reagents selected from the
group consisting: buffer (e.g. restriction enzyme, PCR, storage or washing buffers); DNA recovery reagents or kits (*e.g*., precipitation, ultrafiltration, affinity column) and DNA recovery components.

In a further alternative aspect, the kit may contain, packaged in separate
containers, a polymerase and a reaction buffer optimised for primer extension mediated by the polymerase, such as PCR. In another aspect of the disclosure the kit further comprising
means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of a sequence selected from SEQ ID NO:1 TO SEQ ID NO: 11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO:11); and optionally (d) instructions for use and interpretation of the kit results. In one embodiment said kit further comprises (e) at least one set of oligonucleotides one or a plurality of nucleic acids or peptide nucleic acids which are identical, are complementary, or hybridize under stringent or highly stringent conditions to an at least 9 base long segment of a sequence selected from SEQ ID NO: 12 TO SEQ ID NO: 14.

In an alternative preferred aspect, the kit comprises: (a) a methylation sensitive
restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 TO SEQ ID NO: 11 AND SEQ ID NO:132 TO SEQ ID NO:133 (and more preferably selected from the sub-group thereof consisting of SEQ ID NO: 1; SEQ ID NO: 2; SEQ ID NO: 7 & SEQ ID NO: 11); and optionally (d) instructions for use and interpretation of the kit results. In one aspect, said kit further comprises (e) at least one
set of primer oligonucleotides suitable for the amplification of a sequence comprising at least one CpG dinucleotide of a sequence selected from SEQ ID NO: 12 TO SEQ ID NO: 14.

In an alternative aspect, the kit comprises: (a) a methylation sensitive restriction
enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 TO SEQ ID NO: 11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO: 11); (d) at least one set of oligonucleotides one or a plurality of nucleic acids or peptide nucleic acids which are identical, are complementary, or hybridize under stringent or highly stringent conditions to an at least 9 base long segment of a sequence selected from SEQ ID NO: 1 TO SEQ ID NO:11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO: 11) and optionally (e) instructions for use and interpretation of the kit results. In one embodiment said kit further comprises (f) at least one set of primer oligonucleotides suitable for the amplification of a sequence comprising at least one CpG dinucleotide of a sequence selected from SEQ ID NO: 12 TO SEQ ID NO: 14.

The kit may also contain other components such as buffers or solutions suitable for blocking, washing or coating, packaged in a separate container.

The disclosure further relates to a kit for use in providing a diagnosis of the presence
of a cell proliferative disorder in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for the at leastPCDHGC3 gene or the PCDHGC3 gene and Septin 9 or the PCDHGC3 gene and Septin 9 and one gene selected from the group consisting of RASSF2, TFAP2E, SND1, EDNRB, STOM, GLI3, RXFP3, LimK1, GPR73L1, PCDH10, DOCK10 and MRPS21 and comprises a sequence of at least 15 base pairs in length but no more than 200 bp of a sequence according to one of SEQ ID NO: 1 TO SEQ ID NO: 11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO: 11). Preferably said sequence is at least 15 base pairs in length but no more than 80 bp of a sequence according to one of SEQ ID NO: 1 TO SEQ ID NO: 11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO: 11). It is further preferred that said sequence is at least 20 base pairs in length but no more than 30 bp of a sequence according to one of SEQ ID NO: 1 TO SEQ ID NO: 11 AND SEQ ID NO: 132 TO SEQ ID NO: 133 (and more preferably selected from the sub-group thereof consisting of SEQ ID NO: 1; SEQ ID NO: 2; SEQ ID NO: 7 & SEQ ID NO: 11). In a preferred embodiment said kit further comprises a sequence of at least 15 base pairs in length but no more than 200 bp of a sequence according to one of SEQ ID NO: 12 TO SEQ ID NO: 14. Preferably said sequence is at least 15 base pairs in length but no more than 80 bp of a sequence according to one of SEQ ID NO: 12 TO SEQ ID NO: 14. It is further preferred that said sequence is at least 20 base pairs in length but no more than 30 bp of a sequence according to one of SEQ ID NO: 12 TO SEQ ID NO: 14.

Said test kit preferably further comprises a restriction enzyme component comprising one or a plurality of methylation-sensitive restriction enzymes.

In a further aspect, said test kit is further characterized in that it comprises at
least one methylation-specific restriction enzyme, and wherein the oligonucleotides comprise a restriction site of said at least one methylation specific restriction enzymes. The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The disclosure further provides a composition of matter useful for the
detection of colon cell proliferative disorders. Said composition comprising at least one nucleic acid 18 base pairs in length of a segment of the nucleic acid sequence disclosed in SEQ ID NO: 15 TO SEQ ID NO: 36 AND SEQ ID NO: 43 TO SEQ ID NO: 64 AND SEQ ID NO:134 TO SEQ ID NO:141 (and more preferably selected from the sub-group thereof consisting of SEQ ID NO: 1; SEQ ID NO: 2; SEQ ID NO: 7 & SEQ ID NO: 11), and one or more substances taken from the group comprising : 1-5 mM Magnesium Chloride, 100-500 µM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 15 TO SEQ ID NO: 36 AND SEQ ID NO: 43 TO SEQ ID NO: 64 AND SEQ ID NO:134 TO SEQ ID NO:141 (and more preferably selected from the sub-group thereof consisting of SEQ ID NO:1; SEQ ID NO: 2; SEQ ID NO: 7 & SEQ ID NO: 11) and sequences complementary thereto. In one aspect of the disclosure said composition further comprises an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 37 TO SEQ ID NO: 42 AND SEQ ID NO: 65 TO SEQ ID NO: 70 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.

In further preferred embodiments of the disclosure said at least one oligomer is at least
50, 100, 150, 200, 250 or 500 base pairs in length of a segment of the nucleic acid sequence disclosed in SEQ ID NO: 15 TO SEQ ID NO: 36 AND SEQ ID NO: 43 TO SEQ ID NO: 64 AND SEQ ID NO: 134 TO SEQ ID NO: 141, or SEQ ID NO: 37 TO SEQ ID NO: 42 AND SEQ ID NO: 65 TO SEQ ID NO: 70.

While the present invention has been described with specificity in accordance with certain of its preferred embodiments, the following examples serve only to illustrate the invention.

### EXAMPLES

### Example 1

In the following example a variety of assays suitable for the methylation analysis of the genes and genomic sequences according to tables 1 and 2 were designed, in order to validate the suitable of said markers for the detection of colorectal carcinoma. Furthermore the performance of gene panels (combinations of a plurality of markers) was assessed, of particular interest were panels comprising the gene Septin 9 that provided improved accuracy over the use of Septin 9 alone.

The assays were designed to be run on the LightCycler platform (Roche Diagnostics), but other such instruments commonly used in the art are also suitable. The assays were MSP assays. MSP amplificates were designed to be detected by means of Taqman style fluorescent labelled detection probes.

### Samples

In total 314 samples were analyzed:
198 colorectal carcinoma of the following stages:
   - Stage 0: 4 samples
   - Stage 1:19 samples
   - Stage 2: 84 samples
   - Stage 3: 57 samples
   - Stage 4: 20 samples
   - Stage unknown: 14 samples
22 normal or normal adjacent tissue
26 whole blood samples
40 other cancers (liver, breast & prostate)
28 other normal or normal adjacent tissues (liver, breast & prostate)

### DNA extraction and bisulfite treatment

The DNA was isolated from the all samples according to a modified protocol based on that disclosed in the Qiagen Genomic DNA Handbook (August 2001) (pg 28-31, 44-47). The eluate resulting from the purification was then converted according to the following bisulfite reaction.

The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50° C). The memBrane was turned over according to the manufacturer's instructions. Subsequently a repeated centrifugation was conducted, with which the DNA was removed from the membrane. 10 µL of the eluate was utilized for the Lightcycler Real Time PCR assay.

PCR assay component sequences and thermal cycling conditions are provided in Table 3.

### Control assay

### Control assay

The GSTP1-C3 assay design makes it suitable for quantitating DNAs from different sources, including fresh/frozen samples, remote samples such as plasma or serum, and DNA obtained from archival specimen such as paraffin embedded material. The following oligonucleotides were used in the reaction to amplify the control amplificate:
Control Primer1: GGAGTGGAGGAAATTGAGAT (SEQ ID NO: 71)
Control Primer2: CCACACAACAAATACTCAAAAC (SEQ ID NO: 72)
Control Probe: FAM-TGGGTGTTTGTAATTTTTGTTTTGTGTTAGGTT-TAMRA (SEQ ID NO: 73)

| | |
|---|---|
| Cycle program (40 cycles): | 95 °C, 10 min |
| | 95°C, 15 sec |
| | 58 °C, 1 min |

### Data interpretation

### Calculation of DNA concentration.

The Cp (crossing point values) as calculated by the Lightcycler instrument software were used to determine DNA concentration. The DNA concentration was calculated by reference of the CP value of each well to a standard curve for both the methylation assays and the C3 assay.

### Percentage methylation

For each sample the detected percentage methylation was calculated as the measured concentration of DNA quantified using the methylation assays over the concentration of DNA in the sample as quantified by the C3 assay.

Detection of methylation was determined at multiple different threshold levels, see tables) as well as at all methylation levels (i.e. any samples wherein methylation was detected were deemed positive).

The sensitivity of each assay was determined from the colorectal carcinoma sample positive detection rate, wherein sensitivity was determined as the % samples wherein methylation was positively detected (i.e. true positives).

The specificity of each assay was determined from the whole blood sample negative detection rate (i.e. true negative detection rate) wherein false positives were discounted from the total number of analyzed samples.

### Results

The AUC of each analyzed marker both alone or in some cases in combination with other markers is provided in Tables 4 to 6 (with the exception of MRPS21 & DOCK10). The term 'AUC' is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

Table 4 provides an overview of the measured AUC of each marker in the detection of colorectal carcinoma at all methylation levels, provides information on their complementarity to Septin 9, and also their methylation AUC in whole blood. It is necessary to determine the methylation of a marker in whole blood, as the preferred sample type for a screening assay would be blood, accordingly markers that are methylated in both blood and cancer are not preferred.

Table 5 provides an overview of the measured AUC of each marker in the detection of colorectal carcinoma at methylation cut off values of 10%, 20% and 30%.

Table 6 provides an overview of the measured AUC of panels of markers comprising the gene Septin 9 and 2 further markers in the detection of colorectal carcinoma at methylation cut off values of 0%, 10%, 20% and 30%. The panel data was compiled by determining the proportion or number of the analyzed samples with methylation measured within a given threshold using at least one assay of the panel.

For MRPS21 & DOCK10 assay performance is provided in Figures 27 and 28. Sensitivity of the DOCK10 assay was 0.48 as measured at a specificity of 0.97 (cut off was - 3). Sensitivity of the MRPS21 assay was 0.63 as measured at a specificity of 0.96 (cut off was -1.422).

### Example 2

In the following investigation, the performace of selected markers from example 1, according to Table 7 were selected for further analysis by means of the HM (Heavymethy) assay. Target regions of each gene were bisulfite converted and amplified by means of non-MSP primers, in the presence of a blocker oligonucleotides designed to suppress amplificates that had not been methlated prior to bisulfite treatment. Amplificates were then detected by means of Lightcycler (dual) probes.

Plasma samples from the following patient classes were analyzed:
- Colorectal carcinoma (131 total)
   Stage 0 = 1
   Stage I = 13
   Stage II = 32
   Stage III = 27
   Stage IV = 8
   Unclassified = 50
- Healthy colorectal (colonoscopy verified) = 169
- Non- cancerous diseases (NCD) = 29
- Cancers of non-colorectal origin (NCC) = 31

In total 360 samples were analyzed.

### DNA extraction and bisulfite treatment

The DNA was isolated from the all samples by means of the Magna Pure method (Roche) according to the manufacturer's instructions. The eluate resulting from the purification was then converted according to the following bisulfite reaction. The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50°C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

### Reaction solutions and thermal cycling conditions

PCR assay component sequences are provided in Table 10. Each assay was performed twice (independently) in each sample.

### Thermal cycling conditions were:

| | | |
|---|---|---|
| PCDHGC3 | | |
| activation: | 95°C | 10min |
| 50 cycles: | 95°C | 10 sec (20 ° C/s) |
| | 56°C | 30 sec (20 ° C/s) |
| | 60°C | 3 sec (20° C/s)detection |
| | 72°C | 10 sec (20 C/s) |
| melting curve: | 95°C | 10sec (20 ° C/s) |
| | 40°C | 10sec (20° C/s) |
| | 95°C | 0sec (0,1 °C/s)Continuous |
| cooling: | 40°C 5 sec | |

### All other assays:

| | | |
|---|---|---|
| activation: | 95°C | 10min |
| 55 cycles: | 95°C | 10 sec (20°C/s) |
| | 56°C | 30 sec (20 ° C/s)detection |
| | 72°C | 10 sec (20 ° C/s) |
| melting curve: | 95°C | 10sec (20°C/s) |
| | 40°C | 10sec (20°C/s) |
| | 95°C | 0sec (0,1°C/s)Continuous |
| cooling: | 40°C | 5 sec |

### Results:

In order to predict the presence of CRC tumor DNA in the measured plasma samples we use a logistic regression model. The logistic regression model is build as follows.

First the measurement data for each marker assay is encoded in a qualitative way by the following 3 levels:
- Leve10 both replicate PCR reactions showed no amplification
- Level exactly one of the two PCR replicates showed an amplification curve
- Level2 both of the two PCR replicates showed amplification curves

If any of the two PCR replicates could not be successfully measured the respective marker measurement was regarded as invalid.

The five different DNA methylation markers were used as independent factors with 3 levels in a logistic regression model. An additional intercept factor but no factor interactions were included in the model. The logistic regression model was trained and optimal weights for all factor levels were determined by using the maximum likelihood procedure.

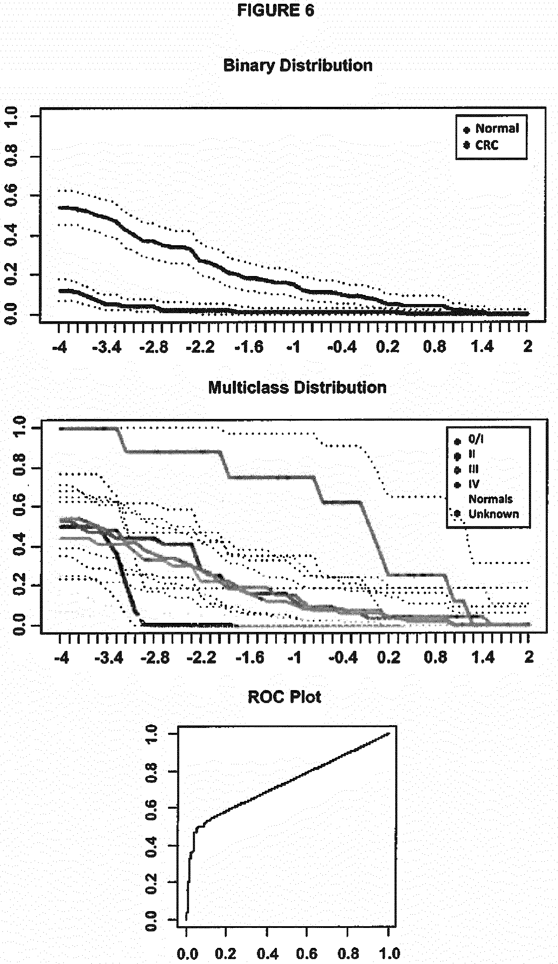

Figures 1 to 10 provide the plots of the measured log mean methylation of the individual assays. Each figures consists of three plots, the plots at the top and in the middle provide the binary and multiclass analysis respectively, the plot at the bottom provides an ROC wherein sensitivity is shown on the Y-axis and 1-specificity is shown on the X-axis. Table 8 and figures 1 to 5 provide an overview of marker performances in all sample groups. Table 9 and figures 6 to 10 provide an overview of marker performances in the colorectal carcinoma and normal colorectal groups.

Figures 12 to 21 provide the plots of the measured log majority mean (analyzed sample is only counted as positive if both replicates are positive, the mean of the two measurements is taken as the quantitative methylation measurement) methylation of the individual assays. Each figures consists of three plots, the plots at the top and in the middle provide the binary and multiclass analysis respectively, the plot at the bottom provides an ROC wherein sensitivity is shown on the Y-axis and 1-specificity is shown on the X-axis. Table 12 and figures 12 to 16 provide an overview of marker performances in all sample groups. Table 13 and figures 18 to 21 provide an overview of marker performances in the colorectal carcinoma and normal colorectal groups.

Table 11 provides an overview of the AUC and snd sensitivities of the single assays at 95% specificity (all p-values were less than 0.00001). Wherein said classes are:
All: Normal + NCD +NCC vs. CRC stages I to IV
I-IV: Normal vs. CRC stages I to IV
I-III: Normal vs. CRC stages I to III

From the multiclass distribution of figure 6 (plot in the middle) and table 11 it can be determined that the gene RASSF2 is particularly effective at detecting Stage 1 and early colorectal carcinomas. Accordingly expression, most preferably CpG methylation, of said gene is in addition to being a preferred diagnostic marker is particularly preferred for the screening of general populations (individuals not displaying any indicators or symptoms of colorectal carcinoma) for the early detection of colorectal carcinomas.

### Marker combinations (panels)

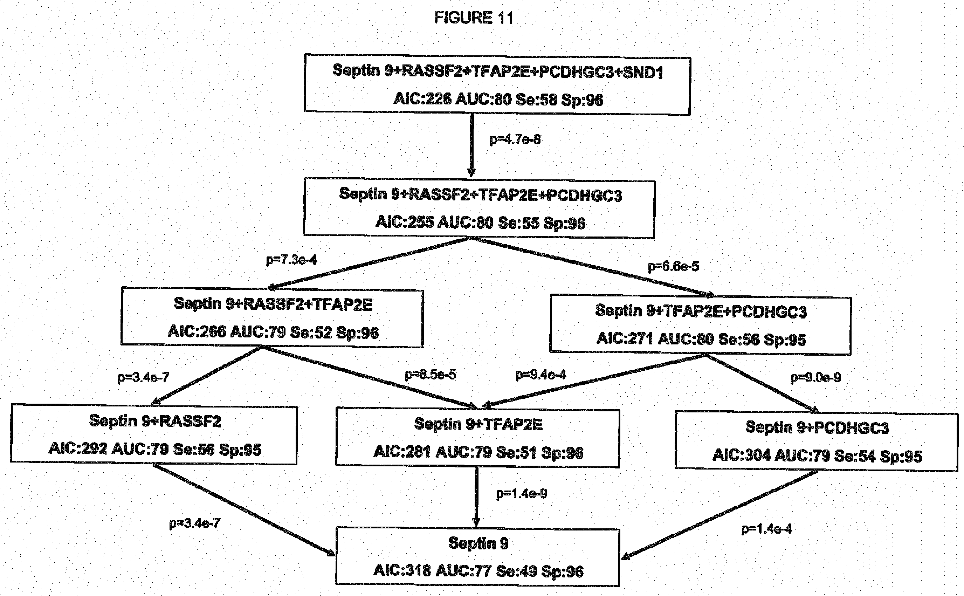

To identify the subset of DNA methylation markers that optimally predicts the presence of CRC we use the backward elimination procedure. In each elimination step the DNA methylation marker with the lowest factor levels was removed from the model. We compared the predictive power of the reduced model with the complete model by using the likelihood ratio test. To identify the subset of DNA methylation markers that optimally predicts the presence of CRC we use the backward elimination procedure. In each elimination step the DNA methylation marker with the lowest factor levels was removed from the model. We compared the predictive power of the reduced model with the complete model by using the likelihood ratio test. Fig. 11 shows that (in the Normal vs. CRC stages I to IV comparison) at each elimination step the predictive power of the logistic regression model was significantly reduced. We conclude that all listed DNA methylation marker models give superior prediction performance as compared to the single marker or the respective simpler marker panels.

We conclude that the following DNA marker models give superior prediction performance as compared to the single marker or the respective simpler marker panels.

Figures 22 to 26 provide an overview of the performance of the following marker combinations:
Septin 9+TFAP2E+RASSF2+PCDHGC3+SND1 (Figure 22)

| | | |
|---|---|---|
| All | AUC | 80 |
| | Sens/Spec | 57/95 |
| All CRC | AUC | 80 |
| | Sens/Spec | 58/96 |
| CRC I-III | AUC | 76 |
| | Sens/Spec | 50/96 |
| Septin 9+TFAP2E+RASSF2+PCDHGC3 (Figure 23) | | |
| All | AUC | 80 |
| | Sens/Spec | 53/95 |
| All CRC | AUC | 80 |
| | Sens/Spec | 55/96 |
| CRC I-III | AUC | 77 |
| | Sens/Spec | 48/96 |
| Septin 9+TFAP2E+RASSF2 (Figure 24) | | |
| All | AUC | 77 |
| | Sens/Spec | 48/96 |
| All CRC | AUC | 79 |
| | Sens/Spec | 52/96 |
| CRC I-III | AUC | 75 |
| | Sens/Spec | 42/96 |
| Septin 9+TFAP2E (Figure 25) | | |
| All | AUC | 77 |
| | Sens/Spec | 45/96 |
| All CRC | AUC | 79 |
| | Sens/Spec | 51/96 |
| CRC I-III | AUC | 75 |
| | Sens/Spec | 41/96 |
| Septin 9+RASSF2 (Figure 26) | | |
| All | AUC | 77 |
| | Sens/Spec | 43/96 |
| All CRC | AUC | 79 |
| | Sens/Spec | 56/95 |
| CRC I-III | AUC | 74 |
| | Sens/Spec | 46/95 |

In each case the upper plot shows all samples (Normals, Non Colorectal Disease, Non Coloretal Cancers and all CRC stages), the lower plot shows only Normal and CRC samples.

**Table 1: Sequences according to the present invention.**

| Genomic SEQ ID NO: | Gene | Methylated bisulfite converted sequence (sense) | Methylated bisulfite converted sequence (antisense) | Unmethylated bisulfite converted sequence (sense) | Unmethylated bisulfite converted sequence (antisense) |
|---|---|---|---|---|---|
| 1 | SND1 | 15 | 16 | 43 | 44 |
| 2 | PCDHGC3 | 17 | 18 | 45 | 46 |
| 3 | EDNRB | 19 | 20 | 47 | 48 |
| 4 | STOM | 21 | 22 | 49 | 50 |
| 5 | GLI3 | 23 | 24 | 51 | 52 |
| 6 | RXFP3 | 25 | 26 | 53 | 54 |
| 7 | RASSF2 | 27 | 28 | 55 | 56 |
| 8 | Q8N2B6 | 29 | 3C | 57 | 58 |
| 9 | PCDH10 | 31 | 32 | 59 | 60 |
| 10 | LIMK1 | 33 | 34 | 61 | 62 |
| 11 | TFAP2E | 35 | 36 | 63 | 64 |
| 12 | Septin 9 | 37 | 38 | 65 | 66 |
| 13 | Septin 9 CpG island | 39 | 40 | 67 | 68 |
| 14 | Septin 9 CpG island | 41 | 42 | 69 | 70 |
| 132 | MRPS21 | 134 | 135 | 138 | 139 |
| 133 | DOCK10 | 136 | 137 | 140 | 141 |

**Table 2: Genes according to the present invention**

| Genomic SEQ ID NO: | Gene | Abbreviation | Chromosomal location* | Contig location* |
|---|---|---|---|---|
| 1 | Staphylococcal nuclease domain-containing protein 1 (p100 co- activator) (100 kDa coactivator) | | 7 | |
| | (EBNA2 | | 127530240 to 127532750 (+) | AC008039.1.1.197346 |
| | coactivator p100) | SND1 | | 141230 to 143740 (+) |
| 2 | Protocadherin gamma C5 precursor | | 5 | |
| | (PCDH-gamma-C5) | | | AC008781.7.1.205516 |
| | | PCDHGC3 | 140835658 to 140837940(+) | 172908 to 175190 (-) |
| 3 | Endothelin B | | 13 | AL1 |
| | receptor | | 77390505 to 77392740 (+) | 39002.18.1.183337 |
| | precursor | EDNRB | | 92863 to 95098 (+) |
| | Erythrocyte band | STOM | 9 | |
| | 7 integral | STOM | 123171727 to | x |
| | membrane protein (Stomatin) (Protein 7.2b) | | 123172921 (+) | |
| 5 | | | 7 | |
| | Zinc finger | | 42234131 to | AC005158.3.1.266344 |
| | protein GLI3 | GLI3 | 42234433(+) | 110482 to 110788 (+) |
| 6 | | | 5 | |
| | Relaxin 3 | | 33970963 to 33973309(+) | AC139777.3.1.169254 |
| | receptor 1 | RXFP3 | | 141119 to 143465 (+) |
| 7 | | | 20 | |
| | Ras association | | 4750982 to 4752901(+) | AL133354.14.1.59726 |
| | domain family 2 | RASSF2 | | 44336 to 46255 (+) |
| 8 | Prokineticin receptor 2 (PK-R2) (G protein-coupled receptor 73-like 1) | | 20 | |
| | (GPR73b) | | 5245129 to | AL121757.7.1.167013 |
| | | GPR73L1 | 5245935(+) | 166111 to 166917 (-) |
| 9 | Protocadherin gamma C5 precursor (PCDH-gamma-C5) | | 4 | |
| | | | 134291880 to | AC105383.3.1.173956 |
| | | PCDH10 | 134294130(+) | 116476 to 118726 (+) |
| 10 | LIM domain kinase 1 (EC 2.7.1.37) | | 7 | |
| | | | 73145461 to | AC005056.2.1.98188 |
| | | LIMK1 | 73146401 (+) | 7 to 947 (-) |
| 11 | transcription factor AP-2 epsilon (activating enhancer blinding protein 2 epsilon) | | 1 | |
| | | | 35814650 to 35816448(+) | AC004865.1.1.120088 |
| | | TFAP2E | | 1091 to 2889 (-) |
| 12 | Septin-9 (MLL septin-like fusion protein) (MLL septin-like fusion protein MSF-A) (Ovarian/Breast septin) (Ov/Br septin) (Septin D1) | | | AC068594.15.1.168501 |
| | | | 17 72789082 to 73008258 (+)** | 150580 to 151086 (+) to AC111170:11.1.158988 |
| | | Septin 9 | | 137268 to 138151 (+)** |
| 13 | Septin 9 CpG island | | 17 72789082 | AC068594.15.1.168501 |
| | | Septin 9 CpG island | to 72789757 (+)** | 150580 to 151255 (+)** |
| 14 | Septin 9 CpG island | Septin 9 CpG island | | |
| 132 | MRPS21 | Dedicator of cytokinesis | | |
| | | protein 10 | | |
| | | (Protein zizimin 3) | | |
| 133 | | Mitochondrial 28S ribosomal protein S21 | | |
| | | (MRP-S21) | | |
| | DOCK10 | (MDS016) | | |
| * Unless otherwise stated all locations refer to Ensembl database v39 (June 2006) | | | | |
| ** Ensembl database v31.35d (8 July 2005) | | | | |

**Table 3: Assay components according to Example 1**

| Gene name | Forward primer SEQ ID NO: | Reverse primer SEQ ID NO: | Probe SEQ ID NO: | PCR conditions | | | | | |
|---|---|---|---|---|---|---|---|---|---|
| | | | Activation | Activation | | Cycling (50x) | | | |
| LHFPL3 | 74 | 75 | 76 | degrees ; C | time | degrees C | time | degrees C | time |
| PCDHGC3 | 77 | 78 | 79 | | 10 min | 95 | 15 sec | 60 | 60 sec |
| EDNRB | 80 | 81 | 82 | 95 | 10 min | 96 | 15 sec | 60 | 60 see |
| RASSF2 | 83 | 84 | 85 | 95 | 10 min | 95 | 15 sec | 60 | 60 see |
| LHFPL3 | 74 | 75 | 76 | degrees C | time | degrees C | time | degrees C | time |
| STOM | 86 | 87 | 88 | 95 | 10 min | 95 | 15 sec | 60 | 60 sec |
| TFAP2E | 89 | 90 | 91 | 95 | 10 min | 95 | 15 sec | 60 | 60 sec |
| GLI3 | 92 | 93 | 94 | 95 | 10 min | 95 | 15 sec | 62 | 60 sec |
| RXFP3 | 95 | 96 | 97 | 95 | 10 min | 95 | 15 sec | 60 | 60 sec |
| LimK1 | 98 | 99 | 100 | 95 | 10 min | 95 | 15 sec | 60 | 60 sec |
| GPR73L1 | 101 | 102 | 103 | | 10 min | 95 | 15 sec | 60 | 60 sec |
| PCDH10 | 104 | 105 | 106 | 95 | 10 min | 95 | 15 sec | 62 | 60 sec |
| MRPS21 | 142 | 143 | 144 | 95 | 10 min | 95 | 15 sec | 60 | 60 sec |
| DOCK10 | 145 | 146 | 147/148 | 95 | 10 min | 95 | 15 sec | 62 | 60 sec |

**Table 4: AUC of colorectal carcinoma detection according to Example 1 with methylation above 0% (single genes).**

| Marker | AUC | Complementarity to Septin 9 | Whole Blood |
|---|---|---|---|
| SND1 | 0.92 | 0.04 | 0.00 |
| PCDHGC3 | 0.91 | 0.04 | 0.08 |
| RASSF2 | 0.86 | 0.04 | 0.00 |
| EDNRB | 0.94 | 0.04 | 0.00 |
| STOM | 0.86 | 0.03 | 0.04 |
| Septin 9 | 0.97 | 0.04 | 0.04 |
| LimK1 | 0.83 | 0.07 | 0.00 |
| TFAP2E | 0.76 | 0.06 | 0.00 |
| GLI3 | 0.71 | 0.04 | 0.00 |
| GPR73L1 | 0.97 | 0.03 | 0.08 |
| RXFP3 | 0.96 | 0.02 | 0.08 |
| PCDH10 | 0.95 | 0.03 | 0.00 |
| MRPS21 | 0.85 | | |
| DOCK10 | 0.73 | | |

**Table 5: AUC of colorectal carcinoma detection according to Example 1 with methylation within various thresholds single genes).**

| Marker | CRC detection rate at various methylation thresholds | | |
|---|---|---|---|
| | 10% | 20% | 30% |
| Septin 9 | 0.92 | 0.85 | 0.74 |
| SND1 | 0.70 | 0.57 | 0.46 |
| PCDHGC3 | 0.73 | 0.54 | 0.38 |
| RASSF2 | 0.73 | 0.54 | 0.33 |
| EDNRB | 0.71 | 0.54 | 0.41 |
| STOM | 0.71 | 0.57 | 0.42 |
| LimK1 | 0.91 | 0.85 | 0.73 |
| TFAP2E | 0.97 | 0.87 | 0.59 |
| GLI3 | 0.80 | 0.64 | 0.44 |
| GPR73L1 | 0.80 | 0.57 | 0.37 |
| RXFP3 | 0.66 | 0.38 | 0.18 |
| PCDH10 | 0.91 | 0.70 | 0.37 |

**Table 6: AUC of colorectal carcinoma detection samples according to Example 1 with methylation within various thresholds (Septin 9 marker panels).**

| Septin 9+2 markers | CRC detection rate at various methylation thresholds | | | |
|---|---|---|---|---|
| | 0% | 10% | 20% | 30% |
| Septin 9 | 0.97 | 0.92 | 0.85 | 0.74 |
| TFAP2E+LimK1 | 0.84 | 0.98 | 0.97 | 0.89 |
| GL13+LimK1 | 0.88 | 0.97 | 0.95 | 0.88 |
| EDNRB+SND1 | 0.94 | 0.96 | 0.90 | 0.83 |
| PGDHGC3+RASSF2 | 0.93 | 0.96 | 0.92 | 0.83 |
| PCDHGC3+SND1 | 0.94 | 0.96 | 0.92 | 0.84 |

**Table 7: Particularly preferred genes and sequences thereof according to the present invention**

| Genomic SEQ ID NO: | Gene | Methylated bisulfite converted sequence (sense) | Methylated bisulfite converted sequence (antisense) | Unmethylat bisulfite converted sequence (sense) | Unmethylated bisulfite converted sequence (antisense) |
|---|---|---|---|---|---|
| 1 | SND1 | 15 | 16 | 43 | 44 |
| 2 | PGDHGG3 | 17 | 18 | 45 | 46 |
| 7 | RASSF2 | 27 | 28 | 55 | 56 |
| 11 | TFAP2E | 35 | 36 | 63 | 64 |

**Table 8: HM assay (Example 2) performance in all tissue samples.**

| | RASSF2 | Septin 9 | SND1 | PCDHGC3 | TFAP2E |
|---|---|---|---|---|---|
| AUC (95% confidence interval) | 4.72 (0.6 7, 0.77) | 0.75(0.7, 0.79) | 0.66(0.6, 0.71) | 0.66(0.61, 0.72) | 0.69 (0.63, 0.74) |
| Sens/Spec | 0.4/0.95 | 0.47/0.95 | 0.25/0.95 | 0.32/0.95 | 0.29/0.95 |
| Sens/Spec cut off | -3.029 | -2.706 | -3.089 | -2.378 | -2.692 |
| Wilcoxon P | 0 | 0 | 0 | 0 | 0 |
| CRC+Adenoma-(pos) | 131 | 131 | 118 | 119 | 119 |
| Normal+non-cancerous diseases (NCD)+carcinoma other than colorectal (NCC) - (neg) | 228 | 228 | 205 | 206 | 206 |

**Table 9: HM assay (Example 2) performance in colorectal carcinoma and normal colorectal tissue samples.**

| | RASSF2 | Septin 9 | SND1 | PCDHGC3 | TFAP2E |
|---|---|---|---|---|---|
| AUC (95% confidence interval) | 0.73(0.67,0.78 ) | 0.76(0.7.0.8 ) | 0.67(0.61,0.73 ) | 0.68(0.62,0.73 ) | 0.71(0.65.0.76 ) |
| Sens/Spec | 0.47/0.95 | 0.48/0.95 | 0.39/0.95 | 4.32/0.95 | 0.39/0.95 |
| Sens/Spec cut off | -3.272 | -2.858 | -3.473 | -2.417 | -3.446 |
| Wilcoxon P | | 0 | 0 | 0 | 0 |
| CRC+Adenom a (pos) | 131 | 131 | 118 | 119 | 119 |
| Normal (neg) | 168 | 169 | 148 | 1481 | 148 |

**Table 10: Primer, blocker and probe sequences according to Example 2.**

| | Septin 9 | RASSF2 | SND1 | PCDHGC3 | TFAP2E |
|---|---|---|---|---|---|
| Forward primer SEQ ID NO: | 107 | 112 | 117 | 122 | 127 |
| Reverse primer SEQ ID NO: | 108 | 113 | 118 | 123 | 128 |
| Blocker SEQ ID NO: | 109 | 114 | 119 | 124 | 129 |
| Probe SEQ ID NO: | 110 | 115 | 124 | 125 | 130 |
| Probe SEQ ID NO: | 111 | 116 | 121 | 126 | 131 |

**Table 11: AUC and sensitivity at 95% specificity) for single le assays of markers according to dass.***

| | AUC | | | Sensitivity | | |
|---|---|---|---|---|---|---|
| | All | I-IV | I-II | All | I-IV | I-II |
| Septin 9 (Majority mean) | 73 | 73 | 67 | 49 | 49 | 37 |
| RASSF2 (Log Mean) | 72 | 73 | 70 | 45 | 48 | 41 |
| TFAP2E (Log Mean) | 68 | 71 | 67 | 32 | 38 | 30 |
| SND 1 (Log Mean) | 64 | 65 | 621 | 25 | 35 | 29 |
| PCDHGC3 (Log Mean) | 65 | 66 | 64 | 30 | 32 | 29 |
| *all p-values were less than 0.00001 | | | | | | |

**Table 12:HM assay (Example 2) performance in all tissue samples.**

| RASSF2 | RASSF2 | Septin 9 | SND1 | PCDHGC3 | TFAP2E |
|---|---|---|---|---|---|
| AUC (95% confidence Interval) | 0.67(0.62,0.72 ) | 0.74(0.69,0.79 ) | 0.63(0.57,0.68 ) | 10.65(0.6,0.7 ) | 05(0.6,0.7 ) |
| Sens/Spec | 0.37(0.96) | 0.51 | 0.28/0.95 | 0.34/0.95 | 10.34/0.9 6 |
| Sens/Spec cut off | -4 | -4 | -3.45 | -2.523 | -4 |
| Wilcoxon P | 0 | 0 | 0 | 0 | 0 |
| CRC+Adenoma-(pos) | 121 | 127 | 113 | 127 | 120 |
| Normal+non-cancerous diseases (NCD)+carcinom a other than colorectal (NCC) - (neg) | 206 | 220 | 194 | 224 | 203 |

**Table 13:HM assay (Example 2) performance in colorectal carcinoma and normal colorectal tissue samples.**

| | RASSF2 | Septin 9 | SND1 | PCDHGC3 | TFAP2E |
|---|---|---|---|---|---|
| AUC (95% confidence interval) | 0.67(0.61.0.73 ) | 0.74(0.69,0.79 ) | 0.64(0.58,0.7 ) | 0 66(0.6,0.71 ) | 0.66(0.6.0.72 ) |
| Sens/Spec | 0.37/0.97 | 0.51/0.97 | 0.3/0.97 | 0.35/0.95 | 0.34/0.98 |
| Sens/Spec cut off | -4 | -4 | -4 | -2.599 | -4 |
| Wlicoxon P | 0 | 0 | 0 | 0 | 0 |
| CRC+Adenom a (pos) | 121 | 121 | 113 | 127 | 120 |
| Normal (neg) | 154 | 164 | 146 | 167 | 154 |

## Claims

1. A method for detecting colorectal cancer in a subject comprising determining the expression level of at least the PCDHGC3 gene in a biological sample isolated from said subject wherein CpG methylation and/or underexpression is indicative of the presence or
class of colorectal cancer.

2. A method for detecting colorectal cancer in a subject comprising determining the expression level of the gene Septin 9 and at least the PCDHGC3 gene in a biological sample isolated from said subject wherein CpG methylation and/or underexpression is indicative of the presence of colorectal cancer.

3. A method according to any of claims 1 or 2 wherein the expression level of 2, 3 or 4 genes selected from the group consisting of RASSF2, TFAP2E, SND1, EDNRB, STOM, GLI3, RXFP3, LimK1, GPR73L1, PCDH10, DOCK10 and MRPS21 are determined.

4. A method according to claim 3 wherein said selected plurality of genes is selected from the group consisting of: Septin 9+RASSF2+TFAP2E+PCDHGC3+SND1; Septin 9+RASSF2+TFAP2E +PCDHGC3; Septin 9+TFAP2E+PCDHGC3; and Septin 9+PCDHGC3

5. The method according to any of claims 1 to 4 wherein said expression level is determined by:
(i) detecting the presence, absence or level of mRNA transcribed from said gene;
(ii) detecting the presence, absence or level of a polypeptide encoded by said gene or sequence thereof; or
(iii) detecting the presence or absence of CpG methylation within said gene, wherein the presence of methylation indicates the presence of colorectal cancer.

6. A method for detecting colorectal cancer in a subject, according to claim 1, comprising contacting genomic
DNA isolated from a biological sample obtained from said subject with at least one reagent, or series of reagents that distinguishes between methylated and non-methylated CpG dinucleotides:
(i) within at least one target region of the genomic DNA, wherein the target region comprises, or hybridizes under stringent conditions to a sequence of at least 16 contiguous nucleotides of at least the sequence of SEQ ID NO: 2;
(ii) within at least two target region of the genomic DNA, wherein a first target region comprises, or hybridizes under stringent conditions to a sequence of at least 16 contiguous nucleotides of at least one sequence selected from the group consisting of SEQ ID NO: 12 TO SEQ ID NO: 14 respectively and wherein a second target region comprises, or hybridizes under stringent conditions to a sequence of at least 16 contiguous nucleotides of at least the sequence of SEQ ID NO: 2,
wherein said contiguous nucleotides comprise at least one CpG dinucleotide sequence, and whereby detecting colorectal carcinoma is, at least in part, afforded.

7. A method for detecting colorectal cancer, according to claim 1, comprising:
a. extracting or otherwise isolating genomic DNA from a biological sample obtained from the subject
b. treating the genomic DNA of a), or a fragment thereof, with one or more reagents to convert cytosine bases that are unmethylated in the 5-position thereof to uracil or to another base that is detectably dissimilar to cytosine in terms of hybridization properties;
c. contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 17, 18, 45 and 46, and complements thereof, wherein the treated genomic DNA or the fragment thereof is either amplified to produce at least one amplificate, or is not amplified; and
d. determining, based on a presence or absence of, or on a property of said amplificate, the methylation state or level of at least one CpG dinucleotide of the sequence of SEQ ID NO: 2, or an average, or a value reflecting an average methylation state or level of a plurality of CpG dinucleotides of the sequence of SEQ ID NO: 2, whereby detecting colorectal cancer, at least in part, afforded.

8. The method of any of claim 1 to 7, wherein the biological sample obtained from the subject is selected from the group comprising cell lines, histological slides, biopsies, paraffin-embedded tissue, body fluids, stool, colonic effluent, urine, blood plasma, blood serum, whole blood, isolated blood cells, cells isolated from the blood and combinations thereof.

9. The method of claim 7,
(i) further comprising in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 15, 16, SEQ ID NO: 19 TO SEQ ID NO: 36 AND SEQ ID NO: 43, 44, SEQ ID NO: 47 TO SEQ ID NO: 64 AND SEQ ID NO: 134 TO SEQ ID NO: 141, and complements thereof, wherein said nucleic acid molecule or peptide nucleic acid molecule suppresses amplification of the nucleic acid to which it is hybridized
(ii) wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NO: 15, 16, SEQ ID NO: 19 TO SEQ ID NO: 36 AND SEQ ID NO: 43, 44, SEQ ID NO: 47 TO SEQ ID NO: 64 AND SEQ ID NO: 134 TO SEQ ID NO: 141, and complements thereof, preferably wherein at least one such hybridizing nucleic acid molecule or peptide nucleic acid molecule is bound to a solid phase or further comprising extending at least one such hybridized nucleic acid molecule by at least one nucleotide base; and/or
(iii) wherein determining in d), comprises sequencing of the amplificate.
(iv) wherein contacting or amplifying in c), comprises use of methylation-specific primers.

10. A method for detecting colorectal cancer, according to claim 1, comprising:
a. extracting or otherwise isolating genomic DNA from a biological sample obtained from the subject
b. digesting the genomic DNA of a), or a fragment thereof, with one or more methylation sensitive restriction enzymes;
contacting the DNA restriction enzyme digest of b), with an amplification enzyme and at least two primers suitable for the amplification of a sequence comprising at least one CpG dinucleotide of the sequence of SEQ ID NO: 2 ;
c. determining, based on a presence or absence of an amplificate the methylation state or level of at least one CpG dinucleotide of the sequence of SEQ ID NO: 2, whereby detecting colorectal cancer is, at least in part, afforded.

11. Use of a nucleic acid:
(i) derived from the genomic DNA sequence of SEQ ID NO: 2 by treatment that is suitable to convert at least one unmethylated cytosine base of the genomic DNA sequence to uracil or another base that is detectably dissimilar to cytosine in terms of hybridization;
(ii) comprising at least 16 contiguous nucleotides of a treated genomic DNA sequence selected from the group consisting of SEQ ID NO: 17, 18, 45 and 46, and sequences complementary thereto, wherein the treatment is suitable to convert at least one unmethylated cytosine base of the genomic DNA sequence to uracil or another base that is detectably dissimilar to cytosine in terms of hybridization;or
(iii) comprising at least 50 contiguous nucleotides of a DNA sequence selected from the group consisting of SEQ ID NO: 17, 18, 45 and 46, and sequences complementary thereto
wherein the base sequence of the nucleic acid preferably comprises at least one CpG, TpG or CpA dinucleotide sequence or
(iv) comprising at least 16 contiguous nucleotides of a treated genomic DNA sequence selected from the group consisting of SEQ ID NO: 17, 18, 45 and 46, and sequences complementary thereto, wherein the treatment is suitable to convert at least one unmethylated cytosine base of the genomic DNA sequence to uracil or another base that is detectably dissimilar to cytosine in terms of hybridization wherein the base sequence of the nucleic acid comprises at least one CpG, or CpA dinucleotide sequence and sequences complementary thereto, for diagnosing or
detecting colorectal cancer.

12. Use of a kit suitable for performing the method according to claims 1-3 comprising:
(i) a) a plurality of oligonucleotides or polynucleotides able to hybridise under stringent or moderately stringent conditions to the transcription products of at least the PCDHGC3 gene; (b) a container suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results;
(ii) (a) a means for detecting polypeptides of at least the PCDHGC3 gene; (b) a container suitable for containing the said means and the biological sample of the patient comprising the polypeptides wherein the means can form complexes with the polypeptides; (c) a means to detect the complexes of (b); or
(iii) (a) a bisulfite reagent; (b) a container suitable for containing the said bisulfite reagent and the biological sample of the patient; (c) at least one set of oligonucleotides containing two oligonucleotides whose sequences in each case are identical, are complementary, or hybridize under stringent or highly stringent conditions to a 9 or more preferably 18 base long segment of a sequence selected from SEQ ID NO: 17, 18, 45 and 46
for detecting or diagnosing colorectal cancer.

13. A kit suitable for performing the method according to claim 10 comprising (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of the sequence of SEQ ID NO: 2; and optionally (d) instructions for use and interpretation of the kit results.

14. The use of a kit according to claim 13 in the diagnosis
of colorectal cancer.

## Patentansprüche

1. Verfahren zum Nachweisen von Dickdarmkrebs in einem Subjekt umfassend ein Bestimmen des Expressionsniveaus wenigstens eines PCDHGC3-Gens in einer biologischen, aus dem Subjekt isolierten Probe, wobei eine CpG-Methylierung und/oder eine Unterexpression das Vorliegen oder die Klasse von Dickdarmkrebs anzeigt.

2. Verfahren zum Nachweisen von Dickdarmkrebs in einem Subjekt umfassend ein Bestimmen des Expressionsniveaus des Gens Septin 9 und wenigstens des PCDHGC3-Gens in einer aus dem Subjekt isolierten Probe, wobei eine CpG-Methylierung und/oder eine Unterexpression das Vorliegen von Dickdarmkrebs anzeigt.

3. Verfahren nach einem der Ansprüche 1 oder 2, wobei das Expressionsniveau von 2, 3 oder 4 Genen ausgewählt aus der Gruppe bestehend aus RASSF2, TFAP2E, SND1, EDNRB, STOM, GLI3, RXFP3, LimK1, GPR73L1, PCDH10, DOCK10 und MRPS21 bestimmt wird.

4. Verfahren nach Anspruch 3, wobei die ausgewählte Vielzahl von Genen ausgewählt ist aus der Gruppe bestehend aus: Septin 9+RASSF2+TFAP2E+PCDHGC3+SND1; Septin 9+RASSF2+TFAP2E +PCDHGC3; Septin 9+TFAP2E+PCDHGC3; und Septin 9+PCDHGC3.

5. Verfahren nach einem der Ansprüche 1 bis 4, wobei das Expressionsniveau bestimmt wird durch:
(i) Nachweisen des Vorliegens, des Fehlens oder des Niveaus von mRNA, die von dem Gen transkribiert wird;
(ii) Nachweisen des Vorliegens, des Fehlens oder des Niveaus eines Polypeptids, das von dem Gen oder einer Sequenz davon kodiert wird; oder
(iii) Nachweisen des Vorliegens oder des Fehlens einer CpG-Methylierung innerhalb des Gens, wobei das Vorliegen einer Methylierung das Vorliegen von Dickdarmkrebs anzeigt.

6. Verfahren zum Nachweisen von Dickdarmkrebs in einem Subjekt nach Anspruch 1, umfassend In-Kontakt-Bringen von genomischer DNA, die aus einer biologischen Probe isoliert wurde, die von dem Subjekt erhalten wurde, mit wenigstens einem Reagenz oder einer Reihe von Reagenzien, die zwischen methylierten und unmethylierten CpG-Dinukleotiden unterscheiden:
(i) innerhalb wenigstens einer Zielregion der genomischen DNA, wobei die Zielregion wenigstens eine Sequenz von 16 zusammenhängenden Nukleotiden wenigstens der Sequenz der SEQ ID NO: 2 umfasst oder unter stringenten Bedingungen daran hybridisiert;
(ii) innerhalb wenigstens zwei Zielregionen der genomischen DNA, wobei eine erste Zielregion eine Sequenz von wenigstens 16 zusammenhängenden Nukleotiden wenigstens einer Sequenz ausgewählt aus der Gruppe bestehend aus SEQ ID NO: 12 bis SEQ ID NO: 14 umfasst oder unter stringenten Bedingungen daran hybridisiert, und wobei eine zweite Zielregion eine Sequenz von wenigstens 16 zusammenhängenden Nukleotiden wenigstens der Sequenz der SEQ ID NO: 2 umfasst oder unter stringenten Bedingungen daran hybridisiert,
wobei die zusammenhängenden Nukleotide wenigstens eine CpG-Dinukleotidsequenz umfassen und wobei ein Nachweisen eines Dickdarmkarzinoms wenigstens teilweise stattfindet.

7. Verfahren zum Nachweisen von Dickdarmkrebs nach Anspruch 1 umfassend:
(a) Extrahieren oder anderweitiges Isolieren von genomischer DNA aus einer biologischen Probe, die von dem Subjekt erhalten wurde;
(b) Behandeln der genomischen DNA aus a) oder eines Fragmentes davon mit einem oder mehreren Reagenzien, um Cytosinbasen, die in der 5-Position unmethyliert sind in Uracil oder eine weitere Base, die sich hinsichtlich von Hybridisierungseigenschaften nachweisbar von Cytosin unterscheidet, umzuwandeln;
(c) In-Kontakt-bringen der behandelten genomischen DNA oder des behandelten Fragments davon mit einem Amplifikationsenzym und wenigstens einem Primer, der eine zusammenhängende Sequenz von wenigstens 9 Nukleotiden umfasst, die zu einer Sequenz ausgewählt aus der Gruppe bestehend aus SEQ ID NO: 17, 18, 45 und 46 und Komplementen davon komplementär ist oder unter moderat stringenten oder stringenten Bedingungen daran hybridisiert, wobei die behandelte genomische DNA oder das Fragment davon entweder amplifiziert wird, um wenigstens ein Amplifikat zu erzeugen, oder nicht amplifiziert werden; und
(d) Bestimmen, basierend auf dem Vorliegen oder dem Fehlen des Amplifikats oder einer Eigenschaft davon, des Methylierungszustands oder Methylierungsniveaus wenigstens eines CpG-Nukleotids der Sequenz der SEQ ID NO: 2 oder eines Durchschnitts davon oder eines Wertes, die einen durchschnittlichen Methylierungszustand oder ein durchschnittliches Methylierungsniveau einer Vielzahl von CpG-Nukleotiden der Sequenz der SEQ ID NO: 2 wiedergeben, wobei ein Nachweisen von Dickdarmkrebs wenigstens teilweise stattfindet.

8. Verfahren nach einem der Ansprüche 1 bis 7, wobei die biologische Probe, die von dem Subjekt erhalten wird, ausgewählt ist aus der Gruppe umfassend Zelllinien, histologische Schnitte, Biopsien, in Paraffin eingebettetes Gewebe, Körperfluide, Stuhl, Dickdarmausfluss, Urin, Blutplasma, Blutserum, Vollblut, isolierte Blutzellen, aus Blut isolierte Zellen und Kombinationen davon.

9. Verfahren nach Anspruch 7,
(i) ferner umfassend in Schritt d) die Verwendung wenigstens eines Nukleinsäuremoleküls oder Peptidnukleinsäuremoleküls jeweils umfassend eine zusammenhängende Sequenz mit wenigstens 9 Nukleotiden Länge, die zu einer Sequenz ausgewählt aus der Gruppe bestehend aus SEQ ID NO: 15, 16, SEQ ID NO: 19 bis SEQ ID NO: 36 und SEQ ID NO: 43, 44, SEQ ID NO: 47 bis SEQ ID NO: 64 und SEQ ID NO: 134 bis SEQ ID NO: 141 und Komplementen davon komplementär ist oder unter moderat stringenten oder stringenten Bedingungen daran hybridisiert, wobei das Nukleinsäuremolekül oder das Peptidnukleinsäuremolekül eine Amplifikation der Nukleinsäure, an die sie hybridisiert sind, unterdrücken;
(ii) wobei das Bestimmen in d) eine Hybridisierung wenigstens eines Nukleinsäuremoleküls oder Peptidnukleinsäuremoleküls jeweils umfassend eine zusammenhängende Sequenz mit wenigstens 9 Nukleotiden Länge, die zu einer Sequenz ausgewählt aus der Gruppe bestehend aus SEQ ID NO: 15, 16, SEQ ID NO: 19 bis SEQ ID NO: 36 und SEQ ID NO: 43, 44, SEQ ID NO: 47 bis SEQ ID NO: 64 und SEQ ID NO: 134 bis SEQ ID NO: 141 und Komplementen davon komplementär ist oder unter moderat stringenten oder stringenten Bedingungen daran hybridisiert, bevorzugt wobei wenigstens ein solches hybridisierendes Nukleinsäuremolekül oder Peptidnukleinsäuremolekül an einer Festphase gebunden ist, umfasst, oder ferner umfassend ein Erweitern wenigstens eines solchen hybridisierten Nukleinsäuremoleküls um wenigstens eine Nukleotidbase; und/oder
(iii) wobei das Bestimmen in d) ein Sequenzieren des Amplifikats umfasst,
(iv) wobei das In-Kontakt-bringen oder Amplifizieren in c) die Verwendung von methylierungsspezifischen Primern umfasst.

10. Verfahren zum Nachweisen von Dickdarmkrebs nach Anspruch 1 umfassend:
(a) Extrahieren oder anderweitiges Isolieren von genomischer DNA aus einer von einem Subjekt erhaltenen biologischen Probe;
(b) Verdauen der genomischen DNA aus a) oder eines Fragments davon mit einem oder mehreren methylierungsempfindlichen Restriktionsenzymen; In-Kontakt-bringen des DNA-Restriktionsenzymverdaus aus b) mit einem Amplifikationsenzym und wenigstens zwei Primern, die für eine Amplifikation einer Sequenz umfassend wenigstens ein CpG-Dinukleotid der Sequenz der SEQ ID NO: 2 geeignet ist;
(c) Bestimmen, basierend auf dem Vorliegen oder dem Fehlen eines Amplifikats, des Methylierungszustandes oder des Methylierungsniveaus wenigstens eines CpG-Dinukleotids der Sequenz der SEQ ID NO: 2, wobei ein Nachweisen von Dickdarmkrebs wenigstens teilweise stattfindet.

11. Verwendung einer Nukleotidsäure:
(i) abgeleitet von der genomischen DNA-Sequenz der SEQ ID NO: 2 durch eine Behandlung, die geeignet ist, wenigstens eine unmethylierte Cytosinbase der genomischen DNA-Sequenz in Uracil oder eine weitere Base, die hinsichtlich einer Hybridisierung nachweisbar von Cytosin verschieden ist, umzuwandeln;
(ii) umfassend wenigstens 16 zusammenhängende Nukleotide einer behandelten genomischen DNA-Sequenz ausgewählt aus der Gruppe bestehend aus SEQ ID NO: 17, 18, 45 und 46 und dazu komplementären Sequenzen, wobei die Behandlung geeignet ist, um wenigstens eine unmethylierte Cytosinbase der genomischen DNA-Sequenz in Uracil oder eine weitere Base, die hinsichtlich einer Hybridisierung nachweisbar von Cytosin verschieden ist, umzuwandeln; oder
(iii) umfassend wenigstens 50 zusammenhängende Nukleotide einer DNA-Sequenz ausgewählt aus der Gruppe bestehend aus SEQ ID NO: 17, 18, 45 und 46 und dazu komplementären Sequenzen,
wobei die Basensequenz der Nukleinsäure bevorzugt wenigstens eine CpG-, TpG- oder CpA-Dinukleotidsequenz umfasst, oder
(iv) umfassend wenigstens 16 zusammenhängende Nukleotide einer behandelten genomischen DNA-Sequenz ausgewählt aus der Gruppe bestehend aus SEQ ID NO: 17, 18, 45 und 46 und dazu komplementären Sequenzen, wobei die Behandlung geeignet ist, um wenigstens eine unmethylierte Cytosinbase der genomischen DNA-Sequenz in Uracil oder eine weitere Base, die hinsichtlich einer Hybridisierung nachweisbar von Cytosin verschieden ist, umzuwandeln, wobei die Basensequenz der Nukleinsäure wenigstens eine CpG- oder CpA-Dinukleotidsequenz und dazu komplementäre Sequenzen umfasst,
zum Diagnostizieren oder Nachweisen von Dickdarmkrebs.

12. Verwendung eines Kits, welches zum Durchführen der Verfahren gemäß den Ansprüchen 1 bis 3 geeignet ist, umfassend:
(i) (a) eine Vielzahl von Oligonukleotiden oder Polynukleotiden, die in der Lage sind, unter stringenten oder moderat stringenten Bedingungen an die Transkriptionsprodukte wenigstens des PCDHGC3-Gens zu hybridisieren; (b) einen Behälter, der geeignet ist, die Oligonukleotide oder Polynukleotide und eine biologische Probe des Patienten umfassend die Transkuptionsprodukte aufzunehmen, wobei die Oligonukleotide oder Polynukleotide unter stringenten oder moderat stringenten Bedingungen an die Transkriptionsprodukte hybridisieren können, (c) Mittel zum Nachweisen der Hybridisierung von (b); und gegebenenfalls (d) Instruktionen zur Verwendung und Interpretation der Kitergebnisse;
(ii) (a) Mittel zum Nachweisen von Polypeptiden wenigstens des PCDHGC3-Gens; (b) einen Behälter, der geeignet ist, die Mittel und die biologische Probe des Patienten umfassend die Polypeptide aufzunehmen, wobei das Mittel Komplexe mit den Polypeptiden bilden kann; (c) Mittel zum Nachweisen der Komplexe aus (b); oder
(iii) (a) ein Bisulfitreagenz; (b) einen Behälter, der geeignet ist, das Bisulfitreagenz und die biologische Probe des Patienten aufzunehmen; (c) wenigstens einen Satz von Oligonukleotiden enthaltend zwei Oligonukleotide, deren Sequenzen jeweils mit einem 9 oder mehr bevorzugt 18 Basenpaare langen Segment einer Sequenz ausgewählt aus SEQ ID NO: 17, 18, 45 und 46 identisch sind, komplementär sind oder unter stringenten oder hochstringenten Bedingungen daran hybridisieren,
zum Nachweisen oder Diagnostizieren von Dickdarmkrebs.

13. Kit, welches zum Durchführen des Verfahrens nach Anspruch 10 geeignet ist, umfassend
(a) ein methylierungsempfindliches Restriktionsenzymreagenz; (b) einen Behälter, der geeignet ist, das Reagenz und die biologische Probe des Patienten aufzunehmen; (c) wenigstens einen Satz von Oligonukleotiden oder eine Vielzahl von Nukleinsäuren oder Peptidnukleinsäuren, die mit einem 9 Basenpaare langen Segment der SEQ ID NO: 2 identisch sind, komplementär sind oder unter stringenten oder hochstringenten Bedingungen daran hybridisieren; und gegebenenfalls (d) Instruktionen zur Verwendung und Interpretation der Kitergebnisse.

14. Verwendung eines Kits nach Anspruch 13 in der Diagnose von Dickdarmkrebs.

## Revendications

1. Procédé pour la détection d'un cancer colorectal chez un sujet, comprenant la détermination du niveau d'expression d'au moins le gène PCDHGC3 dans un échantillon biologique isolé à partir dudit sujet, tandis que la méthylation et/ou la sous-expression de CpG est indicatrice de la présence ou de la classe d'un cancer colorectal.

2. Procédé pour la détection d'un cancer colorectal chez un sujet, comprenant la détermination du niveau d'expression du gène Septine 9 et d'au moins le gène PCDHGC3 dans un échantillon biologique isolé à partir dudit sujet, tandis que la méthylation et/ou la sous-expression de CpG est indicatrice de la présence d'un cancer colorectal.

3. Procédé selon l'une quelconque des revendications 1 ou 2, dans lequel les niveaux d'expression de 2, 3 ou 4 gènes choisis dans le groupe consistant en RASSF2, TFAP2E, SND1, EDNRB, STOM, GL13, RXFP3, LimK1, GPR73L1, PCDH10, DOCK10 et MRPS21 sont déterminés.

4. Procédé selon la revendication 3, dans lequel ladite pluralité sélectionnée de gènes est choisie dans le groupe consistant en
Septine 9+RASSF2+TFAP2E+PCDHGC3+SND1;
Septine 9+RASSF2+TFAP2E+PCDHGC3; Septine 9+TFAP2E+PCDHGC3; et Septine 9+PCDHGC3.

5. Procédé selon l'une quelconque des revendications 1 à 4, dans lequel ledit niveau d'expression est déterminé par:
(i) détection de la présence, de l'absence ou du niveau d'ARNm transcrit à partir dudit gène;
(ii) détection de la présence, de l'absence ou du niveau d'un polypeptide codé par ledit gène ou ladite séquence de celui-ci; ou
(iii) détection de la présence ou de l'absence d'une méthylation de CpG dans ledit gène, tandis que la présence d'une méthylation indique la présence d'un cancer colorectal.

6. Procédé pour la détection d'un cancer colorectal chez un sujet, selon la revendication 1, comprenant la mise en contact d'ADN génomique isolé à partir d'un échantillon biologique obtenu à partir dudit sujet avec au moins un réactif, ou une série de réactifs qui fait la distinction entre des dinucléotides méthylés et non-méthylés:
(i) dans au moins une région cible de l'ADN génomique, tandis que la région cible comprend, ou hybride dans des conditions stringentes à, une séquence d'au moins 16 nucléotides contigus d'au moins la séquence selon SEQ ID NO: 2;
(ii) dans au moins deux régions cibles de l'ADN génomique, une première région cible comprenant, ou hybridant dans des conditions stringentes, à une séquence d'au moins 16 nucléotides contigus d'au moins une séquence choisie dans le groupe consistant en SEQ ID NO: 12 à SEQ ID NO: 14 respectivement et une deuxième région cible comprenant, ou hybridant dans des conditions stringentes à, une séquence d'au moins 16 nucléotides contigus d'au moins la séquence selon SEQ ID NO: 2,
tandis que lesdits nucléotides contigus comprennent au moins une séquence dinucléotidique CpG, et ce par quoi la détection d'un carcinome colorectal est, au moins en partie, procurée.

7. Procédé pour la détection d'un cancer colorectal, selon la revendication 1, comprenant:
a. l'extraction ou l'isolement d'une autre manière d'ADN génomique à partir d'un échantillon biologique obtenu à partir d'un sujet
b. le traitement de l'ADN génomique selon a), ou d'un fragment de celui-ci, avec un ou plusieurs réactifs pour convertir les bases cytosine qui sont non-méthylées sur leur position 5 en uracile ou en une autre base qui est détectable comme n'étant pas similaire à la cytosine en termes de propriétés d'hybridation;
c. la mise en contact de l'ADN génomique traité, ou du fragment traité de celui-ci, avec une enzyme d'amplification et au moins une amorce comprenant une séquence contiguë d'au moins 9 nucléotides qui est complémentaire de, ou hybride dans des conditions modérément stringentes ou stringentes à une séquence choisie dans le groupe consistant en SEQ ID NO: 17, 18, 45 et 46, et leurs compléments, tandis que l'ADN génomique traité ou le fragment de celui-ci soit est amplifié pour produire au moins un produit d'amplification, soit n'est pas amplifié; et
d. la détermination, sur la base de la présence ou de l'absence de, ou sur la base d'une propriété dudit produit d'amplification, de l'état ou du niveau de méthylation d'au moins un dinucléotide CpG de la séquence selon SEQ ID NO: 2, ou d'une moyenne, ou d'une valeur reflétant un état ou un niveau moyen de méthylation d'une pluralité de dinucléotides CpG de la séquence SEQ ID NO: 2, procurant ainsi la détection, au moins en partie, d'un cancer colorectal.

8. Procédé selon l'une quelconque des revendications 1 à 7, dans lequel l'échantillon biologique obtenu à partir du sujet est choisi dans le groupe comprenant lignées cellulaires, coupes histologiques, biopsies, tissus enrobés dans la paraffine, fluides corporels, selles, effluents du côlon, urine, plasma sanguin, sérum sanguin, sang total, cellules sanguines isolées, cellules isolées à partir du sang, et leurs combinaisons.

9. Procédé selon la revendication 7,
(i) comprenant en outre dans l'étape d) l'utilisation d'au moins une molécule d'acide nucléique ou molécule d'acide nucléique de peptide comprenant dans chaque cas une séquence contiguë d'au moins 9 nucléotides de longueur qui est complémentaire de, ou hybride dans des conditions modérément stringentes ou stringentes à une séquence choisie dans le groupe consistant en SEQ ID NO: 15, 16, SEQ ID NO:19 à SEQ ID NO: 36 et SEQ ID NO: 43, 44, SEQ ID NO: 47 à SEQ ID NO: 64 et SEQ ID NO: 134 à SEQ ID NO: 141, et leurs compléments, ladite molécule d'acide nucléique ou molécule d'acide nucléique de peptide supprimant l'amplification de l'acide nucléique auquel elle est hybridée
(ii) tandis que la détermination dans d) comprend l'hybridation d'au moins une molécule d'acide nucléique ou molécule d'acide nucléique de peptide dans chaque cas comprenant une séquence contiguë d'au moins 9 nucléotides de longueur qui est complémentaire de, ou hybride dans des conditions modérément stringentes ou stringentes à une séquence choisie dans le groupe consistant en SEQ ID NO: 15, 16, SEQ ID NO:19 à SEQ ID NO: 36 et SEQ ID NO: 43, 44, SEQ ID NO: 47 à SEQ ID NO: 64 et SEQ ID NO: 134 à SEQ ID NO: 141, et leurs compléments, tandis que de préférence au moins une telle molécule d'acide nucléique ou molécule d'acide nucléique de peptide hybridante est liée à une phase solide ou comprenant en outre l'extension d'au moins une telle molécule d'acide nucléique hybridée par au moins une base nucléotidique; et/ou
(iii) tandis que la détermination dans d) comprend le séquençage du produit d'amplification,
(iv) tandis que la mise en contact ou l'amplification dans c) comprend l'utilisation d'amorces spécifiques vis-à-vis de la méthylation.

10. Procédé pour la détection d'un cancer colorectal, selon la revendication 1, comprenant:
a. l'extraction ou l'isolement d'une autre manière d'ADN génomique à partir d'un échantillon biologique obtenu à partir du sujet;
b. la digestion de l'ADN génomique de a), ou d'un fragment de celui-ci, avec une ou plusieurs enzymes de restriction sensibles à la méthylation; la mise en contact du produit de digestion de l'enzyme de restriction d'ADN de b), avec une enzyme d'amplification et au moins deux amorces convenant pour l'amplification d'une séquence comprenant au moins un dinucléotide CpG de la séquence selon SEQ ID ON: 2;
c. la détermination sur la base d'une présence ou absence d'un produit d'amplification de l'état ou du niveau de méthylation d'au moins un dinucléotide CpG de la séquence SEQ ID NO: 2, ce par quoi la détection d'un cancer colorectal est, au moins en partie procurée.

11. Utilisation d'un acide nucléique:
(i) dérivé de la séquence d'ADN génomique selon SEQ ID NO: 2 par un traitement qui convient pour convertir au moins une base cytosine non-méthylée de la séquence d'ADN génomique en uracile ou une autre base qui est détectable comme n'étant pas similaire à la cytosine en termes d'hybridation;
(ii) comprenant au moins 16 nucléotides contigus d'une séquence d'ADN génomique traitée choisie dans le groupe consistant en SEQ ID NO: 17, 18, 45 et 46, et des séquences complémentaires de celles-ci, tandis que le traitement convient pour la conversion d'au moins une base cytosine non-méthylée de la séquence d'ADN génomique en uracile ou une autre base qui est détectable comme n'étant pas similaire à la cytosine en termes d'hybridation; ou
(iii) comprenant au moins 50 nucléotides contigus d'une séquence d'ADN choisie dans le groupe consistant en SEQ ID NO: 17, 18, 45 et 46, et des séquences complémentaires de celles-ci,
la séquence de bases de l'acide nucléique comprend de préférence au moins une séquence dinucléotidique CpG, TpG ou CpA ou
(iv) comprenant au moins 16 nucléotides contigus d'une séquence d'ADN génomique traitée, choisie dans le groupe consistant en SEQ ID NO: 17, 18, 45 et 46, et des séquences complémentaires de celles-ci, tandis que le traitement convient pour la conversion d'au moins une base cytosine non-méthylée de la séquence d'ADN génomique en uracile ou en une autre base qui est détectable comme n'étant pas similaire à la cytosine en termes d'hybridation,
tandis que la séquence de bases de l'acide nucléique comprend au moins une séquence dinucléotidique CpG, ou CpA et des séquences qui leur sont complémentaires,
pour le diagnostic ou la détection d'un cancer colorectal.

12. Utilisation d'un kit approprié pour la mise en oeuvre du procédé selon les revendications 1-3, comprenant:
(i) (a) une pluralité d'oligonucléotides ou de polynucléotides aptes à hybrider dans des conditions stringentes ou modérément stringentes aux produits de transcription d'au moins le gène PCDHGC3; (b) un récipient approprié pour contenir les oligonucléotides ou polynucléotides et un échantillon biologique du patient comprenant les produits de transcription dans lequel les oligonucléotides ou polynucléotides peuvent hybrider dans des conditions modérément stringentes ou stringentes aux produits de transcription, (c) des moyens pour détecter l'hybridation de (b); et, en option, (d) des instructions pour l'utilisation et l'interprétation des résultats du kit;
(ii) (a) un moyen pour la détection de polypeptides d'au moins le gène PCDHGC3; (b) un récipient approprié pour contenir ledit moyen et l'échantillon biologique du patient comprenant les polypeptides, tandis que le moyen peut former des complexes avec les polypeptides; (c) un moyen pour détecter les complexes de (b); ou
(iii) (a) un réactif du bisulfite; (b) un récipient approprié pour contenir ledit réactif au bisulfite et l'échantillon biologique du patient; (c) au moins un ensemble d'oligonucléotides contenant deux oligonucléotides dont les séquences dans chaque cas sont identiques, sont complémentaires, ou hybrident dans des conditions stringentes ou fortement stringentes à un segment ayant 9 ou plus préférablement 18 bases de longueur d'une séquence choisie parmi SEQ ID NO: 17, 18, 45 et 46
pour la détection ou le diagnostic d'un cancer colorectal.

13. Kit approprié pour la mise en oeuvre du procédé selon la revendication 10, comprenant (a) un réactif enzyme de restriction sensible à la méthylation; (b) un récipient approprié pour contenir ledit réactif et l'échantillon biologique du patient; (c) au moins un ensemble d'oligonucléotides dont l'un ou une pluralité des acides nucléiques ou des acides nucléiques de peptide sont identiques, sont complémentaires, ou hybrident dans des conditions stringentes ou fortement stringentes à un segment d'au moins 9 bases de long de la séquence selon SEQ ID NO: 2, et en option (d) des instructions pour l'utilisation et l'interprétation des résultats du kit.

14. Utilisation d'un kit selon la revendication 13 dans le diagnostic du cancer colorectal.
